# EUROPEAN PATENT APPLICATION

(11) **EP 4 501 924 A1**
(43) Date of publication of application: **05.02.2025**
(21) Application number: 23780296.2
(22) Date of filing: 27.03.2023
(51) Int. Cl.: C07D 401/14, A01N 43/50, A01N 43/54, A01N 43/653, A01N 43/836, A01P 7/04, A61K 31/444, A61K 31/4709, A61K 31/501, A61K 31/5395, A61P 33/00, C07D 413/04, C07D 413/14, C07D 471/04

(54) **PEST CONTROLLING AGENT**

(30) Priority: 28.03.2022 JP 2022051251
(71) Applicant: Nippon Kayaku Kabushiki Kaisha, Tokyo 100-0005 (JP)
(72) Inventor: ATARASHI, Taiki, Kamisu-shi, Ibaraki 314-0255 (JP); HASEGAWA, Shinji, Kamisu-shi, Ibaraki 314-0255 (JP); KAMO, Tomohiro, Kamisu-shi, Ibaraki 314-0255 (JP); UENO, Shotaro, Kamisu-shi, Ibaraki 314-0255 (JP); TANAKA, Yutaro, Kamisu-shi, Ibaraki 314-0255 (JP); TAKUBO, Tatsuaki, Kamisu-shi, Ibaraki 314-0255 (JP); YOSHIDA, Aoi, Kamisu-shi, Ibaraki 314-0255 (JP); KOBAYASHI, Takeru, Kamisu-shi, Ibaraki 314-0255 (JP); SUMI, Takuto, Kamisu-shi, Ibaraki 314-0255 (JP); FUJITA, Tadahide, Kamisu-shi, Ibaraki 314-0255 (JP); AHAREN, Isao, Kamisu-shi, Ibaraki 314-0255 (JP); MURAMOTO, Hiroki, Kamisu-shi, Ibaraki 314-0255 (JP)
(74) Representative: Gille Hrabal Partnerschaftsgesellschaft mbB Patentanwälte
(86) International application number: PCT/JP2023/012099
(87) International publication number: WO 2023/190286

(57) **Abstract**

A compound represented by formula (1) or a salt thereof, or an N-oxide thereof. [In formula (1), X₁ represents an oxygen atom, a sulfur atom, NY₁, or C(J₅J₆), n represents 0 or 1, J₁-J₄ each represent a hydrogen atom, a halogen atom, a (halo)alkyl group, an alkoxy group, a hydroxy group, or a group that is obtained as a result of combining of two bonds of C-J₁ and C-J₂ or C-J₃ and C-J₄ and that is selected from the group consisting of an O atom in a carbonyl group, an S atom in a thiocarbonyl group, an imino group, and a methylene group, D represents a group selected from the group consisting of a phenyl group and a heterocyclic group, B represents a structure represented by any one of B-1 to B-6 and B-8, R₁₂ represents a group selected from the group consisting of (halo)alkyl groups and halo(C₃-C₆) cycloalkyl groups, m represents 0, 1, or 2, G₂ represents a nitrogen atom or C(R₂) and G₄ represents a nitrogen atom or C(R₄), but one or both of G₂ and G₄ are nitrogen atoms, G₅ represents a nitrogen atom or C(R₅), G₆ represents a nitrogen atom or C(R₆), G₇ represents a nitrogen atom or C(R₇), and G₈ represents a nitrogen atom or C(R₈)].

## Description

### TECHNICAL FIELD

The present invention relates to a diazole compound represented by formula (1) or a salt thereof or an N-oxide thereof, and a pest control agent containing the same as an active ingredient.

### BACKGROUND ART

Various azole compounds have so far been disclosed for the purpose of controlling a harmful arthropod. For example, Patent Documents 1 and 2 disclose certain diazole compounds. In addition, various compounds characterized by a sulfur-containing substituent have been disclosed for the purpose of controlling a harmful arthropod, and for example, Patent Documents 3 to 7 disclose arylazole compounds having a sulfur-containing substituent. However, these prior art documents do not disclose the diazole compound according to the present invention at all, and do not disclose any usefulness thereof as a pest control agent at all.

### PRIOR ART DOCUMENTS

### PATENT DOCUMENT

**Patent Document 1:** International Publication No. WO2010/134478
**Patent Document 2:** International Publication No. WO2012/025460
**Patent Document 3:** International Publication No. WO2017/104741
**Patent Document 4:** International Publication No. WO2020/071304
**Patent Document 5:** JP 2017-511378 A
**Patent Document 6:** JP 2017-512835 A
**Patent Document 7:** International Publication No. WO2015/163478

### SUMMARY OF INVENTION

### TECHNICAL PROBLEM

An object of the present invention is to provide a compound that exhibits an excellent control activity against various pests or a salt thereof or an N-oxide thereof, and a pest control agent containing the same as an active ingredient.

### SOLUTION TO PROBLEM

The present inventors have carried out extensive research and as a result found that the compound represented by formula (1) has a high pest control activity and completed the present invention.

That is, the present invention relates to the following, but is not limited thereto.
<1> A compound represented by formula (1) or a salt thereof or an N-oxide thereof: 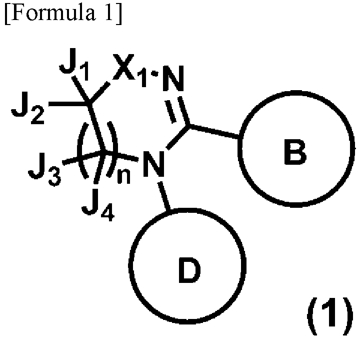 wherein
   X₁ represents an oxygen atom, a sulfur atom, NY₁, or C(J₅J₆),
   n represents 0 or 1,
   J₁, J₂, J₃, and J₄ each independently represent
      a hydrogen atom,
      a halogen atom,
      a (C₁ to C₆) alkyl group unsubstituted or arbitrarily substituted with Tₐ,
      a halo (C₁ to C₆) alkyl group unsubstituted or arbitrarily substituted with Tₐ,
      a (C₁ to C₆) alkoxy group unsubstituted or arbitrarily substituted with Tₐ,
      a halo (C₁ to C₆) alkoxy group unsubstituted or arbitrarily substituted with Tₐ,
      a hydroxy group, or
      a group selected from the group consisting of
         an O atom in a carbonyl group,
         a S atom in a thiocarbonyl group,
         an imino group substituted with Y₂, and
         a methylene group substituted with J₇ and J₈, formed by two bonds of C-J₁ and
   C-J₂, or C-J₃ and C-J₄ together,
   J₅ and J₆ each independently represent
      a hydrogen atom,
      a halogen atom,
      a (C₁ to C₆) alkyl group unsubstituted or arbitrarily substituted with Tₐ,
      a halo (C₁ to C₆) alkyl group unsubstituted or arbitrarily substituted with Tₐ, or
      a group selected from the group consisting of
         an O atom in a carbonyl group,
         a S atom in a thiocarbonyl group,
         an imino group substituted with Y₃, and
         a methylene group substituted with J₇ and J₈, formed by two bonds of C-J₅ and
   C-J₆ together,
   J₇ and J₈ each independently represent a group selected from the group consisting of
      a hydrogen atom,
      a halogen atom,
      a (C₁ to C₆) alkyl group unsubstituted or arbitrarily substituted with Tₐ,
      a halo (C₁ to C₆) alkyl group unsubstituted or arbitrarily substituted with Tₐ,
      a (C₁ to C₆) alkoxy group unsubstituted or arbitrarily substituted with Tₐ,
      a halo (C₁ to C₆) alkoxy group unsubstituted or arbitrarily substituted with Tₐ,
      an amino group optionally substituted with one or two (C₁ to C₆) alkyl groups and/or halo (C₁ to C₆) alkyl groups, and
      a hydroxy group,
   D represents a group selected from the group consisting of
      a phenyl group unsubstituted or arbitrarily substituted with Z₁, and
      a heterocyclic group unsubstituted or arbitrarily substituted with Z₁,
   B is a structure represented by B-1, B-2, B-3, B-4, B-5, B-6, or B-8 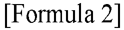 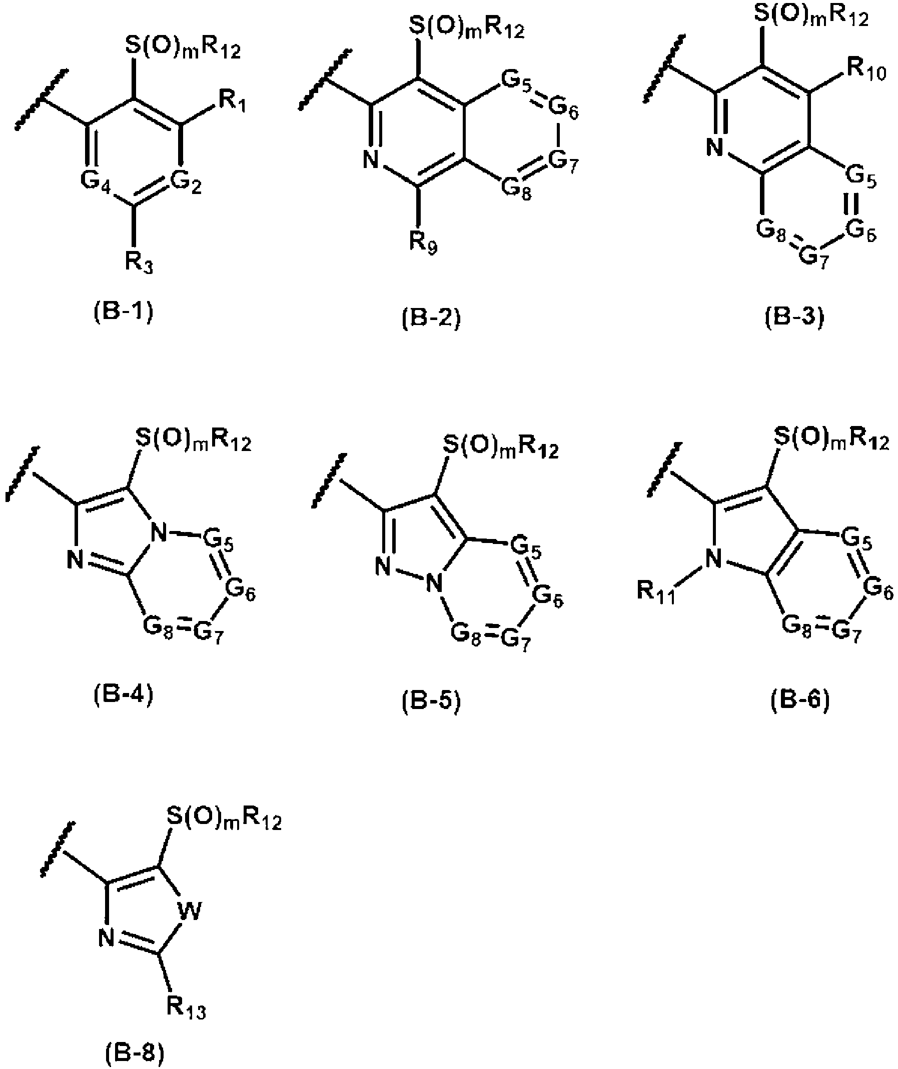
   R₁₂ represents a group selected from the group consisting of
      a (C₁ to C₆) alkyl group,
      a halo (C₁ to C₆) alkyl group,
      a (C₃ to C₆) cycloalkyl group,
      a halo (C₃ to C₆) cycloalkyl group,
      a (C₁ to C₆) alkoxy group,
      a halo (C₁ to C₆) alkoxy group, and
      an NYₐY_{b} group,
   m represents 0, 1, or 2,
   Yₐ and Y_{b} each independently represent a group selected from the group consisting of
      a hydrogen atom,
      a (C₁ to C₆) alkyl group,
      a halo (C₁ to C₆) alkyl group,
      a (C₃ to C₆) cycloalkyl group,
      a halo (C₃ to C₆) cycloalkyl group,
      a (C₁ to C₆) alkoxy group,
      a halo (C₁ to C₆) alkoxy group,
      a (C₁ to C₆) alkenyl group, and
      a halo (C₁ to C₆) alkenyl group, or
      a group forming an aliphatic 3- to 10-membered cyclic amino group or cyclic amide group unsubstituted or optionally substituted with one or more halogen atoms or (C₁ to C₆) alkyl groups, formed by connecting Yₐ and Y_{b} to each other,
   provided that when R₁₂ is an NYₐY_{b} group, m represents 1 or 2,
   G₂ represents a nitrogen atom or C(R₂),
   G₄ represents a nitrogen atom or C(R₄),
   provided that either or both of G₂ and G₄ is/are a nitrogen atom(s),
   G₅ represents a nitrogen atom or C(R₅),
   G₆ represents a nitrogen atom or C(R₆),
   G₇ represents a nitrogen atom or C(R₇),
   G₈ represents a nitrogen atom or C(R₈),
   R₁, R₂, R₃, R₄, R₅, R₆, R₇, R₈, and R₁₃ each independently represent a group selected from the group consisting of
      a hydrogen atom,
      a halogen atom,
      a (C₁ to C₆) alkyl group unsubstituted or arbitrarily substituted with T₁,
      a halo (C₁ to C₆) alkyl group unsubstituted or arbitrarily substituted with T₁,
      a (C₃ to C₆) cycloalkyl group unsubstituted or arbitrarily substituted with T₁,
      a halo (C₃ to C₆) cycloalkyl group unsubstituted or arbitrarily substituted with T₁,
      a (C₁ to C₆) alkoxy group unsubstituted or arbitrarily substituted with T₁,
      a halo (C₁ to C₆) alkoxy group unsubstituted or arbitrarily substituted with T₁,
      a (C₂ to C₆) alkenyl group unsubstituted or arbitrarily substituted with T₁,
      a halo (C₂ to C₆) alkenyl group unsubstituted or arbitrarily substituted with T₁,
      a (C₂ to C₆) alkynyl group unsubstituted or arbitrarily substituted with T₁,
      a halo (C₂ to C₆) alkynyl group unsubstituted or arbitrarily substituted with T₁,
      a (C₂ to C₆) alkenyloxy group unsubstituted or arbitrarily substituted with T₁,
      a halo (C₂ to C₆) alkenyloxy group unsubstituted or arbitrarily substituted with T₁,
      a (C₁ to C₆) alkylthio group unsubstituted or arbitrarily substituted with T₁,
      a halo (C₁ to C₆) alkylthio group unsubstituted or arbitrarily substituted with T₁,
      a (C₁ to C₆) alkylsulfinyl group unsubstituted or arbitrarily substituted with T₁,
      a halo (C₁ to C₆) alkylsulfinyl group unsubstituted or arbitrarily substituted with T₁,
      a (C₁ to C₆) alkylsulfonyl group unsubstituted or arbitrarily substituted with T₁,
      a halo (C₁ to C₆) alkylsulfonyl group unsubstituted or arbitrarily substituted with T₁,
      a (C₁ to C₆) alkylcarbonyl group unsubstituted or arbitrarily substituted with T₁,
      a halo (C₁ to C₆) alkylcarbonyl group unsubstituted or arbitrarily substituted with T₁,
      a (C₁ to C₆) alkylcarbonyloxy group unsubstituted or arbitrarily substituted with T₁,
      a halo (C₁ to C₆) alkylcarbonyloxy group unsubstituted or arbitrarily substituted with T₁,
      a (C₁ to C₆) alkoxycarbonyl group unsubstituted or arbitrarily substituted with T₁,
      a halo (C₁ to C₆) alkoxycarbonyl group unsubstituted or arbitrarily substituted with T₁,
      a (C₁ to C₆) alkoxycarbonyloxy group unsubstituted or arbitrarily substituted with T₁,
      a halo (C₁ to C₆) alkoxycarbonyloxy group unsubstituted or arbitrarily substituted with T₁,
      a (C₁ to C₆) alkylsulfonyloxy group unsubstituted or arbitrarily substituted with T₁,
      a halo (C₁ to C₆) alkylsulfonyloxy group unsubstituted or arbitrarily substituted with T₁,
      a phenyl group unsubstituted or arbitrarily substituted with Z₂,
      a heterocyclic group unsubstituted or arbitrarily substituted with Z₂,
      a phenoxy group unsubstituted or arbitrarily substituted with Z₂,
      a pyridyloxy group unsubstituted or arbitrarily substituted with Z₂,
      an NY₄Y₅ group,
      a C(O)NY₄Y₅ groups,
      a CH=NY₆ groups,
      a cyano group,
      a nitro group,
      a hydroxy group,
      a mercapto group,
      a formyl group,
      a carboxy group, and
      an SF₅ group,
   R₉ and R₁₀ each independently represent a group selected from the group consisting of
      a hydrogen atom,
      a halogen atom,
      a (C₁ to C₆) alkyl group,
      a halo (C₁ to C₆) alkyl group,
      a (C₃ to C₆) cycloalkyl group,
      a halo (C₃ to C₆) cycloalkyl group,
      a (C₁ to C₆) alkoxy group,
      a halo (C₁ to C₆) alkoxy group,
      an NY₄Y₅ group,
      a C(O)NY₄Y₅ group,
      a cyano group,
      a nitro group,
      a hydroxy group,
      a (C₁ to C₆) alkylcarbonyloxy group,
      a halo (C₁ to C₆) alkylcarbonyloxy group,
      a (C₁ to C₆) alkoxycarbonyloxy group, and
      a halo (C₁ to C₆) alkoxycarbonyloxy group,
   R₁₁ represents a group selected from the group consisting of
      a hydrogen atom,
      a (C₁ to C₆) alkyl group,
      a halo (C₁ to C₆) alkyl group,
      a (C₃ to C₆) cycloalkyl group,
      a halo (C₃ to C₆) cycloalkyl group,
      a (C₁ to C₆) alkoxy group,
      a halo (C₁ to C₆) alkoxy group,
      a (C₁ to C₆) alkylcarbonyl group,
      a halo (C₁ to C₆) alkylcarbonyl group,
      a (C₄ to C₇) cycloalkylcarbonyl group, and
      a halo (C₄ to C₇) cycloalkylcarbonyl group,
   Z₁ and Z₂ each independently represent a group selected from the group consisting of
      a halogen atom,
      a (C₁ to C₆) alkyl group unsubstituted or arbitrarily substituted with T₂,
      a halo (C₁ to C₆) alkyl group unsubstituted or arbitrarily substituted with T₂,
      a (C₃ to C₆) cycloalkyl group unsubstituted or arbitrarily substituted with T₂,
      a halo (C₃ to C₆) cycloalkyl group unsubstituted or arbitrarily substituted with T₂,
      a (C₁ to C₆) alkoxy group unsubstituted or arbitrarily substituted with T₂,
      a halo (C₁ to C₆) alkoxy group unsubstituted or arbitrarily substituted with T₂,
      a (C₂ to C₆) alkenyl group unsubstituted or arbitrarily substituted with T₂,
      a halo (C₂ to C₆) alkenyl group unsubstituted or arbitrarily substituted with T₂,
      a (C₂ to C₆) alkenyloxy group unsubstituted or arbitrarily substituted with T₂,
      a halo (C₂ to C₆) alkenyloxy group unsubstituted or arbitrarily substituted with T₂,
      a (C₁ to C₆) alkylthio group unsubstituted or arbitrarily substituted with T₂,
      a halo (C₁ to C₆) alkylthio group unsubstituted or arbitrarily substituted with T₂,
      a (C₁ to C₆) alkylsulfinyl group unsubstituted or arbitrarily substituted with T₂,
      a halo (C₁ to C₆) alkylsulfinyl group unsubstituted or arbitrarily substituted with T₂,
      a (C₁ to C₆) alkylsulfonyl group unsubstituted or arbitrarily substituted with T₂,
      a halo (C₁ to C₆) alkylsulfonyl group unsubstituted or arbitrarily substituted with T₂,
      a (C₁ to C₆) alkylcarbonyl group unsubstituted or arbitrarily substituted with T₂,
      a halo (C₁ to C₆) alkylcarbonyl group unsubstituted or arbitrarily substituted with T₂,
      a (C₁ to C₆) alkoxycarbonyl group unsubstituted or arbitrarily substituted with T₂,
      a halo (C₁ to C₆) alkoxycarbonyl group that is unsubstituted or arbitrarily substituted with T₂,
      a (C₁ to C₆) alkylcarbonyloxy group unsubstituted or arbitrarily substituted with T₂,
      a halo (C₁ to C₆) alkylcarbonyloxy group unsubstituted or arbitrarily substituted with T₂,
      a (C₁ to C₆) alkoxycarbonyloxy group unsubstituted or arbitrarily substituted with T₂,
      a halo (C₁ to C₆) alkoxycarbonyloxy group unsubstituted or arbitrarily substituted with T₂,
      a (C₁ to C₆) alkylsulfonyloxy group unsubstituted or arbitrarily substituted with T₂,
      a halo (C₁ to C₆) alkylsulfonyloxy group unsubstituted or arbitrarily substituted with T₂,
      an NY₄Y₅ group,
      a C(O)NY₄Y₅ group,
      a cyano group,
      a nitro group,
      a hydroxy group,
      a mercapto group,
      a formyl group,
      a carboxy group,
      an SF₅ group,
         or
      a group forming a 5- or 6-membered alicyclic ring unsubstituted or optionally substituted with one or more halogen atoms or (C₁ to C₆) alkyl groups, formed by connecting two adjacent Z₁ or two adjacent Z₂ to each other, or a group forming a 1,3-dioxole group or a 1,4-dioxin group formed by two adjacent Z₁ or two adjacent Z₂ together with a carbon atom to which the two Z₁ or the two Z₂ are attached, wherein the 1,3-dioxole group and the 1,4-dioxin group are optionally substituted with one or two or more independent halogen atoms,
   Y₁, Y₂, and Y₃ each independently represent a group selected from the group consisting of
      a hydrogen atom,
      a (C₁ to C₆) alkyl group unsubstituted or arbitrarily substituted with T₃,
      a halo (C₁ to C₆) alkyl group unsubstituted or arbitrarily substituted with T₃,
      a (C₃ to C₆) cycloalkyl group unsubstituted or arbitrarily substituted with T₃,
      a halo (C₃ to C₆) cycloalkyl group unsubstituted or arbitrarily substituted with T₃,
      a (C₂ to C₆) alkenyl group unsubstituted or arbitrarily substituted with T₃,
      a halo (C₂ to C₆) alkenyl group unsubstituted or arbitrarily substituted with T₃,
      a (C₁ to C₆) alkoxy group unsubstituted or arbitrarily substituted with T₃,
      a halo (C₁ to C₆) alkoxy group unsubstituted or arbitrarily substituted with T₃,
      a (C₁ to C₆) alkylcarbonyl group unsubstituted or arbitrarily substituted with T₃,
      a halo (C₁ to C₆) alkylcarbonyl group unsubstituted or arbitrarily substituted with T₃,
      a (C₃ to C₆) cycloalkylcarbonyl group unsubstituted or arbitrarily substituted with T₃,
      a halo (C₃ to C₆) cycloalkylcarbonyl group unsubstituted or arbitrarily substituted with T₃,
      a (C₁ to C₆) alkoxycarbonyl group unsubstituted or arbitrarily substituted with T₃,
      a halo (C₁ to C₆) alkoxycarbonyl group unsubstituted or arbitrarily substituted with T₃,
      a (C₁ to C₆) alkylsulfonyl group unsubstituted or arbitrarily substituted with T₃,
      a halo (C₁ to C₆) alkylsulfonyl group unsubstituted or arbitrarily substituted with T₃, and
      a hydroxy group,
   Tₐ and T₁ each independently represent a group selected from the group consisting of
      a (C₃ to C₆) cycloalkyl group,
      a halo (C₃ to C₆) cycloalkyl group,
      a (C₁ to C₆) alkoxy group,
      a halo (C₁ to C₆) alkoxy group,
      a (C₁ to C₆) alkylcarbonyl group,
      a halo (C₁ to C₆) alkylcarbonyl group,
      a (C₁ to C₆) alkoxycarbonyl group,
      a halo (C₁ to C₆) alkoxycarbonyl group,
      a (C₁ to C₆) alkylthio group,
      a halo (C₁ to C₆) alkylthio group,
      a (C₁ to C₆) alkylsulfinyl group,
      a halo (C₁ to C₆) alkylsulfinyl group,
      a (C₁ to C₆) alkylsulfonyl group,
      a halo (C₁ to C₆) alkylsulfonyl group,
      an NY₄Y₅ group,
      a C(O)NY₄Y₅ group,
      a cyano group,
      a nitro group,
      a hydroxy group,
      a mercapto group,
      a formyl group,
      a carboxy group,
      a phenyl group unsubstituted or arbitrarily substituted with Z₃,
      a heterocycle unsubstituted or arbitrarily substituted with Z₃,
      a phenoxy group unsubstituted or arbitrarily substituted with Z₃, and
      a pyridyloxy group unsubstituted or arbitrarily substituted with Z₃,
   T₂ and T₃ each independently represents a group selected from the group consisting of
      a (C₃ to C₆) cycloalkyl group,
      a halo (C₃ to C₆) cycloalkyl group,
      a (C₁ to C₆) alkoxy group,
      a halo (C₁ to C₆) alkoxy group,
      a (C₁ to C₆) alkylcarbonyl group,
      a halo (C₁ to C₆) alkylcarbonyl group,
      a (C₁ to C₆) alkoxycarbonyl group,
      a halo (C₁ to C₆) alkoxycarbonyl group,
      a (C₁ to C₆) alkylthio group,
      a halo (C₁ to C₆) alkylthio group,
      a (C₁ to C₆) alkylsulfinyl group,
      a halo (C₁ to C₆) alkylsulfinyl group,
      a (C₁ to C₆) alkylsulfonyl group,
      a halo (C₁ to C₆) alkylsulfonyl group,
      an NY₄Y₅ group,
      a C(O)NY₄Y₅ group,
      a cyano group,
      a nitro group,
      a hydroxy group,
      a mercapto group,
      a formyl group, and
      a carboxy group,
   Z₃, or when a plurality thereof is present, each independently, represents a group selected from the group consisting of
      a halogen atom,
      a (C₁ to C₆) alkyl group,
      a halo (C₁ to C₆) alkyl group,
      a (C₃ to C₆) cycloalkyl group,
      a halo (C₃ to C₆) cycloalkyl group,
      a 1-cyano-(C₃ to C₆) cycloalkyl group,
      a (C₁ to C₆) alkoxy group,
      a halo (C₁ to C₆) alkoxy group,
      a (C₁ to C₆) alkylthio group,
      a halo (C₁ to C₆) alkylthio group,
      a (C₁ to C₆) alkylsulfinyl group,
      a halo (C₁ to C₆) alkylsulfinyl group,
      a (C₁ to C₆) alkylsulfonyl group,
      a halo (C₁ to C₆) alkylsulfonyl group,
      a (C₁ to C₆) alkylcarbonyl group,
      a halo (C₁ to C₆) alkylcarbonyl group,
      a (C₁ to C₆) alkoxycarbonyl group,
      a halo (C₁ to C₆) alkoxycarbonyl group,
      an NY₄Y₅ group,
      a C(O)NY₄Y₅ group,
      a cyano group,
      a nitro group,
      a hydroxy group,
      a mercapto group,
      a formyl group,
      a carboxy group, and
      an SF₅ group,
   Y₄ and Y₅ each independently represent a group selected from the group consisting of
      a hydrogen atom,
      a (C₁ to C₆) alkyl group,
      a halo (C₁ to C₆) alkyl group,
      a (C₃ to C₆) cycloalkyl group,
      a halo (C₃ to C₆) cycloalkyl group,
      a (C₁ to C₆) alkoxy group,
      a halo (C₁ to C₆) alkoxy group,
      a (C₁ to C₆) alkylcarbonyl group,
      a halo (C₁ to C₆) alkylcarbonyl group,
      a (C₃ to C₆) cycloalkylcarbonyl group,
      a halo (C₃ to C₆) cycloalkylcarbonyl group,
      a (C₁ to C₆) alkoxycarbonyl group,
      a halo (C₁ to C₆) alkoxycarbonyl group,
      a (C₁ to C₆) alkylsulfonyl group,
      a halo (C₁ to C₆) alkylsulfonyl group, and
      a phenyl group unsubstituted or arbitrarily substituted with Z₃,
   Y₆, or when a plurality thereof is present, each independently, represents a group selected from the group consisting of
      a hydrogen atom,
      a (C₁ to C₆) alkyl group unsubstituted or arbitrarily substituted with T₃,
      a halo (C₁ to C₆) alkyl group unsubstituted or arbitrarily substituted with T₃,
      a (C₃ to C₆) cycloalkyl group,
      a halo (C₃ to C₆) cycloalkyl group,
      a (C₁ to C₆) alkoxy group unsubstituted or arbitrarily substituted with a cyano group,
      a halo (C₁ to C₆) alkoxy group,
      a (C₃ to C₆) cycloalkoxy group,
      a halo (C₃ to C₆) cycloalkoxy group,
      a halo (C₂ to C₆) alkenyl group,
      a (C₂ to C₆) alkenyloxy group, and
      a halo (C₂ to C₆) alkenyloxy group,
   W represents an oxygen atom, a sulfur atom, or NY₇, and
   Y₇ is a group selected from the group consisting of
      a hydrogen atom,
      a (C₁ to C₆) alkyl group,
      a halo (C₁ to C₆) alkyl group,
      a (C₁ to C₆) alkylcarbonyl group, and
      a halo (C₁ to C₆) alkylcarbonyl group.
<2> A compound represented by formula (1) or a salt thereof or an N-oxide thereof: 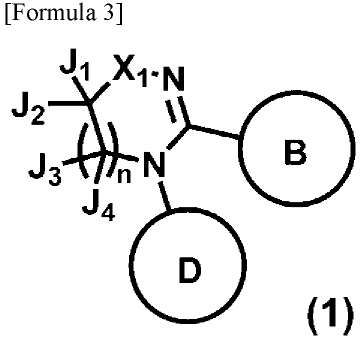 wherein
   X₁ represents an oxygen atom, a sulfur atom, NY₁, or C(J₅J₆),
   n represents 0 or 1,
   J₁, J₂, J₃, and J₄ each independently represent a hydrogen atom, a halogen atom, a (C₁ to C₆) alkyl group unsubstituted or arbitrarily substituted with Tₐ, a halo (C₁ to C₆) alkyl group unsubstituted or arbitrarily substituted with Tₐ, a (C₁ to C₆) alkoxy group unsubstituted or arbitrarily substituted with Tₐ, a halo (C₁ to C₆) alkoxy group unsubstituted or arbitrarily substituted with Tₐ, a hydroxy group, or
      a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
   J₅ and J₆ each independently represent a hydrogen atom, a halogen atom, a (C₁ to C₆) alkyl group unsubstituted or arbitrarily substituted with Tₐ, a halo (C₁ to C₆) alkyl group unsubstituted or arbitrarily substituted with Tₐ, or
      a group selected from the group consisting of an O atom in a carbonyl group, a S atom in a thiocarbonyl group, an imino group substituted with Y₃, and a methylene group substituted with J₇ and J₈, formed by two bonds of C-J₅ and C-J₆ together,
   J₇ and J₈ each independently represent a group selected from the group consisting of a hydrogen atom, a halogen atom, a (C₁ to C₆) alkyl group unsubstituted or arbitrarily substituted with Tₐ, a halo (C₁ to C₆) alkyl group unsubstituted or arbitrarily substituted with Tₐ, a (C₁ to C₆) alkoxy group unsubstituted or arbitrarily substituted with Tₐ, a halo (C₁ to C₆) alkoxy group unsubstituted or arbitrarily substituted with Tₐ, an amino group optionally substituted with one or two (C₁ to C₆) alkyl groups and/or halo (C₁ to C₆) alkyl groups, and a hydroxy group,
   D represents a group selected from the group consisting of a phenyl group unsubstituted or arbitrarily substituted with Z₁, and a heterocyclic group unsubstituted or arbitrarily substituted with Z₁,
   B is a structure represented by B-1, B-2, B-3, B-4, B-5, or B-6 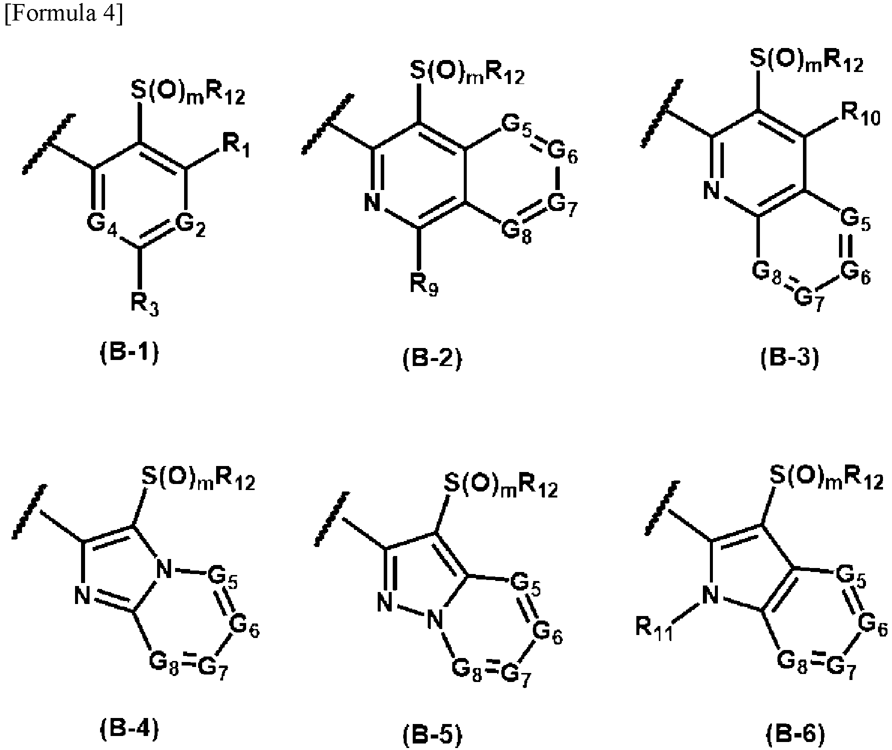
   R₁₂ represents a group selected from the group consisting of a (C₁ to C₆) alkyl group, a halo (C₁ to C₆) alkyl group, a (C₃ to C₆) cycloalkyl group, and a halo (C₃ to C₆) cycloalkyl group,
   m represents 0, 1, or 2,
   G₂ represents a nitrogen atom or C(R₂),
   G₄ represents a nitrogen atom or C(R₄),
   provided that either or both of G₂ and G₄ is/are a nitrogen atom(s),
   G₅ represents a nitrogen atom or C(R₅),
   G₆ represents a nitrogen atom or C(R₆),
   G₇ represents a nitrogen atom or C(R₇),
   G₈ represents a nitrogen atom or C(R₈),
   R₁, R₂, R₃, R₄, R₅, R₆, R₇, and R₈ each independently represent a group selected from the group consisting of
      a hydrogen atom, a halogen atom, a (C₁ to C₆) alkyl group unsubstituted or arbitrarily substituted with T₁, a halo (C₁ to C₆) alkyl group unsubstituted or arbitrarily substituted with T₁, a (C₃ to C₆) cycloalkyl group unsubstituted or arbitrarily substituted with T₁, a halo (C₃ to C₆) cycloalkyl group unsubstituted or arbitrarily substituted with T₁, a (C₁ to C₆) alkoxy group unsubstituted or arbitrarily substituted with T₁, a halo (C₁ to C₆) alkoxy group unsubstituted or arbitrarily substituted with T₁, a (C₂ to C₆) alkenyl group unsubstituted or arbitrarily substituted with T₁, a halo (C₂ to C₆) alkenyl group unsubstituted or arbitrarily substituted with T₁, a (C₂ to C₆) alkynyl group unsubstituted or arbitrarily substituted with T₁, a halo (C₂ to C₆) alkynyl group unsubstituted or arbitrarily substituted with T₁, a (C₂ to C₆) alkenyloxy group unsubstituted or arbitrarily substituted with T₁, a halo (C₂ to C₆) alkenyloxy group unsubstituted or arbitrarily substituted with T₁, a (C₁ to C₆) alkylthio group unsubstituted or arbitrarily substituted with T₁, a halo (C₁ to C₆) alkylthio group unsubstituted or arbitrarily substituted with T₁, a (C₁ to C₆) alkylsulfinyl group unsubstituted or arbitrarily substituted with T₁, a halo (C₁ to C₆) alkylsulfinyl group unsubstituted or arbitrarily substituted with T₁, a (C₁ to C₆) alkylsulfonyl group unsubstituted or arbitrarily substituted with T₁, a halo (C₁ to C₆) alkylsulfonyl group unsubstituted or arbitrarily substituted with T₁, a (C₁ to C₆) alkylcarbonyl group unsubstituted or arbitrarily substituted with T₁, a halo (C₁ to C₆) alkylcarbonyl group unsubstituted or arbitrarily substituted with T₁, a (C₁ to C₆) alkylcarbonyloxy group unsubstituted or arbitrarily substituted with T₁, a halo (C₁ to C₆) alkylcarbonyloxy group substituted or arbitrarily substituted with T₁, a (C₁ to C₆) alkoxycarbonyl group unsubstituted or arbitrarily substituted with T₁, a halo (C₁ to C₆) alkoxycarbonyl group unsubstituted or arbitrarily substituted with T₁, a (C₁ to C₆) alkylsulfonyloxy group unsubstituted or arbitrarily substituted with T₁, a (C₁ to C₆) alkoxycarbonyloxy group unsubstituted or arbitrarily substituted with T₁, a halo (C₁ to C₆) alkoxycarbonyloxy group unsubstituted or arbitrarily substituted with T₁, a halo (C₁ to C₆) alkylsulfonyloxy group unsubstituted or arbitrarily substituted with T₁, a phenyl group unsubstituted or arbitrarily substituted with Z₂, a heterocyclic group unsubstituted or arbitrarily substituted with Z₂, a phenoxy group unsubstituted or arbitrarily substituted with Z₂, a pyridyloxy group unsubstituted or arbitrarily substituted with Z₂, an NY₄Y₅ group, a C(O)NY₄Y₅ group, a CH=NY₆ group, a cyano group, a nitro group, a hydroxy group, a mercapto group, a formyl group, a carboxy group, and an SF₅ group,
   R₉ and R₁₀ each independently represent a group selected from the group consisting of
      a hydrogen atom, a halogen atom, a (C₁ to C₆) alkyl group, a halo (C₁ to C₆) alkyl group, a (C₃ to C₆) cycloalkyl group, a halo (C₃ to C₆) cycloalkyl group, a (C₁ to C₆) alkoxy group, a halo (C₁ to C₆) alkoxy group, a (C₁ to C₆) alkylcarbonyloxy group, a halo (C₁ to C₆) alkylcarbonyloxy group, a (C₁ to C₆) alkoxycarbonyloxy group, a halo (C₁ to C₆) alkoxycarbonyloxy group, an NY₄Y₅ group, a C(O)NY₄Y₅ group, a cyano group, a nitro group, and a hydroxy group,
   R₁₁ represents a group selected from the group consisting of a hydrogen atom, a (C₁ to C₆) alkyl group, a halo (C₁ to C₆) alkyl group, a (C₃ to C₆) cycloalkyl group, a halo (C₃ to C₆) cycloalkyl group, a (C₁ to C₆) alkoxy group, a halo (C₁ to C₆) alkoxy group, a (C₁ to C₆) alkylcarbonyl group, a halo (C₁ to C₆) alkylcarbonyl group, a (C₄ to C₇) cycloalkylcarbonyl group, and a halo (C₄ to C₇) cycloalkylcarbonyl group,
   Z₁ and Z₂ each independently represent a group selected from the group consisting of a halogen atom, a (C₁ to C₆) alkyl group unsubstituted or arbitrarily substituted with T₂, or a halo (C₁ to C₆) alkyl group unsubstituted or arbitrarily substituted with T₂, a (C₃ to C₆) cycloalkyl group unsubstituted or arbitrarily substituted with T₂, a halo (C₃ to C₆) cycloalkyl group unsubstituted or arbitrarily substituted with T₂, a (C₁ to C₆) alkoxy group unsubstituted or arbitrarily substituted with T₂, a halo (C₁ to C₆) alkoxy group unsubstituted or arbitrarily substituted with T₂, a (C₂ to C₆) alkenyl group unsubstituted or arbitrarily substituted with T₂, a halo (C₂ to C₆) alkenyl group unsubstituted or arbitrarily substituted with T₂, a (C₂ to C₆) alkenyloxy group unsubstituted or arbitrarily substituted with T₂, a halo (C₂ to C₆) alkenyloxy group unsubstituted or arbitrarily substituted with T₂, a (C₁ to C₆) alkylthio group unsubstituted or arbitrarily substituted with T₂, a halo (C₁ to C₆) alkylthio group unsubstituted or arbitrarily substituted with T₂, a (C₁ to C₆) alkylsulfinyl group unsubstituted or arbitrarily substituted with T₂, a halo (C₁ to C₆) alkylsulfinyl group unsubstituted or arbitrarily substituted with T₂, a (C₁ to C₆) alkylsulfonyl group unsubstituted or arbitrarily substituted with T₂, a halo (C₁ to C₆) alkylsulfonyl group unsubstituted or arbitrarily substituted with T₂, a (C₁ to C₆) alkylcarbonyl group unsubstituted or arbitrarily substituted with T₂, a halo (C₁ to C₆) alkylcarbonyl group unsubstituted or arbitrarily substituted with T₂, a (C₁ to C₆) alkoxycarbonyl group unsubstituted or arbitrarily substituted with T₂, a halo (C₁ to C₆) alkoxycarbonyl group unsubstituted or arbitrarily substituted with T₂, a (C₁ to C₆) alkylcarbonyloxy group unsubstituted or arbitrarily substituted with T₂, a halo (C₁ to C₆) alkylcarbonyloxy group unsubstituted or arbitrarily substituted with T₂, a (C₁ to C₆) alkoxycarbonyloxy group unsubstituted or arbitrarily substituted with T₂, a halo (C₁ to C₆) alkoxycarbonyloxy group unsubstituted or arbitrarily substituted with T₂, a (C ₁ to C₆) alkylsulfonyloxy group unsubstituted or arbitrarily substituted with T₂, a halo (C₁ to C₆) alkylsulfonyloxy group unsubstituted or arbitrarily substituted with T₂, an NY₄Y₅ group, a C(O)NY₄Y₅ group, a cyano group, a nitro group, a hydroxy group, a mercapto group, a formyl group, a carboxy group, and an SF₅ group, or
      a group forming a 5- or 6-membered alicyclic ring unsubstituted or optionally substituted with one or more halogen atoms or (C₁ to C₆) alkyl groups, formed by connecting two adjacent Z₁ or Z₂ to each other, or a group forming a 1,3-dioxole group or a 1,4-dioxin group formed by two adjacent Z₁ or Z₂ together with a carbon atom to which the two Z₁ or Z₂ are attached, wherein the 1,3-dioxole group and the 1,4-dioxin group are optionally substituted with one or two or more independent halogen atoms,
   Y₁, Y₂, and Y₃ each independently represent a group selected from the group consisting of a hydrogen atom, a (C₁ to C₆) alkyl group unsubstituted or arbitrarily substituted with T₃, a halo (C₁ to C₆) alkyl group unsubstituted or arbitrarily substituted with T₃, a (C₃ to C₆) cycloalkyl group unsubstituted or arbitrarily substituted with T₃, a halo (C₃ to C₆) cycloalkyl group unsubstituted or arbitrarily substituted with T₃, a (C₂ to C₆) alkenyl group unsubstituted or arbitrarily substituted with T₃, a halo (C₂ to C₆) alkenyl group unsubstituted or arbitrarily substituted with T₃, a (C₁ to C₆) alkoxy group unsubstituted or arbitrarily substituted with T₃, a halo (C₁ to C₆) alkoxy group unsubstituted or arbitrarily substituted with T₃, a (C₁ to C₆) alkylcarbonyl group unsubstituted or arbitrarily substituted with T₃, a halo (C₁ to C₆) alkylcarbonyl group unsubstituted or arbitrarily substituted with T₃, a (C₃ to C₆) cycloalkylcarbonyl group unsubstituted or arbitrarily substituted with T₃, a halo (C₃ to C₆) cycloalkylcarbonyl group unsubstituted or arbitrarily substituted with T₃, a (C₁ to C₆) alkoxycarbonyl group unsubstituted or arbitrarily substituted with T₃, a halo (C₁ to C₆) alkoxycarbonyl group unsubstituted or arbitrarily substituted with T₃, a (C₁ to C₆) alkylsulfonyl group unsubstituted or arbitrarily substituted with T₃, a halo (C₁ to C₆) alkylsulfonyl group unsubstituted or arbitrarily substituted with T₃, and a hydroxy group,
   Tₐ and T₁ each independently represent a group selected from the group consisting of a (C₃ to C₆) cycloalkyl group, a halo (C₃ to C₆) cycloalkyl group, a (C₁ to C₆) alkoxy group, a halo (C₁ to C₆) alkoxy group, a (C₁ to C₆) alkylcarbonyl group, a halo (C₁ to C₆) alkylcarbonyl group, a (C₁ to C₆) alkoxycarbonyl group, a halo (C₁ to C₆) alkoxycarbonyl group, a (C₁ to C₆) alkylthio group, a halo (C₁ to C₆) alkylthio group, a (C₁ to C₆) alkylsulfinyl group, a halo (C₁ to C₆) alkylsulfinyl group, a (C₁ to C₆) alkylsulfonyl group, a halo (C₁ to C₆) alkylsulfonyl group, an NY₄Y₅ group, a C(O)NY₄Y₅ group, a cyano group, a nitro group, a hydroxy group, a mercapto group, a formyl group, a carboxy group, a phenyl group unsubstituted or arbitrarily substituted with Z₃, a heterocyclic group unsubstituted or arbitrarily substituted with Z₃, a phenoxy group unsubstituted or arbitrarily substituted with Z₃, and a pyridyloxy group unsubstituted or arbitrarily substituted with Z₃,
   T₂ and T₃ each independently represents a group selected from the group consisting of a (C₃ to C₆) cycloalkyl group, a halo (C₃ to C₆) cycloalkyl group, a (C₁ to C₆) alkoxy group, a halo (C₁ to C₆) alkoxy group, a (C₁ to C₆) alkylcarbonyl group, a halo (C₁ to C₆) alkylcarbonyl group, a (C₁ to C₆) alkoxycarbonyl group, a halo (C₁ to C₆) alkoxycarbonyl group, a (C₁ to C₆) alkylthio group, a halo (C₁ to C₆) alkylthio group, a (C₁ to C₆) alkylsulfinyl group, a halo (C₁ to C₆) alkylsulfinyl group, a (C₁ to C₆) alkylsulfonyl group, a halo (C₁ to C₆) alkylsulfonyl group, an NY₄Y₅ group, a C(O)NY₄Y₅ group, a cyano group, a nitro group, a hydroxy group, a mercapto group, a formyl group, and a carboxy group,
   Z₃, or when a plurality thereof is present, each independently, represents a group selected from the group consisting of a halogen atom, a (C₁ to C₆) alkyl group, a halo (C₁ to C₆) alkyl group, a (C₃ to C₆) cycloalkyl group, a halo (C₃ to C₆) cycloalkyl group, a 1-cyano-(C₃ to C₆) cycloalkyl group, a (C₁ to C₆) alkoxy group, a halo (C₁ to C₆) alkoxy group, a (C₁ to C₆) alkylthio group, a halo (C₁ to C₆) alkylthio group, a (C₁ to C₆) alkylsulfinyl group, a halo (C₁ to C₆) alkylsulfinyl group, a (C₁ to C₆) alkylsulfonyl group, a halo (C₁ to C₆) alkylsulfonyl group, a (C₁ to C₆) alkylcarbonyl group, a halo (C₁ to C₆) alkylcarbonyl group, a (C₁ to C₆) alkoxycarbonyl group, a halo (C₁ to C₆) alkoxycarbonyl group, an NY₄Y₅ group, a C(O)NY₄Y₅ group, a cyano group, a nitro group, a hydroxy group, a mercapto group, a formyl group, a carboxy group, and an SF₅ group,
   Y₄ and Y₅ each independently represent a group selected from the group consisting of a hydrogen atom, a (C₁ to C₆) alkyl group, a halo (C₁ to C₆) alkyl group, a (C₃ to C₆) cycloalkyl group, a halo (C₃ to C₆) cycloalkyl group, a (C₁ to C₆) alkoxy group, a halo (C₁ to C₆) alkoxy group, a (C₁ to C₆) alkylcarbonyl group, a halo (C₁ to C₆) alkylcarbonyl group, a (C₃ to C₆) cycloalkylcarbonyl group, a halo (C₃ to C₆) alkylcarbonyl group, a (C₁ to C₆) alkoxycarbonyl group, a halo (C₁ to C₆) alkoxycarbonyl group, a (C₁ to C₆) alkylsulfonyl group,
   a halo (C₁ to C₆) alkylsulfonyl group, and a phenyl group unsubstituted or arbitrarily substituted with Z₃, and
   Y₆, or when a plurality thereof is present, each independently, represents a group selected from the group consisting of a hydrogen atom, a (C₁ to C₆) alkyl group unsubstituted or arbitrarily substituted with T₃, a halo (C₁ to C₆) alkyl group unsubstituted or arbitrarily substituted with T₃, a (C₃ to C₆) cycloalkyl group, a halo (C₃ to C₆) cycloalkyl group, a (C₁ to C₆) alkoxy group unsubstituted or arbitrarily substituted with a cyano group, a halo (C₁ to C₆) alkoxy group, a (C₃ to C₆) cycloalkoxy group, a halo (C₃ to C₆) cycloalkoxy group, a (C₂ to C₆) alkenyl group, a halo (C₂ to C₆) alkenyl group, a (C₂ to C₆) alkenyloxy group, and a halo (C₂ to C₆) alkenyloxy group.
<3> The compound according to <1> or <2> or a salt thereof or an N-oxide thereof,
   wherein
   X₁ is an oxygen atom, a sulfur atom, NY₁, or C(J₅J₆),
   two bonds of C-J₅ and C-J₆ are taken together to be a group selected from the group consisting of an O atom in a carbonyl group, a S atom in a thiocarbonyl group, an imino group substituted with Y₃, and a methylene group substituted with J₇ and J₈, and
   J₇ and J₈ are each independently a group selected from the group consisting of a hydrogen atom, a halogen atom, a (C₁ to C₆) alkyl group unsubstituted or arbitrarily substituted with Tₐ, a (C₁ to C₆) alkoxy group unsubstituted or arbitrarily substituted with Tₐ, an amino group optionally substituted with one or two (C₁ to C₆) alkyl groups and/or halo (C₁ to C₆) alkyl groups, and a hydroxy group.
<4> The compound according to any one of <1> to <3> or a salt thereof or an N-oxide thereof, wherein
   D is D-1, D-2, D-3, D-4, D-5, D-6, D-7, D-8, D-9, or D-10 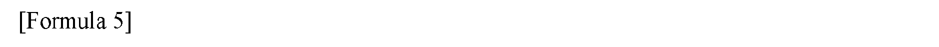 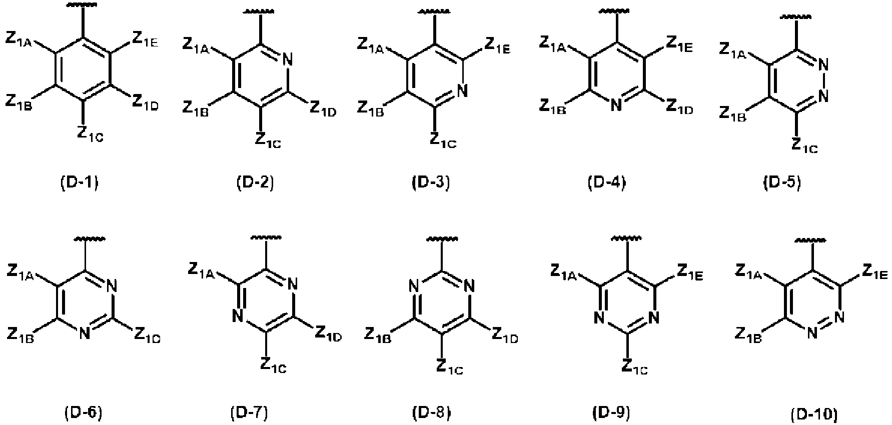 wherein
   Z_{1A}, Z_{1B}, Z_{1C}, Z_{1D}, and Z_{1E} are each independently a group selected from the group consisting of a hydrogen atom, a halogen atom, a (C₁ to C₆) alkyl group unsubstituted or arbitrarily substituted with T₂, a halo (C₁ to C₆) alkyl group unsubstituted or arbitrarily substituted with T₂, a (C₃ to C₆) cycloalkyl group unsubstituted or arbitrarily substituted with T₂, a halo (C₃ to C₆) cycloalkyl group unsubstituted or arbitrarily substituted with T₂, a (C₁ to C₆) alkoxy group unsubstituted or arbitrarily substituted with T₂, a halo (C₁ to C₆) alkoxy group unsubstituted or arbitrarily substituted with T₂, a (C₂ to C₆) alkenyl group unsubstituted or arbitrarily substituted with T₂, a halo (C₂ to C₆) alkenyl group unsubstituted or arbitrarily substituted with T₂, a (C₂ to C₆) alkenyloxy group unsubstituted or arbitrarily substituted with T₂, a halo (C₂ to C₆) alkenyloxy group unsubstituted or arbitrarily substituted with T₂, a (C₁ to C₆) alkylthio group unsubstituted or arbitrarily substituted with T₂, a halo (C₁ to C₆) alkylthio group unsubstituted or arbitrarily substituted with T₂, a (C₁ to C₆) alkylsulfinyl group unsubstituted or arbitrarily substituted with T₂, a halo (C₁ to C₆) alkylsulfinyl group unsubstituted or arbitrarily substituted with T₂, a (C₁ to C₆) alkylsulfonyl group unsubstituted or arbitrarily substituted with T₂, a halo (C₁ to C₆) alkylsulfonyl group unsubstituted or arbitrarily substituted with T₂, a (C₁ to C₆) alkylcarbonyl group unsubstituted or arbitrarily substituted with T₂, a halo (C₁ to C₆) alkylcarbonyl group unsubstituted or arbitrarily substituted with T₂, a (C₁ to C₆) alkoxycarbonyl group unsubstituted or arbitrarily substituted with T₂, a halo (C₁ to C₆) alkoxycarbonyl group unsubstituted or arbitrarily substituted with T₂, a (C₁ to C₆) alkylcarbonyloxy group unsubstituted or arbitrarily substituted with T₂, a halo (C₁ to C₆) alkylcarbonyloxy group unsubstituted or arbitrarily substituted with T₂, a (C₁ to C₆) alkoxycarbonyloxy group unsubstituted or arbitrarily substituted with T₂, a halo (C₁ to C₆) alkoxycarbonyloxy group unsubstituted or arbitrarily substituted with T₂, a (C₁ to C₆) alkylsulfonyloxy group unsubstituted or arbitrarily substituted with T₂, a halo (C₁ to C₆) alkylsulfonyloxy group unsubstituted or arbitrarily substituted with T₂, an NY₄Y₅ group, a C(O)NY₄Y₅ group, a cyano group, a nitro group, a hydroxy group, a mercapto group, a formyl group, a carboxy group, and an SF₅ group.
<5> The compound according to any one of <1> to <4> or a salt thereof or an N-oxide thereof, wherein B is a structure represented by B-1. 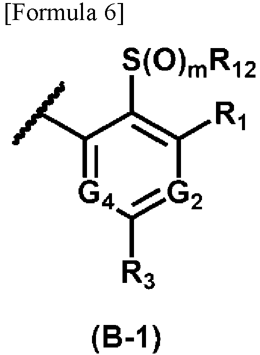
<6> The compound according to any one of <1> to <4> or a salt thereof or an N-oxide thereof, wherein B is a structure represented by B-2. 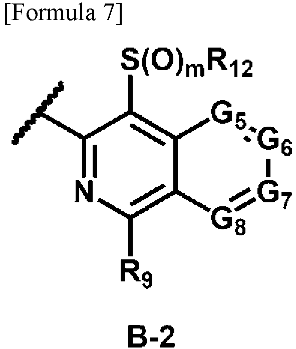
<7> The compound according to any one of <1> to <4> or a salt thereof or an N-oxide thereof, wherein B is a structure represented by B-3. 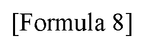 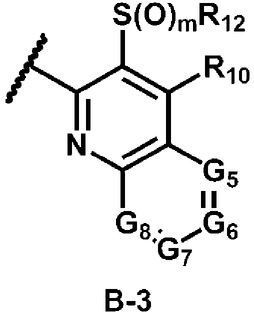
<8> The compound according to any one of <1> to <4> or a salt thereof or an N-oxide thereof, wherein B is a structure represented by B-4. 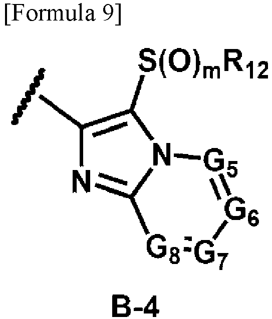
<9> The compound according to any one of <1> to <4> or a salt thereof or an N-oxide thereof, wherein B is a structure represented by B-5. 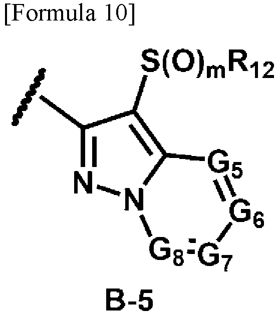
<10> The compound according to any one of <1> to <4> or a salt thereof or an N-oxide thereof, wherein B is a structure represented by B-6. 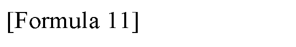 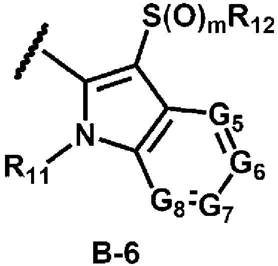
<11> The compound according to any one of <1> to <4> or a salt thereof or an N-oxide thereof, wherein B is a structure represented by B-7 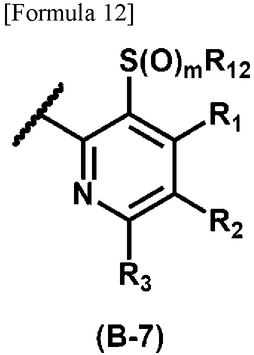 wherein R₁ is a hydrogen atom.
<12> A pest control agent comprising the compound according to any one of <1> to <11> or a salt thereof or an N-oxide thereof.

### ADVANTAGEOUS EFFECTS OF INVENTION

The compound of the present invention represented by formula (1) or a salt thereof or an N-oxide thereof exhibits a quite excellent pest control effect and is useful as a pest control agent.

### DESCRIPTION OF EMBODIMENTS

Herein, the definitions and meanings of the following terms are as follows.

A compound encompassed by the present invention has an optically active form due to the presence of one or two or more asymmetric carbon atoms or asymmetric sulfur atoms, or axial asymmetry, and the present invention encompasses all optically active forms or racemic forms.

A compound encompassed by the present invention may have a geometric isomer depending on the type of a substituent, and the present invention encompasses all geometric isomers or a mixture of geometric isomers included in any proportion.

In addition, a compound encompassed in the present invention may have a tautomer due to a carbonyl group or an imino group as shown below, and the present invention encompasses all tautomers or a mixture of tautomers included in any proportion. In the following formulas, moiety B and moiety D are the same as above.

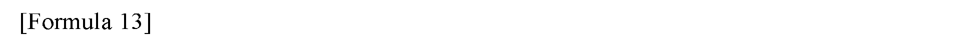

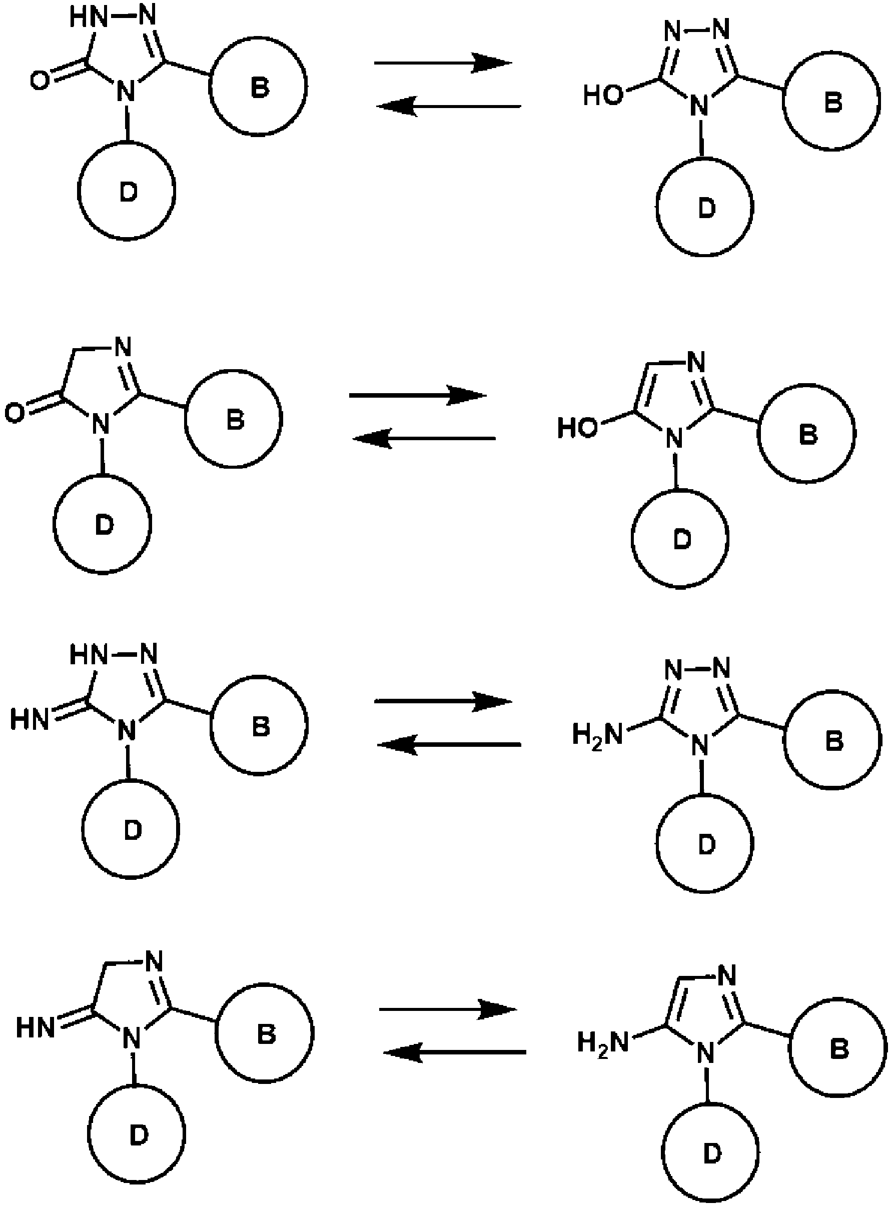

A salt of the compound encompassed by the present invention is a salt that can be formed from the compound according to a conventional method. Examples thereof include a salt of a hydrohalic acid such as hydrofluoric acid, hydrochloric acid, hydrobromic acid, or hydroiodic acid, a salt of an inorganic acid such as nitric acid, sulfuric acid, phosphoric acid, chloric acid, or perchloric acid, a salt of a sulfonic acid such as methanesulfonic acid, ethanesulfonic acid, trifluoromethanesulfonic acid, benzenesulfonic acid, or p-toluenesulfonic acid, a salt of a carboxylic acid such as formic acid, acetic acid, propionic acid, trifluoroacetic acid, fumaric acid, tartaric acid, oxalic acid, maleic acid, malic acid, succinic acid, benzoic acid, mandelic acid, ascorbic acid, lactic acid, gluconic acid, or citric acid, and a salt of an amino acid such as glutamic acid or aspartic acid. Further examples thereof include a salt of an alkali metal such as lithium, sodium, or potassium, a salt of an alkaline earth metal such as calcium, barium, or magnesium, a salt of aluminum, and a quaternary ammonium salt such as a tetramethylammonium salt, a tetrabutylammonium salt, or a benzyltrimethylammonium salt.

In the compound of the present invention, the N-oxide is a compound obtained by oxidizing a nitrogen atom of a tertiary amine or an annular nitrogen atom in a heterocycle. Examples of the heterocycle that can form an N-oxide include a pyridine ring, a pyridazine ring, a pyrimidine ring, a pyrazine ring, and a fused ring including the above nitrogen-containing heterocycle.

Next, specific examples of each substituent shown herein will be shown below. Here, n- means normal, i- means iso, s- means secondary, tert- means tertiary, and c- means cyclo. In addition, Me means a methyl group, Et means an ethyl group, Pr means a propyl group, nPr means a n-propyl group, i-Pr means an i-propyl group, cPr means a cyclopropyl group, Bu means a butyl group, Ph means a phenyl group, and Ac means an acetyl group.

As used herein, examples of the "halogen atom" include a fluorine atom, a chlorine atom, a bromine atom, and an iodine atom. As used herein, the notation "halo" also represents a halogen atom of these.

As used herein, the notation "(Cₐ to C_{b}) alkyl" represents a linear or branched hydrocarbon group having a to b carbon atoms, specific examples thereof include a methyl group, an ethyl group, a n-propyl group, an i-propyl group, a n-butyl group, an i-butyl group, a s-butyl group, a tert-butyl group, a n-pentyl group, a 1,1-dimethylpropyl group, and a n-hexyl group, and those having a number of carbon atoms in the specified range are selected.

As used herein, the notation "halo (Cₐ to C_{b}) alkyl" represents a linear or branched hydrocarbon group having a to b carbon atoms in which a hydrogen atom bonded to a carbon atom is arbitrarily substituted with a halogen atom. At this time, when hydrogen atoms are substituted with two or more halogen atoms, these halogen atoms may be the same or different from each other. Specific examples thereof include a fluoromethyl group, a chloromethyl group, a bromomethyl group, an iodomethyl group, a difluoromethyl group, a dichloromethyl group, a trifluoromethyl group, a chlorodifluoromethyl group, a trichloromethyl group, a bromodifluoromethyl group, a 1-fluoroethyl group, a 2-fluoroethyl group, a 2-chloroethyl group, a 2-bromoethyl group, a 2,2-difluoroethyl group, a 2,2,2-trifluoroethyl group, a 2-chloro-2,2-difluoroethyl group, a 2,2,2-trichloroethyl group, a 2-bromo-2,2-difluoroethyl group, a 1,1,2,2-tetrafluoroethyl group, a 2-chloro-1,1,2-trifluoroethyl group, a 2-chloro-1,1,2,2-tetrafluoroethyl group, a pentafluoroethyl group, a 2,2-difluoropropyl group, a 3,3,3-trifluoropropyl group, a 3-bromo-3,3-difluoropropyl group, a 2,2,3,3-tetrafluoropropyl group, a 2,2,3,3,3-pentafluoropropyl group, a 1,1,2,3,3,3-hexafluoropropyl group, a heptafluoropropyl group, a 2,2,2-trifluoro-1-(methyl)ethyl group, a 2,2,2-trifluoro-1-(trifluoromethyl)ethyl group, a 1,2,2,2-tetrafluoro-1-(trifluoromethyl)ethyl group, a 2,2,3,4,4,4-hexafluorobutyl group, a 2,2,3,3,4,4,4-heptafluorobutyl group, and a nonafluorobutyl group. Those having a number of carbon atoms in the specified range are selected.

As used herein, the notation "(Cₐ to C_{b}) alkenyl" represents a linear or branched unsaturated hydrocarbon group having a to b carbon atoms and having one or two or more double bonds in the molecule. Specific examples thereof include a vinyl group, a 1-propenyl group, a 2-propenyl group, a 1-methylethenyl group, a 2-butenyl group, a 2-methyl-2-propenyl group, a 3-methyl-2-butenyl group, and a 1,1-dimethyl-2-propenyl group, and those having a number of carbon atoms in the specified range are selected.

As used herein, the notation "halo (Cₐ to C_{b}) alkenyl" represents a linear or branched unsaturated hydrocarbon group having a to b carbon atoms in which a hydrogen atom bonded to a carbon atom is arbitrarily substituted with a halogen atom and having one or two or more double bonds in the molecule. At this time, when hydrogen atoms are substituted with two or more halogen atoms, these halogen atoms may be the same or different from each other. Specific examples thereof include a 2,2-dichlorovinyl group, a 2-fluoro-2-propenyl group, a 2-chloro-2-propenyl group, a 2-bromo-2-propenyl group, a 3,3-difluoro-2-propenyl group, a 2,3-dichloro-2-propenyl group, a 3,3-dichloro-2-propenyl group, a 2,3,3-trifluoro-2-propenyl group, a 2,3,3-trichloro-2-propenyl group, a 1-(trifluoromethyl)ethenyl group, a 4,4-difluoro-3-butenyl group, a 3,4,4-trifluoro-3-butenyl group, and a 3-chloro-4,4,4-trifluoro-2-butenyl group. Those having a number of carbon atoms in the specified range are selected.

As used herein, the notation "(Cₐ to C_{b}) alkenyloxy" represents an alkenyl-O- group in which the alkenyl is a previously defined alkenyl having a to b carbon atoms. Specific examples thereof include a vinyloxy group, an allyloxy group, a 1-propenyloxy group, a 2-propenyloxy group, a 1-methylethenyloxy group, a 2-butenyloxy group, a 2-methyl-2-propenyloxy group, a 3-methyl-2-butenyloxy group, and a 1,1-dimethyl-2-propenyloxy group. Those having a number of carbon atoms in the specified range are selected.

As used herein, the notation "halo (Cₐ to C_{b}) alkenyloxy" represents an alkenyl-O-group in which the alkenyl is a previously defined alkenyl having a to b carbon atoms in which a hydrogen atom bonded to a carbon atom is arbitrarily substituted with a halogen atom. Specific examples thereof include a 1-fluorovinyloxy group, a 1-chlorovinyloxy group, a 1-bromo-1-propenyloxy group, a 3-iodo-butenyloxy group, a 1-fluoro-2-methyl-propenyloxy group, and a 3,3-dichloroallyloxy group. Those having a number of carbon atoms in the specified range are selected.

As used herein, the notation "(Cₐ to C_{b}) alkynyl" represents a linear or branched unsaturated hydrocarbon group having a to b carbon atoms and having one or two or more triple bonds in the molecule. Specific examples thereof include an ethynyl group, a propargyl group, a 2-butynyl group, a 1-pentynyl group, a 1-hexynyl group, and a 4,4,4-trifluoro-2-butynyl group. Those having a number of carbon atoms in the specified range are selected.

As used herein, the notation "halo (Cₐ to C_{b}) alkynyl" represents a linear or branched unsaturated hydrocarbon group having a to b carbon atoms in which a hydrogen atom bonded to a carbon atom is arbitrarily substituted with a halogen atom and having one or two or more triple bonds in the molecule. At this time, when hydrogen atoms are substituted with two or more halogen atoms, these halogen atoms may be the same or different from each other. Specific examples thereof include a 3-fluoro-1-propynyl group, a 3-chloro-1-propynyl group, a 3-bromo-1-butynyl group, a 3-bromo-2-propynyl group, a 3-iodo-2-propynyl group, a 3-bromo-1-hexynyl group, a 5,5-dichloro-2-methyl-3-pentynyl group, and a 4-chloro-1,1-dimethyl-2-butynyl group. Those having a number of carbon atoms in the specified range are selected.

As used herein, the notation "(Cₐ to C_{b}) cycloalkyl" represents a cyclic hydrocarbon group having a to b carbon atoms and can form a 3- to 6-membered monocyclic or combined ring structure. In addition, each ring may be arbitrarily substituted with an alkyl group having a number of carbon atoms in the specified range. Specific examples thereof include a cyclopropyl group, a 1-methylcyclopropyl group, a 2-methylcyclopropyl group, a 2,2-dimethylcyclopropyl group, a cyclobutyl group, a cyclopentyl group, and a cyclohexyl group. Those having a number of carbon atoms in the specified range are selected.

As used herein, the notation "halo (Cₐ to C_{b}) cycloalkyl" represents a cyclic hydrocarbon group having a to b carbon atoms in which a hydrogen atom bonded to a carbon atom is arbitrarily substituted with a halogen atom and can form a 3- to 6-membered monocyclic or combined ring structure. In addition, each ring may be arbitrarily substituted with an alkyl group having a number of carbon atoms in the specified range. Specific examples thereof include a 1-fluorocyclopropyl group, a 2-fluorocyclopropyl group, a 1-chlorocyclopropyl group, a 1-bromocyclopropyl group, a 1-iodocyclopropyl group, a 2,2-dichlorocyclopropyl group, a 1-fluorocyclobutyl group, 1-chlorocyclopentyl group, and a 1-bromocyclohexyl group. Those having a number of carbon atoms in the specified range are selected.

As used herein, the notation "1-cyano-(Cₐ to C_{b}) cycloalkyl" represents a cyclic hydrocarbon group having a to b carbon atoms, which is substituted with a cyano group at position 1 and can form a 3- to 6-membered monocyclic or combined ring structure. In addition, each ring may be arbitrarily substituted with an alkyl group having a number of carbon atoms in the specified range. Specific examples thereof include a 1-cyano-cyclopropyl group, a 1-cyano-2-methylcyclopropyl group, a 1-cyano-2,2-dimethylcyclopropyl group, a 1-cyano-cyclobutyl group, a 1-cyano-cyclopentyl group, and a 1-cyano-cyclohexyl group. Those having a number of carbon atoms in the specified range are selected.

As used herein, the notation "(Cₐ to C_{b}) alkoxy" represents an alkyl-O- group in which the alkyl is a previously defined alkyl having a to b carbon atoms. Specific examples thereof include a methoxy group, an ethoxy group, a n-propyloxy group, an i-propyloxy group, a n-butyloxy group, an i-butyloxy group, a s-butyloxy group, a tert-butyloxy group, and a 2-ethylhexyloxy group. Those having a number of carbon atoms in the specified range are selected.

As used herein, the notation "halo (Cₐ to C_{b}) alkoxy" represents a haloalkyl-O- group in which the haloalkyl is a previously defined haloalkyl having a to b carbon atoms. Specific examples thereof include a difluoromethoxy group, a trifluoromethoxy group, a chlorodifluoromethoxy group, a bromodifluoromethoxy group, a 2-fluoroethoxy group, a 2-chloroethoxy group, a 2,2,2-trifluoroethoxy group, a 1,1,2,2-tetrafluoroethoxy group, a 2-chloro-1,1,2-trifluoroethoxy group, and a 1, 1,2,3,3,3-hexafluoropropyloxy group. Those having a number of carbon atoms in the specified range are selected.

As used herein, the notation "(Cₐ to C_{b}) alkylthio" represents an alkyl-S- group in which the alkyl is a previously defined alkyl having a to b carbon atoms. Specific examples thereof include a methylthio group, an ethylthio group, a n-propylthio group, an i-propylthio group, a n-butylthio group, an i-butylthio group, a s-butylthio group, and a tert-butylthio group. Those having a number of carbon atoms in the specified range are selected.

As used herein, the notation "halo (Cₐ to C_{b}) alkylthio" represents a haloalkyl-S- group in which the haloalkyl is a previously defined haloalkyl having a to b carbon atoms. Specific examples thereof include a difluoromethylthio group, a trifluoromethylthio group, a chlorodifluoromethylthio group, a bromodifluoromethylthio group, a 2,2,2-trifluoroethylthio group, a 1,1,2,2-tetrafluoroethylthio group, a 2-chloro-1,1,2-trifluoroethylthio group, a pentafluoroethylthio group, a 1,1,2,3,3,3-hexafluoropropylthio group, a heptafluoropropylthio group, a 1,2,2,2-tetrafluoro-1-(trifluoromethyl)ethylthio group, and a nonafluorobutylthio group. Those having a number of carbon atoms in the specified range are selected.

As used herein, the notation "(Cₐ to C_{b}) alkylsulfinyl" represents an alkyl-S(O)- group in which the alkyl is a previously defined alkyl having a to b carbon atoms. Specific examples thereof include a methylsulfinyl group, an ethylsulfinyl group, a n-propylsulfinyl group, an i-propylsulfinyl group, a n-butylsulfinyl group, an i-butylsulfinyl group, a s-butylsulfinyl group, and a tert-butylsulfinyl group. Those having a number of carbon atoms in the specified range are selected.

As used herein, the notation "halo (Cₐ to C_{b}) alkylsulfinyl" represents a haloalkyl-S(O)- group in which the haloalkyl is a previously defined haloalkyl having a to b carbon atoms. Specific examples thereof include a difluoromethylsulfinyl group, a trifluoromethylsulfinyl group, a chlorodifluoromethylsulfinyl group, a bromodifluoromethylsulfinyl group, a 2,2,2-trifluoroethylsulfinyl group, a 1,2,2,2-tetrafluoro-1-(trifluoromethyl)ethylsulfinyl group, and a nonafluorobutylsulfinyl group. Those having a number of carbon atoms in the specified range are selected.

As used herein, the notation "(Cₐ to C_{b}) alkylsulfonyl" represents an alkyl-SO₂- group in which the alkyl is a previously defined alkyl having a to b carbon atoms. Specific examples thereof include a methylsulfonyl group, an ethylsulfonyl group, a n-propylsulfonyl group, an i-propylsulfonyl group, a n-butylsulfonyl group, an i-butylsulfonyl group, a s-butylsulfonyl group, and a tert-butylsulfonyl group. Those having a number of carbon atoms in the specified range are selected.

As used herein, the notation "halo (Cₐ to C_{b}) alkylsulfonyl" represents a haloalkyl-SO₂-group in which the haloalkyl is a previously defined haloalkyl having a to b carbon atoms. Specific examples thereof include a difluoromethylsulfonyl group, a trifluoromethylsulfonyl group, a chlorodifluoromethylsulfonyl group, a bromodifluoromethylsulfonyl group, a 2,2,2-trifluoroethylsulfonyl group, a 1,1,2,2-tetrafluoroethylsulfonyl group, and a 2-chloro-1,1,2-trifluoroethylsulfonyl group. Those having a number of carbon atoms in the specified range are selected.

As used herein, the notation "(Cₐ to C_{b}) alkylcarbonyl" represents an alkyl-C(O)- group in which the alkyl is a previously defined alkyl having a to b carbon atoms. Specific examples thereof include an acetyl group, a propionyl group, a butyryl group, an isobutyryl group, a valeryl group, an isovaleryl group, a 2-methylbutanoyl group, a pivaloyl group, a hexanoyl group, and a heptanoyl group. Those having a number of carbon atoms in the specified range are selected.

As used herein, the notation "halo (Cₐ to C_{b}) alkylcarbonyl" represents a haloalkyl-C(O)- group in which the haloalkyl is a previously defined haloalkyl having a to b carbon atoms. Specific examples thereof include a fluoroacetyl group, a chloroacetyl group, a difluoroacetyl group, a dichloroacetyl group, a trifluoroacetyl group, a chlorodifluoroacetyl group, a bromodifluoroacetyl group, a trichloroacetyl group, a pentafluoropropionyl group, a heptafluorobutanoyl group, and 3-chloro-2,2-dimethylpropanoyl group. Those having a number of carbon atoms in the specified range are selected.

As used herein, the notation "(Cₐ to C_{b}) alkoxycarbonyl" represents an alkyl-O-C(O)-group in which the alkyl is a previously defined alkyl having a to b carbon atoms. Specific examples thereof include a methoxycarbonyl group, an ethoxycarbonyl group, a n-propyloxycarbonyl group, an i-propyloxycarbonyl group, a n-butoxycarbonyl group, an i-butoxycarbonyl group, a s-butoxycarbonyl group, a tert-butoxycarbonyl group, and a 2-ethylhexyloxycarbonyl group. Those having a number of carbon atoms in the specified range are selected.

As used herein, the notation "halo (Cₐ to C_{b}) alkoxycarbonyl" represents a haloalkyl-O-C(O)- group in which the haloalkyl is a previously defined haloalkyl having a to b carbon atoms. Specific examples thereof include a chloromethoxycarbonyl group, a 2-chloroethoxycarbonyl group, a 2,2-difluoroethoxycarbonyl group, a 2,2,2-trifluoroethoxycarbonyl group, and a 2,2,2-trichloroethoxycarbonyl group, and those having a number of carbon atoms in the specified range are selected.

As used herein, the notation "(Cₐ to C_{b}) alkylcarbonyloxy" represents an alkyl-C(O)-O-group in which the alkyl is a previously defined alkyl having a to b carbon atoms. Specific examples thereof include a methylcarbonyloxy group, an ethylcarbonyloxy group, a n-propylcarbonyloxy group, an i-propylcarbonyloxy group, a n-butylcarbonyloxy group, an i-butylcarbonyloxy group, a s-butylcarbonyloxy group, a tert-butylcarbonyloxy group, and a 2-ethylhexylcarbonyloxy group. Those having a number of carbon atoms in the specified range are selected.

As used herein, the notation "halo (Cₐ to C_{b}) alkylcarbonyloxy" represents a haloalkyl-C(O)-O- group in which the haloalkyl is a previously defined haloalkyl having a to b carbon atoms. Specific examples thereof include a chloromethylcarbonyloxy group, a 2-chloroethylcarbonyloxy group, a 2,2-difluoroethylcarbonyloxy group, a 2,2,2-trifluoroethylcarbonyloxy group, and a 2,2,2-trichloroethylcarbonyloxy group. Those having a number of carbon atoms in the specified range are selected.

As used herein, the notation "(Cₐ to C_{b}) alkoxycarbonyloxy" represents an alkoxy-C(O)-O- group in which the alkoxy is a previously defined alkoxy having a to b carbon atoms. Specific examples thereof include a methoxycarbonyloxy group, an ethoxycarbonyloxy group, a n-propyloxycarbonyloxy group, an i-propyloxycarbonyloxy group, a n-butoxycarbonyloxy group, an i-butoxycarbonyloxy group, a s-butoxycarbonyloxy group, a tert-butoxycarbonyloxy group, and a 2-ethylhexyloxycarbonyloxy group. Those having a number of carbon atoms in the specified range are selected.

As used herein, the notation "halo (Cₐ to C_{b}) alkoxycarbonyloxy" represents a haloalkoxy-C(O)-O- group in which the haloalkoxy is a previously defined haloalkoxy having a to b carbon atoms. Specific examples thereof include a chloromethoxycarbonyloxy group, a 2-chloroethoxycarbonyloxy group, a 2,2-difluoroethoxycarbonyloxy group, a 2,2,2-trifluoroethoxycarbonyloxy group, and a 2,2,2-trichloroethoxycarbonyloxy group. Those having a number of carbon atoms in the specified range are selected.

As used herein, the notation "(Cₐ to C_{b}) alkylsulfonyloxy" represents an alkyl-SO₂-O-group in which the alkyl is a previously defined alkyl having a to b carbon atoms. Specific examples thereof include a methylsulfonyloxy group, an ethylsulfonyloxy group, a n-propylsulfonyloxy group, an i-propylsulfonyloxy group, a n-butylsulfonyloxy group, an i-butylsulfonyloxy group, a s-butylsulfonyloxy group, and a tert-butylsulfonyloxy group. Those having a number of carbon atoms in the specified range are selected.

As used herein, the notation "halo (Cₐ to C_{b}) alkylsulfonyloxy" represents a haloalkyl-SO₂-O- group in which the haloalkyl is a previously defined haloalkyl having a to b carbon atoms. Specific examples thereof include a difluoromethylsulfonyloxy group, a trifluoromethylsulfonyloxy group, a chlorodifluoromethylsulfonyloxy group, a bromodifluoromethylsulfonyloxy group, a 2,2,2-trifluoroethylsulfonyloxy group, a 1,1,2,2-tetrafluoroethylsulfonyloxy group, and a 2-chloro-1,1,2-trifluoroethylsulfonyloxy group. Those having a number of carbon atoms in the specified range are selected.

As used herein, the notation "heterocyclic group" represents a cyclic functional group including one or more of a nitrogen atom, an oxygen atom, and a sulfur atom. The heterocyclic group is preferably an aromatic group including one or more of a nitrogen atom, an oxygen atom, and a sulfur atom. Specific examples thereof include thiophen-2-yl, thiophen-3-yl, furan-2-yl, furan-3-yl, pyrrol-1-yl, pyrrol-2-yl, pyrrol-3-yl, oxazol-2-yl, oxazol-4-yl, oxazol-5-yl, isoxazol-3-yl, isoxazol-4-yl, isoxazol-5-yl, isoxazolin-3-yl, isoxazolin-4-yl, isoxazolin-5-yl, thiazol-2-yl, thiazol-4-yl, thiazol-5-yl, isothiazol-3-yl, isothiazol-4-yl, isothiazol-5-yl, pyrazol-1-yl, pyrazol-3-yl, pyrazol-4-yl, pyrazol-5-yl, imidazol-1-yl, imidazol-2-yl, imidazol-4-yl, 1,3,4-oxadiazol-2-yl, 1,2,4-oxadiazol-3-yl, 1,2,4-oxadiazol-5-yl, 1,3,4-thiadiazol-2-yl, 1,2,4-thiadiazol-3-yl, 1,2,4-thiadiazol-5-yl, 1,2,4-triazol-1-yl, 1,2,4-triazol-3-yl, 1,2,4 -triazol-5-yl, 1,2,3-thiadiazol-4-yl, 1,2,3-thiadiazol-5-yl, 1,2,3-triazol-1-yl, 1,2,3-triazol-2-yl, 1,2,3-triazol-4-yl, 1,2,3,4-tetrazol-1-yl, 1,2,3,4-tetrazol-2-yl, 1,2,3,4-tetrazol-5-yl, pyridin-2-yl, pyridin-3-yl, pyridin-4-yl, pyrimidin-2-yl, pyrimidin-4-yl, pyrimidin-5-yl, pyrazin-2-yl, pyridazin-3-yl, pyridazin-4-yl, 1,3,5-triazin-2-yl, 1,2,4-triazin-3-yl, 1,2,4-triazin-5-yl, 1,2,4-triazin-6-yl, benzothiophen-2-yl, benzothiophen-3-yl, benzothiophen-4-yl, benzothiophen-5-yl, benzothiophen-6-yl, benzothiophen-7-yl, benzofuran-2-yl, benzofuran-3-yl, benzofuran-4-yl, benzofuran-5-yl, benzofuran-6-yl, benzofuran-7-yl, indol-1-yl, indol-2-yl, indol-3-yl, indol-4-yl, indol-5-yl, indol-6-yl, indol-7-yl, benzothiazol-2-yl, benzothiazol-4-yl, benzothiazol-5-yl, benzothiazol-6-yl, benzothiazol-7-yl, benzimidazol-1-yl, benzimidazol-2-yl, benzimidazol-4-yl, benzimidazol-5-yl, benzimidazol-6-yl, benzimidazol-7-yl, benzisoxazol-3-yl, benzisoxazol-4-yl, benzisoxazol-5-yl, benzisoxazol-6-yl, benzisoxazol-7-yl, benzisothiazol-3-yl, benzisothiazol-4-yl, benzisothiazol-5-yl, benzisothiazol-6-yl, benzisothiazol-7-yl, indazol-1-yl, indazol-3-yl, indazol-4-yl, indazol-5-yl, indazol-6-yl, indazol-7-yl, benzoxazol-2-yl, benzoxazol-4-yl, benzoxazol-5-yl, benzoxazol-6-yl, benzoxazol-7-yl, quinolin-2-yl, quinolin-3-yl, quinolin-4-yl, quinolin-5-yl, quinolin-6-yl, quinolin-7-yl, quinolin-8-yl, isoquinolin-1-yl, isoquinolin-3-yl, isoquinolin-4-yl, isoquinolin-5-yl, isoquinolin-6-yl, isoquinolin-7-yl, isoquinolin-8-yl, quinoxalin-2-yl, quinoxalin-3-yl, quinoxalin-5-yl, quinoxalin-6-yl, quinoxalin-7-yl, quinoxalin-8-yl, phthalazin-1-yl, phthalazin-4-yl, phthalazin-5-yl, phthalazin-6-yl, phthalazin-7-yl, phthalazin-8-yl, cinnolin-3-yl, cinnolin-4-yl, cinnolin-5-yl, cinnolin-6-yl, cinnolin-7-yl, cinnolin-8-yl, quinazolin-2-yl, quinazolin-4-yl, quinazolin-5-yl, quinazolin-6-yl, quinazolin-7-yl, and quinazolin-8-yl.

As used herein, the notation "arbitrarily substituted (Cₐ to C_{b}) alkyl" represents a previously defined alkyl group having a to b carbon atoms in which a hydrogen atom bonded to a carbon atom is arbitrarily substituted with a substituent arbitrarily selected from the corresponding substituent group, and those having a number of carbon atoms in the specified range are selected. At this time, when two or more substituents are present on each (Cₐ to C_{b}) alkyl group, the substituents may be the same or different from each other.

As used herein, the notation "arbitrarily substituted halo (Cₐ to C_{b}) alkyl" represents a previously defined haloalkyl group having a to b carbon atoms in which a hydrogen atom or a halogen atom bonded to a carbon atom is arbitrarily substituted with a substituent arbitrarily selected from the corresponding substituent group, and those having a number of carbon atoms in the specified range are selected. At this time, when two or more substituents are present on each halo (Cₐ to C_{b}) alkyl group, the substituents may be the same or different from each other.

As used herein, the notation "arbitrarily substituted (Cₐ to C_{b}) cycloalkyl" represents a previously defined cycloalkyl group having a to b carbon atoms in which a hydrogen atom bonded to a carbon atom is arbitrarily substituted with a substituent arbitrarily selected from the corresponding substituent group, and those having a number of carbon atoms in the specified range are selected. At this time, when two or more substituents are present on each (Cₐ to C_{b}) cycloalkyl group, the substituents may be the same or different from each other.

As used herein, the notation "arbitrarily substituted halo (Cₐ to C_{b}) cycloalkyl" represents a previously defined halocycloalkyl group having a to b carbon atoms in which a hydrogen atom or a halogen atom bonded to a carbon atom is arbitrarily substituted with a substituent arbitrarily selected from the corresponding substituent group, and those having a number of carbon atoms in the specified range are selected. At this time, when two or more substituents are present on each halo (Cₐ to C_{b}) cycloalkyl group, the substituents may be the same or different from each other.

As used herein, the notation "arbitrarily substituted (Cₐ to C_{b}) alkenyl" represents a previously defined alkenyl group having a to b carbon atoms in which a hydrogen atom bonded to a carbon atom is arbitrarily substituted with a substituent arbitrarily selected from the corresponding substituent group, and those having a number of carbon atoms in the specified range are selected. At this time, when two or more substituents are present on each (Cₐ to C_{b}) alkenyl group, the substituents may be the same or different from each other.

As used herein, the notation "arbitrarily substituted halo (Cₐ to C_{b}) alkenyl" represents a previously defined haloalkenyl group having a to b carbon atoms in which a hydrogen atom or a halogen atom bonded to a carbon atom is arbitrarily substituted with a substituent arbitrarily selected from the corresponding substituent group, and those having a number of carbon atoms in the specified range are selected. At this time, when two or more substituents are present on each halo (Cₐ to C_{b}) alkenyl group, the substituents may be the same or different from each other.

As used herein, the notation "arbitrarily substituted (Cₐ to C_{b}) alkenyloxy" represents a previously defined alkenyloxy group having a to b carbon atoms in which a hydrogen atom bonded to a carbon atom is arbitrarily substituted with a substituent arbitrarily selected from the corresponding substituent group, and those having a number of carbon atoms in the specified range are selected. At this time, when two or more substituents are present on each (Cₐ to C_{b}) alkenyloxy group, the substituents may be the same or different from each other.

As used herein, the notation "arbitrarily substituted halo (Cₐ to C_{b}) alkenyloxy" represents a previously defined haloalkenyloxy group having a to b carbon atoms in which a hydrogen atom or a halogen atom bonded to a carbon atom is arbitrarily substituted with a substituent arbitrarily selected from the corresponding substituent group, and those having a number of carbon atoms in the specified range are selected. At this time, when two or more substituents are present on each halo (Cₐ to C_{b}) alkenyloxy group, the substituents may be the same or different from each other.

As used herein, the notation "arbitrarily substituted (Cₐ to C_{b}) alkynyl" represents a previously defined alkynyl group having a to b carbon atoms in which a hydrogen atom bonded to a carbon atom is arbitrarily substituted with a substituent arbitrarily selected from the corresponding substituent group, and those having a number of carbon atoms in the specified range are selected. At this time, when two or more substituents are present on each (Cₐ to C_{b}) alkynyl group, the substituents may be the same or different from each other.

As used herein, the notation "arbitrarily substituted halo (Cₐ to C_{b}) alkynyl" represents a previously defined haloalkynyl group having a to b carbon atoms in which a hydrogen atom or a halogen atom bonded to a carbon atom is arbitrarily substituted with a substituent arbitrarily selected from the corresponding substituent group, and those having a number of carbon atoms in the specified range are selected. At this time, when two or more substituents are present on each halo (Cₐ to C_{b}) alkynyl group, the substituents may be the same or different from each other.

As used herein, the notation "arbitrarily substituted (Cₐ to C_{b}) alkoxy" represents a previously defined alkoxy group having a to b carbon atoms in which a hydrogen atom bonded to a carbon atom is arbitrarily substituted with a substituent arbitrarily selected from the corresponding substituent group, and those having a number of carbon atoms in the specified range are selected. At this time, when two or more substituents are present on each (Cₐ to C_{b}) alkoxy group, the substituents may be the same or different from each other.

As used herein, the notation "arbitrarily substituted halo (Cₐ to C_{b}) alkoxy" represents a previously defined haloalkoxy group having a to b carbon atoms in which a hydrogen atom or a halogen atom bonded to a carbon atom is arbitrarily substituted with a substituent arbitrarily selected from the corresponding substituent group, and those having a number of carbon atoms in the specified range are selected. At this time, when two or more substituents are present on each halo (Cₐ to C_{b}) alkoxy group, the substituents may be the same or different from each other.

As used herein, the notation "arbitrarily substituted (Cₐ to C_{b}) alkylthio" represents a previously defined alkylthio group having a to b carbon atoms in which a hydrogen atom bonded to a carbon atom is arbitrarily substituted with a substituent arbitrarily selected from the corresponding substituent group, and those having a number of carbon atoms in the specified range are selected. At this time, when two or more substituents are present on each (Cₐ to C_{b}) alkylthio group, the substituents may be the same or different from each other.

As used herein, the notation "arbitrarily substituted halo (Cₐ to C_{b}) alkylthio" represents a previously defined haloalkylthio group having a to b carbon atoms in which a hydrogen atom or a halogen atom bonded to a carbon atom is arbitrarily substituted with a substituent arbitrarily selected from the corresponding substituent group, and those having a number of carbon atoms in the specified range are selected. At this time, when two or more substituents are present on each halo (Cₐ to C_{b}) alkylthio group, the substituents may be the same or different from each other.

As used herein, the notation "arbitrarily substituted (Cₐ to C_{b}) alkylsulfinyl" represents a previously defined alkylsulfinyl group having a to b carbon atoms in which a hydrogen atom bonded to a carbon atom is arbitrarily substituted with a substituent arbitrarily selected from the corresponding substituent group, and those having a number of carbon atoms in the specified range are selected. At this time, when two or more substituents are present on each (Cₐ to C_{b}) alkylsulfinyl group, the substituents may be the same or different from each other.

As used herein, the notation "arbitrarily substituted halo (Cₐ to C_{b}) alkylsulfinyl" represents a previously defined haloalkylsulfinyl group having a to b carbon atoms in which a hydrogen atom or a halogen atom bonded to a carbon atom is arbitrarily substituted with a substituent arbitrarily selected from the corresponding substituent group, and those having a number of carbon atoms in the specified range are selected. At this time, when two or more substituents are present on each halo (Cₐ to C_{b}) alkylsulfinyl group, the substituents may be the same or different from each other.

As used herein, the notation "arbitrarily substituted (Cₐ to C_{b}) alkylsulfonyl" represents a previously defined alkylsulfonyl group having a to b carbon atoms in which a hydrogen atom bonded to a carbon atom is arbitrarily substituted with a substituent arbitrarily selected from the corresponding substituent group, and those having a number of carbon atoms in the specified range are selected. At this time, when two or more substituents are present on each (Cₐ to C_{b}) alkylsulfonyl group, the substituents may be the same or different from each other.

As used herein, the notation "arbitrarily substituted halo (Cₐ to C_{b}) alkylsulfonyl" represents a previously defined haloalkylsulfonyl group having a to b carbon atoms in which a hydrogen atom or a halogen atom bonded to a carbon atom is arbitrarily substituted with a substituent arbitrarily selected from the corresponding substituent group, and those having a number of carbon atoms in the specified range are selected. At this time, when two or more substituents are present on each halo (Cₐ to C_{b}) alkylsulfonyl group, the substituents may be the same or different from each other.

As used herein, the notation "arbitrarily substituted (Cₐ to C_{b}) alkylcarbonyl" represents a previously defined alkylcarbonyl group having a to b carbon atoms in which a hydrogen atom bonded to a carbon atom is arbitrarily substituted with a substituent arbitrarily selected from the corresponding substituent group, and those having a number of carbon atoms in the specified range are selected. At this time, when two or more substituents are present on each (Cₐ to C_{b}) alkylcarbonyl group, the substituents may be the same or different from each other.

As used herein, the notation "arbitrarily substituted halo (Cₐ to C_{b}) alkylcarbonyl" represents a previously defined haloalkylcarbonyl group having a to b carbon atoms in which a hydrogen atom or a halogen atom bonded to a carbon atom is arbitrarily substituted with a substituent arbitrarily selected from the corresponding substituent group, and those having a number of carbon atoms in the specified range are selected. At this time, when two or more substituents are present on each halo (Cₐ to C_{b}) alkylcarbonyl group, the substituents may be the same or different from each other.

As used herein, the notation "arbitrarily substituted (Cₐ to C_{b}) alkoxycarbonyl" represents a previously defined alkoxycarbonyl group having a to b carbon atoms in which a hydrogen atom bonded to a carbon atom is arbitrarily substituted with a substituent arbitrarily selected from the corresponding substituent group, and those having a number of carbon atoms in the specified range are selected. At this time, when two or more substituents are present on each (Cₐ to C_{b}) alkoxycarbonyl group, the substituents may be the same or different from each other.

As used herein, the notation "arbitrarily substituted halo (Cₐ to C_{b}) alkoxycarbonyl" represents a previously defined haloalkoxycarbonyl group having a to b carbon atoms in which a hydrogen atom or a halogen atom bonded to a carbon atom is arbitrarily substituted with a substituent arbitrarily selected from the corresponding substituent group, and those having a number of carbon atoms in the specified range are selected. At this time, when two or more substituents are present on each halo (Cₐ to C_{b}) alkoxycarbonyl group, the substituents may be the same or different from each other.

As used herein, the notation "arbitrarily substituted (Cₐ to C_{b}) alkylcarbonyloxy" represents a previously defined alkylcarbonyloxy group having a to b carbon atoms in which a hydrogen atom bonded to a carbon atom is arbitrarily substituted with a substituent arbitrarily selected from the corresponding substituent group, and those having a number of carbon atoms in the specified range are selected. At this time, when two or more substituents are present on each (Cₐ to C_{b}) alkylcarbonyloxy group, the substituents may be the same or different from each other.

As used herein, the notation "arbitrarily substituted halo (Cₐ to C_{b}) alkylcarbonyloxy" represents a previously defined haloalkylcarbonyloxy group having a to b carbon atoms in which a hydrogen atom or a halogen atom bonded to a carbon atom is arbitrarily substituted with a substituent arbitrarily selected from the corresponding substituent group, and those having a number of carbon atoms in the specified range are selected. At this time, when two or more substituents are present on each halo (Cₐ to C_{b}) alkylcarbonyloxy group, the substituents may be the same or different from each other.

As used herein, the notation "arbitrarily substituted (Cₐ to C_{b}) alkoxycarbonyloxy" represents a previously defined alkoxycarbonyloxy group having a to b carbon atoms in which a hydrogen atom bonded to a carbon atom is arbitrarily substituted with a substituent arbitrarily selected from the corresponding substituent group, and those having a number of carbon atoms in the specified range are selected. At this time, when two or more substituents are present on each (Cₐ to C_{b}) alkoxycarbonyloxy group, the substituents may be the same or different from each other.

As used herein, the notation "arbitrarily substituted halo (Cₐ to C_{b}) alkoxycarbonyloxy" represents a previously defined haloalkoxycarbonyloxy group having a to b carbon atoms in which a hydrogen atom or a halogen atom bonded to a carbon atom is arbitrarily substituted with a substituent arbitrarily selected from the corresponding substituent group, and those having a number of carbon atoms in the specified range are selected. At this time, when two or more substituents are present on each halo (Cₐ to C_{b}) alkoxycarbonyloxy group, the substituents may be the same or different from each other.

As used herein, the notation "arbitrarily substituted (Cₐ to C_{b}) alkylsulfonyloxy" represents a previously defined alkylsulfonyloxy group having a to b carbon atoms in which a hydrogen atom bonded to a carbon atom is arbitrarily substituted with a substituent arbitrarily selected from the corresponding substituent group, and those having a number of carbon atoms in the specified range are selected. At this time, when two or more substituents are present on each (Cₐ to C_{b}) alkylsulfonyloxy group, the substituents may be the same or different from each other.

As used herein, the notation "arbitrarily substituted halo (Cₐ to C_{b}) alkylsulfonyloxy" represents a previously defined haloalkylsulfonyloxy group having a to b carbon atoms in which a hydrogen atom or a halogen atom bonded to a carbon atom is arbitrarily substituted with a substituent arbitrarily selected from the corresponding substituent group, and those having a number of carbon atoms in the specified range are selected. At this time, when two or more substituents are present on each halo (Cₐ to C_{b}) alkylsulfonyloxy group, the substituents may be the same or different from each other.

As used herein, the notation "arbitrarily substituted phenyl" represents a phenyl group in which a hydrogen atom bonded to a carbon atom on the phenyl ring is arbitrarily substituted with a substituent arbitrarily selected from the corresponding substituent group. At this time, when two or more substituents are present on each phenyl group, the substituents may be the same or different from each other.

As used herein, the notation "arbitrarily substituted heterocyclic group" represents a heterocyclic group in which a hydrogen atom bonded to a carbon atom on a heterocycle is arbitrarily substituted with a substituent arbitrarily selected from the corresponding substituent group. At this time, when two or more substituents are present on each heterocyclic group, the substituents may be the same or different from each other.

As used herein, the notation "arbitrarily substituted phenoxy" represents a phenoxy group in which a hydrogen atom bonded to a carbon atom on the phenyl ring is arbitrarily substituted with a substituent arbitrarily selected from the corresponding substituent group. At this time, when two or more substituents are present on each phenoxy group, the substituents may be the same or different from each other.

As used herein, the notation "arbitrarily substituted pyridyloxy" represents a pyridyloxy group in which a hydrogen atom bonded to a carbon atom on the pyridine ring is arbitrarily substituted with a substituent arbitrarily selected from the corresponding substituent group. At this time, when two or more substituents are present on each pyridyloxy group, the substituents may be the same or different from each other.

As used herein, the notation "a 5- or 6-membered alicyclic group formed by connecting two adjacent Z₁ or two adjacent Z₂ to each other" or "a 1,3-dioxole group or a 1,4-dioxin group formed by two adjacent Z₁ or two adjacent Z₂ together with a carbon atom to which the two Z₁ or the two Z₂ are attached" represents a 5- or 6-membered alicyclic group or a 1,3-dioxole group or a 1,4-dioxin group formed by connecting any atom on each Z₁ or each Z₂ through a covalent bond in two adjacent Z₁ or two adjacent Z₂ with which a phenyl group, a heterocyclic group, a phenoxy group, or a pyridyloxy group is substituted. At this time, the two Z₁ or the two Z₂ may be the same or different from each other. In addition, the atom on each Z₁ or each Z₂ to be covalently bonded can be independently selected arbitrarily from a carbon atom, a nitrogen atom, an oxygen atom, or a sulfur atom. Specific examples thereof include the substituent structures illustrated below. However, the examples are not limited thereto. In the illustrations below, "Het" means a heterocyclic group, and the heterocyclic group is defined as above.

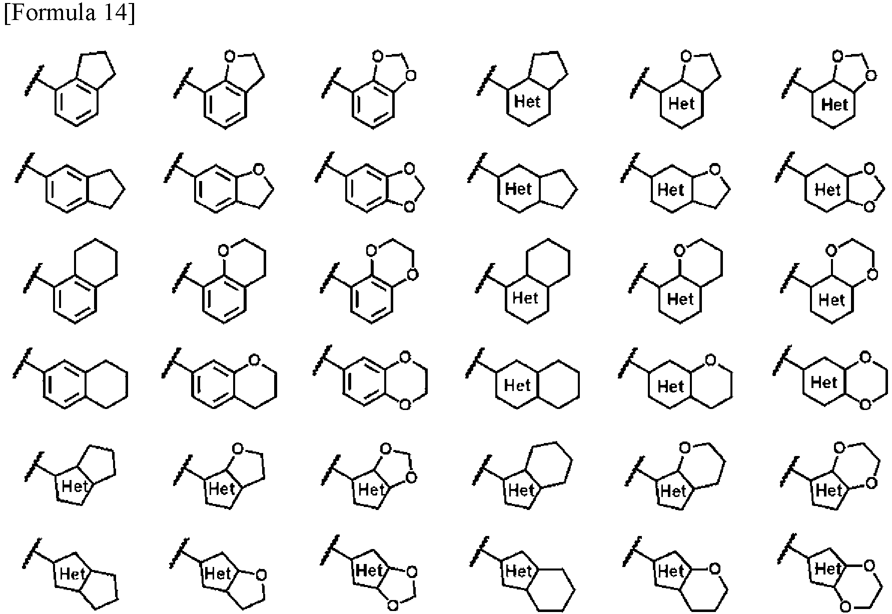

As used herein, the notation "aliphatic 3- to 10-membered cyclic amino group or cyclic amide group formed by connecting Yₐ and Y_{b} to each other" represents an aliphatic 3- to 10-membered cyclic amino group or cyclic amide group formed by connecting any atoms on Yₐ and Y_{b} through a covalent bond.
At this time, the two Yₐ and Y_{b} may be the same or different from each other. In addition, the atom on each of Yₐ and Y_{b} to be covalently bonded can be independently selected arbitrarily from a hydrogen atom, a carbon atom, a nitrogen atom, an oxygen atom, or a sulfur atom. Specific examples thereof include the substituent structures illustrated below. However, the examples are not limited thereto.

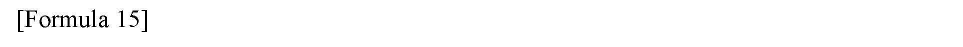

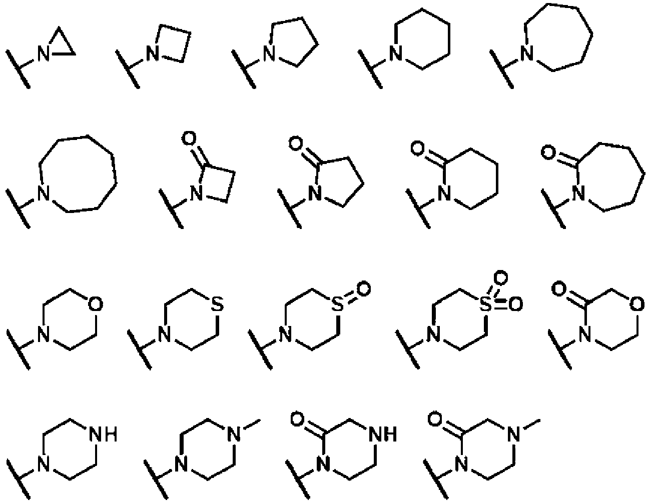

The present invention relates to the compound represented by formula (1) or a salt thereof or an N-oxide thereof. The compound represented by formula (1) is a structural compound in which a cyclic structure moiety D and a cyclic structure moiety B are linked through a ring structure shown in formula (1). Hereinafter, the compound will be described.

In formula (1), the cyclic structure linking moiety D and moiety B is a 5- or 6-membered nitrogen-containing cyclic group including at least two nitrogen atoms. The cyclic structure is, for example, a diazole structure, an oxadiazole structure, a thiadiazole structure, a triazole structure, an imidazole structure, a diazine structure (a pyrazine structure, a pyrimidine structure, or a pyridazine structure), an oxadiazine structure, a thiadiazine structure, or a triazine structure. The cyclic structure is preferably a 5-membered or 6-membered ring diazole structure, oxadiazole structure, thiadiazole structure, or diazine structure, oxadiazine structure, or thiadiazine structure.

In the cyclic structure linking moiety D and moiety B of formula (1), which is the compound of the present invention, Xⱼ is an oxygen atom, a sulfur atom, NY₁, or C(J₅J₆), and J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group (methylidene group) substituted with J₇ and J₈ (=CJ₇J₈), formed by two bonds of C-J₁ and C-J₂, or C-J₃ and C-J₄ together.

C(J₅J₆) is a methylene group (>CJ₅J₆) substituted with J₅ and J₆, and J₅ and J₆ are each independently a hydrogen atom, a halogen atom, a (C₁ to C₆) alkyl group optionally having a substituent, a halo (C₁ to C₆) alkyl group optionally having a substituent, or a group selected from the group consisting of an O atom in a carbonyl group, a S atom in a thiocarbonyl group, an imino group substituted with Y₃, and a methylene group substituted with J₇ and J₈, formed by two bonds of C-J₅ and C-J₆ together. J₅ and J₆ are each preferably a hydrogen atom, a halogen atom, or a group selected from the group consisting of an O atom in a carbonyl group, a S atom in a thiocarbonyl group, an imino group substituted with Y₃, and a methylene group substituted with J₇ and J₈, formed by two bonds of C-J₅ and C-J₆ together.

Y₁, Y₂, and Y₃ are each a group selected from the group consisting of a hydrogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₂ to C₆) alkenyl group optionally having a substituent, a halo (C₂ to C₆) alkenyl group optionally having a substituent, a (C₁ to C₆) alkoxy group optionally having a substituent, a halo (C₁ to C₆) alkoxy group optionally having a substituent, a (C₁ to C₆) alkylcarbonyl group optionally having a substituent, a halo (C₁ to C₆) alkylcarbonyl optionally having a substituent, a (C₃ to C₆) cycloalkylcarbonyl group optionally having a substituent, a halo (C₃ to C₆) cycloalkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylsulfonyl group optionally having a substituent, a halo (C₁ to C₆) alkylsulfonyl group optionally having a substituent, and a hydroxy group.

J₇ and J₈ are each a group selected from the group consisting of a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, and an amino group optionally having a substituent.

In formula (1), specific examples of the cyclic structure linking moiety D and moiety B include a 1,2,4-oxadiazole structure, a 1,2,4-thiadiazole structure, a 1,2,4-triazole structure, an imidazole structure, 1,2,4-oxadiazine structure, a 1,2,4-thiadiazine structure, a 1,2,4-triazine structure, and a pyrimidine structure, illustrated by the following formulas.

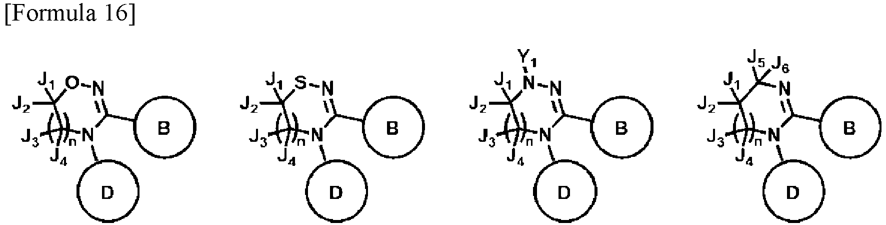

When X₁ in formula (1) is NY₁, Y₁ is preferably a group selected from the group consisting of a hydrogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₁ to C₆) alkylcarbonyl optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, and a hydroxy group. Y₁ is more preferably a group selected from the group consisting of a hydrogen atom, a (C₁ to C₆) alkyl group optionally having a substituent, a halo (C₁ to C₆) alkyl group optionally having a substituent, a (C₁ to C₆) alkoxy group, a halo (C₁ to C₆) alkoxy group optionally having a substituent, a (C₁ to C₆) alkylcarbonyl group optionally having a substituent, a halo (C₁ to C₆) alkylcarbonyl group optionally having a substituent, and a hydroxy group. Y₁ is further preferably a group selected from the group consisting of a hydrogen atom, a (C₁ to C₆) alkyl group optionally having a substituent, and a (C₁ to C₆) alkylcarbonyl group optionally having a substituent.

When X₁ in formula (1) is C(J₅J₆), J₅ and J₆ are each independently preferably a hydrogen atom, a halogen atom, a (C₁ to C₆) alkyl group optionally having a substituent, or a group selected from the group consisting of an O atom in a carbonyl group, a S atom in a thiocarbonyl group, and a methylene group substituted with J₇ and J₈, formed by two bonds of C-J₅ and C-J₆ together. J₅ and J₆ are each further preferably a hydrogen atom or a group selected from the group consisting of an O atom in a carbonyl group, a S atom in a thiocarbonyl group, and a methylene group substituted with J₇ and J₈, formed by two bonds of C-J₅ and C-J₆ together.

In formula (1), J₁, J₂, J₃, and J₄ are each independently preferably a hydrogen atom, a halogen atom, a (C₁ to C₆) alkyl group optionally having a substituent, or a group selected from the group consisting of an O atom in a carbonyl group, a S atom in a thiocarbonyl group, and an imino group substituted with Y₂, formed by two bonds of C-J₁ and C-J₂, or C-J₃ and C-J₄ together. J₁, J₂, J₃, and J₄ are each further preferably a hydrogen atom, a (C₁ to C₆) alkyl group, or a group selected from the group consisting of an O atom in a carbonyl group and a S atom in a thiocarbonyl group, formed by two bonds of C-J₁ and C-J₂, or C-J₃ and C-J₄ together. J₁, J₂, J₃, and J₄ are each more preferably a hydrogen atom, or a group selected from the group consisting of an O atom in a carbonyl group and a S atom in a thiocarbonyl group, formed by two bonds of C-J₁ and C-J₂, or C-J₃ and C-J₄ together.

In formula (1) above, n may be 0. In this case, J₁ and J₂ are each preferably a hydrogen atom, a (C₁ to C₆) alkyl groups optionally having a substituent, or an O atom in a carbonyl group, a S atom in a thiocarbonyl group, and an imino group substituted with Y₂, formed by two bonds of C-J₁ and C-J₂ together. J₁ and J₂ are each more preferably a hydrogen atom, or an O atom in a carbonyl group or a S atom in a thiocarbonyl group, formed by two bonds of C-J₁ and C-J₂ together.

J₇ and J₈ are each independently a group selected from the group consisting of a hydrogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, an amino group optionally substituted with one or two (C₁ to C₆) alkyl groups and/or halo (C₁ to C₆) alkyl groups, and a hydroxy group. J₇ and J₈ are each preferably a group selected from the group consisting of a hydrogen atom, a (C₁ to C₆) alkyl group optionally having a substituent, a (C₁ to C₆) alkoxy group optionally having a substituent, an amino group optionally substituted with one or two (C₁ to C₆) alkyl groups and/or halo (C₁ to C₆) alkyl groups, and a hydroxy group. J₇ and J₈ are each more preferably a group selected from the group consisting of a hydrogen atom, a (C₁ to C₆) alkoxy group optionally having a substituent, an amino group optionally substituted with one or two (C₁ to C₆) alkyl groups, and a hydroxy group.

Y₂ and Y₃ are each preferably a group selected from the group consisting of a hydrogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₁ to C₆) alkylcarbonyl optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, and a hydroxy group. Y₂ and Y₃ are each more preferably a group selected from the group consisting of a hydrogen atom, a (C₁ to C₆) alkyl group optionally having a substituent, a halo (C₁ to C₆) alkyl group optionally having a substituent, a (C₁ to C₆) alkoxy group, a halo (C₁ to C₆) alkoxy group optionally having a substituent, a (C₁ to C₆) alkylcarbonyl group optionally having a substituent, a halo (C₁ to C₆) alkylcarbonyl group optionally having a substituent, and a hydroxy group. Y₂ and Y₃ are each further preferably a group selected from the group consisting of a hydrogen atom, a (C₁ to C₆) alkyl group optionally having a substituent, and a (C₁ to C₆) alkylcarbonyl group optionally having a substituent.

More preferable embodiments of the cyclic structure linking moiety D and moiety B in formula (1) include a 1,2,4-oxadiazole structure, a 1,2,4-thiadiazole structure, a 1,2,4-oxadiazine structure and a 1,2,4-thiadiazine structure. That is, in the cyclic structure, preferably, X₁ is an oxygen atom or a sulfur atom, n is 0 or 1, and J₁, J₂, J₃, and J₄ are defined as above.

D in formula (1) is a phenyl group unsubstituted or arbitrarily substituted with Z₁ or a heterocyclic group unsubstituted or arbitrarily substituted with Z₁. D is preferably a phenyl group unsubstituted or arbitrarily substituted with Z₁, a pyridyl group unsubstituted or arbitrarily substituted with Z₁, a pyridazinyl group unsubstituted or arbitrarily substituted with Z₁, a pyrimidinyl group unsubstituted or arbitrarily substituted with Z₁, or a pyrazinyl group unsubstituted or arbitrarily substituted with Z₁. D is more preferably a pyridyl group unsubstituted or arbitrarily substituted with Z₁. D is more preferably a phenyl group substituted with one or more Z₁ or a nitrogen-containing heterocyclic group substituted with one or more Z₁. The nitrogen-containing heterocycle in this case is a heterocyclic group selected from the group consisting of a pyridyl group, a pyridazinyl group, a pyrimidinyl group, and a pyrazinyl group.

Moiety D is preferably a ring structure group selected from the group consisting of D-1, D-2, D-3, D-4, D-5, D-6, D-7, D-8, D-9, and D-10 and preferably a phenyl group or a heterocyclic group optionally having a substituent defined by Z_{1A}, Z_{1B}, Z_{1C}, Z_{1D}, and Z_{1E}. Moiety D is more preferably a ring structure group selected from the group consisting of D-1, D-2, D-3, D-4, D-5, D-6, D-7, and D-8. Moiety D is particularly preferably a ring structure group selected from the group consisting of D-1, D-2, D-3, D-4, D-5, and D-6. Moiety D is further preferably a ring structure group represented by D-2.

Z₁ is a group selected from the group consisting of a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group, a (C₁ to C₆) alkylcarbonyloxy group optionally having a substituent, a halo (C₁ to C₆) alkylcarbonyloxy optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group, or a group forming a 5- or 6-membered alicyclic group unsubstituted or optionally substituted with one or more halogen atoms or (C₁ to C₆) alkyl groups, formed by connecting adjacent Z₁ to each other, or a group forming a 1,3-dioxole group or a 1,4-dioxin group formed by adjacent Z₁ together with a carbon atom to which these Z₁ are attached.

The substituent on the phenyl group or the heterocyclic group in moiety D is preferably a group selected from the group consisting of a hydrogen atom, or a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, an NY₄Y₅ group, a C(O)NY₄Y₅ group, a cyano group, a nitro group, and a hydroxy group. The substituent is more preferably a group selected from the group consisting of a hydrogen atom, or a halogen atom, a (C₁ to C₆) alkyl group optionally having a substituent, a halo (C₁ to C₆) alkyl group optionally having a substituent, a (C₃ to C₆) cycloalkyl group optionally having a substituent, a (C₁ to C₆) alkoxy group optionally having a substituent, a halo (C₁ to C₆) alkoxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, and a cyano group.

Moiety D is more preferably a phenyl group, a pyridyl group, a pyridazinyl group, a pyrimidinyl group, or a pyrazinyl group substituted with Z_{1A}, Z_{1B}, Z_{1C}, Z_{1D}, and Z_{1E}, represented by D-1 to D-10 above.

Z_{1A}, Z_{1B}, Z_{1C}, Z_{1D}, and Z_{1E} are each a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group, a (C₁ to C₆) alkylcarbonyloxy group optionally having a substituent, a halo (C₁ to C₆) alkylcarbonyloxy optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group.

Z_{1A} to Z_{1E} are each preferably a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, an NY₄Y₅ group, a C(O)NY₄Y₅ group, a cyano group, a nitro group, and a hydroxy group. Z_{1A} to Z_{1E} are each more preferably a group selected from the group consisting of a hydrogen atom, or a halogen atom, a (C₁ to C₆) alkyl group optionally having a substituent, a halo (C₁ to C₆) alkyl group optionally having a substituent, a (C₃ to C₆) cycloalkyl group optionally having a substituent, a (C₁ to C₆) alkoxy group optionally having a substituent, a halo (C₁ to C₆) alkoxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, and a cyano group.

Z_{1A} to Z_{1E} are each, for example, a hydrogen atom, a halogen atom, a methyl group, an ethyl group, an isopropyl group, a trifluoromethyl group, a pentafluoroethyl group, a heptafluoronormalpropyl group, a heptafluoroisopropyl group, a trifluoromethoxy group, a 2,2,2-trifluoroethoxy group, a pentafluoroethoxy group, a trifluoromethylthio group, a 2,2,2-trifluoroethylthio group, a pentafluoroethylthio group, a trifluoromethylsulfinyl group, a 2,2,2-trifluoroethylsulfinyl group, a pentafluoroethylsulfinyl group, a trifluoromethylsulfonyl group, a 2,2,2-trifluoroethylsulfonyl group, or a pentafluoroethylsulfonyl group. Z_{1A} to Z_{1E} are each further preferably a group selected from the group consisting of a hydrogen atom, a halogen atom, a methyl group, a trifluoromethyl group, a pentafluoroethyl group, a heptafluoroisopropyl group, a trifluoromethoxy group, a trifluoromethylthio group, a trifluoromethylsulfinyl group, or a trifluoromethylsulfonyl group.

Moiety B in formula (1) is B-1, B-2, B-3, B-4, B-5, B-6, or B-8 above. That is, moiety B is a nitrogen-containing heterocyclic group having a substituent represented by - S(O)ₘR₁₂ optionally having substituents defined by R₁, R₂, R₃, R₄, R₅, R₆, R₇, R₈, R₁₀, R₁₁, and R₁₃.

The substituents defined by R₁, R₂, R₃, R₄, R₅, R₆, R₇, R₈, and R₁₃ are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkynyl group optionally having a substituent, a halo (C₂ to C₆) alkynyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy optionally having a substituent, a (C₁ to C₆) alkoxycarbonyl group optionally having a substituent, a halo (C₁ to C₆) alkoxycarbonyl group, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, an NY₄Y₅ group, a C(O)NY₄Y₅ group, a CH=NY₆ group, a cyano group, a nitro group, a hydroxy group, a mercapto group, a formyl group, a carboxy group, and an SF₅ group.

R₁ to R₈ and R₁₃ above are each independently preferably a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy optionally having a substituent, a (C₂ to C₆) alkynyl group optionally having a substituent, a halo (C₂ to C₆) alkynyl group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a phenyl group optionally having a substituent, a heterocyclic group optionally having a substituent, an NY₄Y₅ group, a C(O)NY₄Y₅ group, a CH=NY₆ group, a cyano group, a nitro group, and a hydroxy group.

More preferably, R₁ to R₈ and R₁₃ are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a phenyl group optionally having a substituent, a heterocyclic group optionally having a substituent, a -NY₄Y₅ group, a - C(O)NY₄Y₅ group, a -CH=NY₃ group, a cyano group, a nitro group, and a hydroxy group, and at least one of R₂ and R₃ is a group selected from the group consisting of a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkynyl group optionally having a substituent, a halo (C₂ to C₆) alkynyl group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a phenyl group optionally having a substituent, a heterocyclic group optionally having a substituent, -NY₄Y₅, a -C(O)NY₄Y₅ group, and a -CH=NY₆ group.

The substituents defined by R₉ and R₁₀ in moiety B above are each independently a group selected from the group consisting of a hydrogen atom, a halogen atom, a (C₁ to C₆) alkyl group, a halo (C₁ to C₆) alkyl group, a (C₃ to C₆) cycloalkyl group, a halo (C₃ to C₆) cycloalkyl group, a (C₁ to C₆) alkoxy group, a halo (C₁ to C₆) alkoxy group, a (C₁ to C₆) alkylcarbonyloxy group, a halo (C₁ to C₆) alkylcarbonyloxy group, a (C₁ to C₆) alkoxycarbonyloxy group, a halo (C₁ to C₆) alkoxycarbonyloxy group, optionally having a substituent, an NY₄Y₅ group, a C(O)NY₄Y₅ group, a cyano group, a nitro group, and a hydroxy group. The substituents are each independently preferably a group selected from the group consisting of a hydrogen atom, a halogen atom, and a (C₁ to C₆) alkyl group, and further preferably a hydrogen atom.

The substituent defined by R₁₁ is a group selected from the group consisting of a hydrogen atom, a (C₁ to C₆) alkyl group, a halo (C₁ to C₆) alkyl group, a (C₃ to C₆) cycloalkyl group, a halo (C₃ to C₆) cycloalkyl group, a (C₁ to C₆) alkoxy group, a halo (C₁ to C₆) alkoxy group, a (C₂ to C₆) alkylcarbonyl group, a halo (C₂ to C₆) alkylcarbonyl group, a (C₄ to C₇) cycloalkylcarbonyl group, and a halo (C₄ to C₇) cycloalkylcarbonyl group. The substituent is preferably a group selected from the group consisting of a hydrogen atom, a (C₁ to C₆) alkyl group, and a (C₂ to C₆) alkylcarbonyl group.

R₁₂ in the -S(O)ₘR₁₂ group disposed in moiety B is a group selected from the group consisting of a (C₁ to C₆) alkyl group, a halo (C₁ to C₆) alkyl group, or a (C₃ to C₆) cycloalkyl group, a halo (C₃ to C₆) cycloalkyl group, a (C₁ to C₆) alkoxy group, a halo (C₁ to C₆) alkoxy group, and an NYₐY_{b} group. R₁₂ is more preferably a (C₁ to C₆) alkyl group, a halo (C₁ to C₆) alkyl group, or NYₐY_{b}. R₁₂ is further preferably a (C₁ to C₆) alkyl group or a halo (C₁ to C₆) alkyl group.
m is 0, 1, or 2. m is preferably 2.

When R₁₂ in the -S(O)ₘR₁₂ group disposed in moiety B is an NYₐY_{b} group, m is 1 or 2. m is preferably 2.

Moiety B is preferably a cyclic structure moiety represented by B-1, B-2, B-3, or B-4. More preferably, moiety B is preferably a pyridyl group optionally having a substituent wherein in B-1, one of G₂ and G₄ is a nitrogen atom, and the other is a carbon atom group, an isoquinolyl group optionally having a substituent wherein in B-2, G₅, G₆, G₇, and G₈ are each a carbon atom group (C(R_{5 to 8})), a quinolyl group optionally having a substituent wherein in B-3, G₅, G₆, G₇, and G₈ are each a carbon atom group (C(R_{5 to 8})), or an imidazo[1,2-a]pyridyl group structure optionally having a substituent wherein in B-4, G₅, G₆, G₇, and G₈ are each a carbon atom group (C(R_{5 to 8})). R₁ to R₈, R₉ and R₁₀, and R₁₁ in this case are defined as above.

Furthermore, moiety B is preferably a cyclic structure moiety represented by B-1, B-3, or B-4, and is preferably a pyridyl group structure optionally having a substituent wherein in B-1, one of G₂ and G₄ is a nitrogen atom, and the other is a carbon atom group, a quinolyl group structure optionally having a substituent wherein in B-3, G₅, G₆, G₇, and G₈ are each a carbon atom group (C(R_{5 to 8})), or an imidazo[1,2-a]pyridyl group structure optionally having a substituent wherein in B-4, G₅, G₆, G₇, and G₈ are each a carbon atom group (C(R_{5 to 8})). R₁ to R₈, R₉ and R₁₀, and R₁₁ in this case are defined as above.

Moiety B is particularly preferably B-7, which is B-1 wherein G₂ is a C(R₂) group and G₄ is a nitrogen atom. In B-7, preferably, R₁ is a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a -NY₄Y₅ group, a cyano group, a nitro group, a hydroxy group, and an amino group, and R₂ and R₃ is each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkynyl group optionally having a substituent, a halo (C₂ to C₆) alkynyl group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a phenyl group optionally having a substituent, a heterocyclic group optionally having a substituent, a -NY₄Y₅ group, a -CH=NY₆ group, a cyano group, a nitro group, a hydroxy group, and an amino group.

More preferably, R₁ is a hydrogen atom, and R₂ and R₃ are as described above. Moiety B is further preferably B-7 wherein R₁ is a hydrogen atom, and at least one of R₂ and R₃ is a group selected from the group consisting of a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₂ to C₆) alkynyl group optionally having a substituent, a halo (C₂ to C₆) alkynyl group optionally having a substituent, a phenyl group optionally having a substituent, and a heterocyclic group optionally having a substituent. Moiety B is especially preferably B-7 wherein R₁ is a hydrogen atom, and at least one of R₂ and R₃ is a group selected from the group consisting of a phenyl group optionally having a substituent and a heterocyclic group optionally having a substituent.

Tₐ and T₁, and T₂ and T₃ are each independently preferably a (C₃ to C₆) cycloalkyl group, a halo (C₃ to C₆) cycloalkyl group, a (C₁ to C₆) alkoxy group, a halo (C₁ to C₆) alkoxy group, a (C₁ to C₆) alkylthio group, a halo (C₁ to C₆) alkylthio group, a (C₁ to C₆) alkylsulfinyl group, a halo (C₁ to C₆) alkylsulfinyl group, a (C₁ to C₆) alkylsulfonyl group, a halo (C₁ to C₆) alkylsulfonyl group, an NY₄Y₅ group, a C(O)NY₄Y₅ group, a cyano group, a nitro group, or a hydroxy group. Tₐ and T₁, and T₂ and T₃ are each independently more preferably a group selected from the group consisting of a (C₃ to C₆) cycloalkyl group, a (C₁ to C₆) alkoxy group, a halo (C₁ to C₆) alkoxy group, a (C₁ to C₆) alkylthio group, a halo (C₁ to C₆) alkylthio group, a (C₁ to C₆) alkylsulfinyl group, a halo (C₁ to C₆) alkylsulfinyl group, a (C₁ to C₆) alkylsulfonyl group, a halo (C₁ to C₆) alkylsulfonyl group, an amino group, a cyano group, a nitro group, and a hydroxy group. Tₐ and T₁, and T₂ and T₃ are each independently further preferably a group selected from the group consisting of a (C₃ to C₆) cycloalkyl group, a (C₁ to C₆) alkoxy group, a halo (C₁ to C₆) alkoxy group, a (C₁ to C₆) alkylthio group, a halo (C₁ to C₆) alkylthio group, a (C₁ to C₆) alkylsulfinyl group, a halo (C₁ to C₆) alkylsulfinyl group, a (C₁ to C₆) alkylsulfonyl group, a halo (C₁ to C₆) alkylsulfonyl group, an amino group, a cyano group, a nitro group, and a hydroxy group. Tₐ and T₁, and T₂ and T₃ are each independently more preferably a group selected from the group consisting of a (C₁ to C₆) alkoxy group, a halo (C₁ to C₆) alkoxy group, a (C₁ to C₆) alkylthio group, a halo (C₁ to C₆) alkylthio group, a cyano group, and a hydroxy group.

Z₂ is a group selected from the group consisting of a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group, a (C₁ to C₆) alkylcarbonyloxy group optionally having a substituent, a halo (C₁ to C₆) alkylcarbonyloxy optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group, or a group forming a 5- or 6-membered alicyclic group unsubstituted or optionally substituted with one or more halogen atoms or (C₁ to C₆) alkyl groups, formed by connecting adjacent Z₂ to each other, or a group forming a 1,3-dioxole group or a 1,4-dioxin group formed by adjacent Z₂ together with a carbon atom to which these Z₂ are attached. Z₂ is more preferably a group selected from the group consisting of a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, an NY₄Y₅ group, a C(O)NY₄Y₅ group, a cyano group, a nitro group, and a hydroxy group.

Z₃ is preferably a group selected from the group consisting of a halogen atom, a (C₁ to C₆) alkyl group, a halo (C₁ to C₆) alkyl group, a (C₃ to C₆) cycloalkyl group, a halo (C₃ to C₆) cycloalkyl group, a (C₁ to C₆) alkoxy group, a halo (C₁ to C₆) alkoxy group, a (C₁ to C₆) alkylthio group, a halo (C₁ to C₆) alkylthio group, a (C₁ to C₆) alkylsulfinyl group, a halo (C₁ to C₆) alkylsulfinyl group, a (C₁ to C₆) alkylsulfonyl group, a halo (C₁ to C₆) alkylsulfonyl group, an NY₄Y₅ group, a C(O)NY₄Y₅ group, a cyano group, a nitro group, and a hydroxy group.

Y₄ and Y₅ are each independently preferably a group selected from the group consisting of a hydrogen atom, a (C₁ to C₆) alkyl group, a halo (C₁ to C₆) alkyl group, a (C₃ to C₆) cycloalkyl group, a halo (C₃ to C₆) cycloalkyl group, a (C₁ to C₆) alkylcarbonyl group, a halo (C₁ to C₆) alkylcarbonyl group, a (C₃ to C₆) cycloalkylcarbonyl group, a halo (C₃ to C₆) alkylcarbonyl group, a (C₁ to C₆) alkylsulfonyl group, a halo (C₁ to C₆) alkylsulfonyl group, and a phenyl group unsubstituted or arbitrarily substituted with Z₃. Y₄ and Y₅ are each independently more preferably a group selected from the group consisting of a hydrogen atom, a (C₁ to C₆) alkyl group, a halo (C₁ to C₆) alkyl group, a (C₃ to C₆) cycloalkyl group, a halo (C₃ to C₆) cycloalkyl group, a (C₁ to C₆) alkylcarbonyl group, and a halo (C₁ to C₆) alkylcarbonyl group. Y₄ and Y₅ are each independently further preferably a group selected from the group consisting of a hydrogen atom, a (C₁ to C₆) alkyl group, a halo (C₁ to C₆) alkyl group, a (C₁ to C₆) alkylcarbonyl group, and a halo (C₁ to C₆) alkylcarbonyl group.

Y₆ is preferably a group selected from the group consisting of a hydrogen atom, a (C₁ to C₆) alkyl group optionally having a substituent, a halo (C₁ to C₆) alkyl group optionally having a substituent, a (C₁ to C₆) alkoxy group unsubstituted or arbitrarily substituted with a cyano group, a halo (C₁ to C₆) alkoxy group, a (C₂ to C₆) alkenyloxy group, and a halo (C₂ to C₆) alkenyloxy group. Y₆ is more preferably a group selected from the group consisting of a hydrogen atom, a halo (C₁ to C₆) alkyl group, a halo (C₁ to C₆) alkoxy group, and a halo (C₂ to C₆) alkenyloxy group.

Yₐ and Y_{b} each independently represent a group selected from the group consisting of a hydrogen atom, a (C₁ to C₆) alkyl group, a halo (C₁ to C₆) alkyl group, a (C₃ to C₆) cycloalkyl group, a halo (C₃ to C₆) cycloalkyl group, a (C₁ to C₆) alkoxy group, a halo (C₁ to C₆) alkoxy group, a (C₁ to C₆) alkenyl group, and a halo (C₁ to C₆) alkenyl group, or a group forming an aliphatic 3- to 10-membered cyclic amino group or cyclic amide group unsubstituted or optionally substituted with one or more halogen atoms or (C₁ to C₆) alkyl groups, formed by connecting Yₐ and Y_{b} to each other.

Yₐ and Y_{b} each independently preferably represent a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3-to 10-membered cyclic amino group or cyclic amide group unsubstituted or optionally substituted with one or more halogen atoms or (C₁ to C₆) alkyl groups, formed by connecting Yₐ and Y_{b} to each other, and are each independently more preferably a group selected from the group consisting of a hydrogen atom, a (C₁ to C₆) alkyl group optionally having a substituent, a halo (C₁ to C₆) alkyl group optionally having a substituent, and a (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other. Yₐ and Y_{b} are each independently particularly preferably a group selected from the group consisting of a (C₁ to C₆) alkyl group optionally having a substituent, a halo (C₁ to C₆) alkyl group optionally having a substituent, and a (C₃ to C₆) cycloalkyl group.

W represents an oxygen atom, a sulfur atom, or NY₇. W is preferably an oxygen atom or a sulfur atom.

Y₇ is a group selected from the group consisting of a hydrogen atom, a (C₁ to C₆) alkyl group, a halo (C₁ to C₆) alkyl group, a (C₁ to C₆) alkylcarbonyl group, and a halo (C₁ to C₆) alkylcarbonyl group. Y₇ is preferably a group selected from the group consisting of a hydrogen atom, a (C₁ to C₆) alkyl group, and a halo (C₁ to C₆) alkyl group.

Particularly preferable R₁, R₂, R₃, and R₁₂ groups when moiety B is B-1 in formula (1) will be described in detail. X₁, n, J₁, J₂, J₃, J₄, J₅, J₆, J₇, J₈, Y₁, Y₂, Y₃, Y₄, Y₅, Y₆, and moiety D in this case are defined as above.

R₁ is preferably a hydrogen atom, a halogen atom, a (C₁ to C₆) alkyl group optionally having a substituent, or a halo (C₁ to C₆) alkyl group optionally having a substituent. R₁ is more preferably a hydrogen atom, a methyl group, or a halogen atom. R₁ is further preferably a hydrogen atom.

R₂ is preferably a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a halo (C₁ to C₆) alkenyl group optionally having a substituent, a halo (C₁ to C₆) alkenyloxy group optionally having a substituent, a (C₂ to C₆) alkynyl group optionally having a substituent, a halo (C₁ to C₆) alkynyl group optionally having a substituent, a C(O)NY₄Y₅ group, a CH=NY₆ group, a phenyl group unsubstituted or arbitrarily substituted with Z₂, or a heterocyclic group unsubstituted or arbitrarily substituted with Z₂. R₂ is more preferably a (C₃ to C₆) cycloalkyl group optionally having a substituent, a phenyl group arbitrarily substituted with Z₂, or a heterocyclic group arbitrarily substituted with Z₂. R₂ is further preferably a phenyl group arbitrarily substituted with at least one or more Z₂, a thienyl group arbitrarily substituted with at least one or more Z₂, or a pyridyl group arbitrarily substituted with at least one or more Z₂. R₂ is more preferably a phenyl group arbitrarily substituted with at least one or more Z₂, or a pyridyl group arbitrarily substituted with at least one or more Z₂.

A preferable embodiment of the phenyl group arbitrarily substituted with Z₂ is a phenyl group arbitrarily substituted with at least one or more halogen atoms, a phenyl group arbitrarily substituted with at least one or more (C₁ to C₆) alkyl groups, a phenyl group arbitrarily substituted with at least one or more halo (C₁ to C₆) alkyl groups, a phenyl group arbitrarily substituted with at least one or more (C₁ to C₆) alkoxy groups, a phenyl group arbitrarily substituted with at least one or more halo (C₁ to C₆) alkoxy groups, a phenyl group arbitrarily substituted with at least one or more (C₃ to C₆) cycloalkyl groups, a phenyl group arbitrarily substituted with at least one or more (C₁ to C₆) alkylthio group, a phenyl group arbitrarily substituted with at least one or more halo (C₁ to C₆) alkylthio groups, a phenyl group arbitrarily substituted with at least one or more (C₁ to C₆) alkylsulfinyl groups, a phenyl group arbitrarily substituted with at least one or more halo (C₁ to C₆) alkylsulfinyl groups, a phenyl group arbitrarily substituted with at least one or more (C₁ to C₆) alkylsulfonyl groups, a phenyl group arbitrarily substituted with at least one or more halo (C₁ to C₆) alkylsulfonyl groups, a phenyl group arbitrarily substituted with at least one or more 1-cyano-(C₃ to C₆) cycloalkyl groups, a phenyl group arbitrarily substituted with at least one or more cyano groups, a 1,3-benzodioxole group arbitrarily substituted with at least one or more halogen atoms, or a 2,3-dihydro-1,4-benzodioxin group arbitrarily substituted with at least one or more halogen atoms.

A preferable embodiment of the pyridyl group arbitrarily substituted with Z₂ is a pyridyl group arbitrarily substituted with at least one or more halogen atoms, a pyridyl group arbitrarily substituted with at least one or more (C₁ to C₆) alkyl groups, a pyridyl group arbitrarily substituted with at least one or more halo (C₁ to C₆) alkyl groups, a pyridyl group arbitrarily substituted with at least one or more (C₁ to C₆) alkoxy groups, a pyridyl group arbitrarily substituted with at least one or more halo (C₁ to C₆) alkoxy groups, a pyridyl group arbitrarily substituted with at least one or more (C₃ to C₆) cycloalkyl groups, a pyridyl group arbitrarily substituted with at least one or more (C₁ to C₆) alkylthio group, a pyridyl group arbitrarily substituted with at least one or more halo (C₁ to C₆) alkylthio groups, a pyridyl group arbitrarily substituted with at least one or more (C₁ to C₆) alkylsulfinyl groups, a pyridyl group arbitrarily substituted with at least one or more halo (C₁ to C₆) alkylsulfinyl groups, a pyridyl group arbitrarily substituted with at least one or more (C₁ to C₆) alkylsulfonyl groups, a pyridyl group arbitrarily substituted with at least one or more halo (C₁ to C₆) alkylsulfonyl groups, a pyridyl group arbitrarily substituted with at least one or more 1-cyano-(C₃ to C₆) cycloalkyl groups, or a pyridyl group arbitrarily substituted with at least one or more cyano groups.

R₃ is preferably a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a (C₃ to C₆) cycloalkyl group optionally having a substituent, a halo (C₃ to C₆) cycloalkyl group optionally having a substituent, a dimethylamino group, a phenyl group unsubstituted or arbitrarily substituted with Z₂, or a heterocyclic group unsubstituted or arbitrarily substituted with Z₂. R₃ is more preferably a hydrogen atom, a halogen atom, a (C₁ to C₆) alkyl group, a (C₁ to C₆) alkoxy group optionally having a substituent, a (C₃ to C₆) cycloalkyl group optionally having a substituent, or a dimethylamino group. R₃ is further preferably a hydrogen atom, a halogen atom, a methyl group, an ethyl group, an isopropyl group, a cyclopropyl group, a methoxy group, or a dimethylamino group. R₃ is further preferably a hydrogen atom or a methyl group. R₃ is also preferably a phenyl group arbitrarily substituted with at least one or more Z₂.

R₁₂ is preferably a (C₁ to C₆) alkyl group or an NYₐY_{b} group. R₁₂ is more further preferably a (C₁ to C₆) alkyl group, and R₁₂ is further preferably an ethyl group. m is preferably 2.

When R₁₂ is an NYₐY_{b} group, m is 1 or 2. m is preferably 2.

In addition, more preferable embodiments of R₅, R₆, R₇, R₈, and R₁₂ as well as R₉, R₁₀, and R₁₁ when a compound wherein the cyclic structure moiety B is B-2, B-3, B-4, B-5, or B-6 or a salt thereof or an N-oxide thereof is also a preferable embodiment will be described in detail. X₁, n, J₁, J₂, J₃, J₄, J₅, J₆, J₇, J₈, Y₁, Y₂, Y₃, Y₄, Y₅, Y₆, and moiety D in this case are defined as above.

R₅, R₆, R₇, and R₈ are each independently preferably a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₂ to C₆) alkenyl group optionally having a substituent, a halo (C₂ to C₆) alkenyl group optionally having a substituent, a (C₁ to C₆) alkoxy group optionally having a substituent, a halo (C₁ to C₆) alkoxy group optionally having a substituent, a (C₂ to C₆) alkenyloxy group optionally having a substituent, a halo (C₂ to C₆) alkenyloxy optionally having a substituent, a (C₂ to C₆) alkynyl group optionally having a substituent, a halo (C₂ to C₆) alkynyl group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a phenyl group unsubstituted or arbitrarily substituted with Z₂, a heterocyclic group unsubstituted or arbitrarily substituted with Z₂, a cyano group, an amino group, and a hydroxy group.

R₅, R₆, R₇, and R₈ are each more preferably a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₂ to C₆) alkenyl group optionally having a substituent, a halo (C₂ to C₆) alkenyl group optionally having a substituent, a (C₁ to C₆) alkoxy group optionally having a substituent, a halo (C₁ to C₆) alkoxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a cyano group, or a phenyl group unsubstituted or arbitrarily substituted with Z₂.

R₅, R₆, R₇, and R₈ are each further preferably a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a (C₁ to C₆) alkoxy group optionally having a substituent, a halo (C₁ to C₆) alkoxy group optionally having a substituent, or a phenyl group unsubstituted or arbitrarily substituted with Z₂.

The substituents defined by R₉ and R₁₀ are each a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, and a halo (C₁ to C₆) alkoxy group optionally having a substituent. The substituents are each more preferably a hydrogen atom, a halogen atom, or a (C₁ to C₆) alkyl group optionally having a substituent, and further preferably a hydrogen atom.

The substituent defined by R₁₁ is a group selected from the group consisting of a hydrogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, and a halo (C₁ to C₆) alkoxy group optionally having a substituent, a (C₁ to C₆) alkylcarbonyl group optionally having a substituent, and a halo (C₁ to C₆) alkylcarbonyl group optionally having a substituent. The substituent is preferably a hydrogen atom, a (C₁ to C₆) alkyl group optionally having a substituent, or a (C₂ to C₆) alkylcarbonyl group optionally having a substituent.

R₁₂ is preferably a (C₁ to C₆) alkyl group or an NYₐY_{b} group. R₁₂ is more preferably a (C₁ to C₆) alkyl group, and R₁₂ is further preferably an ethyl group. m is preferably 2.

When R₁₂ is an NYₐY_{b} group, m is 1 or 2. m is preferably 2.

In addition, more preferable embodiments of R₁₃ and R₁₂ and W and Y₇ when a compound wherein the cyclic structure moiety B is B-8 or a salt thereof or an N-oxide thereof is also a preferable embodiment will be described in detail. X₁, n, J₁, J₂, J₃, J₄, J₅, J₆, J₇, J₈, Y₁, Y₂, Y₃, Y₄, Y₅, Y₆, and moiety D in this case are defined as above.

The substituent defined by R₁₃ is preferably a hydrogen atom, a halogen atom, a (C₁ to C₃) alkyl group optionally having a substituent, a halo (C₁ to C₃) alkyl group optionally having a substituent, a (C₃ to C₆) cycloalkyl group optionally having a substituent, a halo (C₃ to C₆) cycloalkyl group optionally having a substituent, a (C₂ to C₆) alkenyl group optionally having a substituent, a halo (C₂ to C₆) alkenyl group optionally having a substituent, a (C₁ to C₆) alkoxy group optionally having a substituent, a halo (C₁ to C₆) alkoxy group optionally having a substituent, a (C₂ to C₆) alkynyl group optionally having a substituent, a halo (C₂ to C₆) alkynyl group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a phenyl group unsubstituted or arbitrarily substituted with Z₂, a heterocyclic group unsubstituted or arbitrarily substituted with Z₂, or a cyano group. R₁₃ is more preferably a phenyl group arbitrarily substituted with Z₂ or a heterocyclic group arbitrarily substituted with Z₂. R₂ is further preferably a phenyl group arbitrarily substituted with at least one or more Z₂, or a pyridyl group arbitrarily substituted with at least one or more Z₂. R₂ is more preferably a phenyl group arbitrarily substituted with at least one or more Z₂.

R₁₂ is preferably a (C₁ to C₆) alkyl group or an NYₐY_{b} group. R₁₂ is more further preferably a (C₁ to C₆) alkyl group, and R₁₂ is further preferably an ethyl group. m is preferably 2.

When R₁₂ is an NYₐY_{b} group, m is 1 or 2. m is preferably 2.

W is preferably an oxygen atom or a sulfur atom.

Y₇ is preferably a group selected from the group consisting of a hydrogen atom, a (C₁ to C₆) alkyl group, and a halo (C₁ to C₆) alkyl group.

The compound represented by formula (1) according to the present invention or a salt thereof or an N-oxide thereof can be produced, for example, by the production method presented by the following step a to step n, but the present invention is not limited thereto.

### <Step a>

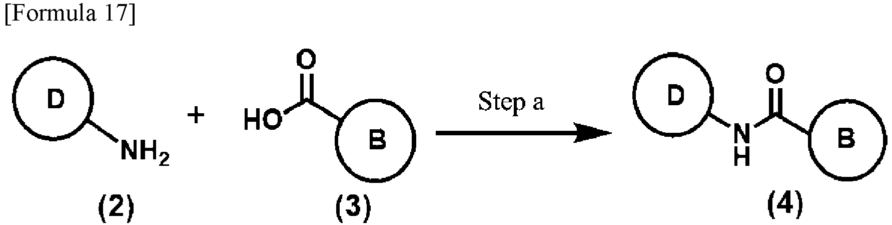

A compound represented by formula (4) can be produced by reacting a compound represented by formula (2) and a compound represented by formula (3) with a reactant in the presence of an inert solvent and optionally a base. In the formulas, moiety D and moiety B are defined as above. Moiety B represents a cyclic structure represented by any of formulas (3-1) to (3-6) or formula (3-8) below, wherein G₂, G₄, G₅, G₆, G₇, G₈, W, R₁, R₃, R₉, R₁₀, R₁₁, R₁₂, and R₁₃ are the same as above, and m is 0, 1, or 2.

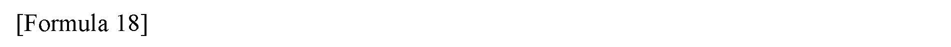

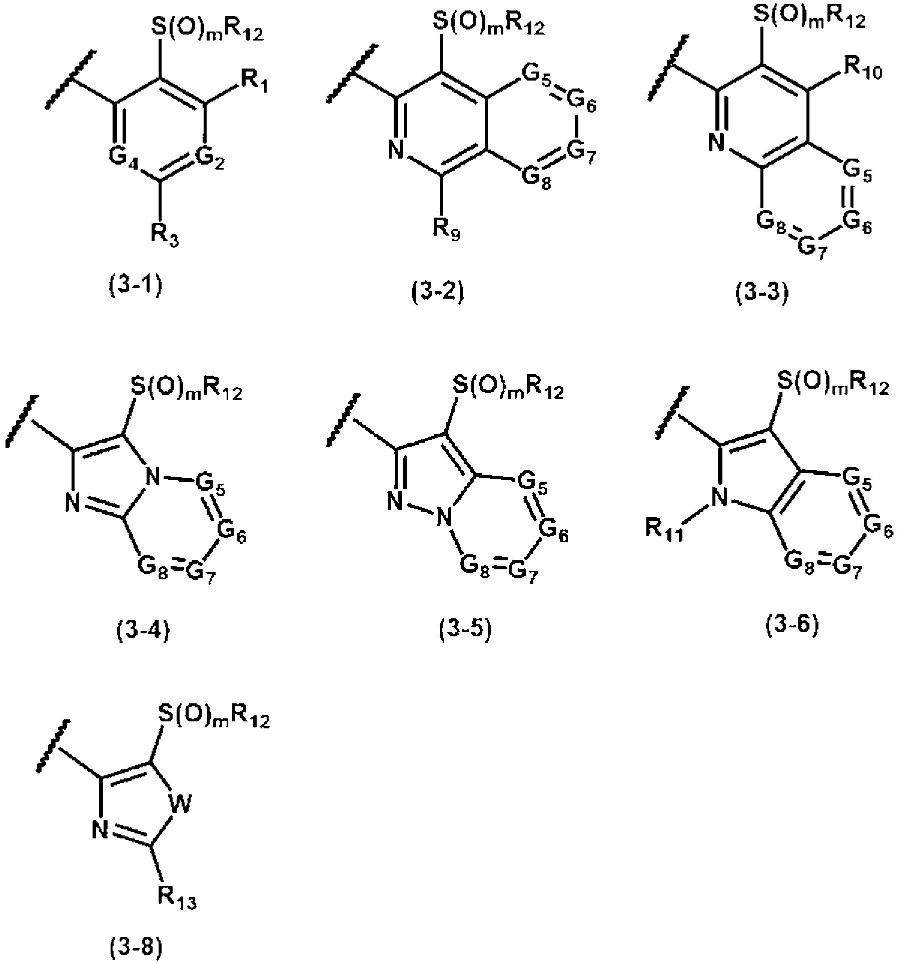

Examples of the reactant that can be used in step a include a condensing agent such as dicyclohexylcarbodiimide, 1-ethyl-3-(3-dimethylaminopropyl)-carbodiimide hydrochloride, HATU, PyBOP, or a Mukaiyama reagent, and the reactants that can be used in the present reaction are not limited thereto. Usually, the amount of the reactant used may be appropriately selected in the range of about 1 to 3 times the number of moles of the compound represented by formula (2) or (3).

Examples of the base that can be used in step a include triethylamine, N,N-diisopropylethylamine, and pyridine, and the amount thereof used may be appropriately selected usually in the range of about 1 to 5 times the number of moles of the compound represented by formula (2) or (3).

The inert solvent that can be used in step a may be any inert solvent that does not remarkably inhibit the present reaction, examples thereof include an aromatic hydrocarbon such as benzene, toluene, or xylene, a halogenated hydrocarbon such as dichloromethane or 1,2-dichloroethane, and a polar solvent such as N,N-dimethylformamide, N,N-dimethylacetamide, dimethylsulfoxide, or 1,3-dimethyl-2-imidazolinone, and these inert solvents can be used on its own or as a mixture of two or more thereof.

The reaction temperature in step a may be usually in the range of about -78°C to the boiling point of the solvent used, and the reaction time may be appropriately selected depending on the reaction scale, the reaction temperature, or the like, and for example, may be appropriately selected in the range of several minutes to 48 hours. The amino compound represented by formula (2) is usually used in an amount in the range of about 0.3 to 3 times the number of moles of the compound represented by formula (3). **In** addition, the present reaction can also be carried out, for example, in an atmosphere of an inert gas such as nitrogen gas or argon gas.

After completion of the reaction, the target product may be isolated from the reaction system including the target product by a conventional method, and if necessary, the target product can be produced by purification processes such as recrystallization and column chromatography. A composition including the target product may be used in the next step without isolation.

### <Step b>

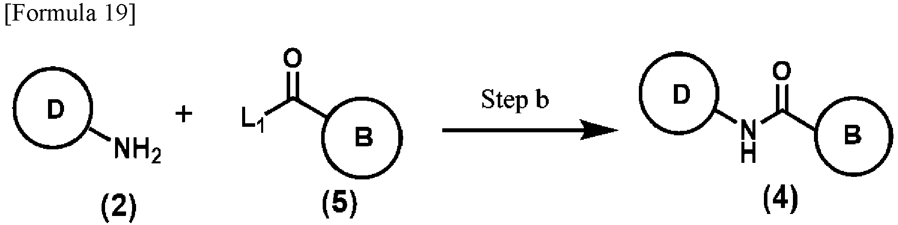

A compound represented by formula (4) can be produced by reacting a compound represented by formula (2) and a compound represented by formula (5) in the presence of an inert solvent and optionally a base. In the formulas, L₁ represents a halogen or an alkoxy group, and moiety D and moiety B are defined as above. Moiety B represents a cyclic structure represented by any of formulas (3-1) to (3-6) or formula (3-8) above, wherein G₂, G₄, G₅, G₆, G₇, G₈, W, R₁, R₃, R₉, R₁₀, R₁₁, R₁₂, and R₁₃ are the same as above, and m is 0, 1, or 2.

Examples of the base that can be used in step b include an alkali metal salt such as sodium hydride, sodium carbonate, or potassium carbonate, a tertiary amine such as triethylamine or N,N-diisopropylethylamine, and a nitrogen-containing aromatic compound such as pyridine or 4-dimethylaminopyridine, and the amount thereof used may be appropriately selected usually in the range of about 1 to 5 times the number of moles of the compound represented by formula (2) or formula (5).

The inert solvent that can be used in step b may be any inert solvent that does not remarkably inhibit the present reaction, examples thereof include an ether such as tetrahydrofuran, ethylene glycol dimethyl ether, or 1,4-dioxane, an aromatic hydrocarbon such as benzene, toluene, or xylene, a halogenated hydrocarbon such as dichloromethane or 1,2-dichloroethane, and a polar solvent such as N,N-dimethylformamide, N,N-dimethylacetamide, dimethylsulfoxide, or 1,3-dimethyl-2-imidazolinone, and these inert solvents can be used on its own or as a mixture of two or more thereof.

The reaction temperature in step b may be usually in the range of about -78°C to the boiling point of the solvent used, and the reaction time may be appropriately selected depending on the reaction scale, the reaction temperature, or the like, and for example, may be appropriately selected in the range of several minutes to 48 hours. The amino compound represented by formula (2) is used usually in an amount in the range of about 0.3 to 3 times the number of moles of the compound represented by formula (5). In addition, the present reaction can also be carried out, for example, in an atmosphere of an inert gas such as nitrogen gas or argon gas.

After completion of the reaction, the target product may be isolated from the reaction system including the target product by a conventional method, and if necessary, the target product can be produced by purification by recrystallization, column chromatography, or the like. A composition including the target product may be used in the next step without isolation.

### <Step c>

A compound represented by formula (7-1), (7-2), (7-3), (7-4), (7-5), (7-6), or (7-8) can be produced by reacting a compound represented by formula (6-1), (6-2), (6-3), (6-4), (6-5), (6-6), or (6-8), respectively, with a reactant in the presence of an inert solvent. In the formulas, moiety D, G₂, G₄, G₅, G₆, G₇, G₈, W, R₁, R₃, R₉, R₁₀, R₁₁, R₁₂, and R₁₃ are the same as above, and m is 1 or 2. The compounds represented by formulas (6-1) to (6-6) and (6-8) can be prepared by the production method of step a or step b above.

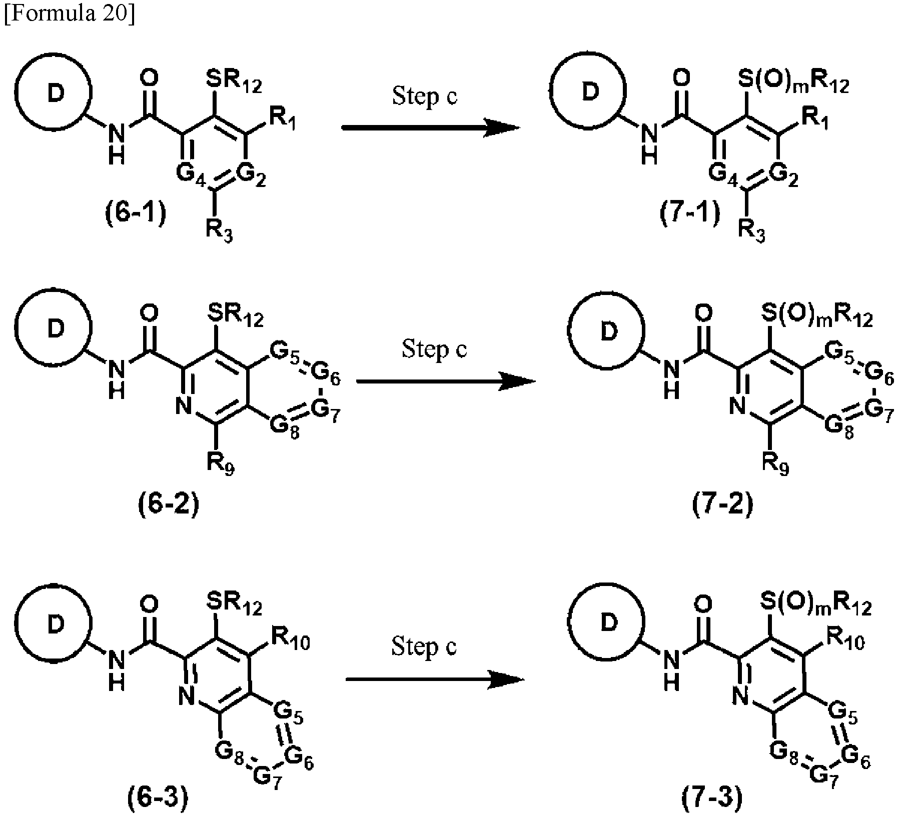

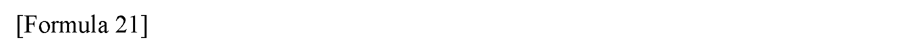

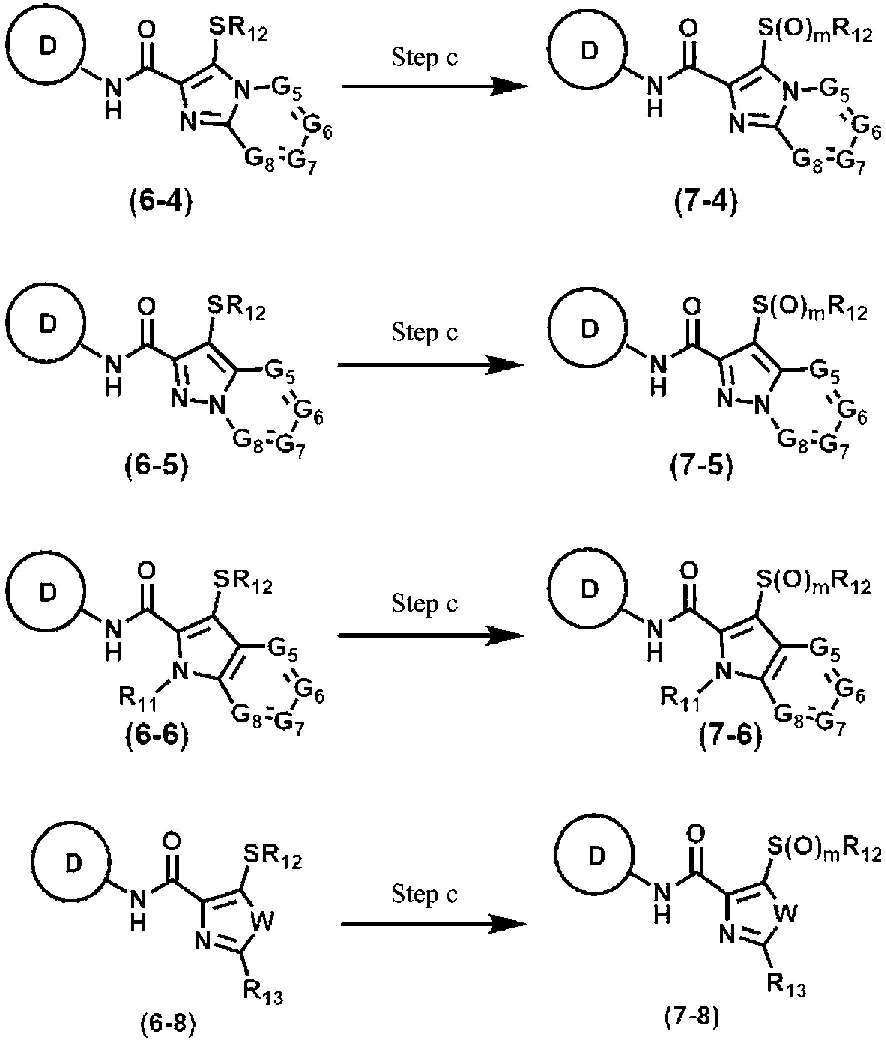

Examples of the reactant that can be used in step c include an oxidizing agent such as 3-chloroperbenzoic acid or aqueous hydrogen peroxide, and the reactants that can be used in the present reaction are not limited thereto. The amount of the reactant used may be appropriately selected usually in the range of about 1 to 3 times the number of moles of the compound represented by formula (6-1), (6-2), (6-3), (6-4), (6-5), (6-6), or (6-8).

The inert solvent that can be used in step c may be any inert solvent that does not remarkably inhibit the present reaction, examples thereof include an aromatic hydrocarbon such as benzene, toluene, or xylene, and a halogenated hydrocarbon such as dichloromethane or 1,2-dichloroethane, and these inert solvents can be used on its own or as a mixture of two or more thereof.

The reaction temperature in step c may be usually in the range of about -78°C to the boiling point of the solvent used, and the reaction time may be appropriately selected depending on the reaction scale, the reaction temperature, or the like, and for example, may be appropriately selected in the range of several minutes to 48 hours. In addition, the present reaction can also be carried out, for example, in an atmosphere of an inert gas such as nitrogen gas or argon gas.

After completion of the reaction, the target product may be isolated from the reaction system including the target product by a conventional method, and if necessary, the target product can be produced by purification processes such as recrystallization and column chromatography. A composition including the target product may be used in the next step without isolation.

### <Step d>

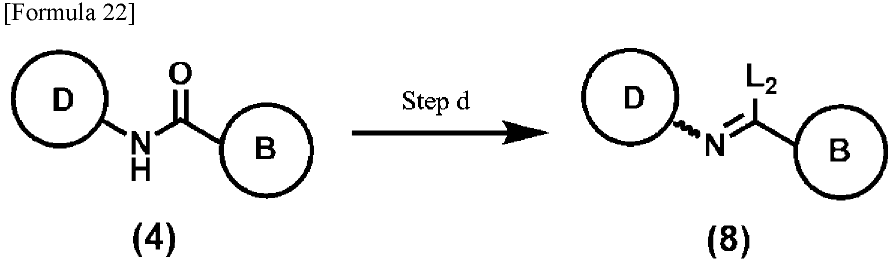

A compound represented by formula (8) can be produced by reacting a compound represented by formula (4) with a reactant in the presence of an inert solvent. In the formulas, L₂ represents a halogen or a trifluoromethanesulfonate group, and moiety D and moiety B are defined as above. Moiety B represents a cyclic structure represented by any of formulas (3-1) to (3-6) or formula (3-8) above, wherein G₂, G₄, G₅, G₆, G₇, G₈, W, R₁, R₃, R₉, R₁₀, R₁₁, R₁₂, and R₁₃ are the same as above, and m is 0, 1, or 2. The compound represented by formula (4) can be prepared by the production method of step a, step b, or step c.

Examples of the reactant that can be used in step d include phosphorus oxychloride, phosphorus pentachloride, carbon tetrachloride-triphenylphosphine, and trifluoromethanesulfonic anhydride, and the reactants that can be used in the present reaction are not limited thereto. The amount of the reactant used may be appropriately selected usually in the range of about 1 to 10 times the number of moles of the compound represented by formula (4), and a reactant such as phosphorus oxychloride can be added in excess to carry out the reaction without a solvent.

The inert solvent that can be used in step d may be any inert solvent that does not remarkably inhibit the present reaction, examples thereof include an aromatic hydrocarbon such as benzene, toluene, or xylene, a halogenated hydrocarbon such as dichloromethane or 1,2-dichloroethane, and a polar solvent such as acetonitrile, and these inert solvents can be used on its own or as a mixture of two or more thereof.

The reaction temperature in step d may be usually in the range of about -78°C to the boiling point of the solvent used, and the reaction time may be appropriately selected depending on the reaction scale, the reaction temperature, or the like, and for example, may be appropriately selected in the range of several minutes to 48 hours. In addition, the present reaction can also be carried out, for example, in an atmosphere of an inert gas such as nitrogen gas or argon gas.

After completion of the reaction, the target product may be isolated from the reaction system including the target product by a conventional method, and if necessary, the target product can be produced by purification processes such as recrystallization and column chromatography. A composition including the target product may be used in the next step without isolation.

### <Step e>

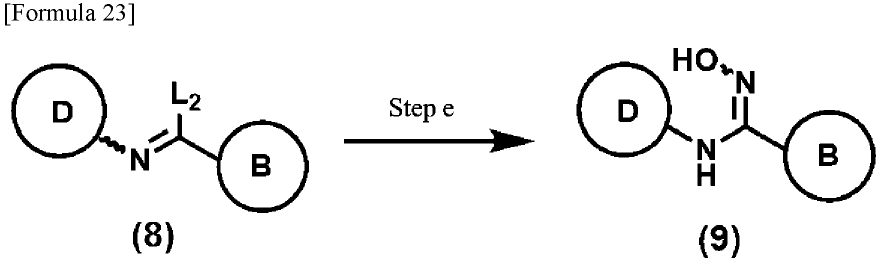

A compound represented by formula (9) can be produced by reacting a compound represented by formula (8) with a reactant in the presence of an inert solvent and optionally a base. Moiety D, moiety B, and L₂ are defined as above. Moiety B is a cyclic structure represented by any of formulas (3-1) to (3-6) or formula (3-8) above, wherein G₂, G₄, G₅, G₆, G₇, G₈, W, R₁, R₃, R₉, R₁₀, R₁₁, R₁₂, and R₁₃ are the same as above, and m is 0, 1, or 2.

Examples of the reactant that can be used in step e include a hydroxylamine aqueous solution and a hydroxylamine acid addition salt, and the amount thereof used may be appropriately selected usually in the range of about 1 to 20 times the number of moles of the compound represented by formula (8).

Examples of the base that can be used in step e include an alkali metal carbonate such as sodium carbonate or potassium carbonate, a tertiary amine such as triethylamine or N,N-diisopropylethylamine, and a nitrogen-containing aromatic compound such as pyridine or 4-dimethylaminopyridine, and the amount thereof used may be appropriately selected usually in the range of about 1 to 20 times the number of moles of the compound represented by formula (8).

The inert solvent that can be used in step e may be any inert solvent that does not remarkably inhibit the present reaction, examples thereof include an ether such as diethyl ether, tetrahydrofuran, or dioxane, an aromatic hydrocarbon such as benzene, toluene, or xylene, a polar solvent such as N,N-dimethylformamide, N,N-dimethylacetamide, dimethylsulfoxide, or 1,3-dimethyl-2-imidazolinone, and a halogenated hydrocarbon such as dichloromethane or 1,2-dichloroethane, and these inert solvents can be used on its own or as a mixture of two or more thereof.

The reaction temperature in step e may be usually in the range of about -78°C to the boiling point of the solvent used, and the reaction time varies depending on the reaction scale, the reaction temperature, or the like, and may be appropriately selected in the range of several minutes to 48 hours. In addition, the present reaction can also be carried out, for example, in an atmosphere of an inert gas such as nitrogen gas or argon gas.

After completion of the reaction, the target product may be isolated from the reaction system including the target product by a conventional method, and if necessary, the target product can be produced by purification by recrystallization, column chromatography, or the like. A composition including the target product may be used in the next step without isolation.

### <Step f>

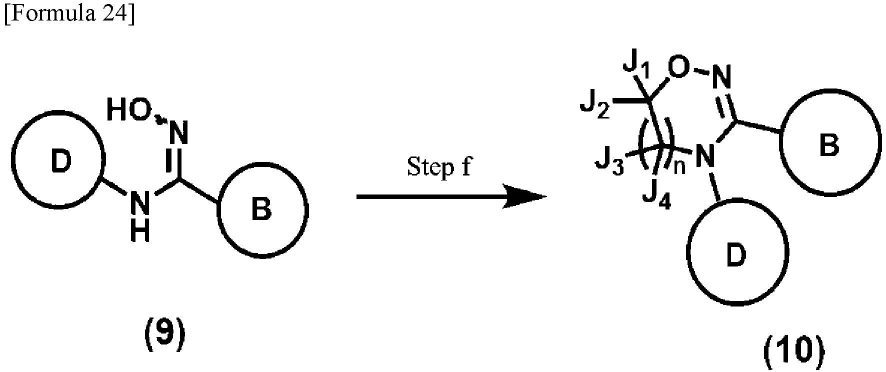

A compound represented by formula (10) can be produced by reacting a compound represented by formula (10) with a reactant in the presence of an inert solvent and optionally a base. Moiety D, moiety B, J₁, J₂, J₃, and J₄ are defined as above, and n is 0 or 1. Moiety B is a cyclic structure represented by any of formulas (3-1) to (3-6) or formula (3-8) above, wherein G₂, G₄, G₅, G₆, G₇, G₈, W, R₁, R₃, R₉, R₁₀, R₁₁, R₁₂, and R₁₃ are the same as above, and m is 0, 1, or 2.

Examples of the reactant that can be used in step f include 1,1-carbonyldiimidazole, 1, 1-thiocarbonyldiimidazole, methyl chloroformate, ethyl chloroformate, phenyl chloroformate, 4-nitrophenyl chloroformate, dibromomethane, bromochloromethane, 1,2-dichloroethane, 1,2-dibromoethane, chloroacetyl chloride, and bromoacetyl chloride, and the amount thereof used may be appropriately selected usually in the range of about 1 to 20 times the number of moles of the compound represented by formula (9).

Examples of the base that can be used in step f include an alkali metal carbonate such as sodium carbonate or potassium carbonate, a tertiary amine such as triethylamine or N,N-diisopropylethylamine, and a nitrogen-containing aromatic compound such as pyridine or 4-dimethylaminopyridine, and the amount thereof used may be appropriately selected usually in the range of about 1 to 20 times the number of moles of the compound represented by formula (9).

The inert solvent that can be used in step f may be any inert solvent that does not remarkably inhibit the present reaction, examples thereof include an ether such as diethyl ether, tetrahydrofuran, or dioxane, an aromatic hydrocarbon such as benzene, toluene, or xylene, a polar solvent such as N,N-dimethylformamide, N,N-dimethylacetamide, dimethylsulfoxide, or 1,3-dimethyl-2-imidazolinone, and a halogenated hydrocarbon such as dichloromethane or 1,2-dichloroethane, and these inert solvents can be used on its own or as a mixture of two or more thereof.

The reaction temperature in step f may be usually in the range of about -78°C to the boiling point of the solvent used, and the reaction time varies depending on the reaction scale, the reaction temperature, or the like, and may be appropriately selected in the range of several minutes to 48 hours. In addition, the present reaction can also be carried out, for example, in an atmosphere of an inert gas such as nitrogen gas or argon gas.

After completion of the reaction, the target product may be isolated from the reaction system including the target product by a conventional method, and if necessary, the target product can be produced by purification by recrystallization, column chromatography, or the like. A composition including the target product may be used in the next step without isolation.

### <Step g>

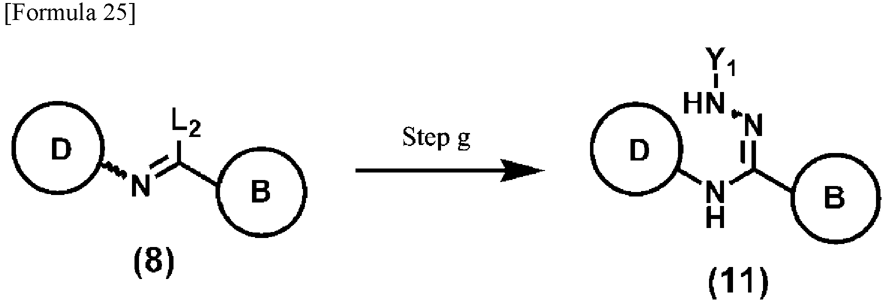

A compound represented by formula (11) can be produced by reacting a compound represented by formula (8) with a reactant in the presence of an inert solvent and optionally a base. Moiety D, moiety B, Y₁, and L₂ are defined as above. Moiety B is a cyclic structure represented by any of formulas (3-1) to (3-6) or formula (3-8) above, wherein G₂, G₄, G₅, G₆, G₇, G₈, W, R₁, R₃, R₉, R₁₀, R₁₁, R₁₂, and R₁₃ are the same as above, and m is 0, 1, or 2.

Examples of the reactant that can be used in step g include hydrazine, a hydrazine aqueous solution, methylhydrazine, and acetohydrazine, and the amount thereof used may be appropriately selected usually in the range of about 1 to 20 times the number of moles of the compound represented by formula (8).

Examples of the base that can be used in step g include an alkali metal carbonate such as sodium carbonate or potassium carbonate, a tertiary amine such as triethylamine or N,N-diisopropylethylamine, and a nitrogen-containing aromatic compound such as pyridine or 4-dimethylaminopyridine, and the amount thereof used may be appropriately selected usually in the range of about 1 to 20 times the number of moles of the compound represented by formula (8).

The inert solvent that can be used in step g may be any inert solvent that does not remarkably inhibit the present reaction, examples thereof include an ether such as diethyl ether, tetrahydrofuran, or dioxane, an aromatic hydrocarbon such as benzene, toluene, or xylene, a polar solvent such as N,N-dimethylformamide, N,N-dimethylacetamide, dimethylsulfoxide, or 1,3-dimethyl-2-imidazolinone, and a halogenated hydrocarbon such as dichloromethane or 1,2-dichloroethane, and these inert solvents can be used on its own or as a mixture of two or more thereof.

The reaction temperature in step g may be usually in the range of about -78°C to the boiling point of the solvent used, and the reaction time varies depending on the reaction scale, the reaction temperature, or the like, and may be appropriately selected in the range of several minutes to 48 hours. In addition, the present reaction can also be carried out, for example, in an atmosphere of an inert gas such as nitrogen gas or argon gas.

After completion of the reaction, the target product may be isolated from the reaction system including the target product by a conventional method, and if necessary, the target product can be produced by purification by recrystallization, column chromatography, or the like. A composition including the target product may be used in the next step without isolation.

### <Step h>

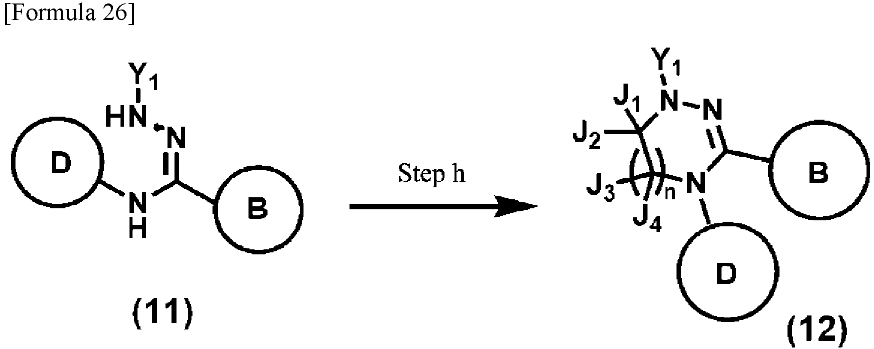

A compound represented by formula (12) can be produced by reacting a compound represented by formula (11) with a reactant in the presence of an inert solvent and optionally a base. Moiety D, moiety B, Y₁, J₁, J₂, J₃, and J₄ are defined as above, and n is 0 or 1. Moiety B is a cyclic structure represented by any of formulas (3-1) to (3-6) or formula (3-8) above, wherein G₂, G₄, G₅, G₆, G₇, G₈, W, R₁, R₃, R₉, R₁₀, R₁₁, R₁₂, and R₁₃ are the same as above, and m is 0, 1, or 2.

Examples of the reactant that can be used in step h include 1,1-carbonyldiimidazole, 1,1-thiocarbonyldiimidazole, methyl chloroformate, ethyl chloroformate, phenyl chloroformate, 4-nitrophenyl chloroformate, dibromomethane, bromochloromethane, 1,2-dichloroethane, 1,2-dibromoethane, chloroacetyl chloride, and bromoacetyl chloride, and the amount thereof used may be appropriately selected usually in the range of about 1 to 20 times the number of moles of the compound represented by formula (11).

Examples of the base that can be used in step h include an alkali metal carbonate such as sodium carbonate or potassium carbonate, a tertiary amine such as triethylamine or N,N-diisopropylethylamine, and a nitrogen-containing aromatic compound such as pyridine or 4-dimethylaminopyridine, and the amount thereof used may be appropriately selected usually in the range of about 1 to 20 times the number of moles of the compound represented by formula (11).

The inert solvent that can be used in step h may be any inert solvent that does not remarkably inhibit the present reaction, examples thereof include an ether such as diethyl ether, tetrahydrofuran, or dioxane, an aromatic hydrocarbon such as benzene, toluene, or xylene, a polar solvent such as N,N-dimethylformamide, N,N-dimethylacetamide, dimethylsulfoxide, or 1,3-dimethyl-2-imidazolinone, and a halogenated hydrocarbon such as dichloromethane or 1,2-dichloroethane, and these inert solvents can be used on its own or as a mixture of two or more thereof.

The reaction temperature in step h may be usually in the range of about -78°C to the boiling point of the solvent used, and the reaction time varies depending on the reaction scale, the reaction temperature, or the like, and may be appropriately selected in the range of several minutes to 48 hours. In addition, the present reaction can also be carried out, for example, in an atmosphere of an inert gas such as nitrogen gas or argon gas.

After completion of the reaction, the target product may be isolated from the reaction system including the target product by a conventional method, and if necessary, the target product can be produced by purification by recrystallization, column chromatography, or the like. A composition including the target product may be used in the next step without isolation.

### <Step i>

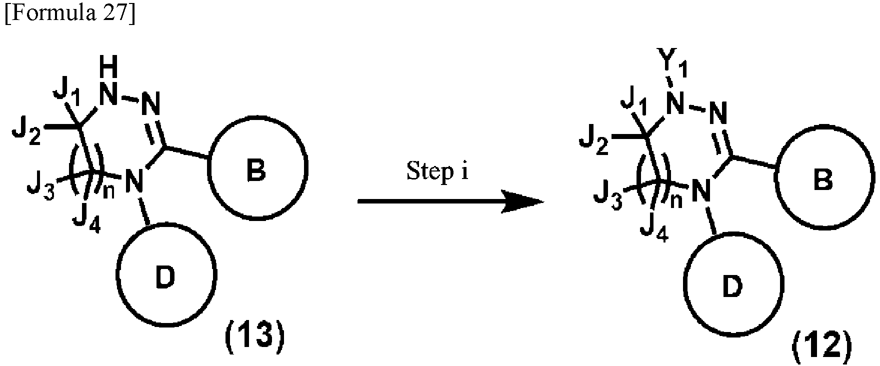

A compound represented by formula (12) can be produced by reacting a compound represented by formula (13) with a reactant in the presence of an inert solvent and optionally a base. Moiety D, moiety B, Y₁, J₁, J₂, J₃, and J₄ are defined as above, and n is 0 or 1.
However, Y₁ is not hydrogen. Moiety B is a cyclic structure represented by any of formulas (3-1) to (3-6) or formula (3-8) above, wherein G₂, G₄, G₅, G₆, G₇, G₈, W, R₁, R₃, R₉, R₁₀, R₁₁, R₁₂, and R₁₃ are the same as above, and m is 0, 1, or 2. The compound represented by formula (13) can be prepared by the production method of step h above.

Examples of the reactant that can be used in step i include iodomethane, iodoethane, dimethyl sulfate, acetic anhydride, acetyl chloride, allyl chloride, methyl chlorocarbonate, dimethyl carbonate, mesyl chloride, trifluoromethanesulfonyl chloride, trifluoromethanesulfonic anhydride, bromoacetonitrile, methoxymethyl chloride, cyclopropylcarbonyl chloride, and methylthiomethyl chloride, and the amount thereof used may be appropriately selected usually in the range of about 1 to 20 times the number of moles of the compound represented by formula (13).

Examples of the base that can be used in step i include an alkali metal carbonate such as sodium carbonate or potassium carbonate, an alkali metal salt such as potassium-t-butoxide, sodium-t-butoxide, or sodium hydride, a tertiary amine such as triethylamine or N,N-diisopropylethylamine, and a nitrogen-containing aromatic compound such as pyridine or 4-dimethylaminopyridine, and the amount thereof used may be appropriately selected usually in the range of about 1 to 20 times the number of moles of the compound represented by formula (13).

The inert solvent that can be used in step i may be any inert solvent that does not remarkably inhibit the present reaction, examples thereof include an ether such as diethyl ether, tetrahydrofuran, or dioxane, an aromatic hydrocarbon such as benzene, toluene, or xylene, a polar solvent such as N,N-dimethylformamide, N,N-dimethylacetamide, dimethylsulfoxide, or 1,3-dimethyl-2-imidazolinone, and a halogenated hydrocarbon such as dichloromethane or 1,2-dichloroethane, and these inert solvents can be used on its own or as a mixture of two or more thereof.

The reaction temperature in step i may be usually in the range of about -78°C to the boiling point of the solvent used, and the reaction time varies depending on the reaction scale, the reaction temperature, or the like, and may be appropriately selected in the range of several minutes to 48 hours. In addition, the present reaction can also be carried out, for example, in an atmosphere of an inert gas such as nitrogen gas or argon gas.

After completion of the reaction, the target product may be isolated from the reaction system including the target product by a conventional method, and if necessary, the target product can be produced by purification by recrystallization, column chromatography, or the like. A composition including the target product may be used in the next step without isolation.

### <Step j>

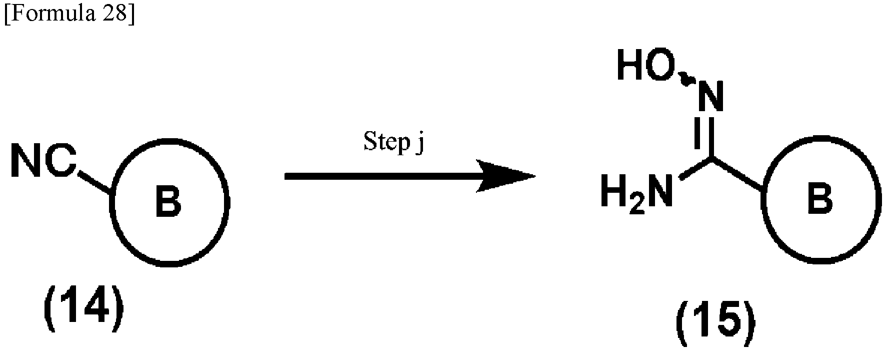

A compound represented by formula (15) can be produced by reacting a compound represented by formula (14) with a reactant in the presence of an inert solvent and optionally a base. Moiety B is defined as above. Moiety B is a cyclic structure represented by any of formulas (3-1) to (3-6) or formula (3-8) above, wherein G₂, G₄, G₅, G₆, G₇, G₈, W, R₁, R₃, R₉, R₁₀, R₁₁, R₁₂, and R₁₃ are the same as above, and m is 0, 1, or 2.

Examples of the reactant that can be used in step j include a hydroxylamine aqueous solution and a hydroxylamine acid addition salt, and the amount thereof used may be appropriately selected usually in the range of about 1 to 20 times the number of moles of the compound represented by formula (14).

Examples of the base that can be used in step j include an alkali metal carbonate such as sodium carbonate or potassium carbonate, an alkali metal salt such as potassium-t-butoxide, sodium-t-butoxide, or sodium hydride, a tertiary amine such as triethylamine or N,N-diisopropylethylamine, and a nitrogen-containing aromatic compound such as pyridine or 4-dimethylaminopyridine, and the amount thereof used may be appropriately selected usually in the range of about 1 to 20 times the number of moles of the compound represented by formula (14).

The inert solvent that can be used in step j may be any inert solvent that does not remarkably inhibit the present reaction, examples thereof include an ether such as diethyl ether, tetrahydrofuran, or dioxane, an aromatic hydrocarbon such as benzene, toluene, or xylene, a polar solvent such as N,N-dimethylformamide, N,N-dimethylacetamide, dimethylsulfoxide, or 1,3-dimethyl-2-imidazolinone, a halogenated hydrocarbon such as dichloromethane or 1,2-dichloroethane, and an alcohol such as methanol, ethanol, or isopropyl alcohol, and these inert solvents can be used on its own or as a mixture of two or more thereof.

The reaction temperature in step j may be usually in the range of about -78°C to the boiling point of the solvent used, and the reaction time varies depending on the reaction scale, the reaction temperature, or the like, and may be appropriately selected in the range of several minutes to 48 hours. In addition, the present reaction can also be carried out, for example, in an atmosphere of an inert gas such as nitrogen gas or argon gas.

After completion of the reaction, the target product may be isolated from the reaction system including the target product by a conventional method, and if necessary, the target product can be produced by purification processes such as recrystallization and column chromatography. A composition including the target product may be used in the next step without isolation.

### <Step k>

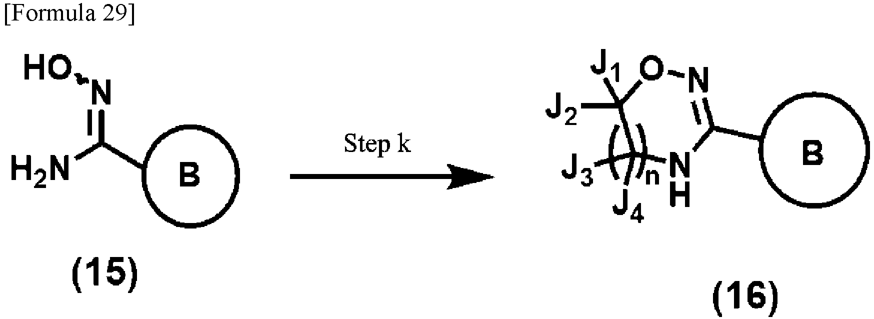

A compound represented by formula (16) can be produced by reacting a compound represented by formula (15) with a reactant in the presence of an inert solvent and optionally a base. Moiety B, J₁, J₂, J₃, and J₄ are defined as above, and n is 0 or 1. Moiety B is a cyclic structure represented by any of formulas (3-1) to (3-6) or formula (3-8) above, wherein G₂, G₄, G₅, G₆, G₇, G₈, W, R₁, R₃, R₉, R₁₀, R₁₁, R₁₂, and R₁₃ are the same as above, and m is 0, 1, or 2.

Examples of the reactant that can be used in step k include 1,1-carbonyldiimidazole, 1, 1-thiocarbonyldiimidazole, methyl chloroformate, ethyl chloroformate, phenyl chloroformate, 4-nitrophenyl chloroformate, dibromomethane, bromochloromethane, 1,2-dichloroethane, 1,2-dibromoethane, chloroacetyl chloride, and bromoacetyl chloride, and the amount thereof used may be appropriately selected usually in the range of about 1 to 20 times the number of moles of the compound represented by formula (15).

Examples of the base that can be used in step k include an alkali metal carbonate such as sodium carbonate or potassium carbonate, a tertiary amine such as triethylamine or N,N-diisopropylethylamine, and a nitrogen-containing aromatic compound such as pyridine or 4-dimethylaminopyridine, and the amount thereof used may be appropriately selected usually in the range of about 1 to 20 times the number of moles of the compound represented by formula (15).

The inert solvent that can be used in step k may be any inert solvent that does not remarkably inhibit the present reaction, examples thereof include an ether such as diethyl ether, tetrahydrofuran, or dioxane, an aromatic hydrocarbon such as benzene, toluene, or xylene, a polar solvent such as N,N-dimethylformamide, N,N-dimethylacetamide, dimethylsulfoxide, or 1,3-dimethyl-2-imidazolinone, and a halogenated hydrocarbon such as dichloromethane or 1,2-dichloroethane, and these inert solvents can be used on its own or as a mixture of two or more thereof.

The reaction temperature in step k may be usually in the range of about -78°C to the boiling point of the solvent used, and the reaction time varies depending on the reaction scale, the reaction temperature, or the like, and may be appropriately selected in the range of several minutes to 48 hours. In addition, the present reaction can also be carried out, for example, in an atmosphere of an inert gas such as nitrogen gas or argon gas.

After completion of the reaction, the target product may be isolated from the reaction system including the target product by a conventional method, and if necessary, the target product can be produced by purification by recrystallization, column chromatography, or the like. A composition including the target product may be used in the next step without isolation.

### <Step 1>

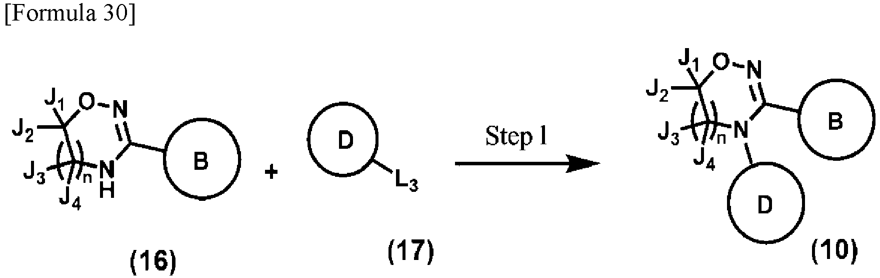

A compound represented by formula (10) can be produced by reacting a compound represented by formula (16) and a compound represented by formula (17) in the presence of an inert solvent and a base, and in the presence of optionally a metal catalyst and optionally a ligand. In the formulas, L₃ represents a halogen, a trifluoromethanesulfonate group, or an alkoxy group, moiety D, moiety B, J₁, J₂, J₃, and J₄ are defined as above, and n is 0 or 1. Moiety B is a cyclic structure represented by any of formulas (3-1) to (3-6) or formula (3-8) above, wherein G₂, G₄, G₅, G₆, G₇, G₈, W, R₁, R₃, R₉, R₁₀, R₁₁, R₁₂, and R₁₃ are the same as above, and m is 0, 1, or 2.

Examples of the base that can be used in step 1 include an alkali metal carbonate such as sodium carbonate or potassium carbonate, an alkali metal salt such as potassium-t-butoxide, sodium-t-butoxide, or sodium hydride, a tertiary amine such as triethylamine or N,N-diisopropylethylamine, and a nitrogen-containing aromatic compound such as pyridine or 4-dimethylaminopyridine, and the amount thereof used may be appropriately selected usually in the range of about 1 to 20 times the number of moles of the compound represented by formula (16).

Examples of the metal catalyst that can be used in step 1 include a palladium catalyst such as tetrakistriphenylphosphinepalladium(0), bis(triphenylphosphine)palladium(II) dichloride, palladium(II) acetate, tris(dibenzylideneacetone)dipalladium(0), or [1,1'-bis(diphenylphosphino)ferrocene]palladium(II) dichloride dichloromethane adduct, and a copper catalyst such as copper(I) iodide, copper(I) chloride, or copper(I) bromide. The amount thereof used may be appropriately selected usually in the range of about 0.001 to 1 time the number of moles of the compound represented by formula (16).

Examples of the ligand that can be used in step 1 include an amine-based ligand such as ethylenediamine, N,N'-dimethylethylenediamine, diethylenetriamine, 1,4,7-triazacyclononane, or triethylenetetramine, and an imine-based ligand such as 2,2'-bipyridyl, 1,8-naphthyridine, or 1,10-phenanthroline, and the amount thereof used may be appropriately selected usually in the range of about 0.001 to 1 time the number of moles of the compound represented by formula (16).

The inert solvent that can be used in step 1 may be any inert solvent that does not remarkably inhibit the present reaction, examples thereof include an ether such as diethyl ether, tetrahydrofuran, or dioxane, an aromatic hydrocarbon such as benzene, toluene, or xylene, a polar solvent such as N,N-dimethylformamide, N,N-dimethylacetamide, dimethylsulfoxide, or 1,3-dimethyl-2-imidazolinone, and a halogenated hydrocarbon such as dichloromethane or 1,2-dichloroethane, and these inert solvents can be used on its own or as a mixture of two or more thereof.

The reaction temperature in step 1 may be usually in the range of about -78°C to the boiling point of the solvent used, and the reaction time varies depending on the reaction scale, the reaction temperature, or the like, and may be appropriately selected in the range of several minutes to 48 hours. In addition, the present reaction can also be carried out, for example, in an atmosphere of an inert gas such as nitrogen gas or argon gas.

After completion of the reaction, the target product may be isolated from the reaction system including the target product by a conventional method, and if necessary, the target product can be produced by purification by recrystallization, column chromatography, or the like. A composition including the target product may be used in the next step without isolation.

### <Step m>

A compound represented by formula (21-1), (21-2), (21-3), (21-4), (21-5), (21-6), or (21-8) can be produced by reacting a compound represented by formula (20-1), (20-2), (20-3), (20-4), (20-5), (20-6), or (20-8), respectively, with a chlorinating agent in the presence of an inert solvent. In the formulas, moiety D, J₁, J₂, J₃, and J₄ are defined as above, and n is 0 or 1. In the following formulas, G₂, G₄, G₅, G₈, G₇, G₈, W, R₁, R₃, R₉, R₁₀, R₁₁, and R₁₃ are the same as above, and m is 1 or 2. The compounds represented by formulas (20-1) to (20-6) and (20-8) can be prepared by the same production methods as in step a to step 1 above.

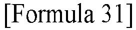

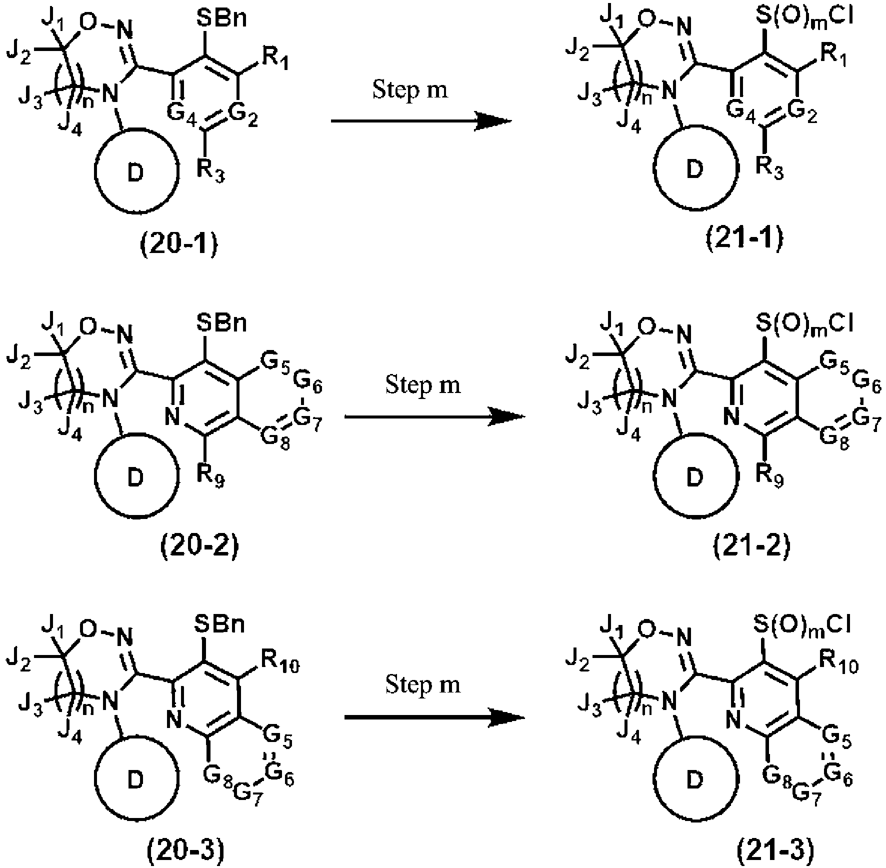

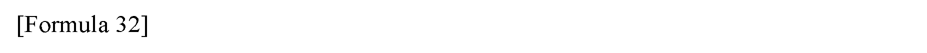

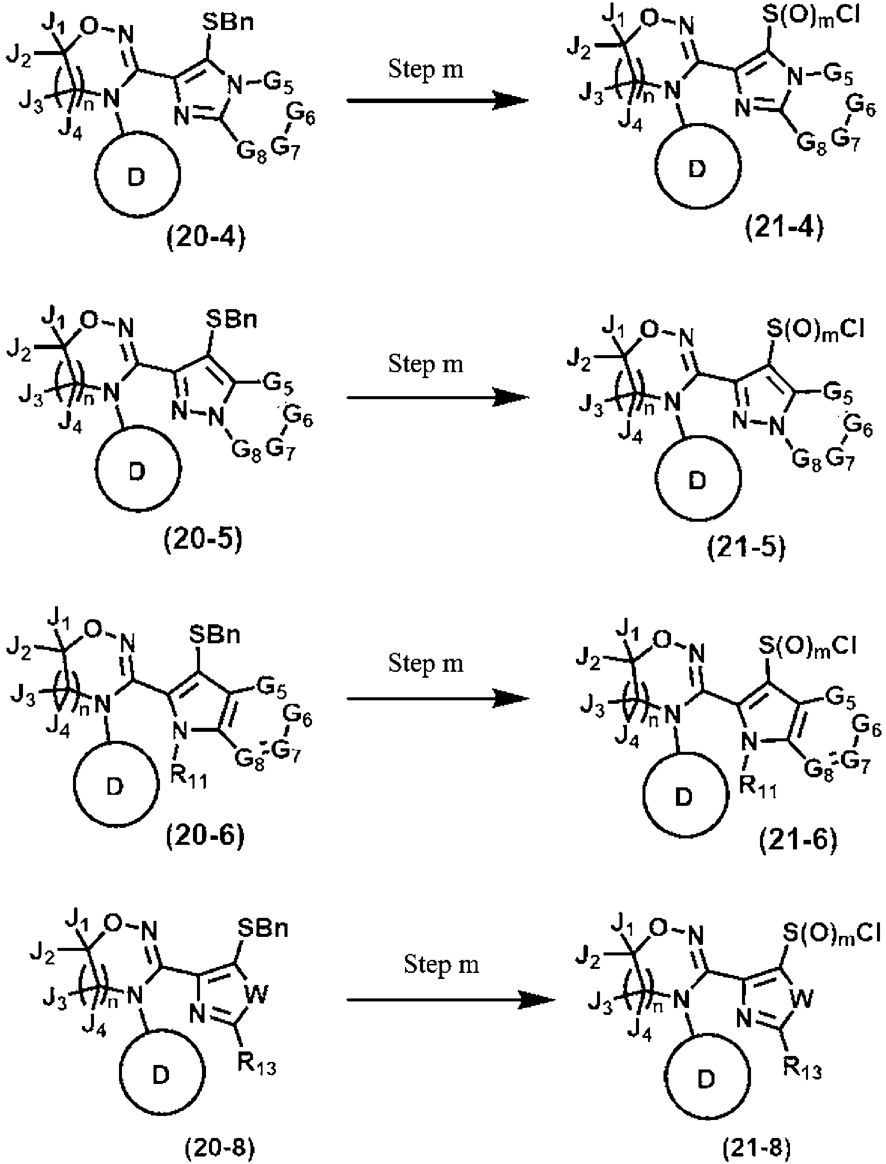

Examples of the chlorinating agent that can be used in step m include chlorine, thionyl chloride, N-chlorosuccinimide, and 1,3-dichloro-5,5-dimethylhydantoin, and the amount thereof used may be appropriately selected usually in the range of about 1 to 20 times the number of moles of the compound represented by any of formulas (20-1) to (20-6) and (20-8).

The inert solvent that can be used in step m may be any inert solvent that does not remarkably inhibit the present reaction, examples thereof include inert solvents such as an ether such as diethyl ether, tetrahydrofuran, or dioxane, an aromatic hydrocarbon such as benzene, toluene, or xylene, a nitrile such as acetonitrile or propionitrile, a halogenated hydrocarbon such as dichloromethane, chloroform, or 1,2-dichloroethane, an organic acid such as acetic acid or propionic acid, and water, and these inert solvents can be used on its own or as a mixture of two or more thereof.

The reaction temperature in step m may be usually in the range of about -78°C to the boiling point of the solvent used, and the reaction time varies depending on the reaction scale, the reaction temperature, or the like, and may be appropriately selected in the range of several minutes to 48 hours. In addition, the present reaction can also be carried out, for example, in an atmosphere of an inert gas such as nitrogen gas or argon gas.

After completion of the reaction, the target product may be isolated from the reaction system including the target product by a conventional method, and if necessary, the target product can be produced by purification by recrystallization, column chromatography, or the like. A composition including the target product may be used in the next step without isolation.

### <Step n>

A compound represented by formula (22-1), (22-2), (22-3), (22-4), (22-5), (22-6), or (22-8) can be produced by reacting a compound represented by formula (21-1), (21-2), (21-3), (21-4), (21-5), (21-6), or (21-8), respectively, with NHYₐY_{b}, wherein YₐY_{b} is the same as above, in the presence of an inert solvent and optionally a base. In the formulas, moiety D, J₁, J₂, J₃, and J₄ are defined as above, and n is 0 or 1. In the following formulas, G₂, G₄, G₅, G₆, G₇, G₈, W, Yₐ, Y_{b}, R₁, R₃, R₉, R₁₀, R₁₁, and R₁₃ are the same as above, and m is 1 or 2. The compounds represented by formulas (21-1) to (21-6) and (21-8) can be prepared by the same production methods as in step a to step m above.

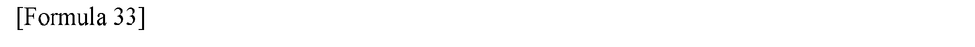

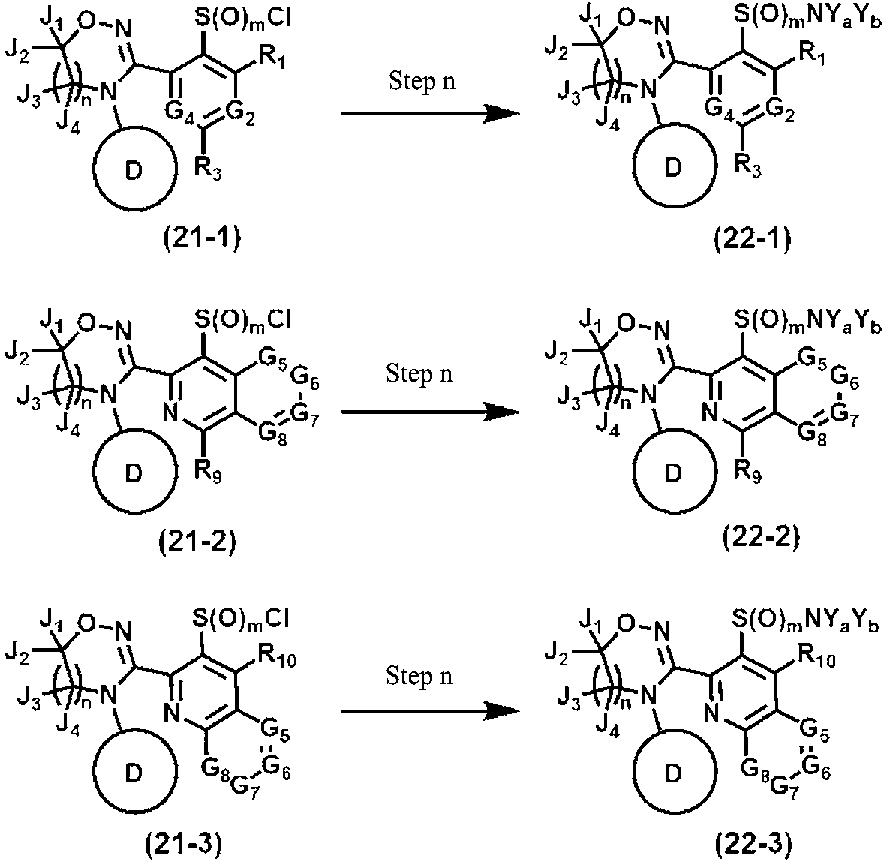

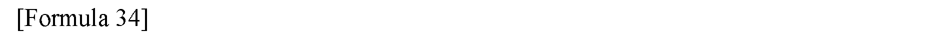

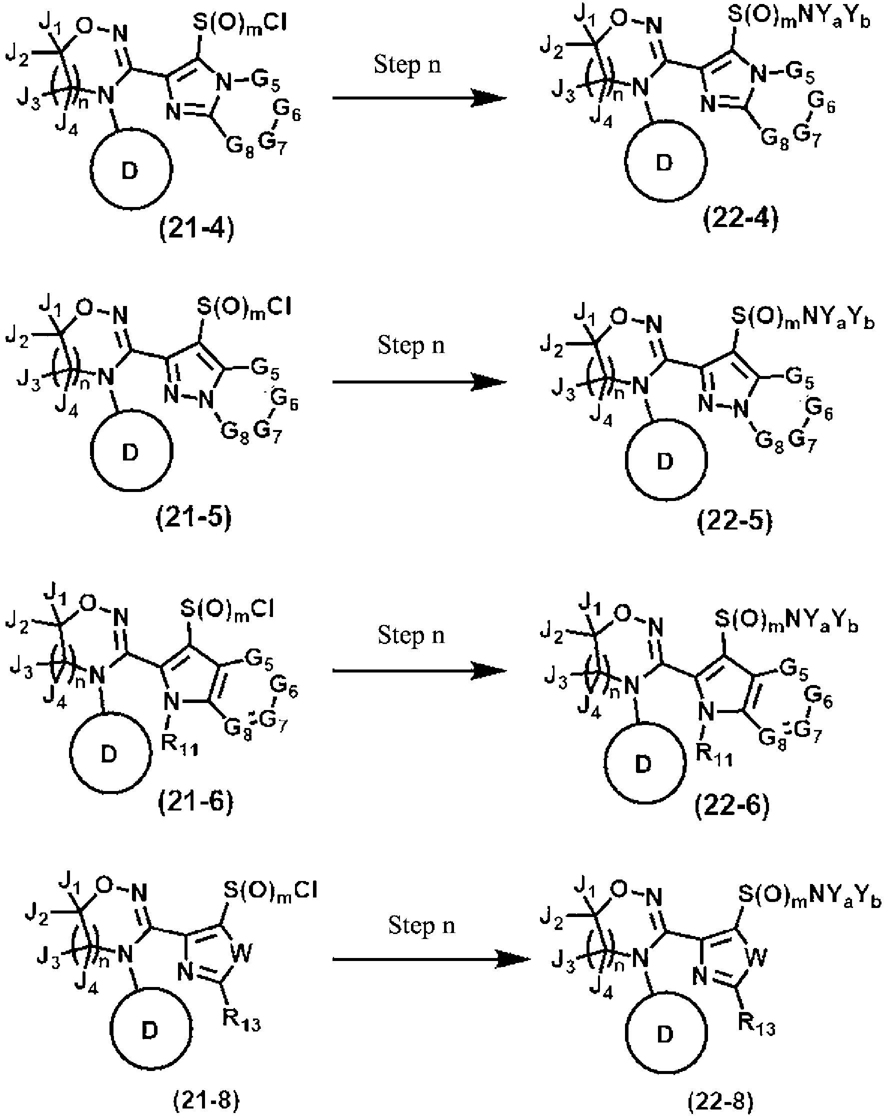

The amount of NHYₐY_{b} used that can be used in step n may be appropriately selected usually in the range of about 1 to 20 times the number of moles of the compound represented by any of formulas (21-1) to (21-6) and (21-8).

The inert solvent that can be used in step n may be any inert solvent that does not remarkably inhibit the present reaction, examples thereof include inert solvents such as an ether such as diethyl ether, tetrahydrofuran, or dioxane, an aromatic hydrocarbon such as benzene, toluene, or xylene, a nitrile such as acetonitrile or propionitrile, a halogenated hydrocarbon such as dichloromethane, chloroform, or 1,2-dichloroethane, an alcohol such as methanol, ethanol, or isopropyl alcohol, an amide such as dimethylformamide or dimethylacetamide, and a polar solvent such as dimethyl sulfoxide or 1,3-dimethyl-2-imidazolidinone, and these inert solvents can be used on its own or as a mixture of two or more thereof.

The reaction temperature in step n may be usually in the range of about -78°C to the boiling point of the solvent used, and the reaction time varies depending on the reaction scale, the reaction temperature, or the like, and may be appropriately selected in the range of several minutes to 48 hours. In addition, the present reaction can also be carried out, for example, in an atmosphere of an inert gas such as nitrogen gas or argon gas.

After completion of the reaction, the target product may be isolated from the reaction system including the target product by a conventional method, and if necessary, the target product can be produced by purification by recrystallization, column chromatography, or the like.

The compound represented by formula (1) according to the present invention or a salt thereof or an N-oxide thereof has a control effect on a pest in agriculture and horticulture. Therefore, the present application also includes an invention of a pest control agent including the compound represented by formula (1) or a salt thereof or an N-oxide thereof as an active ingredient. More particularly, the present application provides a use for a pest control agent in agriculture and horticulture.

The insect species to be controlled in the present invention (insect species on which the compound represented by formula (1) exhibits a control effect) is not particularly limited, and the compound can be used to control a wide range of harmful insect pests in agriculture and horticulture. Examples of preferable insect species to be controlled include the following.

Lepidoptera {for example, Crambidae, such as rice stem borer (Chilo suppressalis), Darkheaded stem borer (Chilo polychrysus), White stem borer (Scirpophaga innotata), yellow paddy borer (Scirpophaga incertulas), Rupela albina, grass leaf roller (Cnaphalocrocis medinalis), Marasmia patnalis, rice leaf roller (Marasmia exigua), cotton leaf roller (Notarcha derogata), corn borer (Ostrinia furnacalis), European corn borer (Ostrinia nubilalis), cabbage webworm (Hellula undalis), grape leaf roller (Herpetogramma luctuosale), bluegrass webworm (Pediasia teterrellus), rice case-worm (Nymphula depunctalis), and Sugarcane borer (Diatraea saccharalis); Pyralidae, such as lesser cornstalk borer (Elasmopalpus lignosellus) and mealworm moth (Plodia interpunctella); Noctuidae, such as tobacco cutworm (Spodoptera litura), beet armyworm (Spodoptera exigua), rice armyworm (Mythimna separata), cabbage moth (Mamestra brassicae), pink borer (Sesamia inferens), lawn armyworm (Spodoptera mauritia), green rice caterpillar (Naranga aenescens), fall armyworm (Spodoptera frugiperda), African armyworm (Spodoptera exempta), black cutworm (Agrotis ipsilon), beet worm (Autographa nigrisigna), gold spot (Plusia festucae), Soybean looper (Chrysodeixis includens), Trichoplusia spp., Heliothis spp. such as tobacco budworm (Heliothis virescens), Helicoverpa spp. such as cotton bollworm (Helicoverpa armigera) and corn earworm (Helicoverpa zea), Velvetbean caterpillar (Anticarsia gemmatalis), Cotton leafworm (Alabama argillacea), and Hop vine borer (Hydraecia immanis); Pieridae, such as small white (Pieris rapae); Tortricidae, such as oriental fruit moth (Grapholita molesta), plum fruit moth (Grapholita dimorpha), soybean moth (Leguminivora glycinivorella), adzuki pod worm (Matsumuraeses azukivora), summer fruit tortrix (Adoxophyes orana fasciata), smaller tea tortrix (Adoxophyes honmai), Japanese tea tortrix (Homona magnanima), apple tortrix (Archips fuscocupreanus), codling moth (Cydia pomonella), sugarcane gray borer (Tetramoera schistaceana), Bean Shoot Borer (Epinotia aporema), and Citrus fruit borer (Ecdytolopha aurantiana); Gracillariidae, such as tea leaf roller (Caloptilia theivora) and Asiatic apple leaf miner (Phyllonorycter ringoniella); Carposinidae, such as Peach fruit moth (Carposina sasakii); Lyonetiidae, such as Coffee Leaf miner (Leucoptera coffeella), apple leaf miner (Lyonetia clerkella), and blackthorn blister moth (Lyonetia prunifoliella); Lymantriidae, such as Lymantria spp. such as gypsy moth (Lymantria dispar), and Euproctis spp. such as tea tussock moth (Euproctis pseudoconspersa); Pluteliidae, such as diamondback moth (Plutella xylostella); Gelechiidae, such as peach twig borer (Anarsia lineatella), sweet potato leaf folder (Helcystogramma triannulella), pink bollworm (Pectinophora gossypiella), potato tuber moth (Phthorimaea operculella), and Tuta absoluta; Arctiidae, such as fall webworm (Hyphantria cunea); Castniidae, such as Giant Sugarcane borer (Telchin licus); Cossidae, such as carpenter moth (Cossus insularis); Geometridae, such as giant looper (Ascotis selenaria); Limacodidae, such as blue-striped nettle grub (Parasa lepida); Stathmopodidae, such as persimmon fruit moth (Stathmopoda masinissa); Sphingidae, such as tobacco hornworm (Acherontia lachesis); Sesiidae, such as Nokona feralis; and Hesperiidae, such as rice skipper (Parnara guttata)}.

Hemiptera {for example, Delphacidae, such as small brown planthopper (Laodelphax striatellus), brown planthopper (Nilaparvata lugens), white-backed planthopper (Sogatella furcifera), corn leafhopper (Peregrinus maidis), cereal leafhopper (Javesella pellucida), sugarcane leafhopper (Perkinsiella saccharicida), and Tagosodes orizicolus; Cicadellidae, such as green rice leafhopper (Nephotettix cincticeps), green paddy leafhopper (Nephotettix virescens), rice leafhopper (Nephotettix nigropictus), zigzag-striped leafhopper (Recilia dorsalis), tea green leafhopper (Empoasca onukii), potato leafhopper (Empoasca fabae), corn leafhopper (Dalbulus maidis), and white paddy leafhopper (Cofana spectra); Cercopidae, such as Mahanarva posticata and Mahanarva fimbriolata; Aphididae, such as black bean aphid (Aphis fabae), soybean aphid (Aphis glycines), cotton aphid (Aphis gossypii), green apple aphid (Aphis pomi), apple aphid (Aphis spiraecola), green peach aphid (Myzus persicae), leaf-curling plum aphid (Brachycaudus helichrysi), cabbage aphid (Brevicoryne brassicae), Rosy apple aphid (Dysaphis plantaginea), turnip aphid (Lipaphis erysimi), potato aphid (Macrosiphum euphorbiae), foxglove aphid (Aulacorthum solani), lettuce aphid (Nasonovia ribisnigri), oat bird-cherry aphid (Rhopalosiphum padi), corn leaf aphid (Rhopalosiphum maidis), brown citrus aphid (Toxoptera citricida), mealy plum aphid (Hyalopterus pruni), cane aphid (Melanaphis sacchari), black rice root aphid (Tetraneura nigriabdominalis), sugarcane cottony aphid (Ceratovacuna lanigera), and apple woolly aphid (Eriosoma lanigerum); Phylloxeridae, such as grape phylloxera (Daktulosphaira vitifoliae), Pecan phylloxera (Phylloxera devastatrix), Pecan leaf phylloxera (Phylloxera notabilis), and Southern pecan leaf phylloxera (Phylloxera russellae); Adelgidae, such as hemlock woolly aphid (Adelges tsugae), Adelges piceae, and Aphrastasia pectinatae; Pentatomidae, such as black rice bug (Scotinophara lurida), Malayan rice black bug (Scotinophara coarctata), common green stink bug (Nezara antennata), white-spotted spined bug (Eysarcoris aeneus), lewis spined bug (Eysarcoris lewisi), white spotted stink bug (Eysarcoris ventralis), Eysarcoris annamita, brown marmorated stink bug (Halyomorpha halys), southern green stink bug (Nezara viridula), Brown stink bug (Euschistus heros), Red banded stink bug (Piezodorus guildinii), Oebalus pugnax, or Dichelops melacanthus; Cydnidae, such as Burrower brown bug (Scaptocoris castanea); Alydidae, such as bean bug (Riptortus pedestris), corbett rice bug (Leptocorisa chinensis), and paddy bug (Leptocorisa acuta); Coreidae, such as rice stinkbug (Cletus punctiger) and Australian leaf-footed bug (Leptoglossus australis); Lygaeidae, such as oriental chinch bug (Caverelius saccharivorus), seed bug (Togo hemipterus), and chinch bug (Blissus leucopterus); Miridae, such as rice leaf bug (Trigonotylus caelestialium), sorghum plant bug (Stenotus rubrovittatus), wheat leaf bug (Stenodema calcarata), and tarnished plant bug (Lygus lineolaris); Aleyrodidae, such as greenhouse whitefly (Trialeurodes vaporariorum), tobacco whitefly (Bemisia tabaci), citrus whitefly (Dialeurodes citri), orange spiny whitefly (Aleurocanthus spiniferus), camellia spiny whitefly (Aleurocanthus camelliae), and Pealius euryae; Diaspididae, such as cyanophyllum scale (Abgrallaspis cyanophylli), California red scale (Aonidiella aurantii), San Jose scale (Diaspidiotus perniciosus), white peach scale (Pseudaulacaspis pentagona), arrowhead scale (Unaspis yanonensis), and citrus snow scale (Unaspis citri); Coccidae, such as pink wax scale (Ceroplastes rubens); Margarodidae, such as cottony cushion scale (Icerya purchasi) and Seychelles scale (Icerya seychellarum); Pseudococcidae, such as solanum mealybug (Phenacoccus solani), cotton mealybug (Phenacoccus solenopsis), Japanese mealybug (Planococcus kraunhiae), white peach scale (Pseudococcus comstocki), citrus mealybug (Planococcus citri), scarlet mealybug (Pseudococcus calceolariae), long-tailed mealybug (Pseudococcus longispinus), and tuttle mealybug (Brevennia rehi); Psyllidae, such as Asian citrus psyllid (Diaphorina citri), African citrus psyllid (Trioza erytreae), pear sucker (Cacopsylla pyrisuga), Chinese pear psyllid (Cacopsylla chinensis), potato psyllid (Bactericera cockerelli), and Pear psylla (Cacopsylla pyricola); Tingidae, such as sycamore lace bug (Corythucha ciliata), chrysanthemum lace bug (Corythucha marmorata), Japanese pear lace bug (Stephanitis nashi), and azalea lace bug (Stephanitis pyrioides); and Cimicidae, such as common bed bug (Cimex lectularius), and Cicadidae, such as Giant Cicada (Quesada gigas)}.

Coleoptera {for example, Chrysomelidae, such as western corn rootworm (Diabrotica virgifera virgifera), southern corn rootworm (Diabrotica undecimpunctata howardi), northern corn rootworm (Diabrotica barberi), Mexican corn rootworm (Diabrotica virgifera zeae), banded cucumber beetle (Diabrotica balteata), Cucurbit Beetle (Diabrotica speciosa), bean leaf beetle (Cerotoma trifurcata), cereal leaf beetle (Oulema melanopus), cucurbit leaf beetle (Aulacophora femoralis), striped flea beetle (Phyllotreta striolata), Cabbage flea beetle (Phyllotreta cruciferae), Western black flea beetle (Phyllotreta pusilla), Cabbage stem flea beetle (Psylliodes chrysocephala), Colorado potato beetle (Leptinotarsa decemlineata), rice leaf beetle (Oulema oryzae), grape colaspis (Colaspis brunnea), corn flea beetle (Chaetocnema pulicaria), sweet-potato flea beetle (Chaetocnema confinis), potato flea beetle (Epitrix cucumeris), rice hispa (Dicladispa armigera), southern corn leaf beetle (Myochrous denticollis), Laccoptera quadrimaculata, and tobacco flea beetle (Epitrix hirtipennis); Carabidae, such as Seedcorn beetle (Stenolophus lecontei) and Slender seedcorn beetle (Clivina impressifrons); Scarabaeidae, such as cupreous chafer (Anomala cuprea), soybean beetle (Anomala rufocuprea), green chafer (Anomala albopilosa), Japanese beetle (Popillia japonica), yellowish elongate chafer (Heptophylla picea), European Chafer (Rhizotrogus majalis), carrot beetle (Tomarus gibbosus), Holotrichia spp., Phyllophaga spp. such as June beetle (Phyllophaga crinita), and Diloboderus spp. such as Diloboderus abderus; Curculionidae, such as coffee bean weevil (Araecerus coffeae), sweet-potato weevil (Cylas formicarius), West Indian Sweet potato weevil (Euscepes postfasciatus), alfalfa weevil (Hypera postica), maize weevil (Sitophilus zeamais), rice water weevil (Echinocnemus squameus), rice water weevil (Lissorhoptrus oryzophilus), Asiatic palm weevil (Rhabdoscelus lineatocollis), boll weevil (Anthonomus grandis), hunting billbug (Sphenophorus venatus), Southern Corn Billbug (Sphenophorus callosus), Soybean stalk weevil (Sternechus subsignatus), Sugarcane weevil (Sphenophorus levis), rusty gourd-shaped weevil (Scepticus griseus), brown gourd-shaped weevil (Scepticus uniformis), Mexican bean weevil (Zabrotes subfasciatus), pine shoot beetle (Tomicus piniperda), Coffee Berry Borer (Hypothenemus hampei), Aracanthus spp. such as Aracanthus mourei, and cotton root borer (Eutinobothrus brasiliensis); Tenebrionidae, such as red flour beetle (Tribolium castaneum) and confused flour beetle (Tribolium confusum); Coccinellidae, such as 28-spotted potato ladybird (Epilachna vigintioctopunctata); Bostrychidae, such as common powder-post beetle (Lyctus brunneus); Ptinidae; Cerambycidae, such as citrus long-horned beetle (Anoplophora malasiaca) and Migdolus fryanus; Elateridae, such as sugarcane click beetle (Melanotus okinawensis), barley wireworm (Agriotes fuscicollis), pectinate-horned click beetle (Melanotus legatus), Anchastus spp., Conoderus spp., Ctenicera spp., Limonius spp., and Aeolus spp.; and Staphylinidae, such as skirt and blouse beetle (Paederus fuscipes)}.

Thysanoptera {for example, Thripidae, such as western flower thrips (Frankliniella occidentalis), southern yellow thrips (Thrips palmi), yellow tea thrips (Scirtothrips dorsalis), onion thrips (Thrips tabaci), eastern flower thrips (Frankliniella intonsa), rice thrips (Stenchaetothrips biformis), and poinsettia thrips (Echinothrips americanus); and Phlaeothripidae, such as grass thrips (Haplothrips aculeatus)}.

Diptera {for example, Anthomyiidae, such as seedcorn maggot (Delia platura) and onion maggot (Delia antiqua); Ulidiidae, such as sugarbeet root maggot (Tetanops myopaeformis); Agromyzidae, such as rice leaf miner (Agromyza oryzae), tomato leaf miner (Liriomyza sativae), American serpentine leaf miner (Liriomyza trifolii), and pea leaf miner (Chromatomyia horticola); Chloropidae, such as rice stem maggot (Chlorops oryzae); Tephritidae, such as melon fly (Bactrocera cucurbitae), oriental fruit fly (Bactrocera dorsalis), Malaysian fruit fly (Bactrocera latifrons), olive fruit fly (Bactrocera oleae), Queensland fruit fly (Bactrocera tryoni), and Mediterranean fruit fly (Ceratitis capitata); Ephydridae, such as smaller rice leaf miner (Hydrellia griseola), whorl maggot (Hydrellia philippina), or paddy stem maggot (Hydrellia sasakii); Drosophilidae, such as spotted-wing drosophila (Drosophila suzukii); Phoridae, such as Megaselia spiracularis; Psychodidae, such as bathroom moth fly (Clogmia albipunctata); Sciaridae, such as Bradysia difformis; Cecidomyiidae, such as hessian fly (Mayetiola destructor) and Asian rice gall midge (Orseolia oryzae); Diopsidae, such as Diopsis macrophthalma; and Tipulidae, such as rice crane fly (Tipula aino), Common cranefly (Tipula oleracea), and European cranefly (Tipula paludosa)}.

Hymenoptera {for example, Tenthredinidae, such as turnip sawfly (Athalia rosae) and nippon cabbage sawfly (Athalia japonica); fire ant (Solenopsis spp.) family, and Formicidae, such as Brown leaf-cutting ant (Atta capiguara)}.

Orthoptera {for example, Acrididae, such as migratory locust (Locusta migratoria), Moroccan locust (Dociostaurus maroccanus), Australian plague locust (Chortoicetes terminifera), red locust (Nomadacris septemfasciata), Brown Locust (Locustana pardalina), Tree Locust (Anacridium melanorhodon), Italian Locust (Calliptamus italicus), Differential grasshopper (Melanoplus differentialis), Two striped grasshopper (Melanoplus bivittatus), Migratory grasshopper (Melanoplus sanguinipes), Red-Legged grasshopper (Melanoplus femurrubrum), Clearwinged grasshopper (Camnula pellucida), desert locust (Schistocerca gregaria), Yellow-winged locust (Gastrimargus musicus), Spur-throated locust (Austracris guttulosa), Japanese grasshopper (Oxya yezoensis), rice grasshopper (Oxya japonica), and Bombay locust (Patanga succincta); Gryllotalpidae, such as oriental mole cricket (Gryllotalpa orientalis); Gryllidae, such as house cricket (Acheta domestica) and emma field cricket (Teleogryllus emma); and Tettigoniidae, such as Mormon cricket (Anabrus simplex)}.

Blattodea {for example, Blattellidae, such as German cockroach (Blattella germanica); Blattidae, such as smoky-brown cockroach (Periplaneta fuliginosa), American cockroach (Periplaneta americana), brown cockroach (Periplaneta brunnea), oriental cockroach (Blatta orientalis), Japanese cockroach (Periplaneta japonica), and Australian cockroach (Periplaneta australasiae); and Termitidae, such as Japanese termite (Reticulitermes speratus), Formosan subterranean termite (Coptotermes formosanus), western drywood termite (Incisitermes minor), Cryptotermes domesticus, black-winged subterranean termite (Odontotermes formosanus), Neotermes koshunensis, Glyptotermes satsumensis, Glyptotermes nakajimai, Glyptotermes fuscus, Hodotermopsis sjostedti, Coptotermes guangzhouensis, Reticulitermes amamianus, Reticulitermes miyatakei, Reticulitermes kanmonensis, Nasutitermes takasagoensis, Pericapritermes nitobei, Sinocapritermes mushae, and Cornitermes cumulans}.

Acari {for example, Tetranychidae, such as common red spider mite (Tetranychus urticae), kanzawa spider mite (Tetranychus kanzawai), red spider mite (Tetranychus evansi), citrus red mite (Panonychus citri), fruit-tree red spider mite (Panonychus ulmi), and Oligonychus spp.; Eriophyidae, such as Japanese citrus rust mite (Aculops pelekassi), Phyllocoptruta citri, tomato rust mite (Aculops lycopersici), ribbed tea mite (Calacarus carinatus), tea rust mite (Acaphylla theavagrans), Japanese pear rust mite (Eriophyes chibaensis), apple bud mite (Aculus schlechtendali), persimmon bud mite (Aceria diospyri), Aceria tosichella, and Shevtchenkella sp.; Tarsonemidae, such as broad mite (Polyphagotarsonemus latus); Tenuipalpidae, such as Red and black flat mite (Brevipalpus phoenicis); Tuckerellidae; Ixodidae, such as bush tick (Haemaphysalis longicornis), northern tick (Haemaphysalis flava), Dermacentor taiwanensis, American dog tick (Dermacentor variabilis), Yamato tick (Ixodes ovatus), taiga tick (Ixodes persulcatus), black-legged tick (Ixodes scapularis), lone star tick (Amblyomma americanum), cattle tick (Boophilus microplus), and brown dog tick (Rhipicephalus sanguineus); Acaridae, such as cereal mite (Tyrophagus putrescentiae) and grassland mite (Tyrophagus similis); Pyroglyphidae, such as American house dust mite (Dermatophagoides farinae) and European house dust mite (Dermatophagoides pteronyssinus); Cheyletidae, such as Cheyletus eruditus, Cheyletus malaccensis, Cheyletus moorei, and Cheyletiella yasguri; Sarcoptidae, such as ear mange mite (Otodectes cynotis) and itch mite (Sarcoptes scabiei); Demodicidae, such as dog follicle mite (Demodex canis); Listrophoridae; Haplochthoniidae; Macronyssidae, such as tropical rat mite (Ornithonyssus bacoti) and feather mite (Ornithonyssus sylviarum); Dermanyssidae, such as bird mite (Dermanyssus gallinae); and Trombiculidae, such as Leptotrombidium akamushi}.

Plant parasitic nematodes {for example, nematodes of Aphelenchida such as rice white tip nematode (Aphelenchoides besseyi), strawberry foliar nematode (Aphelenchoides fragariae), chrysanthemum foliar nematode (Aphelenchoides ritzemabosi), and pine wood nematode (Bursaphelenchus xylophilus); and nematodes of Tylenchida such as white potato cyst nematode (Globodera pallida), potato cyst nematode (Globodera rostochiensis), cereal cyst nematode (Heterodera avenae), soybean cyst nematode (Heterodera glycines), sugarbeet cyst nematode (Heterodera schachtii), clover cyst nematode (Heterodera trifolii), peanut root-knot nematode (Meloidogyne arenaria), northern root-knot nematode (Meloidogyne hapla), southern root-knot nematode (Meloidogyne incognita), Javanese root-knot nematode (Meloidogyne javanica), apple root-knot nematode (Meloidogyne mali), coffee root-lesion nematode (Pratylenchus coffeae), root-lesion nematode (Pratylenchus drenatus), tea root-lesion nematode (Pratylenchus loosi), California root-lesion nematode (Pratylenchus neglectus), northern root-lesion nematode (Pratylenchus penetrans), walnut root-lesion nematode (Pratylenchus vulnus), citrus burrowing nematode (Radopholus citrophilus), and banana burrowing nematode (Radopholus similis)}.

Insect species to be controlled in the present invention (insect species on which the compound represented by formula (1) exhibits a control effect) also include pests such as a sanitary insect pest, a stored grain insect pest, a clothing insect pest, a house insect pest, and a parasite. The compound has an excellent control effect particularly on an ectoparasite that harms a human and an animal. Ectoparasites to be controlled include those that parasitize the back, armpits, lower abdomen, inner thighs, or the like of a host animal and live by obtaining a nutrient source such as blood or dander from the animal or bird, and those that fly to the back, buttocks, or the like of a host animal and live by obtaining a nutrient source such as blood or dander from the animal or bird. Examples of the ectoparasites include a mite, a louse, and a flea.

Examples of the host animal for which the control agent of the present invention is effective include a dog, a cat, a mouse, a rat, a hamster, a guinea pig, a squirrel, a rabbit, and a ferret; a pet bird (for example, a pigeon, a parrot, a hill myna, a Java sparrow, a parakeet, a Japanese pine, or a canary); cattle, a horse, a pig, a sheep, and a goat; poultry (for example, a duck, a chicken, a quail, or a goose); and a honey bee (for example, a Western honey bee or a Japanese honey bee).

That is, the pest control agent of the present invention is effective as an animal ectoparasite control agent intended for protection of the above animals and birds.

Examples of target Acari include the following insect pests.

Mites of Mesostigmata {for example, Dermanyssidae, such as chicken mite (Dermanyssus gallinae); mites of Macronyssidae, including northern fowl mite (Ornithonyssus sylviarum), tropical fowl mite (Ornithonyssus bursa), and tropical rat mite (Ornithonyssus bacoti) of Ornithonyssus spp.; mites of Laelapidae, including spiny rat mite (Laelaps echidninus) and Laelaps jettmari of Laelaps spp., and Tropilaelaps clarae; and mites of Varroidae, including Varroa mite (Varroa destructor), Varroa jacobsoni, and Varroa underwoodi of Varroa spp.}.

Ticks of Metastigmata {for example, ticks of Argasidae, including fowl tick (Argas persicus) and Argas reflexus of Argas spp. and Ornithodoros moubata of Ornithodoros spp.; and ticks of Ixodidae, including Haemaphysalis concinna, Haemaphysalis punctata, Haemaphysalis cinnabarina, Haemaphysalis otophila, Haemaphysalis leachi, Asian long-horned tick (Haemaphysalis longicornis), Haemaphysalis mageshimaensis, Haemaphysalis yeni, Haemaphysalis campanulata, Haemaphysalis pentalagi, Haemaphysalis flava, Haemaphysalis megaspinosa, Haemaphysalis japonica, and Haemaphysalis douglasi of Haemaphysalis spp., Amblyomma americanum, Amblyomma variegatum, Amblyomma maculatum, Amblyomma hebraeum, Amblyomma cajennense, and Amblyomma testudinarium of Amblyomma spp., Ixodes ricinus, Ixodes hexagonus, Ixodes canisuga, Ixodes pilosus, Ixodes rubicundus, Ixodes scapularis, Ixodes holocyclus, Ixodes ovatus, Ixodes persulcatus, and Ixodes nipponensis of Ixodes spp., cattle tick (Rhipicephalus (Boophilus) microplus), Rhipicephalus (Boophilus) decoloratus, Rhipicephalus (Boophilus) annulatus, and Rhipicephalus (Boophilus) calceratus of Boophilus spp., Rhipicephalus evertsi, brown dog tick (Rhipicephalus sanguineus), Rhipicephalus bursa, Rhipicephalus appendiculatus, Rhipicephalus capensis, Rhipicephalus turanicus, and Rhipicephalus zambeziensis of Rhipicephalus spp., and Dermacentor marginatus, Dermacentor reticulatus, Dermacentor pictus, Dermacentor albipictus, Dermacentor andersoni, and Dermacentor variabilis of Dermacentor spp.}.

Acaridida of Astigmata {for example, mites of Psoroptidae, including sheep scab mite (Psoroptes ovis), rabbit ear mite (Psoroptes cuniculi), and horse psoroptic mange mite (Psoroptes equi) of Psoroptidae spp., chorioptic mange mite (Chorioptes bovis) of Chorioptes spp., and ear mange mite (Otodectes cynotis) of Otodectes spp.; mites of Sarcoptidae, including itch mite (Sarcoptes scabiei), dog itch mite (Sarcoptes canis), cattle itch mite (Sarcoptes bovis), sheep itch mite (Sarcoptes ovis), Sarcoptes rupicaprae, horse itch mite (Sarcoptes equi), and pig itch mite (Sarcoptes suis) of Sarcoptes spp., and cat mange mite (Notoedres cati) of Notoedres spp.; and mites of Knemidokoptidae, including scaly leg mite (Knemidokoptes mutans) of Knemidokoptes spp.}.

Actinedida of Prostigmata {for example, mites of Demodixidae, including dog follicle mite (Demodex canis), cattle follicle mite (Demodex bovis), sheep follicle mite (Demodex ovis), goat follicle mite (Demodex caprae), horse follicle mite (Demodex equi), Demodex caballi, pig follicle mite (Demodex suis), and cat follicle mite (Demodex cati) of Demodex spp.; and mites of Trombiculidae, including Trombicula alfreddugesi and Trombicula akamushi of Trombicula spp.}.

Examples of target Phthiraptera include the following insect pests.

Lice of Anoplura {for example, lice of Haematopinidae, including horse sucking louse (Haematopinus asini), short-nosed cattle louse (Haematopinus eurysternus), and hog louse (Haematopinus suis) of Haematopinus spp.; and lice of Linognathidae, including dog sucking louse (Linognathus setosus), long-nosed cattle louse (Linognathus vituli), Linognathus ovillus, Linognathus oviformis, Linognathus pedalis, and goat sucking louse (Linognathus stenopsis) of Linognathus spp. and hairy cattle louse (Solenopotes capillatus) of Solenopotes spp.}.

Biting lice of Amblycera {for example, biting lice of Menoponidae, including chicken body louse (Menacanthus stramineus), Menacanthus cornutus, and Menacanthus pallidulus of Menacanthus spp., and for example, shaft louse (Menopon gallinae) of Menopon spp.}.

Biting lice of Ischnocera {for example, biting lice of Philopteridae, including slender pigeon louse (Columbicola columbae) of Columbicola spp., chicken head louse (Cuclotogaster heterographus) of Cuclotogaster spp., brown chicken louse (Goniodes dissimilis), large chicken louse (Goniodes gigas), and Goniodes gallinae of Goniodes spp., and wing louse (Lipeurus caponis) of Lipeurus spp.; and biting lice of Trichodectidae, including cattle biting louse (Bovicola bovis), sheep biting louse (Bovicola ovis), Bovicola limbata, goat biting louse (Bovicola caprae), and horse biting louse (Bovicola equi) of Bovicola spp., dog biting louse (Trichodectes canis) of Trichodectes spp., and cat biting louse (Felicola subrostrata) of Felicola spp.}.

Examples of target Siphonaptera include the following insect pests. For example, fleas of Tungidae, including chigoe flea (Tunga penetrans) of Tunga spp.; fleas of Pulicidae, including dog flea (Ctenocephalides canis) and cat flea (Ctenocephalides felis) of Ctenocephalides spp., hedgehog flea (Archaeopsylla erinacei) of Archaeopsylla spp., oriental rat flea (Xenopsylla cheopis) of Xenopsylla spp., human flea (Pulex irritans) of Pulex spp., and sticktight flea (Echidnophaga gallinacea) of Echidnophaga spp.; fleas of Ceratophyllidae, including European chicken flea (Ceratophyllus gallinae) and Ceratophyllus anisus of Ceratophyllus spp., and European rat flea (Nosopsyllus fasciatus) of Nosopsyllus spp.; and fleas of Leptopsyllidae, including European mouse flea (Leptopsylla segnis) of Leptopsylla spp.

Other target ectoparasites include insect pests of Hemiptera. Examples of insect pests of Hemiptera include the following. For example, insects of Cimicidae, including common bed bug (Cimex lectularius) of Cimex spp.; and insects of Reduviidae, including Panstrongylus spp., kissing bug (Rhodnius prolixus) of Rhodnius spp., and barber bug (Triatoma infestans) of Triatoma spp.

The present invention is also effective against insect pests of Diptera, which are biting insects (chewing flies, blood-sucking adult flies, mobile dipteran larvae, and parasitic fly maggots). Examples of insect pests of Diptera include the following.

### Nematocera

{For example, (a) mosquitoes of Culicidae, including southern house mosquito (Culex quinquefasciatus), northern house mosquito (Culex pipiens pallens), Culex tarsalis, London Underground mosquito (Culex pipiens molestus), tropical house mosquito (Culex pipiens fatigans), and Culex tritaeniorhynchus summorosus of Culex spp., Armigeres subalbatus of Armigeres spp., African malaria mosquito (Anopheles gambiae), Anopheles maculipennis, Chinese malaria mosquito (Anopheles sinensis), and Anopheles lesteri of Anopheles spp., and yellow fever mosquito (Aedes aegypti), Asian tiger mosquito (Aedes albopictus), Aedes taeniorhynchus, Aedes togoi, and Aedes vexans nipponii of Aedes spp.; and (b) black flies of Simuliidae, including Simulium reptans, Simulium ornatum, white-stockinged black fly (Simulium venustum), and Simulium salopiense of Simulium spp., and Prosimulium yezoense of Prosimulium spp.; and biting midges of Ceratopogonidae, including Culicoides arakawae, Culicoides pictimargo, Culicoides kibunensis, Culicoides homotomus, Culicoides oxystoma, Culicoides nipponensis, Culicoides punctatus, Culicoides maculatus, and Culicoides matsuzawai of Culiodes spp.}.

### Brachycera

{For example, (a) horse flies of Tabanidae, including Tabanus bromius, Tabanus spodopterus, Tabanus atratus, Tabanus sudeticus, Tabanus trigonus, Tabanus chrysurus, Tabanus trigeminus, Tabanus fulvimedioides, and Tabanus iyoensis of Tabanus spp., and Chrysops caecutiens, Chrysops relictus, Chrysops suavis, and Chrysops japonicus of Chrysops spp.; flies of Muscidae, including house fly (Musca domestica), Musca bezzii, Musca hervei, Musca conducens, and false stable fly (Musca stabulans) of Muscina spp., stable fly (Stomoxys calcitrans) of Stomoxys spp., horn fly (Haematobia irritans), Haematobia irritans exigua, and Haematobia stimulans of Haematobia spp., and little house fly (Fannia canisularis) of Fannia spp.; flies of Glossinidae, including Glossina spp.; flies of Hippoboscidae, including sheep ked (Melophagus ovinus) of Melophagus spp.; flies of Calliphoridae, including Calliphora lata of Calliphora spp., Australian sheep blowfly (Lucilia (Phaenicia) cuprina), common green bottle fly (Lucilia (Phaenicia) sericata), and green bottle fly (Lucilia illustris) of Lucilia spp., and Chrysomya hominivorax, Chrysomya chloropyga, and Chrysomya bezziana of Chrysomyia. spp.; and flies of Oestridae, including Cuterebra spp. of Cuterebrinae, northern cattle grub (Hypoderma bovis) and common cattle grub (Hypoderma lineatum) of Hypoderma spp. of Hypodermatinae, horse bot fly (Gasterophilus intestinalis), nose botfly (Gasterophilus haemorroidalis), Gasterophilus inermis, throat botfly (Gasterophilus nasalis), Gasterophilus nigricornis, and dark-winged horse botfly (Gasterophilus pecorum) of Gasterophilus spp. of Gasterophilinae, and sheep nasal botfly (Oestrus ovis) of Oestrus spp. of Oestrinae}.

When the compound represented by formula (1) is used as a pest control agent for agriculture and horticulture, the compound represented by formula (1) may be used as it is, and may be mixed with a suitable carrier such as a solid carrier, a liquid carrier, or a gaseous carrier, formulation adjuvant such as a surfactant or a dispersant, or the like to prepare an agrochemical formulation before use. Preferable examples of the agrochemical formulation include an emulsifiable concentrate, an EW, a soluble concentrate, a suspension, a wettable powder, a wettable powder, a dust, a DL dust, a dust-granule mixture, a granule, a tablet, an oil miscible liquid, an aerosol, a flowable, a dry flowable, and a microcapsule. The compound can be used as a formulation type arbitrarily selected from these agrochemical formulations. The carrier in the present invention refers to a solid carrier, a liquid carrier, a gaseous carrier, or the like.

Examples of the solid carrier include talc, bentonite, clay, kaolin, diatomaceous earth, vermiculite, white carbon, calcium carbonate, acid clay, silica sand, silica stone, zeolite, pearlite, attapulgite, pumice, ammonium sulfate, sodium sulfate, and urea.

Examples of the liquid carrier include an alcohol such as methanol, ethanol, n-hexanol, ethylene glycol, or propylene glycol, a ketone such as acetone, methyl ethyl ketone, or cyclohexanone, an aliphatic hydrocarbon such as n-hexane, kerosine, or kerosene, an aromatic hydrocarbon such as toluene, xylene, or methylnaphthalene, an ether such as diethyl ether, dioxane, or tetrahydrofuran, an ester such as ethyl acetate, a nitrile such as acetonitrile or isobutyronitrile, an acid amide such as dimethylformamide or dimethylacetamide, a vegetable oil such as soybean oil or cottonseed oil, dimethyl sulfoxide, and water.

In addition, examples of the gaseous carrier include LPG, air, nitrogen, carbon dioxide, and dimethyl ether.

Examples of the surfactant and the dispersant include an alkyl sulfate, an alkyl(aryl)sulfonate, a polyoxyalkylene alkyl(aryl) ether, a polyhydric alcohol ester, a lignosulfonate, an alkylsulfosuccinate, a formalin condensate of an alkylnaphthalenesulfonate, a polycarboxylate, a POE polystyrylphenyl ether sulfate and phosphate, and a POE-POP block polymer.

Furthermore, examples of the formulation adjuvant include carboxymethyl cellulose, hydroxypropyl cellulose, polyvinyl alcohol, xanthan gum, pregelatinized starch, gum arabic, polyvinylpyrrolidone, ethylene-acrylic acid copolymer, ethylene-vinyl acetate copolymer, polyethylene glycol, liquid paraffin, calcium stearate, an antifoaming agent, and an antiseptic.

The various carriers, surfactants, dispersants, and formulation adjuvants described above can be used on its own or in combination, if necessary.

The content of the compound represented by formula (1) as an active ingredient in the agrochemical formulation is not particularly limited and is preferably 1 to 75% by weight for an emulsifiable concentrate, 0.3 to 25% by weight for a dust, 1 to 90% by weight for a wettable powder, and 0.1 to 10% by weight for a granule.

When the compound represented by formula (1) is used as an acaricide for controlling a mite parasitic on a domestic animal such as cattle or a pig, or a pet such as a dog or a cat, the compound can be used in an amount of 0.01 to 1000 mg of the active ingredient per kg of the host animal.

An acaricide for pest control can be applied by using a known veterinary method. For example, when the purpose is systemic suppression, examples of the method include a method involving administering the same to an animal by using a tablet, a capsule, an immersion liquid, a feed mixture, a suppository, an injection (intramuscular, subcutaneous, intravenous, intraperitoneal, or the like), or the like, and when the purpose is non-systemic suppression, examples thereof include a method involving administering an oil-based or water-based soluble concentrate by spraying, pour-on, spot-on, or the like, and a method involving kneading an acaricide into a resin, forming the kneaded product into a suitable shape such as a collar or an ear tag, and attaching the same to an animal.

The pest control agent according to the present invention can be used as it is or after diluted. In addition, the pest control agent according to the present invention can be mixed with or used in combination with a further insecticide, nematicide, microbicide, acaricide, herbicide, plant growth regulator, fertilizer, or the like. Examples of an agent that can be mixed or used in combination include those disclosed in Pesticide Manual (18th edition, published by The British Crop Protection Council), SHIBUYA INDEX (17th edition, 2014, published by SHIBUYA INDEX RESEARCH GROUP), IRAC Mode of Action Classification Scheme (Mode of Action Classification Scheme Version 9.3, published by IRAC), and FRAC Code List (FRAC Code List (C) 2020: Fungicides sorted by mode of action, 2020 edition, published by FRAC), and those whose structures can be identified on the Internet (for example, http://www.alanwood.net/pesticides/sitemap.html).

More specifically, examples of the insecticide, nematicide, and acaricide include the following compounds.

Carbamate-based compounds such as alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, oxamyl, pirimicarb, propoxur, thiodicarb, thiofanox, triazamate, trimethacarb, XMC, xylylcarb, metolcarb, fenothiocarb, and fenoxycarb.

Organophosphate ester-based compounds such as acephate, azamethiphos, azinphosethyl, azinphos-methyl, ethylthiometon, chlorethoxyfos, cadusafos, chlorethoxyfos, chlorfenvinphos, chlormephos, chlorpyrifos, chlorpyrifos-methyl, coumaphos, cyanophos, demeton-S-methyl, diazinon, dichlorvos, dicrotophos, dimethoate, dimethylvinphos, EPN, ethion, etoprophos, famphur, fenamifos, fenitrothion, fenthion, fosthiazate, heptenophos, imicyafos, isofenphos, isopropyl O-(methoxyaminothiophosphoryl)salicylate, isoxathion, malathion, mecarbam, methamidophos, methidathion, mevinphos, monocrotophos, naled, omethoate, dioxydemeton ethyl), parathion, parathion-methyl, PAP, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion, chlorpyrifos-ethyl, disulfoton, sulprofos, flupyrazophos, phenthoate, fonofos, and tribufos.

Organochlorine-based compounds such as endosulfan, alpha-endosulfan, gamma-HCH, dicofol, chlordane, dieldrin, and methoxychlor.

Phenylpyrazole-based compounds such as acetoprole, fipronil, ethiprole, pyrafluprole, pyriprole, flufiprole, and nicofluprole.

Metadiamide-based compounds such as broflanilide and cyproflanilide.

Isoxazoline-based compounds such as afoxolaner, fluralaner, sarolaner, fluxametamide, lotilaner, and isocycloseram.

Pyrethroid-based compounds such as acrinathrin, allethrin, d-cis-trans allethrin, d-trans allethrin, bifenthrin, kappa-bifenthrin, bioallethrin S-cyclopentenyl, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin, deltamethrin, empenthrin, esfenvalerate, ethofenprox, fenpropathrin, fenvalerate, flucythrinate, flumethrin, tau-fluvalinate, halfenprox, imiprothrin, kadethrin, permethrin, phenothrin, prallethrin, pyrethrin, resmethrin, silafluofen, tefluthrin, kappa-tefluthrin, phthalthrin, tetramethrin, tralomethrin, transfluthrin, metoxadiazone, metofluthrin, profluthrin, pyrethrum, terallethrin, momfluorothrin, heptafluthrin, meperfluthrin, tetramethylfluthrin, dimefluthrin, and protrifenbut.

Neonicotinoid compounds such as acetamiprid, chlothianidin, dinotefuran, imidacloprid, nitenpyram, thiacloprid, and thiamethoxam.

Sulfoxamine-based compounds such as sulfoxaflor.

Butenolide-based compounds such as flupyradifurone.

Mesoionic compounds such as triflumezopyrim, dicloromezotiaz, and fenmezoditiaz.

2-Aminopyridine-based compounds such as flupyrimin.

Spinosyn-based compounds such as spinosad and spinetoram.

Macrolide-based compounds such as abamectin, ivermectin, emamectin benzoate, milbemectin, and lepimectin.

Juvenile hormone-like compounds such as hydroprene, quinoprene, Diofenolan, and methoprene.

4-Phenoxyphenoxy-based compounds such as pyriproxyfene.

Pyridine azomethine-based compounds such as pymetrozine.

Pyridinecarboxamide-based compounds such as flonicamid.

Oxazole-based compounds such as ethoxazole.

Formulations of Bacillus thuringiensis and Bacillus sphaericus such as B.t. subsp. israelensis, B.t. subsp. aizawai, B.t. subsp. kurstaki, and B.t. subsp. tenebrionis, and insecticidal proteins produced thereby. Insecticidal proteins produced by Bt crops that fall under the above.

Thiourea-based compounds such as diafenthiuron.

Organic metal-based compounds such as azocyclotin, cyhexatin, and fenbutatin oxide.

Sulfite ester-based and diphenyl ether-based compounds such as propargite.

Diphenylsulfone-based compounds such as tetradifon.

Pyrrole-based compounds such as chlorfenapyr and tralopyril.

Dinitro-based compounds such as DNOC.

Nereistoxin analogs such as bensultap, cartap, thiocyclam, thiosultap, and thiosultap sodium.

Benzoylurea-based compounds such as bistrifluron, chlorfluazuron, diflubenzuron, flucycloxuron, flufenoxuron, hexaflumuron, lufenuron, novaluron, noviflumuron, teflubenzuron, triflumuron, and bistrifluron, and thiadiazine-based compounds such as buprofezin.

Triazole-based compounds such as cyromazine.

Diacylhydrazine-based compounds such as chromafenozide, halofenozide, methoxyfenozide, and tebufenozide.

Amidine-based compounds such as amitraz.

Amidinohydrazone-based compounds such as hydramethylnon.

Naphthoquinone-based compounds such as acequinocyl.

Strobilurin-based compounds such as fluacrypyrim, pyriminostrobin, and Flufenoxystrobin.

Quinazoline-based compounds such as fenazaquin.

Phenoxypyrazole-based compounds such as fenpyroxymate.

Phenoxyethylamine-based compounds such as pyrimidifen.

Pyridazinone-based compounds such as pyridaben.

Pyrazole-4-carboxamide-based compounds such as tebufenpyrad, tolfenpyrad, pyflubumide, and dimpropyridaz.

Hydrazinecarboxamide-based compounds such as metaflumizone.

Tetronic acid and tetramic acid-based compounds such as spirodiclofen, spirotetaramat, spiromesifen, spiropidion, and spidoxamat.

Beta-ketonitrile-based compounds such as cyflumetofen, cyenopyrafen, and cyetpyrafen.

Phthalic acid amide-based compounds such as flubendiamide.

Anthranilic acid amide-based compounds such as chlorantraniliprole, cyantraniliprole, cyclaniliprole, tetraniliprole, cyhalodiamide, tetrachlorantraniliprole, and fluchlordiniliprole.

Quinoxaline-based compounds such as quinomethionate.

Thiazolidinone-based compounds such as hexythiazox.

Hydrazine-based compounds such as bifenazate.

Pyridinamine-based compounds such as flufenerim.

Aminoquinazoline-based compounds such as pyrifluquinazon.

6-Phenoxyquinoline-based compounds such as flometoquin.

Pyridinylethylbenzamide-based compounds such as fluopyram.

Pyridinylcyclobutynylamide-based compounds such as cyclobutrifluram.

Sulfonamide-based compounds such as fluazaindolizine and amidoflumet.

Pyridylpyrazole-based compounds such as tyclopyrazoflor.

Oxadiazole-based compounds such as thioxazafen.

Benzoxazole-based compounds such as oxazosulfyl.

Pyridylindazole-based compounds such as indazapyroxamet.

Butyrolactone-based compounds such as trifluenfuronate.

In addition, further examples of the insecticide, nematicide, and acaricide include a compound such as nicotine, chloropicrin, sulfuryl fluoride, crylotie, clofentezine, diflovidazin, rotenone, indoxacarb, piperonyl butoxide, chlordimeform, pyridalyl, azadirachtin, benzoxymate, afidopyropen, fluhexafon, fluensulfone, benclothiaz, carzole, insecticidal soap, dimehypo, nithiazine, a borate salt, metaaldehyde, ryanodine, sulfluramid, acynonapyr, benzpyrimoxan, 3-bromo-N-(2,4-dichloro-6-(methylcarbamoyl)phenylyl)-1-(3,5-dichloropyridin-2-yl)-1H-pyrazole-5-carboxamide, or flupentiophenox. Furthermore, the pest control agent according to the present invention can also be mixed or used in combination with a microbial pesticide such as an entomopathogenic bacterium, an entomopathogenic virus, or an entomopathogenic fungus.

Examples of the microbicide used include the following.

Phenylamide-based compounds such as metalaxyl, metalaxyl-M, oxadixyl, ofurase, benalaxyl, benalaxyl-M, kiralaxyl, ofurace, furalaxyl, and cyprofuram, and hydroxypyrimidinebased compounds such as bupyrimate, dimethilimol, and ethilimol.

Isoxazole-based compounds such as hymexazole and hydroxyisoxazole, piperidinyl thiazole isoxazoline-based compounds such as oxathiapiprolin and fluoxapiprolin, isothiazolonebased compounds such as octhilinone, and carboxylic acid-based compounds such as oxolinic acid.

Benzimidazole and thiophanate-based compounds such as benomyl, thiophanatemethyl, carbendazole, fuberidazole, thiabendazole, and debacarb.

N-phenylcarbamate-based compounds such as diethofencarb.

Toluamide-based compounds such as zoxamide.

Ethylaminothiazolecarboxamide-based compounds such as ethaboxam.

Phenylurea-based compounds such as pencycuron.

Pyridinylmethylbenzamide-based compounds such as fluopicolide and fluopimomide.

Pyrimidinamine-based compounds such as diflumetorim and bupirimate.

Benzanilide-based compounds such as benodanil, flutolanil, mepronil, and flufenoxadiazam.

Phenyloxoethylthiophenamide-based compounds such as isofetamid.

Pyridinylethylbenzamide-based compounds such as fluopyram.

Furancarboxamide-based compounds such as fenfuram.

Oxathiincarboxamide-based compounds such as oxycarboxin and carboxin.

Thiazolecarboxamide-based compounds such as thifluzamide.

Pyrazole-4-carboxamide-based compounds such as fluxapyroxad, furametpyr, penflufen, penthiopyrad, benzovindiflupyr, bixafen, isopyrazam, sedaxane, inpyrfluxam, fluindapyr, isoflucypram, pyrapropoyne, and flubeneteram.

Pyridinecarboxamide-based compounds such as boscalid.

Strobilurin-based compounds such as azoxystrobin, coumetoxystrobin, kresoxymmethyl, trifloxystrobin, picoxystrobin, pyraclostrobin, dimoxystrobin, metominostrobin, orysastrobin, fluoxastrobin, pyraoxystrobin, pyrametostrobin, flufenoxystrobin, fenaminstrobin, enoxastrobin, coumoxystrobin, mandestrobin, and triclopyricarb.

Oxazolidinedione-based compounds such as famoxadon.

Imidazolinone-based compounds such as fenamidone.

Benzylcarbamate-based compounds such as triclopyricarab and pyribencarb.

Cyanoimidazole-based compounds such as cyazofamid.

Sulfamoyltriazole-based compounds such as amisulbrom.

Dinitrophenyl croton-based compounds such as binapacryl, meptyldinocap, and dinocap.

2,6-Dinitroaniline-based compounds such as fluazinam.

Pyrimidinone hydrazone-based compounds such as ferimzone.

Organic and inorganic metal-based compounds such as fentin-acetate, fentin chloride, fentin hydroxide, triphenyltin hydroxide (fenthin hydroxide), triphenyltin acetate (fenthin acetate), and oxine copper.

Thiophenecarboxamide-based compounds such as silthiofam.

Triazolopyrimidinamine-based compounds such as ametoctradin.

Anilinopyrimidine-based compounds such as mepanipyrim, nitrapyrin, pyrimethanil, and cyprodinil.

Enopyranuronic acid antibiotics such as blasticidin-S.

Hexopyranosyl antibiotics such as kasugamycin and kasugamycin hydrochloride hydrate.

Glucopyranosyl antibiotics such as streptomycin.

Tetracycline antibiotics such as oxytetracycline.

Allyloxyquinoline-based compounds such as quinoxyfen.

Quinazoline-based compounds such as proquinazid.

Cyanopyrrole-based compounds such as fludioxonil and fenpiclonil.

Dicarboximide-based compounds such as fluoroimid, procymidone, iprodione, and vinchlozolin.

Phosphorothiolate-based compounds such as edifenphos, iprobenfos, and pyrazophos.

Dithiolane-based compounds such as isoprothiolane.

Propylcarbamate-based compounds such as propamocarb and propamocarb hydrochloride.

Bacillus spp. such as Bacillus subtilis (QST713, FZB24, MBI600, and D747 strains) and produced microbicidal proteins, and microbicidal proteins produced by the Bt crops.

Terpene hydrocarbons and terpene alcohols, such as Melaleuca alternifolia extract.

Piperazine-based compounds such as triforine.

Pyridine-based compounds such as pyrifenox and pyrisoxazole.

Pyrimidine-based compounds such as fenarimol, nuarimol, and flumetylsulforim.

Azole-based compounds such as azaconazole, bromuconazole, diniconazole, diniconazole-M, epoxyconazole, fluquinconazole, oxpoconazole, pefurazoate, difenoconazole, fenbuconazole, imibenconazole, ipconazole, metconazole, tetraconazole, triadimefon, triadimenol, triticonazole, uniconazole, imazalil, bitertanol, triflumizole, etaconazole, propiconazole, penconazole, flusilazole, flutriafol, myclobutanil, paclobutrazol, prothioconazole, cyproconazole, tebuconazole, hexaconazole, prochloraz, simeconazole, ipfentrifluconazole, and fluoxytioconazole.

Morpholine-based compounds such as aldimorph, dodemorph, dodemorph acetate, tridemorph, fenpropimorph, dimethomorph, flumorph, and pyrimorph.

Piperidine-based compounds such as piperalin and fenpropidin.

Spiroketalamine-based compounds such as spiroxamine.

Hydroxyanilide-based compounds such as fenhexamid.

Aminopyrazolinone-based compounds such as fenpyrazamine.

Thiocarbamate and dithiocarbamate-based compounds such as ferbam, metam, metasulphocarb, metiram, thiram, mancozeb, maneb, zineb, ziram, polycarbamate, propineb, thiuram, and pyributicarb.

Glucopyranosyl antibiotics such as validamycin.

Nucleoside-based antibiotics such as mildiomycin and polyoxin.

Valinamide carbamate-based compounds such as benthiavalicarb, benthiavalicarbisopropyl, valifenalate, and iprovalicarb.

Mandelic acid amide-based compounds such as mandipropamid.

Picolinamide-based compounds such as fenpicoxamid, florylpicoxamid, and metarylpicoxamid.

Isobenzofuranone-based compounds such as fthalide.

Pyrroloquinolinone-based compounds such as pyroquilone.

Triazolobenzothiazole-based compounds such as tricyclazole.

Cyclopropanecarboxamide-based compounds such as carpropamid.

Carboxamide-based compounds such as diclocymet.

Propionamide-based compounds such as fenoxanil.

Benzothiadiazole-based compounds such as acibenzolar-S-methyl.

Benzisothiazole-based compounds such as probenazole and dichlobentiazox.

Thiadiazolecarboxamide-based compounds such as tiadinil.

Isothiazolecarboxamide-based compounds such as isotianil.

Cyanoacetamide oxime-based compounds such as cymoxanil.

Ethylphosphonate-based compounds such as fosetyl.

Phthalamic acid-based compounds such as techlophthalam.

Benzotriazine-based compounds such as triazoxide.

Benzenesulfonic acid-based compounds such as flusulfamide.

Pyridazinone-based compounds such as diclomezine.

Phenylacetamide-based compounds such as cyflufenamide.

Benzophenone-based compounds such as metrafenopne.

Benzoylpyridine-based compounds such as pyriofenone.

Cyanomethylenethiazolidine-based compounds such as flutianil.

4-Quinolylacetic acid-based compounds such as tebufloquin.

3-Phenoxyquinoline-based compounds such as ipflufenoquin.

Organophosphorus-based compounds such as fosetyl-aluminium and tolclofos-methyl.

1,2,4-Thiadiazole-based compounds such as echlomezole.

Trifluoroethylcarbamate-based compounds such as tolprocarb.

Copper-based compounds such as Bordeaux mixture, copper acetate, basic copper sulfate, oxy copper chloride, copper hydroxide, oxyquinoline-copper (oxine-copper).

Inorganic compounds such as copper and sulfur.

N-Halogenothioalkyl-based compounds such as captan, captafol, and folpet.

Organochlorine-based compounds such as anilazine, chlorothalonil, dichlorophen, pentachlorophenol and salts thereof, hexachlorobenzene, and quintozene.

Guanidine-based compounds such as iminoctadine triacetate salt, iminoctadine albesilate, guanidine, dodine, dodine free base, guazatine, guazatine acetate salt, and albesilate.

Anthraquinone-based compounds such as dithianon.

Quinoxaline-based compounds such as quinomethionate.

Maleimide-based compounds such as fluoroimide, and sulfenic acid-based compounds such as tolylfluanid and dichlofluanid.

Dinitrophenol-based compounds such as dinobuton.

Cyclic dithiocarbamate-based compounds such as dazomet.

Anilide-based compounds such as pyraziflumid.

Nicotinic acid ester-based compounds such as aminopyrifen.

Tetrazolinone-based compounds such as methyltetraprole.

Pyridazine-based compounds such as pyridachlometyl.

Hydrazide-based compounds such as chloroinconazide.

In addition, further examples of the microbicide include dipymetitrone, picarbutrazox, tecnazen, nitrthal-isopropyl, dicyclomet, acibenzolar, prohexadione-calcium, bronopol, diphenylamine, flumetover, bethoxazin, biphenyl, chloroneb, CNA, iodcarb, prothiocarb, and seboxylamine.

The compound of the present invention represented by formula (1) or a salt thereof or an N-oxide thereof or an agriculturally, horticulturally, and veterinarily acceptable acid addition salt thereof (all of which are also referred to as the "compound of the present invention") can be used to control a target pest by applying an effective amount thereof to a plant, soil, or an animal. That is, a method for controlling a pest in these fields is provided. Here, the pest control method according to the present invention also includes a method involving applying the compound of the present invention by smoking treatment in a closed space.

The compound of the present invention can also enhance the vigor of a crop by treating or contacting the crop, a seed from which the crop grows, or the placenta of the crop with a biologically effective amount thereof.

When the subject to be treated or contacted is a seed, the amount of the compound of the present invention is not limited, and the compound of the present invention is preferably included in an amount of about 0.0001 to about 1% by mass of the whole seed after treatment.

The present invention further provides a use of the compound of the present invention as an active ingredient in a composition for protecting an animal or a bird from a harmful parasitic invertebrate. The composition includes the compound of the present invention in an amount that is parasiticidally effective and does not harm the target animal or bird. In the above use, the compound of the present invention is contacted with a harmful invertebrate or a habitat thereof in a biologically effective amount to control the harmful invertebrate.

Next, specific examples of the compound of the present invention will be shown below.

A compound represented by formula (1-1), which is formula (1) where B is B-7 and D is D-1, a salt thereof or an N-oxide thereof 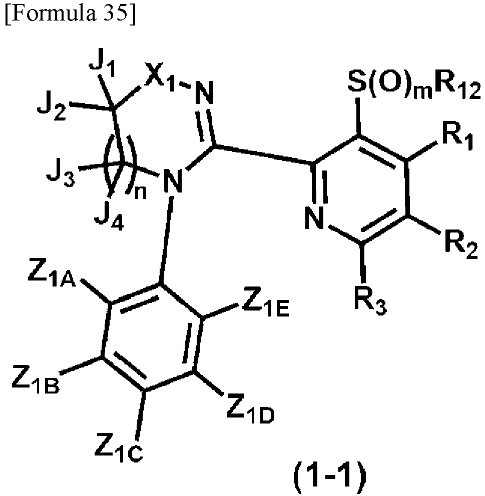 wherein
X₁ is an oxygen atom, a sulfur atom, NY₁, or C(J₅J₆), n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1C}, Z_{1D}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₁, R₂, and R₃ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group, a halo (C₂ to C₆) alkenyl group optionally having a substituent, a (C₂ to C₆) alkynyl group optionally having a substituent, a halo (C₂ to C₆) alkynyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-1), which is formula (1) where B is B-7 and D is D-1, Z_{1A}, Z_{1B}, Z_{1C}, Z_{1D}, and Z_{1E} are substituents shown in Table 1-1, Table 1-2, Table 1-3, Table 1-4, and Table 1-5, respectively, X₁ is a structure shown in Table 2, J₁ and J₂ are substituents shown in Table 3-1, J₃ and J₄ are substituents shown in Table 3-2, n is 0 or 1, R₁ is a substituent shown in Table 4, R₂ is a substituent shown in Table 5-1 to Table 5-5, R₃ is a substituent shown in Table 6-1 to Table 6-3, R₁₂ is a substituent shown in Table 7, and m is combined from 0, 1, and 2.

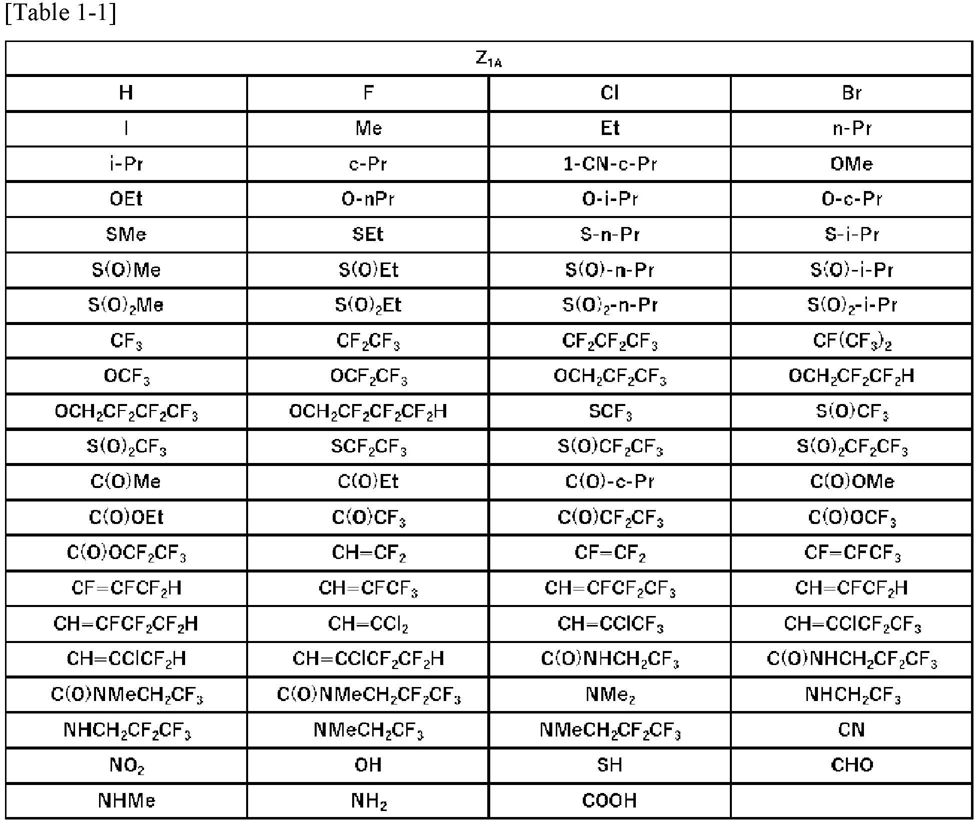

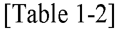

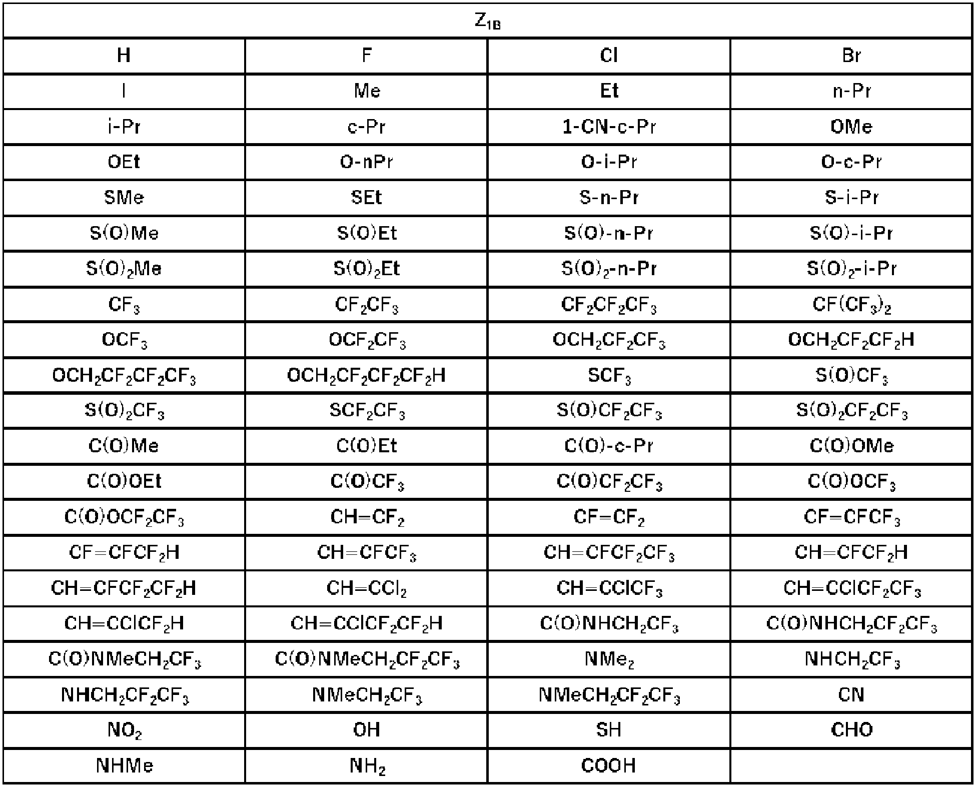

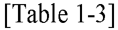

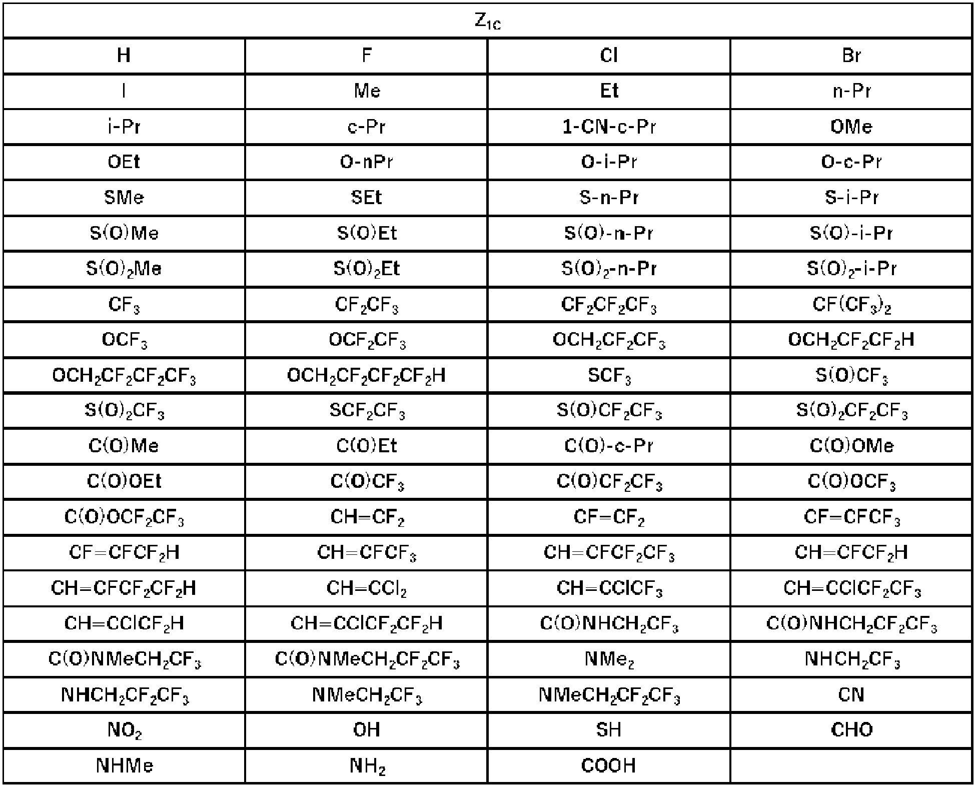

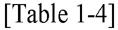

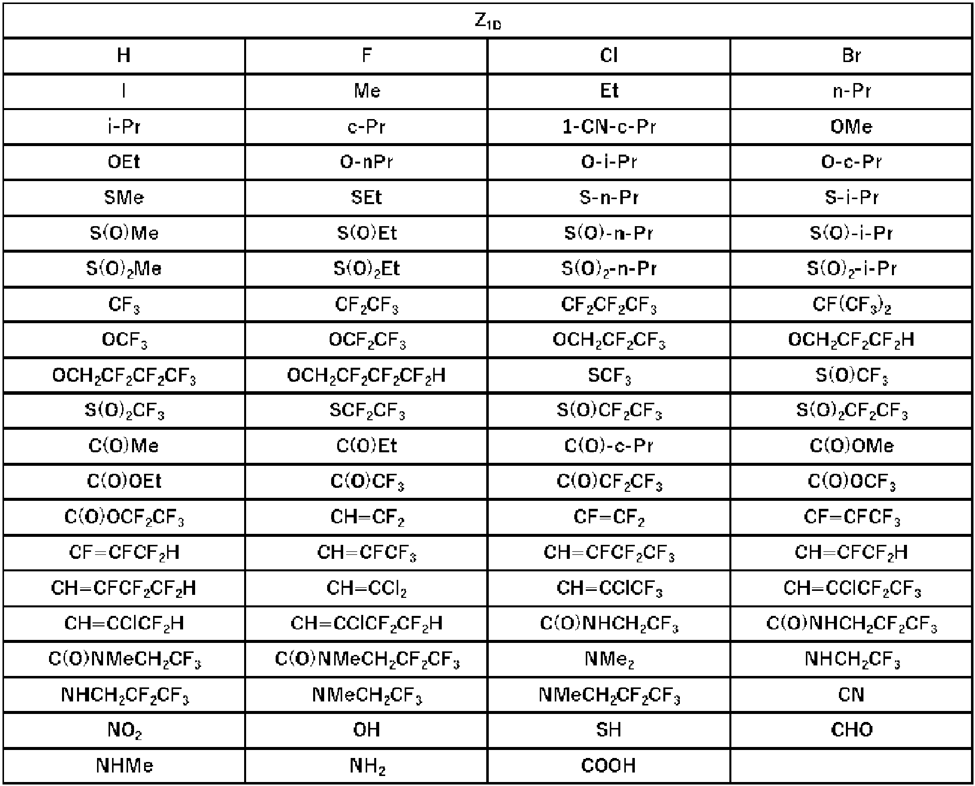

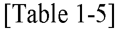

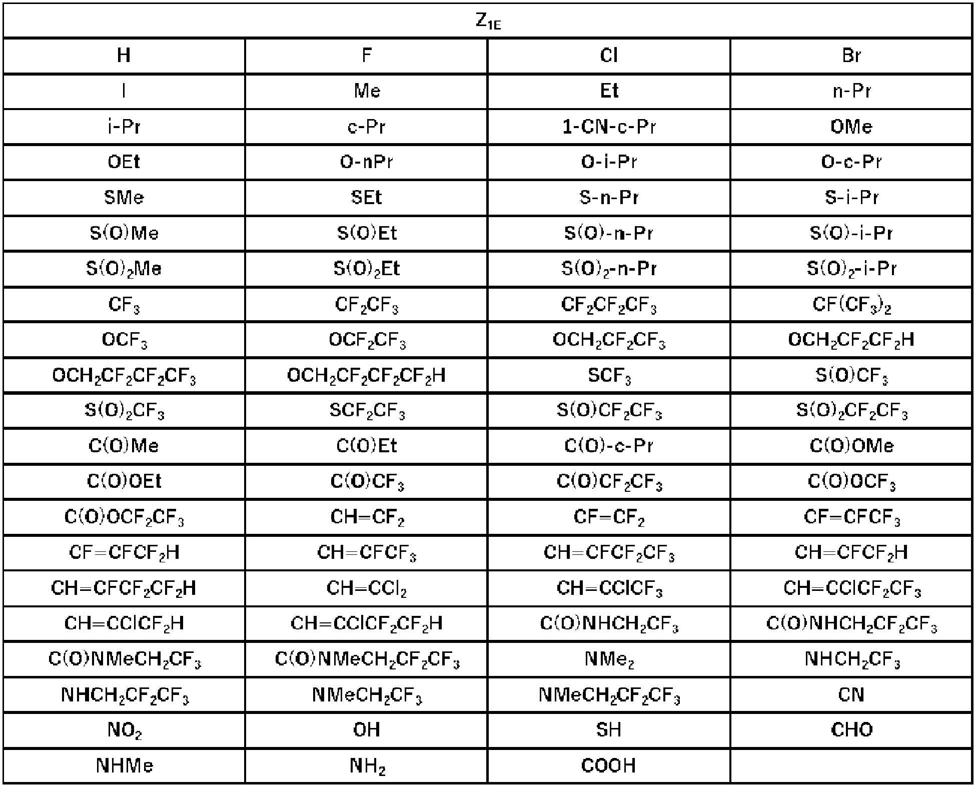

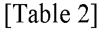

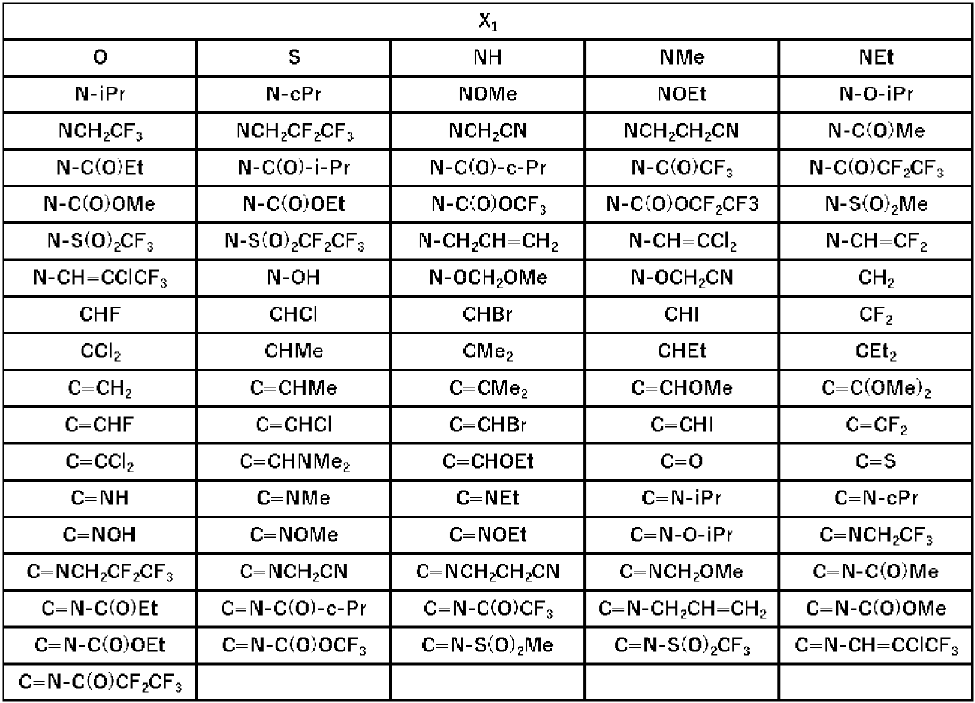

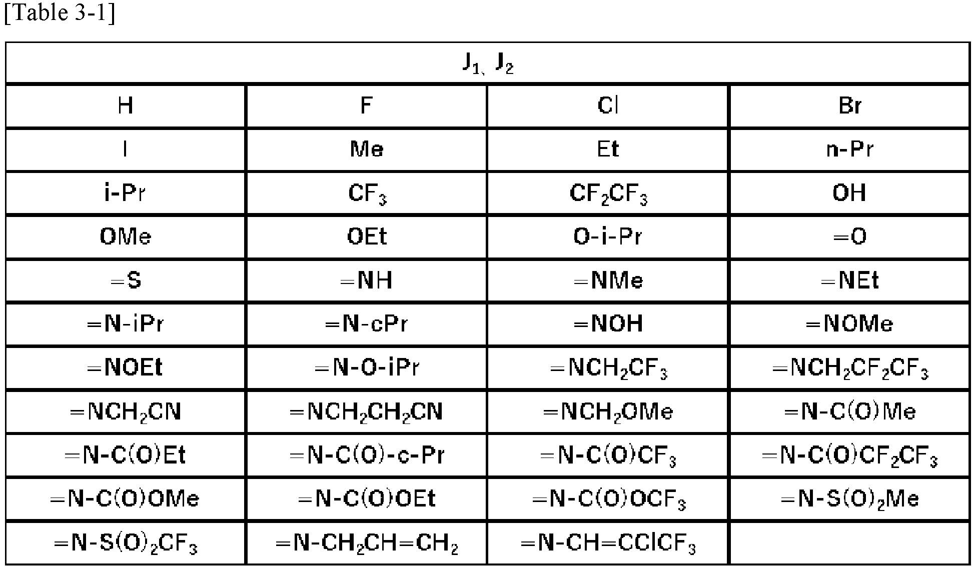

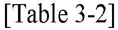

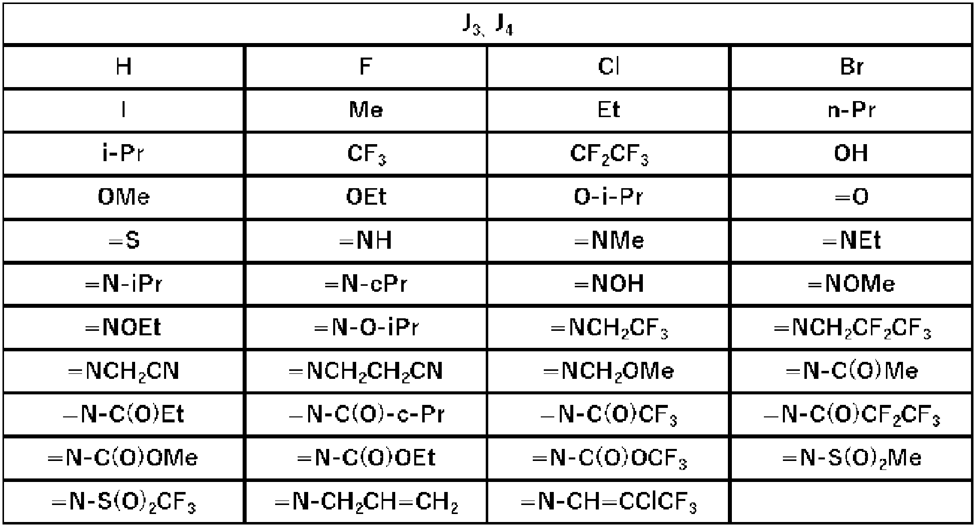

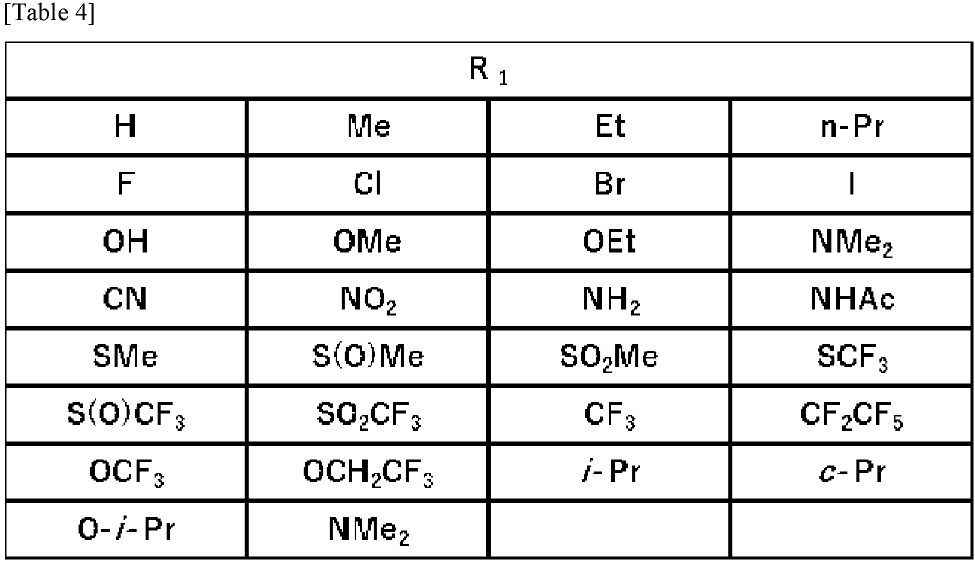

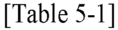

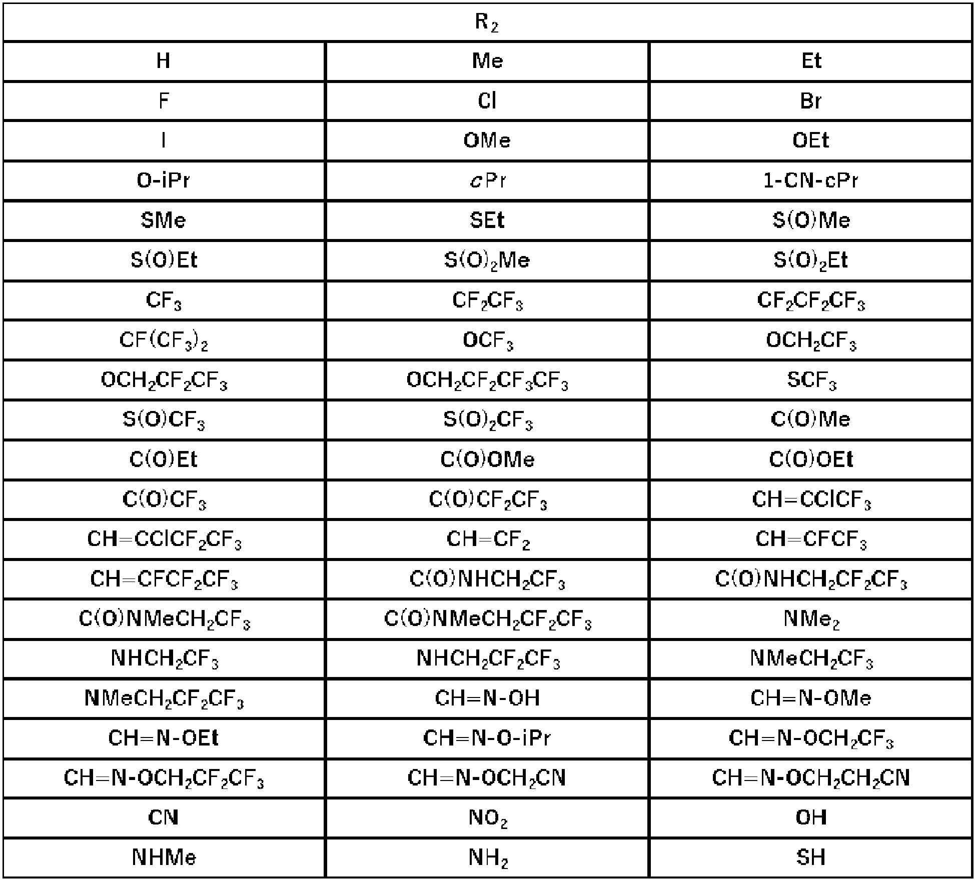

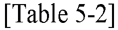

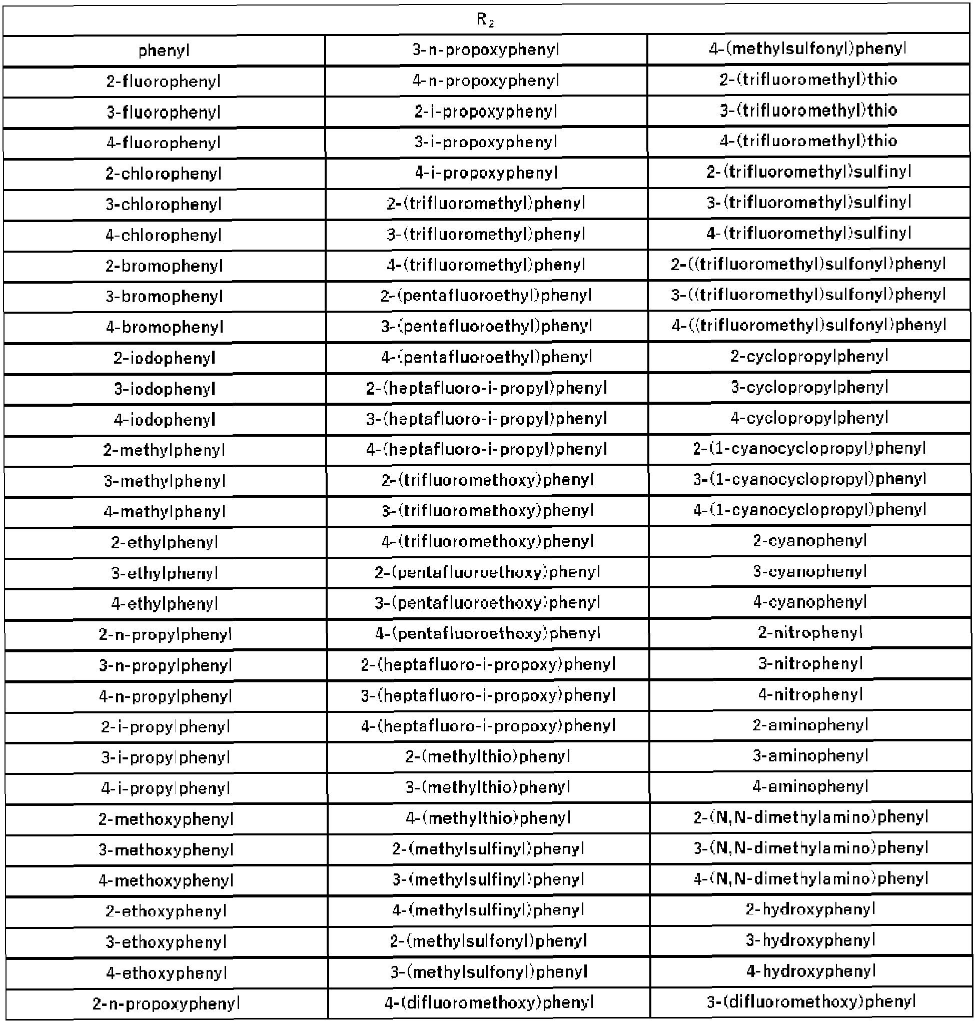

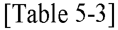

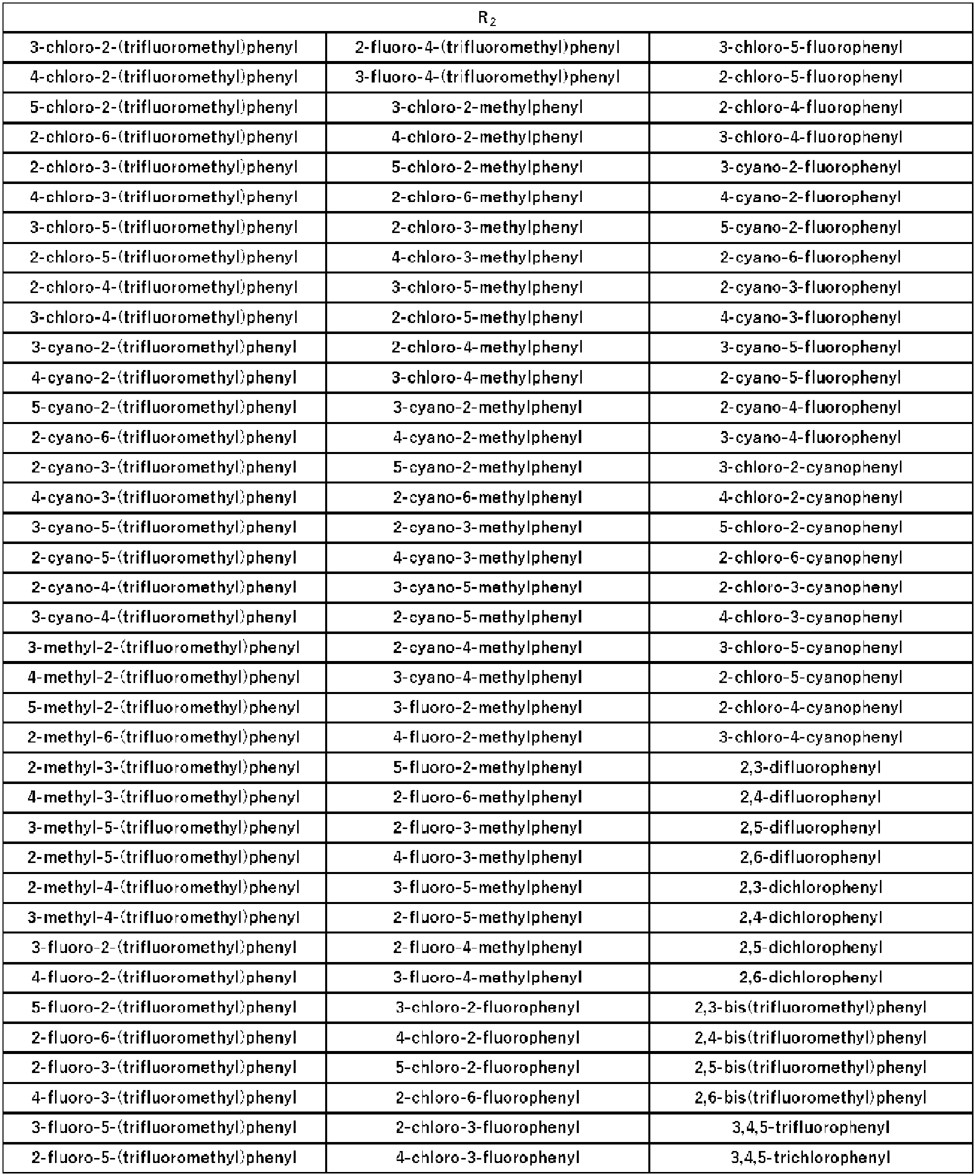

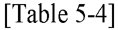

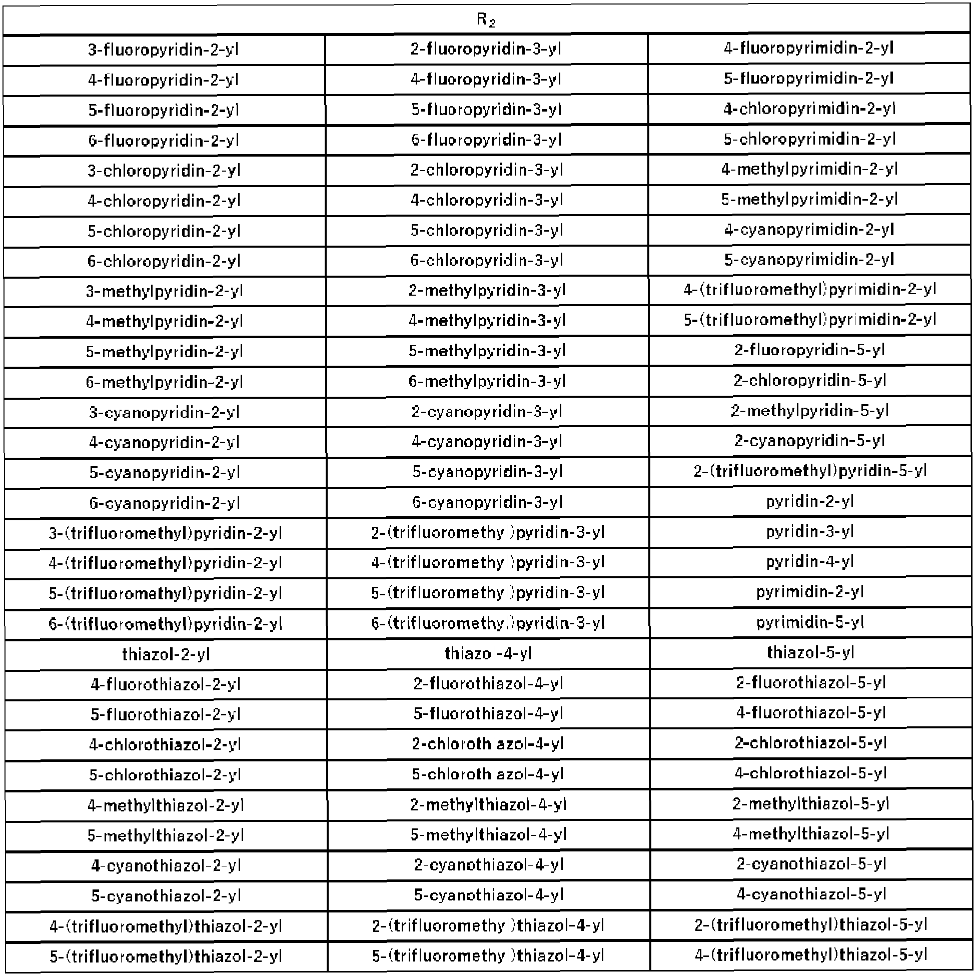

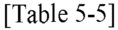

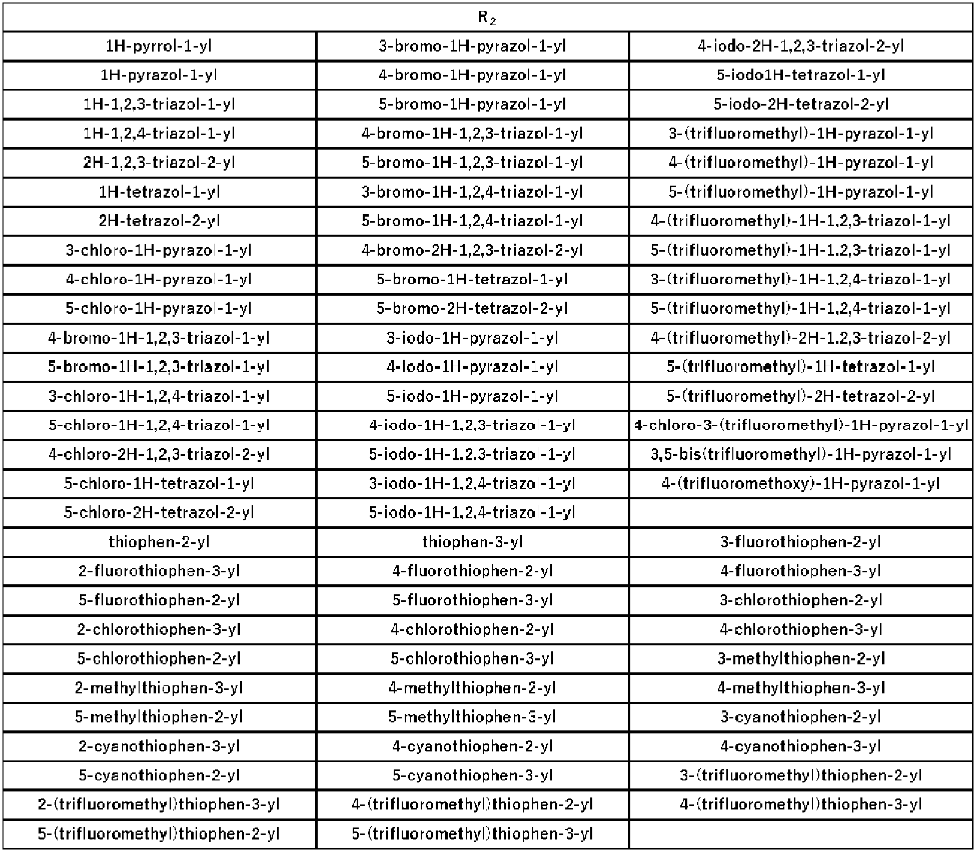

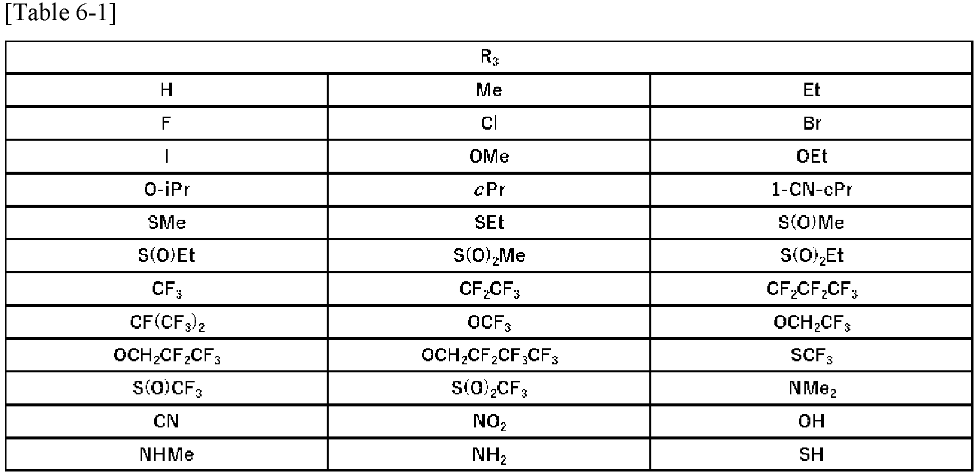

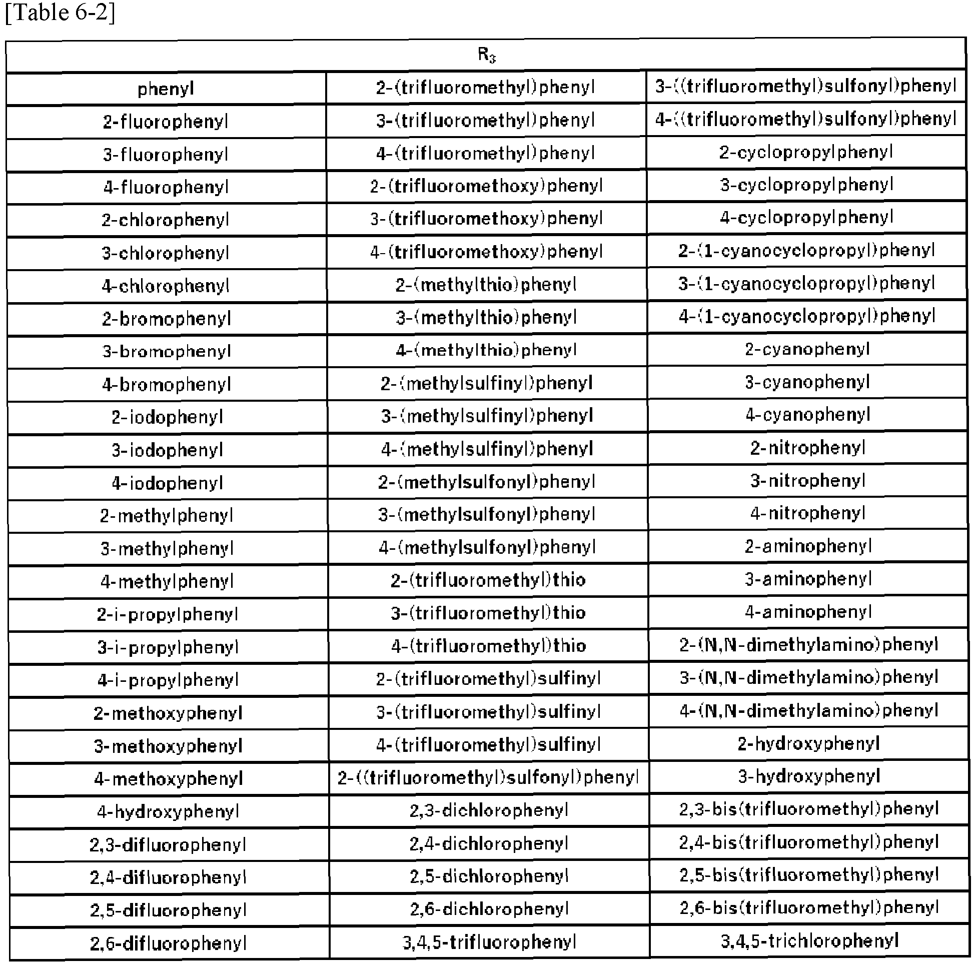

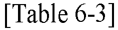

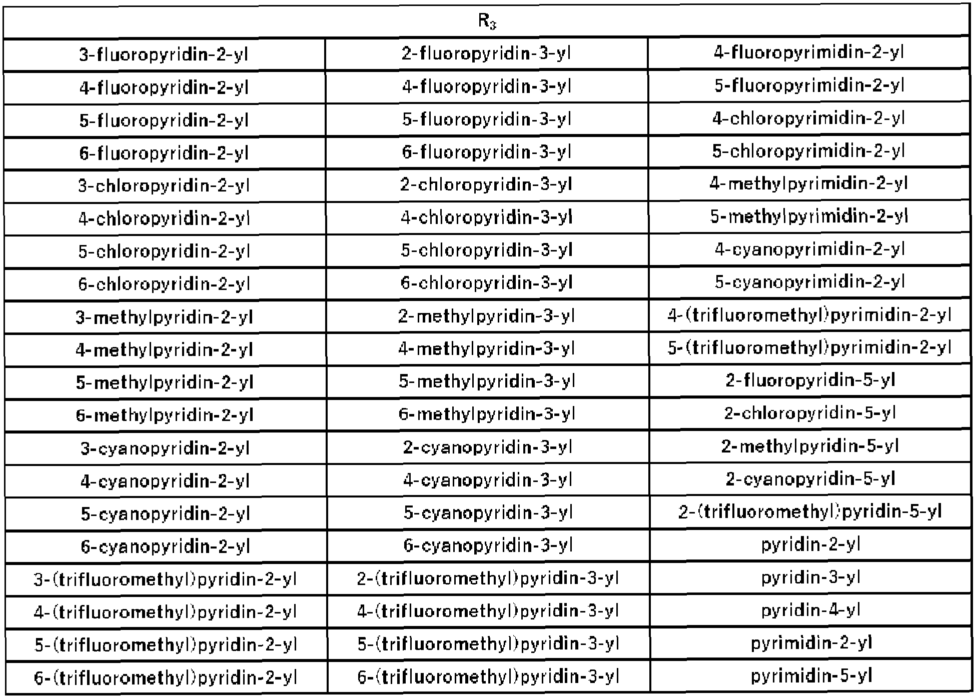

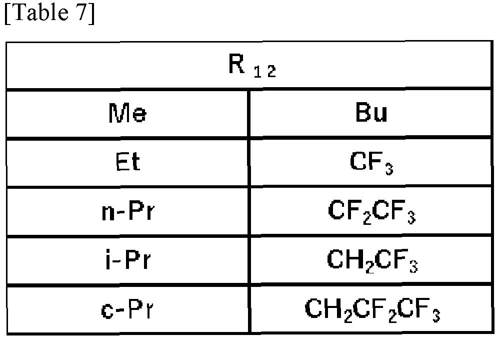

A compound represented by formula (1-2), which is formula (1) where B is B-7 and D is D-2, a salt thereof or an N-oxide thereof 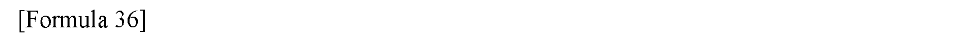 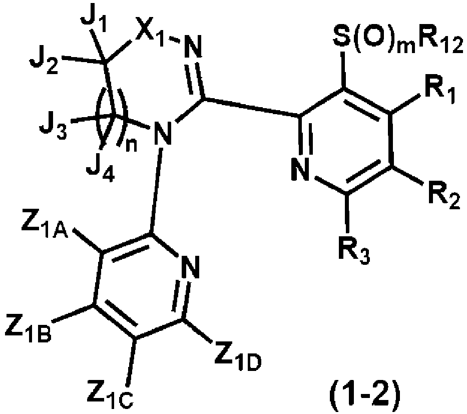 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1C}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₁, R₂, and R₃ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkynyl group optionally having a substituent, a halo (C₂ to C₆) alkynyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-2), which is formula (1) where B is B-7 and D is D-2, Z_{1A}, Z_{1B}, Z_{1C}, and Z_{1D} are substituents shown in Table 1-1, Table 1-2, Table 1-3, and Table 1-4, respectively, X₁ is a structure shown in Table 2, J₁ and J₂ are substituents shown in Table 3-1, J₃ and J₄ are substituents shown in Table 3-2, n is 0 or 1, R₁ is a substituent shown in Table 4, R₂ is a substituent shown in Table 5-1 to Table 5-5, R₃ is a substituent shown in Table 6-1 to Table 6-3, R₁₂ is a substituent shown in Table 7, and m is combined from 0, 1, and 2.

A compound represented by formula (1-3), which is formula (1) where B is B-7 and D is D-3, a salt thereof or an N-oxide thereof 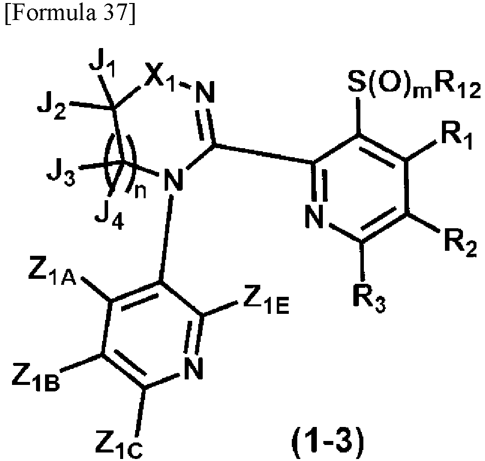 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1C}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₁, R₂, and R₃ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkynyl group optionally having a substituent, a halo (C₂ to C₆) alkynyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-3), which is formula (1) where B is B-7 and D is D-3, Z_{1A}, Z_{1B}, Z_{1C}, and Z_{1E} are substituents shown in Table 1-1, Table 1-2, Table 1-3, and Table 1-5, respectively, X₁ is a structure shown in Table 2, J₁ and J₂ are substituents shown in Table 3-1, J₃ and J₄ are substituents shown in Table 3-2, n is 0 or 1, R₁ is a substituent shown in Table 4, R₂ is a substituent shown in Table 5-1 to Table 5-5, R₃ is a substituent shown in Table 6-1 to Table 6-3, R₁₂ is a substituent shown in Table 7, and m is combined from 0, 1, and 2.

A compound represented by formula (1-4), which is formula (1) where B is B-7 and D is D-4, a salt thereof or an N-oxide thereof 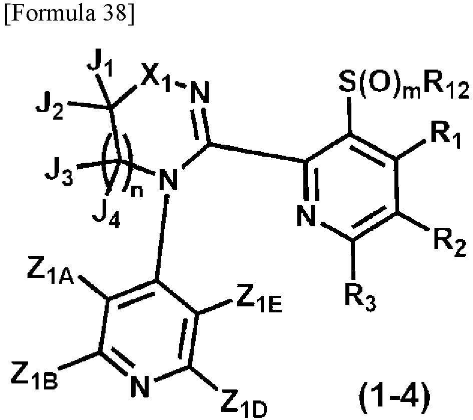 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1D}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₁, R₂, and R₃ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkynyl group optionally having a substituent, a halo (C₂ to C₆) alkynyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-4), which is formula (1) where B is B-7 and D is D-4, Z_{1A}, Z_{1B}, Z_{1D}, and Z_{1E} are substituents shown in Table 1-1, Table 1-2, Table 1-4, and Table 1-5, respectively, X₁ is a structure shown in Table 2, J₁ and J₂ are substituents shown in Table 3-1, J₃ and J₄ are substituents shown in Table 3-2, n is 0 or 1, R₁ is a substituent shown in Table 4, R₂ is a substituent shown in Table 5-1 to Table 5-5, R₃ is a substituent shown in Table 6-1 to Table 6-3, R₁₂ is a substituent shown in Table 7, and m is combined from 0, 1, and 2.

A compound represented by formula (1-5), which is formula (1) where B is B-7 and D is D-5, a salt thereof or an N-oxide thereof 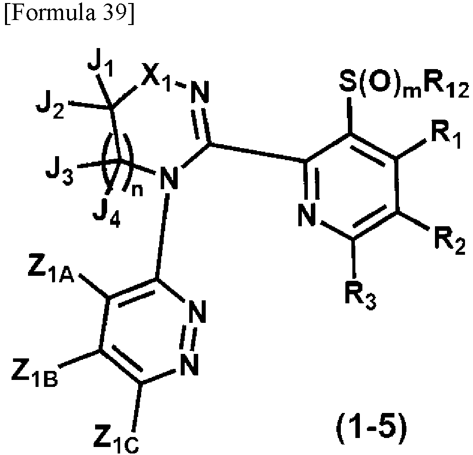 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, and Z_{1C} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₁, R₂, and R₃ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(O)NY₄Y_{S} group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or
   a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-5), which is formula (1) where B is B-7 and D is D-5, Z_{1A}, Z_{1B}, and Z_{1C} are substituents shown in Table 1-1, Table 1-2, and Table 1-3, respectively, X₁ is a structure shown in Table 2, J₁ and J₂ are substituents shown in Table 3-1, J₃ and J₄ are substituents shown in Table 3-2, n is 0 or 1, R₁ is a substituent shown in Table 4, R₂ is a substituent shown in Table 5-1 to Table 5-5, R₃ is a substituent shown in Table 6-1 to Table 6-3, R₁₂ is a substituent shown in Table 7, and m is combined from 0, 1, and 2.

A compound represented by formula (1-6), which is formula (1) where B is B-7 and D is D-6, a salt thereof or an N-oxide thereof 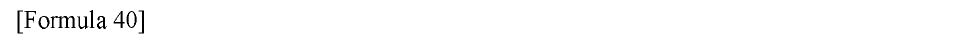 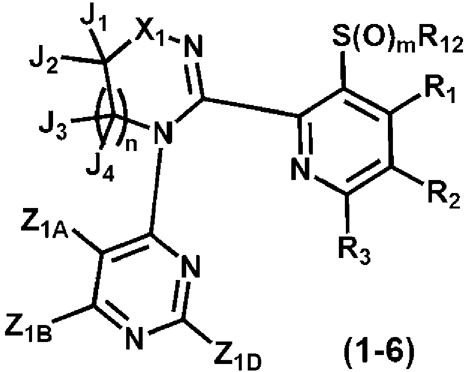 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₁, R₂, and R₃ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(O)NY₄Y_{S} group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-6), which is formula (1) where B is B-7 and D is D-6, Z_{1A}, Z_{1B}, and Z_{1D} are substituents shown in Table 1-1, Table 1-2, and Table 1-4, respectively, X₁ is a structure shown in Table 2, J₁ and J₂ are substituents shown in Table 3-1, J₃ and J₄ are substituents shown in Table 3-2, n is 0 or 1, R₁ is a substituent shown in Table 4, R₂ is a substituent shown in Table 5-1 to Table 5-5, R₃ is a substituent shown in Table 6-1 to Table 6-3, R₁₂ is a substituent shown in Table 7, and m is combined from 0, 1, and 2.

A compound represented by formula (1-7), which is formula (1) where B is B-7 and D is D-7, a salt thereof or an N-oxide thereof 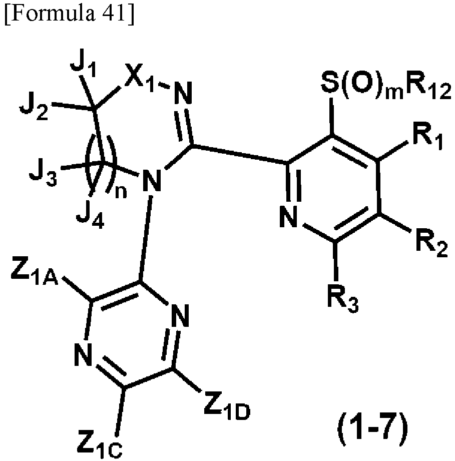 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1C}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₁, R₂, and R₃ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-7), which is formula (1) where B is B-7 and D is D-7, Z_{1A}, Z_{1C}, and Z_{1D} are substituents shown in Table 1-1, Table 1-3, and Table 1-4, respectively, X₁ is a structure shown in Table 2, J₁ and J₂ are substituents shown in Table 3-1, J₃ and J₄ are substituents shown in Table 3-2, n is 0 or 1, R₁ is a substituent shown in Table 4, R₂ is a substituent shown in Table 5-1 to Table 5-5, R₃ is a substituent shown in Table 6-1 to Table 6-3, R₁₂ is a substituent shown in Table 7, and m is combined from 0, 1, and 2.

A compound represented by formula (1-8), which is formula (1) where B is B-7 and D is D-8, a salt thereof or an N-oxide thereof 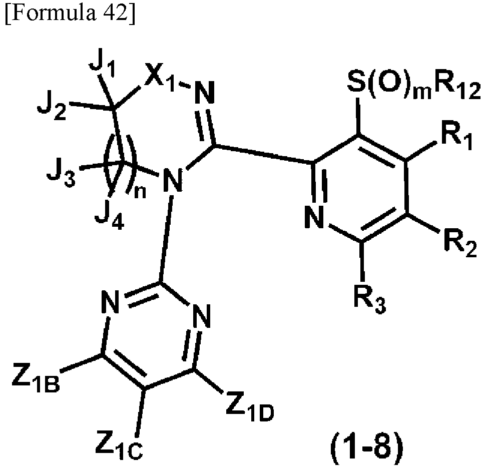 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1B}, Z_{1C}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₁, R₂, and R₃ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-8), which is formula (1) where B is B-7 and D is D-8, Z_{1B}, Z_{1C}, and Z_{1D} are substituents shown in Table 1-2, Table 1-3, and Table 1-4, respectively, X₁ is a structure shown in Table 2, J₁ and J₂ are substituents shown in Table 3-1, J₃ and J₄ are substituents shown in Table 3-2, n is 0 or 1, R₁ is a substituent shown in Table 4, R₂ is a substituent shown in Table 5-1 to Table 5-5, R₃ is a substituent shown in Table 6-1 to Table 6-3, R₁₂ is a substituent shown in Table 7, and m is combined from 0, 1, and 2.

A compound represented by formula (1-9), which is formula (1) where B is B-7 and D is D-9, a salt thereof or an N-oxide thereof 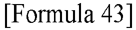 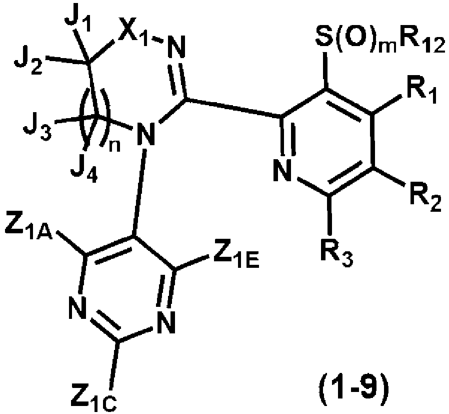 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1C}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₁, R₂, and R₃ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-9), which is formula (1) where B is B-7 and D is D-9, Z_{1A}, Z_{1C}, and Z_{1E} are substituents shown in Table 1-1, Table 1-3, and Table 1-5, respectively, X₁ is a structure shown in Table 2, J₁ and J₂ are substituents shown in Table 3-1, J₃ and J₄ are substituents shown in Table 3-2, n is 0 or 1, R₁ is a substituent shown in Table 4, R₂ is a substituent shown in Table 5-1 to Table 5-5, R₃ is a substituent shown in Table 6-1 to Table 6-3, R₁₂ is a substituent shown in Table 7, and m is combined from 0, 1, and 2.

A compound represented by formula (1-10), which is formula (1) where B is B-7 and D is D-10, a salt thereof or an N-oxide thereof 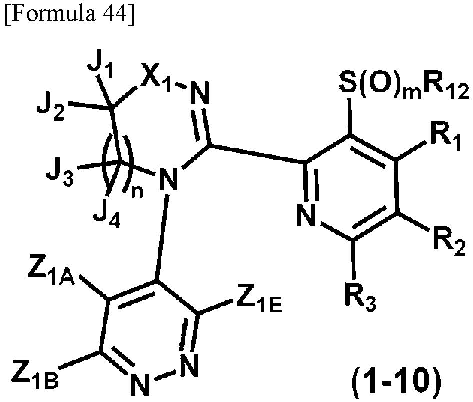 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₁, R₂, and R₃ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-10), which is formula (1) where B is B-7 and D is D-10, Z_{1A}, Z_{1B}, and Z_{1E} are substituents shown in Table 1-1, Table 1-2, and Table 1-5, respectively, X₁ is a structure shown in Table 2, J₁ and J₂ are substituents shown in Table 3-1, J₃ and J₄ are substituents shown in Table 3-2, n is 0 or 1, R₁ is a substituent shown in Table 4, R₂ is a substituent shown in Table 5-1 to Table 5-5, R₃ is a substituent shown in Table 6-1 to Table 6-3, R₁₂ is a substituent shown in Table 7, and m is combined from 0, 1, and 2.

A compound represented by formula (1-11), which is formula (1) where B is B-1, G₄ is CR₄, G₂ is a nitrogen atom, and D is D-1, a salt thereof or an N-oxide thereof 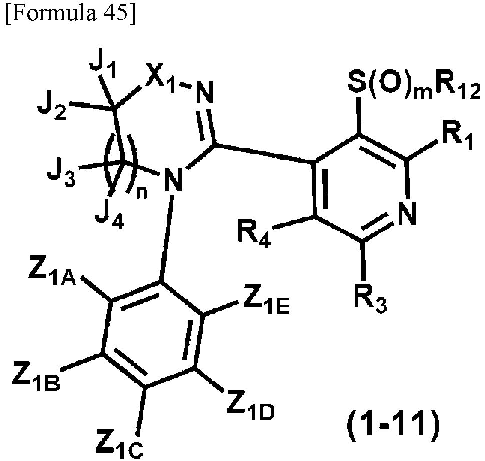 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1C}, Z_{1D}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₁, R₃, and R₄ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or
   a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-11), which is formula (1) where B is B-1, G₄ is CR₄, G₂ is a nitrogen atom, and D is D-1, Z_{1A}, Z_{1B}, Z_{1C}, Z_{1D}, and Z_{1E} are substituents shown in Table 1-1, Table 1-2, Table 1-3, Table 1-4, and Table 1-5, respectively, X₁ is a structure shown in Table 2, J₁ and J₂ are substituents shown in Table 3-1, J₃ and J₄ are substituents shown in Table 3-2, n is 0 or 1, R₁ is a substituent shown in Table 4, R₃ is a substituent shown in Table 6-1 to Table 6-3, R₄ is a substituent shown in Table 8, R₁₂ is a substituent shown in Table 7, and m is combined from 0, 1, and 2.

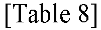

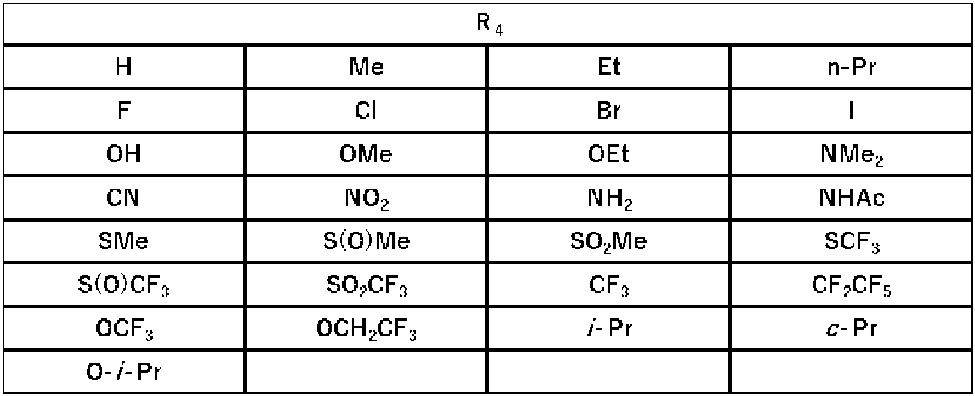

A compound represented by formula (1-12), which is formula (1) where B is B-1, G₄ is CR₄, G₂ is a nitrogen atom, and D is D-2, a salt thereof or an N-oxide thereof 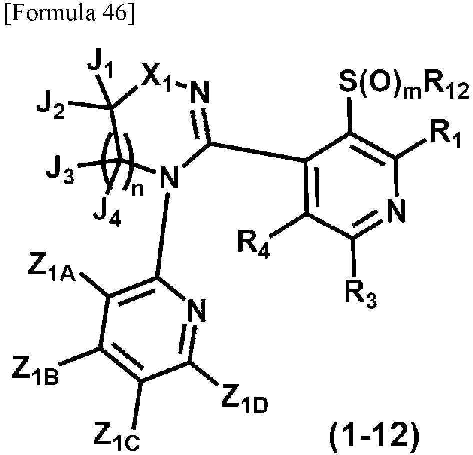 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1C}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₁, R₃, and R₄ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(0)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-12), which is formula (1) where B is B-1, G₄ is CR₄, G₂ is a nitrogen atom, and D is D-2, Z_{1A}, Z_{1B}, Z_{1C}, and Z_{1D} are substituents shown in Table 1-1, Table 1-2, Table 1-3, and Table 1-4, respectively, X₁ is a structure shown in Table 2, J₁ and J₂ are substituents shown in Table 3-1, J₃ and J₄ are substituents shown in Table 3-2, n is 0 or 1, R₁ is a substituent shown in Table 4, R₃ is a substituent shown in Table 6-1 to Table 6-3, R₄ is a substituent shown in Table 8, R₁₂ is a substituent shown in Table 7, and m is combined from 0, 1, and 2.

A compound represented by formula (1-13), which is formula (1) where B is B-1, G₄ is CR₄, G₂ is a nitrogen atom, and D is D-3, a salt thereof or an N-oxide thereof 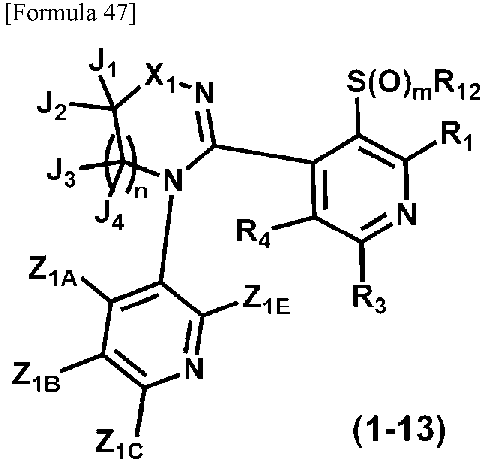 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1C}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(0)NY₄Y₅ group, a cyano group, a nitro group, a hydroxy group, a mercapto group, a formyl group, a carboxy group, and an SF₅ group,
R₁, R₃, and R₄ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(0)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-13), which is formula (1) where B is B-1, G₄ is CR₄, G₂ is a nitrogen atom, and D is D-3, Z_{1A}, Z_{1B}, Z_{1C}, and Z_{1E} are substituents shown in Table 1-1, Table 1-2, Table 1-3, and Table 1-5, respectively, X₁ is a structure shown in Table 2, J₁ and J₂ are substituents shown in Table 3-1, J₃ and J₄ are substituents shown in Table 3-2, n is 0 or 1, R₁ is a substituent shown in Table 4, R₃ is a substituent shown in Table 6-1 to Table 6-3, R₄ is a substituent shown in Table 8, R₁₂ is a substituent shown in Table 7, and m is combined from 0, 1, and 2.

A compound represented by formula (1-14), which is formula (1) where B is B-1, G₄ is CR₄, G₂ is a nitrogen atom, and D is D-4, a salt thereof or an N-oxide thereof 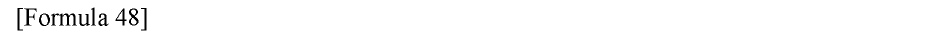 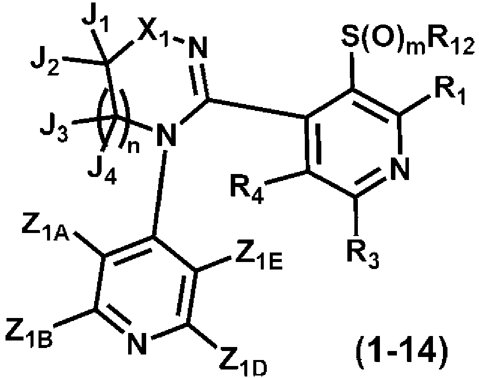 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1D}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(0)NY₄Y₅ group, a cyano group, a nitro group, a hydroxy group, a mercapto group, a formyl group, a carboxy group, and an SF₅ group,
R₁, R₃, and R₄ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(0)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-14), which is formula (1) where B is B-1, G₄ is CR₄, G₂ is a nitrogen atom, and D is D-4, Z_{1A}, Z_{1B}, Z_{1D}, and Z_{1E} are substituents shown in Table 1-1, Table 1-2, Table 1-4, and Table 1-5, respectively, X₁ is a structure shown in Table 2, J₁ and J₂ are substituents shown in Table 3-1, J₃ and J₄ are substituents shown in Table 3-2, n is 0 or 1, R₁ is a substituent shown in Table 4, R₃ is a substituent shown in Table 6-1 to Table 6-3, R₄ is a substituent shown in Table 8, R₁₂ is a substituent shown in Table 7, and m is combined from 0, 1, and 2.

A compound represented by formula (1-15), which is formula (1) where B is B-1, G₄ is CR₄, G₂ is a nitrogen atom, and D is D-5, a salt thereof or an N-oxide thereof 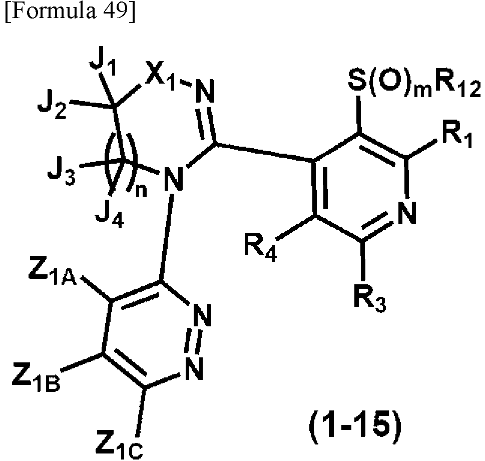 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, and Z_{1C} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(0)NY₄Y₅ group, a cyano group, a nitro group, a hydroxy group, a mercapto group, a formyl group, a carboxy group, and an SF₅ group,
R₁, R₃, and R₄ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(0)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-15), which is formula (1) where B is B-1, G₄ is CR₄, G₂ is a nitrogen atom, and D is D-5, Z_{1A}, Z_{1B}, and Z_{1C} are substituents shown in Table 1-1, Table 1-2 and Table 1-3, respectively, X₁ is a structure shown in Table 2, J₁ and J₂ are substituents shown in Table 3-1, J₃ and J₄ are substituents shown in Table 3-2, n is 0 or 1, R₁ is a substituent shown in Table 4, R₃ is a substituent shown in Table 6-1 to Table 6-3, R₄ is a substituent shown in Table 8, R₁₂ is a substituent shown in Table 7, and m is combined from 0, 1, and 2.

A compound represented by formula (1-16), which is formula (1) where B is B-1, G₄ is CR₄, G₂ is a nitrogen atom, and D is D-6, a salt thereof or an N-oxide thereof 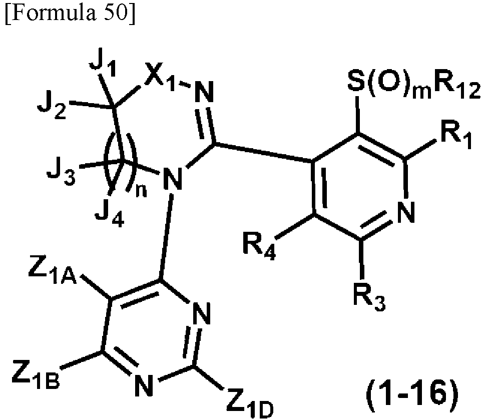 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(0)NY₄Y₅ group, a cyano group, a nitro group, a hydroxy group, a mercapto group, a formyl group, a carboxy group, and an SF₅ group,
R₁, R₃, and R₄ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(0)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or
   a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-16), which is formula (1) where B is B-1, G₄ is CR₄, G₂ is a nitrogen atom, and D is D-6, Z_{1A}, Z_{1B}, and Z_{1D} are substituents shown in Table 1-1, Table 1-2, and Table 1-4, respectively, X₁ is a structure shown in Table 2, J₁ and J₂ are substituents shown in Table 3-1, J₃ and J₄ are substituents shown in Table 3-2, n is 0 or 1, R₁ is a substituent shown in Table 4, R₃ is a substituent shown in Table 6-1 to Table 6-3, R₄ is a substituent shown in Table 8, R₁₂ is a substituent shown in Table 7, and m is combined from 0, 1, and 2.

A compound represented by formula (1-17), which is formula (1) where B is B-1, G₄ is CR₄, G₂ is a nitrogen atom, and D is D-7, a salt thereof or an N-oxide thereof 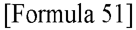 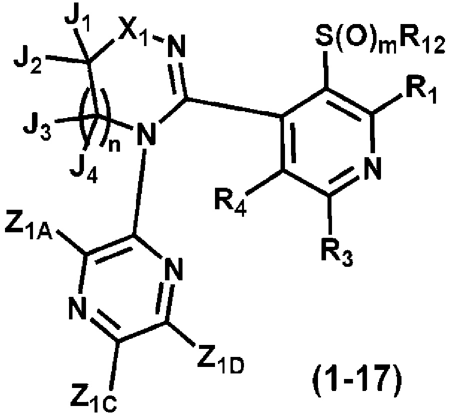 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1C}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(0)NY₄Y₅ group, a cyano group, a nitro group, a hydroxy group, a mercapto group, a formyl group, a carboxy group, and an SF₅ group,
R₁, R₃, and R₄ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(0)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or
   a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-17), which is formula (1) where B is B-1, G₄ is CR₄, G₂ is a nitrogen atom, and D is D-7, Z_{1A}, Z_{1C}, and Z_{1D} are substituents shown in Table 1-1, Table 1-3, and Table 1-4, respectively, X₁ is a structure shown in Table 2, J₁ and J₂ are substituents shown in Table 3-1, J₃ and J₄ are substituents shown in Table 3-2, n is 0 or 1, R₁ is a substituent shown in Table 4, R₃ is a substituent shown in Table 6-1 to Table 6-3, R₄ is a substituent shown in Table 8, R₁₂ is a substituent shown in Table 7, and m is combined from 0, 1, and 2.

A compound represented by formula (1-18), which is formula (1) where B is B-1, G₄ is CR₄, G₂ is a nitrogen atom, and D is D-8, a salt thereof or an N-oxide thereof 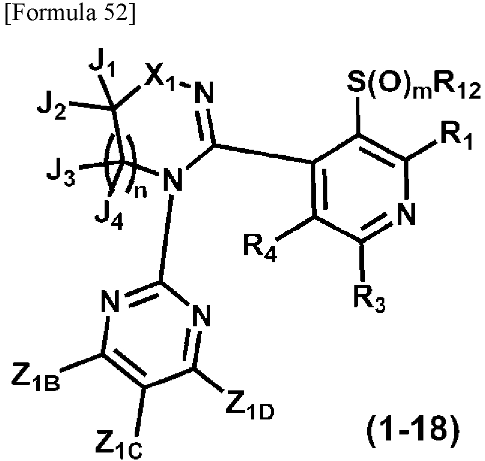 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1B}, Z_{1C}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₁, R₃, and R₄ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-18), which is formula (1) where B is B-1, G₄ is CR₄, G₂ is a nitrogen atom, and D is D-8, Z_{1B}, Z_{1C}, and Z_{1D} are substituents shown in Table 1-2, Table 1-3, and Table 1-4, respectively, X₁ is a structure shown in Table 2, J₁ and J₂ are substituents shown in Table 3-1, J₃ and J₄ are substituents shown in Table 3-2, n is 0 or 1, R₁ is a substituent shown in Table 4, R₃ is a substituent shown in Table 6-1 to Table 6-3, R₄ is a substituent shown in Table 8, R₁₂ is a substituent shown in Table 7, and m is combined from 0, 1, and 2.

A compound represented by formula (1-19), which is formula (1) where B is B-1, G₄ is CR₄, G₂ is a nitrogen atom, and D is D-9, a salt thereof or an N-oxide thereof 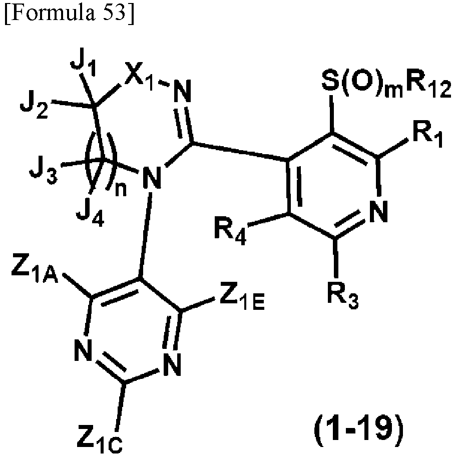 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1C}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyloxy group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₁, R₃, and R₄ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-19), which is formula (1) where B is B-1, G₄ is CR₄, G₂ is a nitrogen atom, and D is D-9, Z_{1A}, Z_{1C}, and Z_{1E} are substituents shown in Table 1-1, Table 1-3, and Table 1-5, respectively, X₁ is a structure shown in Table 2, J₁ and J₂ are substituents shown in Table 3-1, J₃ and J₄ are substituents shown in Table 3-2, n is 0 or 1, R₁ is a substituent shown in Table 4, R₃ is a substituent shown in Table 6-1 to Table 6-3, R₄ is a substituent shown in Table 8, R₁₂ is a substituent shown in Table 7, and m is combined from 0, 1, and 2.

A compound represented by formula (1-20), which is formula (1) where B is B-1, G₄ is CR₄, G₂ is a nitrogen atom, and D is D-10, a salt thereof or an N-oxide thereof 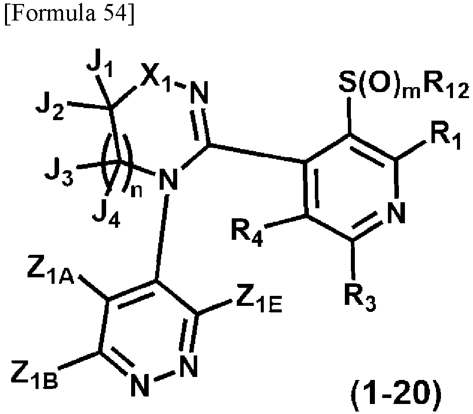 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₁, R₃, and R₄ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or
   a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-20), which is formula (1) where B is B-1, G₄ is CR₄, G₂ is a nitrogen atom, and D is D-10, Z_{1A}, Z_{1B}, and Z_{1E} are substituents shown in Table 1-1, Table 1-2, and Table 1-5, respectively, X₁ is a structure shown in Table 2, J₁ and J₂ are substituents shown in Table 3-1, J₃ and J₄ are substituents shown in Table 3-2, n is 0 or 1, R₁ is a substituent shown in Table 4, R₃ is a substituent shown in Table 6-1 to Table 6-3, R₄ is a substituent shown in Table 8, R₁₂ is a substituent shown in Table 7, and m is combined from 0, 1, and 2.

A compound represented by formula (1-21), which is formula (1) where B is B-2, G₅, G₆, G₇, and G₈ are CR₅, CR₆, CR₇, and CR₈, respectively, and D is D-1, a salt thereof or an N-oxide thereof 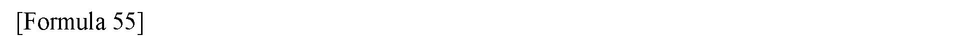 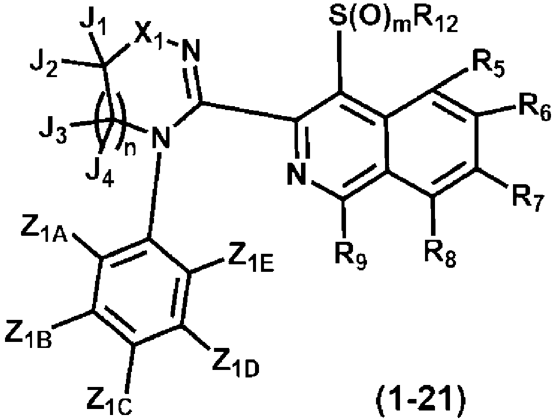 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1C}, Z_{1D}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇ and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₉ is a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a -NY₄Y₅ group, a - C(O)NY₄Y₅ group, a cyano group, a nitro group, a hydroxy group, a (C₁ to C₆) alkylcarbonyloxy group, a halo (C₁ to C₆) alkylcarbonyloxy group, a (C₁ to C₆) alkoxycarbonyloxy group, and a halo (C₁ to C₆) alkylcarbonyloxy group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or
   a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-21), which is formula (1) where B is B-2, G₅, G₆, G₇, and G₈ are CR₅, CR₆, CR₇, and CR₈, respectively, and D is D-1, Z_{1A}, Z_{1B}, Z_{1C}, Z_{1D}, and Z_{1E} are substituents shown in Table 1-1, Table 1-2, Table 1-3, Table 1-4, and Table 1-5, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₉ is a substituent shown in Table 10, R₁₂ is a substituent shown in Table 7, and m is combined from 0, 1, and 2.

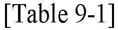

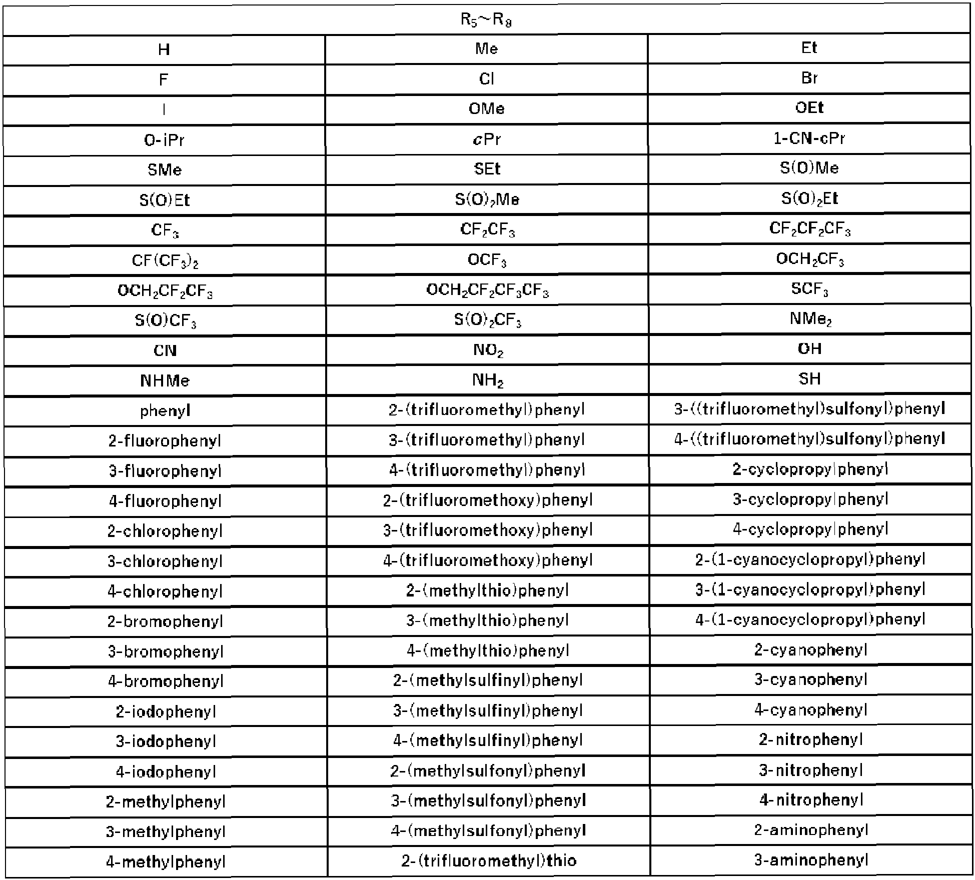

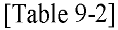

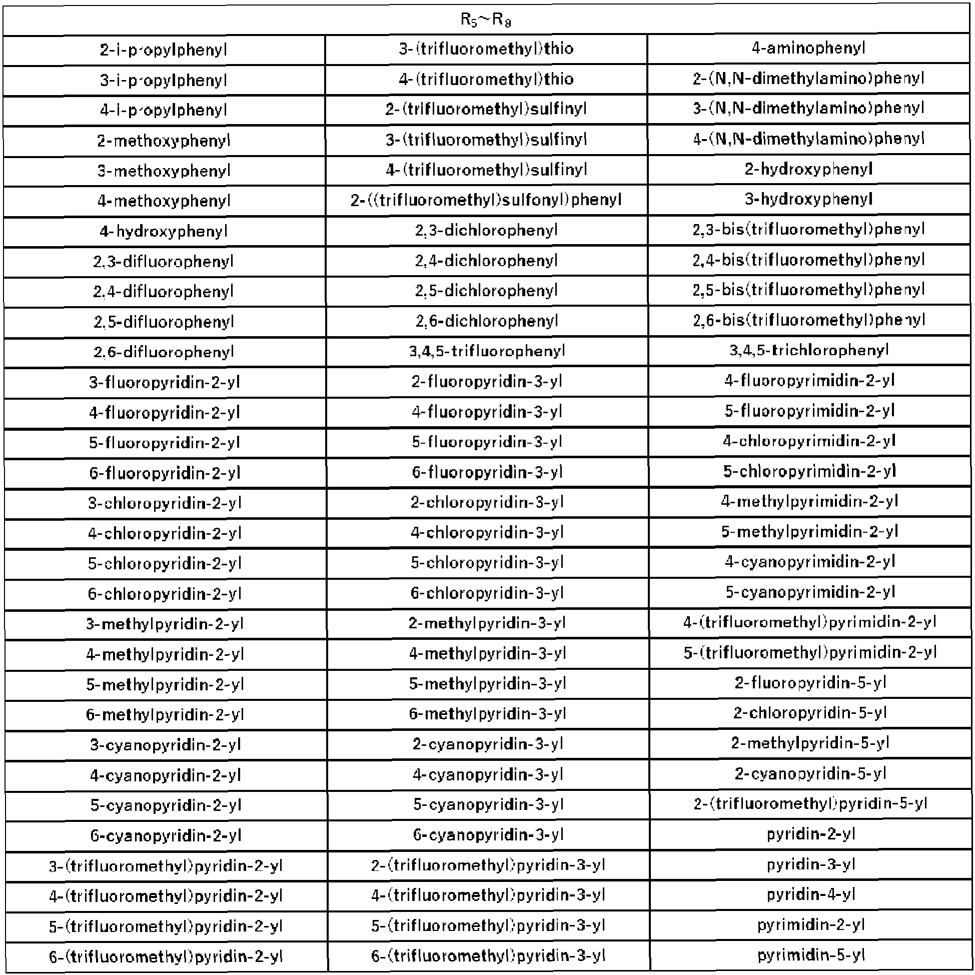

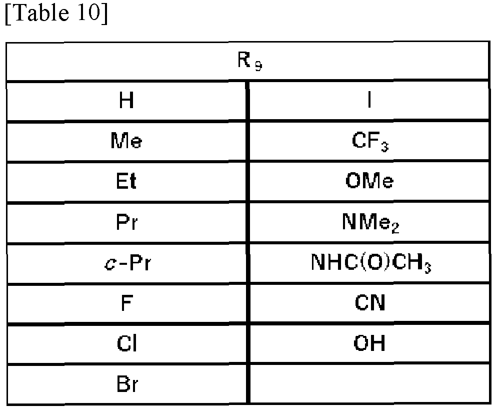

A compound represented by formula (1-22), which is formula (1) where B is B-2, G₅, G₆, G₇, and G₈ are CR₅, CR₆, CR₇, and CR₈, respectively, and D is D-2, a salt thereof or an N-oxide thereof 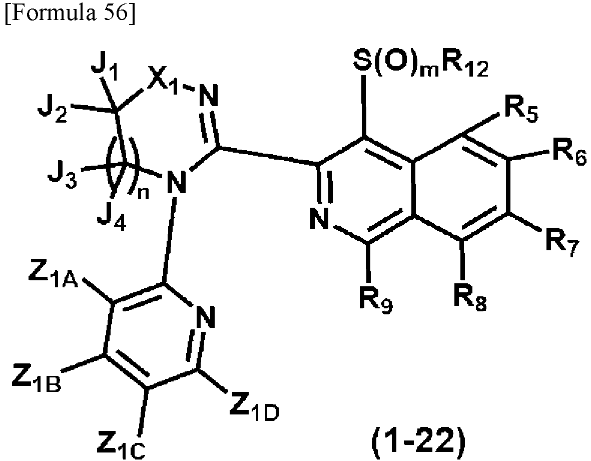 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1C}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇ and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₉ is a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a -NY₄Y₅ group, a - C(O)NY₄Y₅ group, a cyano group, a nitro group, a hydroxy group, a (C₁ to C₆) alkylcarbonyloxy group, a halo (C₁ to C₆) alkylcarbonyloxy group, a (C₁ to C₆) alkoxycarbonyloxy group, and a halo (C₁ to C₆) alkylcarbonyloxy group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-22), which is formula (1) where B is B-2, G₅, G₆, G₇, and G₈ are CR₅, CR₆, CR₇, and CR₈, respectively, and D is D-2, Z_{1A}, Z_{1B}, Z_{1C}, and Z_{1D} are substituents shown in Table 1-1, Table 1-2, Table 1-3, and Table 1-4, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₉ is a substituent shown in Table 10, R₁₂ is a substituent shown in Table 7, and m is combined from 0, 1, and 2.

A compound represented by formula (1-23), which is formula (1) where B is B-2, G₅, G₆, G₇, and G₈ are CR₅, CR₆, CR₇, and CR₈, respectively, and D is D-3, a salt thereof or an N-oxide thereof 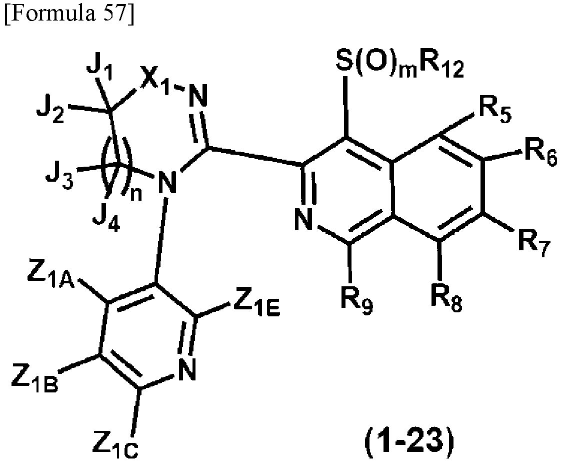 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1C}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇ and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₉ is a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a -NY₄Y₅ group, a - C(O)NY₄Y₅ group, a cyano group, a nitro group, a hydroxy group, a (C₁ to C₆) alkylcarbonyloxy group, a halo (C₁ to C₆) alkylcarbonyloxy group, a (C₁ to C₆) alkoxycarbonyloxy group, and a halo (C₁ to C₆) alkylcarbonyloxy group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or
   a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-23), which is formula (1) where B is B-2, G₅, G₆, G₇, and G₈ are CR₅, CR₆, CR₇, and CR₈, respectively, and D is D-3, Z_{1A}, Z_{1B}, Z_{1C}, and Z_{1E} are substituents shown in Table 1-1, Table 1-2, Table 1-3, and Table 1-5, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₉ is a substituent shown in Table 10, R₁₂ is a substituent shown in Table 7, and m is combined from 0, 1, and 2.

A compound represented by formula (1-24), which is formula (1) where B is B-2, G₅, G₆, G₇, and G₈ are CR₅, CR₆, CR₇, and CR₈, respectively, and D is D-4, a salt thereof or an N-oxide thereof 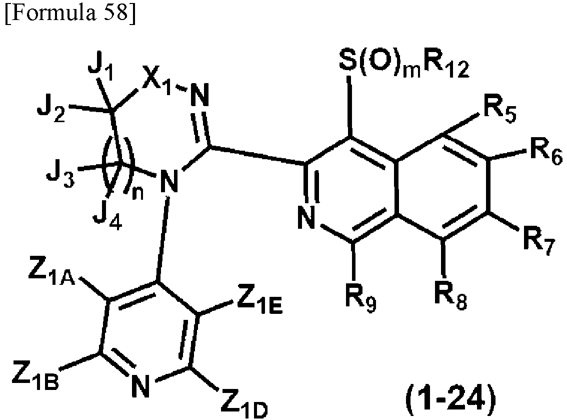 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1D}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇ and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₉ is a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a -NY₄Y₅ group, a - C(O)NY₄Y₅ group, a cyano group, a nitro group, a hydroxy group, a (C₁ to C₆) alkylcarbonyloxy group, a halo (C₁ to C₆) alkylcarbonyloxy group, a (C₁ to C₆) alkoxycarbonyloxy group, and a halo (C₁ to C₆) alkylcarbonyloxy group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-24), which is formula (1) where B is B-2, G₅, G₆, G₇, and G₈ are CR₅, CR₆, CR₇, and CR₈, respectively, and D is D-4, Z_{1A}, Z_{1B}, Z_{1D}, and Z_{1E} are substituents shown in Table 1-1, Table 1-2, Table 1-4, and Table 1-5, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₉ is a substituent shown in Table 10, R₁₂ is a substituent shown in Table 7, and m is combined from 0, 1, and 2.

A compound represented by formula (1-25), which is formula (1) where B is B-2, G₅, G₆, G₇, and G₈ are CR₅, CR₆, CR₇, and CR₈, respectively, and D is D-5, a salt thereof or an N-oxide thereof 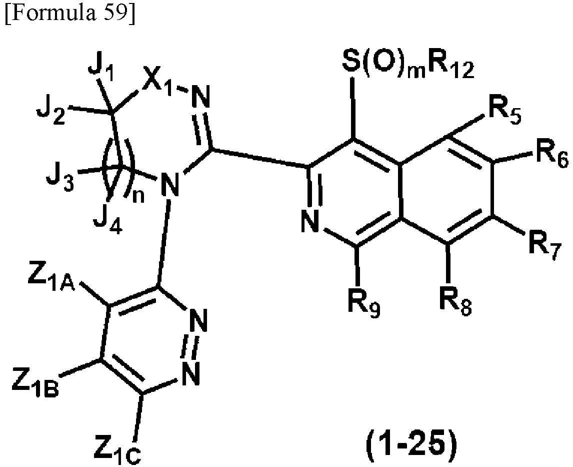 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, and Z_{1C} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇ and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₉ is a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a -NY₄Y₅ group, a - C(O)NY₄Y₅ group, a cyano group, a nitro group, a hydroxy group, a (C₁ to C₆) alkylcarbonyloxy group, a halo (C₁ to C₆) alkylcarbonyloxy group, a (C₁ to C₆) alkoxycarbonyloxy group, and a halo (C₁ to C₆) alkylcarbonyloxy group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-25), which is formula (1) where B is B-2, G₅, G₆, G₇, and G₈ are CR₅, CR₆, CR₇, and CR₈, respectively, and D is D-5, Z_{1A}, Z_{1B}, and Z_{1C} are substituents shown in Table 1-1, Table 1-2, and Table 1-3, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₉ is a substituent shown in Table 10, R₁₂ is a substituent shown in Table 7, and m is combined from 0, 1, and 2.

A compound represented by formula (1-26), which is formula (1) where B is B-2, G₅, G₆, G₇, and G₈ are CR₅, CR₆, CR₇, and CR₈, respectively, and D is D-6, a salt thereof or an N-oxide thereof 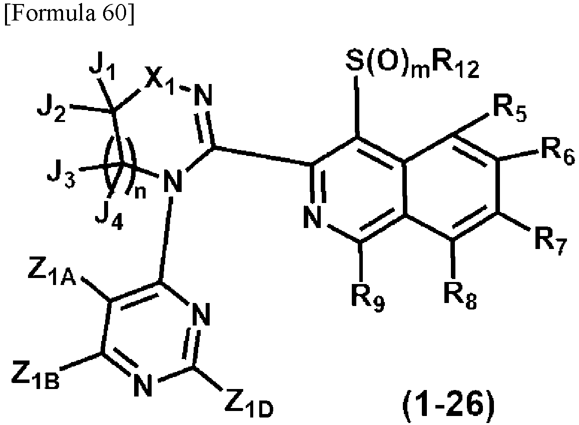 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇ and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₉ is a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a -NY₄Y₅ group, a - C(O)NY₄Y₅ group, a cyano group, a nitro group, a hydroxy group, a (C₁ to C₆) alkylcarbonyloxy group, a halo (C₁ to C₆) alkylcarbonyloxy group, a (C₁ to C₆) alkoxycarbonyloxy group, and a halo (C₁ to C₆) alkylcarbonyloxy group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-26), which is formula (1) where B is B-2, G₅, G₆, G₇, and G₈ are CR₅, CR₆, CR₇, and CR₈, respectively, and D is D-6, Z_{1A}, Z_{1B}, and Z_{1D} are substituents shown in Table 1-1, Table 1-2, and Table 1-4, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₉ is a substituent shown in Table 10, R₁₂ is a substituent shown in Table 7, and m is combined from 0, 1, and 2.

A compound represented by formula (1-27), which is formula (1) where B is B-2, G₅, G₆, G₇, and G₈ are CR₅, CR₆, CR₇, and CR₈, respectively, and D is D-7, a salt thereof or an N-oxide thereof 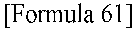 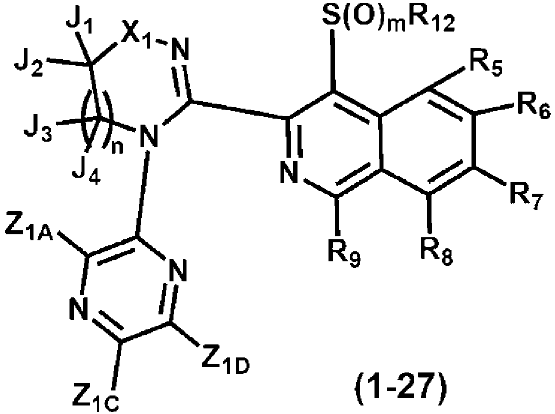 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1C}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇ and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group,
a carboxy group, and an SF₅ group,
Y₃, Y₄, Y₅, and Y₆ are defined as above,
R₉ is a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a -NY₄Y₅ group, a - C(O)NY₄Y₅ group, a cyano group, a nitro group, a hydroxy group, a (C₁ to C₆) alkylcarbonyloxy group, a halo (C₁ to C₆) alkylcarbonyloxy group, a (C₁ to C₆) alkoxycarbonyloxy group, and a halo (C₁ to C₆) alkylcarbonyloxy group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-27), which is formula (1) where B is B-2, G₅, G₆, G₇, and G₈ are CR₅, CR₆, CR₇, and CR₈, respectively, and D is D-7, Z_{1A}, Z_{1C}, and Z_{1D} are substituents shown in Table 1-1, Table 1-3, and Table 1-4, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₉ is a substituent shown in Table 10, R₁₂ is a substituent shown in Table 7, and m is combined from 0, 1, and 2.

A compound represented by formula (1-28), which is formula (1) where B is B-2, G₅, G₆, G₇, and G₈ are CR₅, CR₆, CR₇, and CR₈, respectively, and D is D-8, a salt thereof or an N-oxide thereof 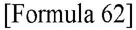 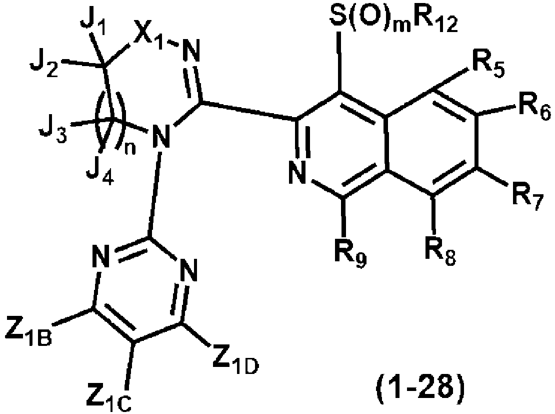 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1B}, Z_{1C}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇ and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₉ is a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a -NY₄Y₅ group, a - C(O)NY₄Y₅ group, a cyano group, a nitro group, a hydroxy group, a (C₁ to C₆) alkylcarbonyloxy group, a halo (C₁ to C₆) alkylcarbonyloxy group, a (C₁ to C₆) alkoxycarbonyloxy group, and a halo (C₁ to C₆) alkylcarbonyloxy group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-28), which is formula (1) where B is B-2, G₅, G₆, G₇, and G₈ are CR₅, CR₆, CR₇, and CR₈, respectively, and D is D-8, Z_{1B}, Z_{1C}, and Z_{1D} are substituents shown in Table 1-2, Table 1-3, and Table 1-4, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₉ is a substituent shown in Table 10, R₁₂ is a substituent shown in Table 7, and m is combined from 0, 1, and 2.

A compound represented by formula (1-29), which is formula (1) where B is B-2, G₅, G₆, G₇, and G₈ are CR₅, CR₆, CR₇, and CR₈, respectively, and D is D-9, a salt thereof or an N-oxide thereof 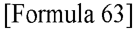 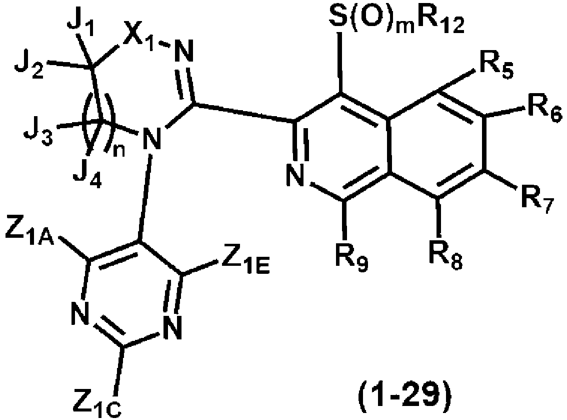 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1C}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇ and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₉ is a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a -NY₄Y₅ group, a - C(O)NY₄Y₅ group, a cyano group, a nitro group, a hydroxy group, a (C₁ to C₆) alkylcarbonyloxy group, a halo (C₁ to C₆) alkylcarbonyloxy group, a (C₁ to C₆) alkoxycarbonyloxy group, and a halo (C₁ to C₆) alkylcarbonyloxy group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-29), which is formula (1) where B is B-2, G₅, G₆, G₇, and G₈ are CR₅, CR₆, CR₇, and CR₈, respectively, and D is D-9, Z_{1A}, Z_{1C}, and Z_{1E} are substituents shown in Table 1-1, Table 1-3, and Table 1-5, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₉ is a substituent shown in Table 10, R₁₂ is a substituent shown in Table 7, and m is combined from 0, 1, and 2.

A compound represented by formula (1-30), which is formula (1) where B is B-2, G₅, G₆, G₇, and G₈ are CR₅, CR₆, CR₇, and CR₈, respectively, and D is D-10, a salt thereof or an N-oxide thereof 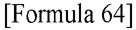 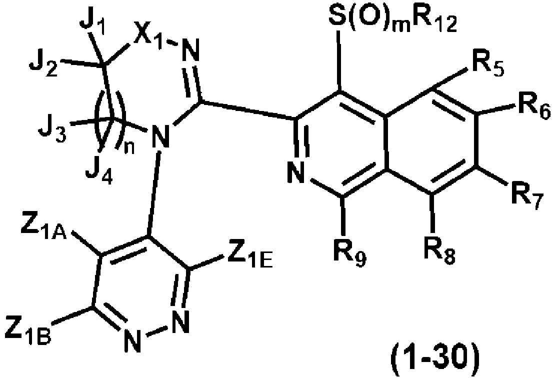 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇ and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₉ is a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a -NY₄Y₅ group, a - C(O)NY₄Y₅ group, a cyano group, a nitro group, a hydroxy group, a (C₁ to C₆) alkylcarbonyloxy group, a halo (C₁ to C₆) alkylcarbonyloxy group, a (C₁ to C₆) alkoxycarbonyloxy group, and a halo (C₁ to C₆) alkylcarbonyloxy group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-30), which is formula (1) where B is B-2, G₅, G₆, G₇, and G₈ are CR₅, CR₆, CR₇, and CR₈, respectively, and D is D-10, Z_{1A}, Z_{1B}, and Z_{1E} are substituents shown in Table 1-1, Table 1-2, and Table 1-5, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₉ is a substituent shown in Table 10, R₁₂ is a substituent shown in Table 7, and m is combined from 0, 1, and 2.

A compound represented by formula (1-31), which is formula (1) where B is B-3, G₅, G₆, G₇, and G₈ are CR₅, CR₆, CR₇, and CR₈, respectively, and D is D-1, a salt thereof or an N-oxide thereof 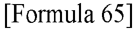 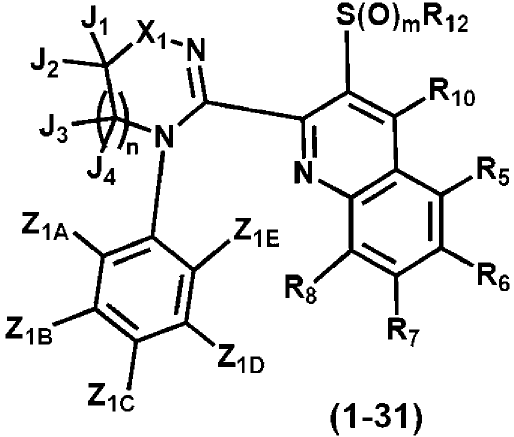 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1C}, Z_{1D}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇ and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₀ is a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a -NY₄Y₅ group, a - C(O)NY₄Y₅ group, a cyano group, a nitro group, a hydroxy group, a (C₁ to C₆) alkylcarbonyloxy group, a halo (C₁ to C₆) alkylcarbonyloxy group, a (C₁ to C₆) alkoxycarbonyloxy group, and a halo (C₁ to C₆) alkylcarbonyloxy group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-31), which is formula (1) where B is B-3, G₅, G₆, G₇, and G₈ are CR₅, CR₆, CR₇, and CR₈, respectively, and D is D-1, Z_{1A}, Z_{1B}, Z_{1C}, Z_{1D}, and Z_{1E} are substituents shown in Table 1-1, Table 1-2, Table 1-3, Table 1-4, and Table 1-5, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₀ is a substituent shown in Table 11, R₁₂ is a substituent shown in Table 7, and m is combined from 0, 1, and 2.

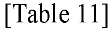

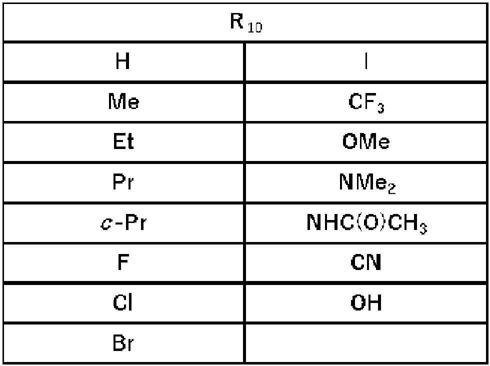

A compound represented by formula (1-32), which is formula (1) where B is B-3, G₅, G₆, G₇, and G₈ are CR₅, CR₆, CR₇, and CR₈, respectively, and D is D-2, a salt thereof or an N-oxide thereof 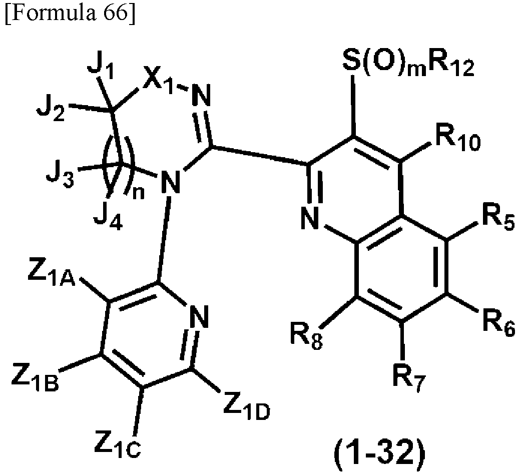 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1C}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇ and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₀ is a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a -NY₄Y₅ group, a - C(O)NY₄Y₅ group, a cyano group, a nitro group, a hydroxy group, a (C₁ to C₆) alkylcarbonyloxy group, a halo (C₁ to C₆) alkylcarbonyloxy group, a (C₁ to C₆) alkoxycarbonyloxy group, and a halo (C₁ to C₆) alkylcarbonyloxy group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-32), which is formula (1) where B is B-3, G₅, G₆, G₇, and G₈ are CR₅, CR₆, CR₇, and CR₈, respectively, and D is D-2, Z_{1A}, Z_{1B}, Z_{1C}, and Z_{1D} are substituents shown in Table 1-1, Table 1-2, Table 1-3, and Table 1-4, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₀ is a substituent shown in Table 11, R₁₂ is a substituent shown in Table 7, and m is combined from 0, 1, and 2.

A compound represented by formula (1-33), which is formula (1) where B is B-3, G₅, G₆, G₇, and G₈ are CR₅, CR₆, CR₇, and CR₈, respectively, and D is D-3, a salt thereof or an N-oxide thereof 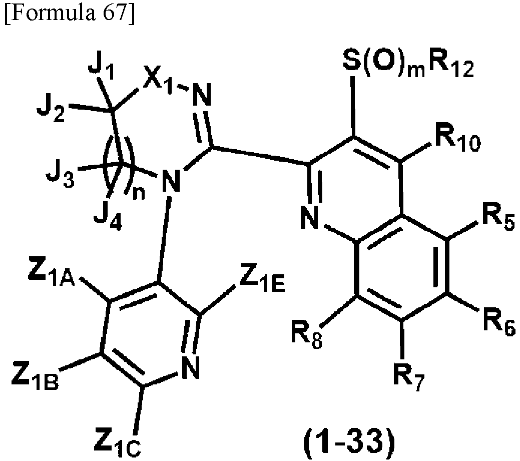 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1C}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇ and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₀ is a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a -NY₄Y₅ group, a - C(O)NY₄Y₅ group, a cyano group, a nitro group, a hydroxy group, a (C₁ to C₆) alkylcarbonyloxy group, a halo (C₁ to C₆) alkylcarbonyloxy group, a (C₁ to C₆) alkoxycarbonyloxy group, and a halo (C₁ to C₆) alkylcarbonyloxy group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-33), which is formula (1) where B is B-3, G₅, G₆, G₇, and G₈ are CR₅, CR₆, CR₇, and CR₈, respectively, and D is D-3, Z_{1A}, Z_{1B}, Z_{1C}, and Z_{1E} are substituents shown in Table 1-1, Table 1-2, Table 1-3, and Table 1-5, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₀ is a substituent shown in Table 11, R₁₂ is a substituent shown in Table 7, and m is combined from 0, 1, and 2.

A compound represented by formula (1-34), which is formula (1) where B is B-3, G₅, G₆, G₇, and G₈ are CR₅, CR₆, CR₇, and CR₈, respectively, and D is D-4, a salt thereof or an N-oxide thereof 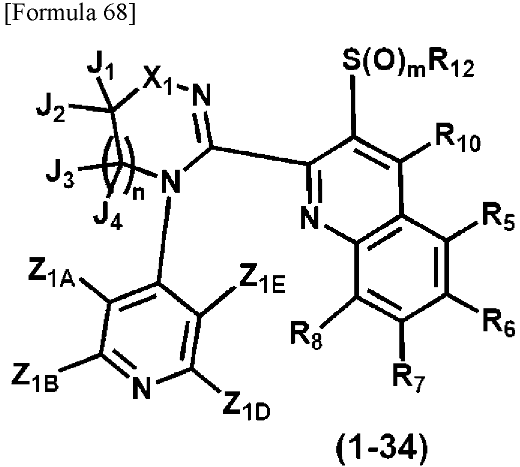 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1D}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇ and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₀ is a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a -NY₄Y₅ group, a - C(O)NY₄Y₅ group, a cyano group, a nitro group, a hydroxy group, a (C₁ to C₆) alkylcarbonyloxy group, a halo (C₁ to C₆) alkylcarbonyloxy group, a (C₁ to C₆) alkoxycarbonyloxy group, and a halo (C₁ to C₆) alkylcarbonyloxy group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-34), which is formula (1) where B is B-3, G₅, G₆, G₇, and G₈ are CR₅, CR₆, CR₇, and CR₈, respectively, and D is D-4, Z_{1A}, Z_{1B}, Z_{1D}, and Z_{1E} are substituents shown in Table 1-1, Table 1-2, Table 1-4, and Table 1-5, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₀ is a substituent shown in Table 11, R₁₂ is a substituent shown in Table 7, and m is combined from 0, 1, and 2.

A compound represented by formula (1-35), which is formula (1) where B is B-3, G₅, G₆, G₇, and G₈ are CR₅, CR₆, CR₇, and CR₈, respectively, and D is D-5, a salt thereof or an N-oxide thereof 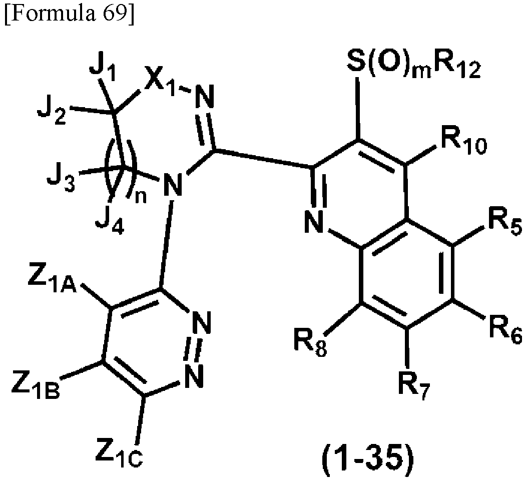 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, and Z_{1C} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇ and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₀ is a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a -NY₄Y₅ group, a - C(O)NY₄Y₅ group, a cyano group, a nitro group, a hydroxy group, a (C₁ to C₆) alkylcarbonyloxy group, a halo (C₁ to C₆) alkylcarbonyloxy group, a (C₁ to C₆) alkoxycarbonyloxy group, and a halo (C₁ to C₆) alkylcarbonyloxy group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-35), which is formula (1) where B is B-3, G₅, G₆, G₇, and G₈ are CR₅, CR₆, CR₇, and CR₈, respectively, and D is D-5, Z_{1A}, Z_{1B}, and Z_{1C} are substituents shown in Table 1-1, Table 1-2, and Table 1-3, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₀ is a substituent shown in Table 11, R₁₂ is a substituent shown in Table 7, and m is combined from 0, 1, and 2.

A compound represented by formula (1-36), which is formula (1) where B is B-3, G₅, G₆, G₇, and G₈ are CR₅, CR₆, CR₇, and CR₈, respectively, and D is D-6, a salt thereof or an N-oxide thereof 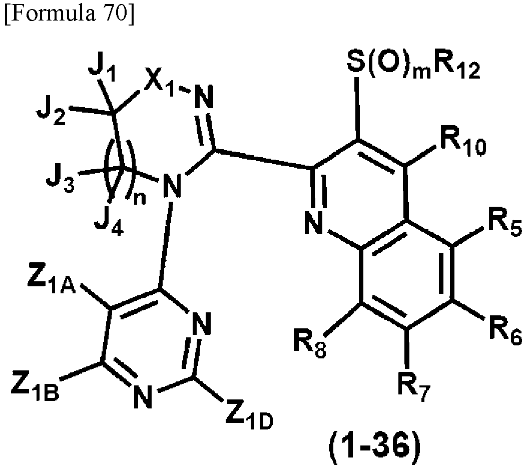 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇ and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₀ is a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a -NY₄Y₅ group, a - C(O)NY₄Y₅ group, a cyano group, a nitro group, a hydroxy group, a (C₁ to C₆) alkylcarbonyloxy group, a halo (C₁ to C₆) alkylcarbonyloxy group, a (C₁ to C₆) alkoxycarbonyloxy group, and a halo (C₁ to C₆) alkylcarbonyloxy group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-36), which is formula (1) where B is B-3, G₅, G₆, G₇, and G₈ are CR₅, CR₆, CR₇, and CR₈, respectively, and D is D-6, Z_{1A}, Z_{1B}, and Z_{1D} are substituents shown in Table 1-1, Table 1-2, and Table 1-4, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₀ is a substituent shown in Table 11, R₁₂ is a substituent shown in Table 7, and m is combined from 0, 1, and 2.

A compound represented by formula (1-37), which is formula (1) where B is B-3, G₅, G₆, G₇, and G₈ are CR₅, CR₆, CR₇, and CR₈, respectively, and D is D-7, a salt thereof or an N-oxide thereof 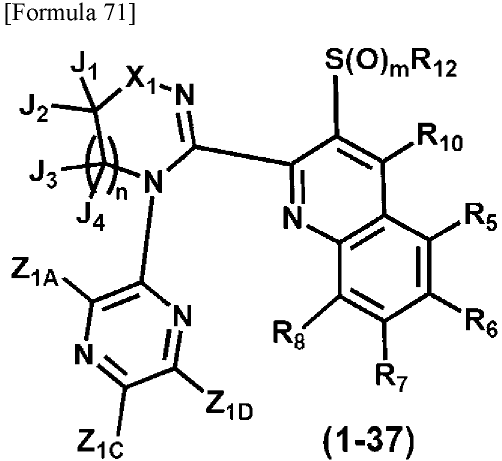 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1C}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇ and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₀ is a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a -NY₄Y₅ group, a - C(O)NY₄Y₅ group, a cyano group, a nitro group, a hydroxy group, a (C₁ to C₆) alkylcarbonyloxy group, a halo (C₁ to C₆) alkylcarbonyloxy group, a (C₁ to C₆) alkoxycarbonyloxy group, and a halo (C₁ to C₆) alkylcarbonyloxy group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-37), which is formula (1) where B is B-3, G₅, G₆, G₇, and G₈ are CR₅, CR₆, CR₇, and CR₈, respectively, and D is D-7, Z_{1A}, Z_{1C}, and Z_{1D} are substituents shown in Table 1-1, Table 1-3, and Table 1-4, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₀ is a substituent shown in Table 11, R₁₂ is a substituent shown in Table 7, and m is combined from 0, 1, and 2.

A compound represented by formula (1-38), which is formula (1) where B is B-3, G₅, G₆, G₇, and G₈ are CR₅, CR₆, CR₇, and CR₈, respectively, and D is D-8, a salt thereof or an N-oxide thereof 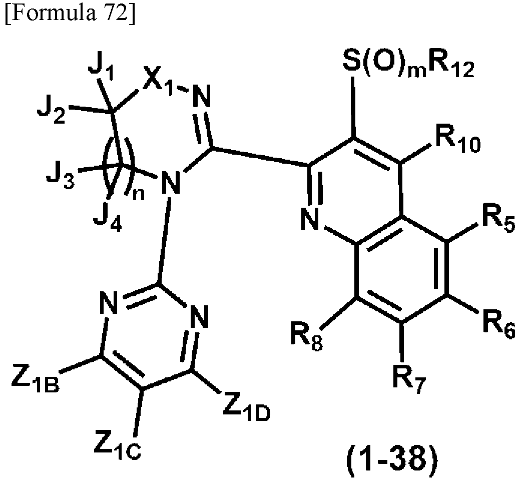 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1B}, Z_{1C}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇ and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₀ is a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a -NY₄Y₅ group, a - C(O)NY₄Y₅ group, a cyano group, a nitro group, a hydroxy group, a (C₁ to C₆) alkylcarbonyloxy group, a halo (C₁ to C₆) alkylcarbonyloxy group, a (C₁ to C₆) alkoxycarbonyloxy group, and a halo (C₁ to C₆) alkylcarbonyloxy group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-38), which is formula (1) where B is B-3, G₅, G₆, G₇, and G₈ are CR₅, CR₆, CR₇, and CR₈, respectively, and D is D-8, Z_{1B}, Z_{1C}, and Z_{1D} are substituents shown in Table 1-2, Table 1-3, and Table 1-4, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₀ is a substituent shown in Table 11, R₁₂ is a substituent shown in Table 7, and m is combined from 0, 1, and 2.

A compound represented by formula (1-39), which is formula (1) where B is B-3, G₅, G₆, G₇, and G₈ are CR₅, CR₆, CR₇, and CR₈, respectively, and D is D-9, a salt thereof or an N-oxide thereof 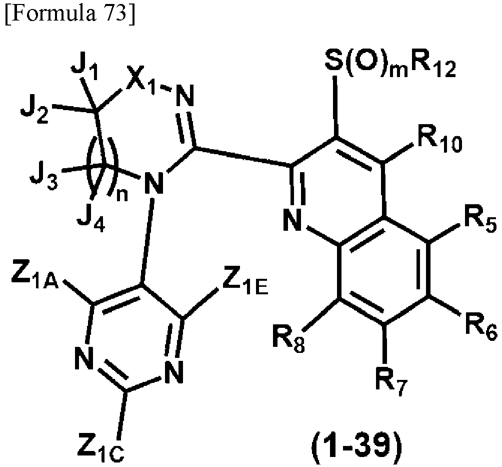 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1C}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇ and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₀ is a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a -NY₄Y₅ group, a - C(O)NY₄Y₅ group, a cyano group, a nitro group, a hydroxy group, a (C₁ to C₆) alkylcarbonyloxy group, a halo (C₁ to C₆) alkylcarbonyloxy group, a (C₁ to C₆) alkoxycarbonyloxy group, and a halo (C₁ to C₆) alkylcarbonyloxy group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-39), which is formula (1) where B is B-3, G₅, G₆, G₇, and G₈ are CR₅, CR₆, CR₇, and CR₈, respectively, and D is D-9, Z_{1A}, Z_{1C}, and Z_{1E} are substituents shown in Table 1-1, Table 1-3, and Table 1-5, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₀ is a substituent shown in Table 11, R₁₂ is a substituent shown in Table 7, and m is combined from 0, 1, and 2.

A compound represented by formula (1-40), which is formula (1) where B is B-3, G₅, G₆, G₇, and G₈ are CR₅, CR₆, CR₇, and CR₈, respectively, and D is D-10, a salt thereof or an N-oxide thereof 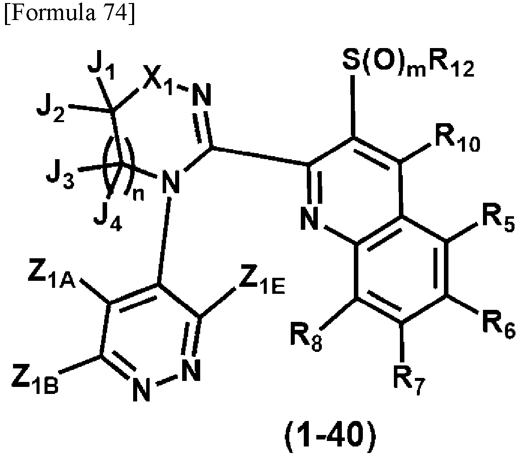 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇ and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₀ is a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a -NY₄Y₅ group, a - C(O)NY₄Y₅ group, a cyano group, a nitro group, a hydroxy group, a (C₁ to C₆) alkylcarbonyloxy group, a halo (C₁ to C₆) alkylcarbonyloxy group, a (C₁ to C₆) alkoxycarbonyloxy group, and a halo (C₁ to C₆) alkylcarbonyloxy group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-40), which is formula (1) where B is B-3, G₅, G₆, G₇, and G₈ are CR₅, CR₆, CR₇, and CR₈, respectively, and D is D-10, Z_{1A}, Z_{1B}, and Z_{1E} are substituents shown in Table 1-1, Table 1-2, and Table 1-5, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₀ is a substituent shown in Table 11, R₁₂ is a substituent shown in Table 7, and m is combined from 0, 1, and 2.

A compound represented by formula (1-41), which is formula (1) where B is B-4, G₅, G₆, G₇, and G₈ are CR₅, CR₆, CR₇, and CR₈, respectively, and D is D-1, a salt thereof or an N-oxide thereof 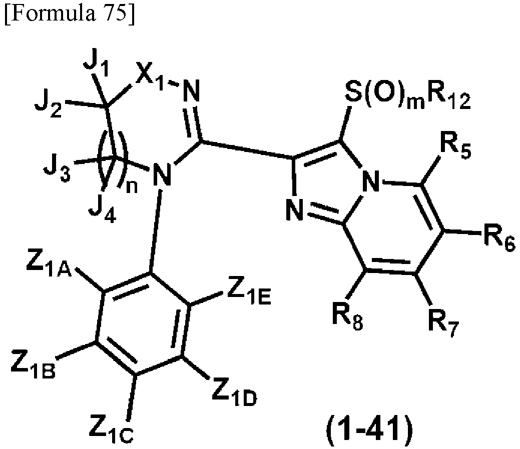 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1C}, Z_{1D}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-41), which is formula (1) where B is B-4, G₅, G₆, G₇, and G₈ are CR₅, CR₆, CR₇, and CR₈, respectively, and D is D-1, Z_{1A}, Z_{1B}, Z_{1C}, Z_{1D}, and Z_{1E} are substituents shown in Table 1-1, Table 1-2, Table 1-3, Table 1-4, and Table 1-5, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₂ is a substituent shown in Table 7, and m is combined from 0, 1, and 2.

A compound represented by formula (1-42), which is formula (1) where B is B-4, G₅, G₆, G₇, and G₈ are CR₅, CR₆, CR₇, and CR₈, respectively, and D is D-2, a salt thereof or an N-oxide thereof 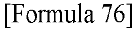 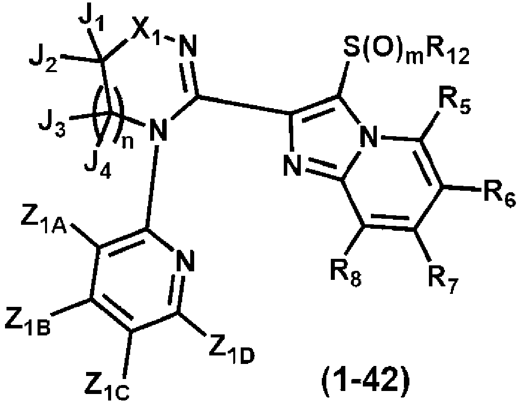 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1C}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-42), which is formula (1) where B is B-4, G₅, G₆, G₇, and G₈ are CR₅, CR₆, CR₇, and CR₈, respectively, and D is D-2, Z_{1A}, Z_{1B}, Z_{1C}, and Z_{1D} are substituents shown in Table 1-1, Table 1-2, Table 1-3, and Table 1-4, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₂ is a substituent shown in Table 7, and m is combined from 0, 1, and 2.

A compound represented by formula (1-43), which is formula (1) where B is B-4, G₅, G₆, G₇, and G₈ are CR₅, CR₆, CR₇, and CR₈, respectively, and D is D-3, a salt thereof or an N-oxide thereof 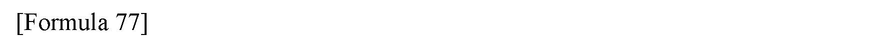 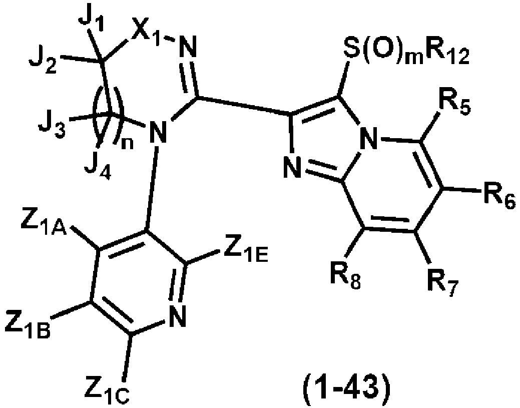 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1C}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-43), which is formula (1) where B is B-4, G₅, G₆, G₇, and G₈ are CR₅, CR₆, CR₇, and CR₈, respectively, and D is D-3, Z_{1A}, Z_{1B}, Z_{1C}, and Z_{1E} are substituents shown in Table 1-1, Table 1-2, Table 1-3, and Table 1-5, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₂ is a substituent shown in Table 7, and m is combined from 0, 1, and 2.

A compound represented by formula (1-44), which is formula (1) where B is B-4, G₅, G₆, G₇, and G₈ are CR₅, CR₆, CR₇, and CR₈, respectively, and D is D-4, a salt thereof or an N-oxide thereof 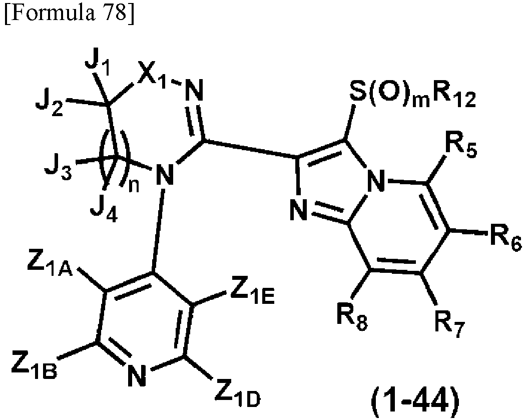 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1D}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and m is an integer of 0 to 2,
provided that when R₁₂ is an NYₐY_{b} group, m is 1 or 2.

The compound of the present invention wherein in formula (1-44), which is formula (1) where B is B-4, G₅, G₆, G₇, and G₈ are CR₅, CR₆, CR₇, and CR₈, respectively, and D is D-4, Z_{1A}, Z_{1B}, Z_{1D}, and Z_{1E} are substituents shown in Table 1-1, Table 1-2, Table 1-4, and Table 1-5, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₂ is a substituent shown in Table 7, and m is combined from 0, 1, and 2.

A compound represented by formula (1-45), which is formula (1) where B is B-4, G₅, G₆, G₇, and G₈ are CR₅, CR₆, CR₇, and CR₈, respectively, and D is D-5, a salt thereof or an N-oxide thereof 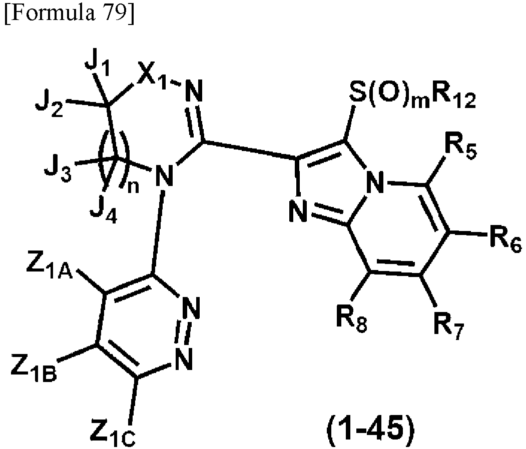 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, and Z_{1C} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-45), which is formula (1) where B is B-4, G₅, G₆, G₇, and G₈ are CR₅, CR₆, CR₇, and CR₈, respectively, and D is D-5, Z_{1A}, Z_{1B}, and Z_{1C} are substituents shown in Table 1-1, Table 1-2, and Table 1-3, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₂ is a substituent shown in Table 7, and m is combined from 0, 1, and 2.

A compound represented by formula (1-46), which is formula (1) where B is B-4, G₅, G₆, G₇, and G₈ are CR₅, CR₆, CR₇, and CR₈, respectively, and D is D-6, a salt thereof or an N-oxide thereof 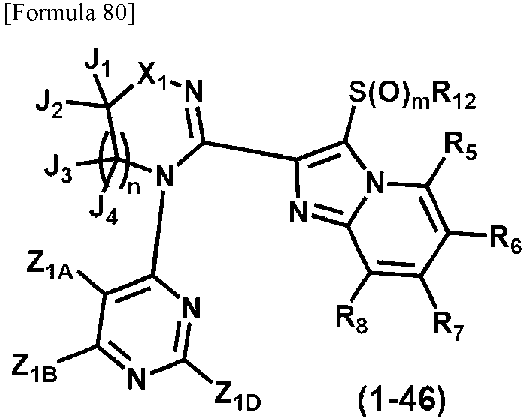 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-46), which is formula (1) where B is B-4, G₅, G₆, G₇, and G₈ are CR₅, CR₆, CR₇, and CR₈, respectively, and D is D-6, Z_{1A}, Z_{1B}, and Z_{1D} are substituents shown in Table 1-1, Table 1-2, and Table 1-4, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₂ is a substituent shown in Table 7, and m is combined from 0, 1, and 2.

A compound represented by formula (1-47), which is formula (1) where B is B-4, G₅, G₆, G₇, and G₈ are CR₅, CR₆, CR₇, and CR₈, respectively, and D is D-7, a salt thereof or an N-oxide thereof 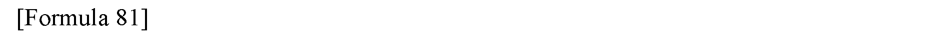 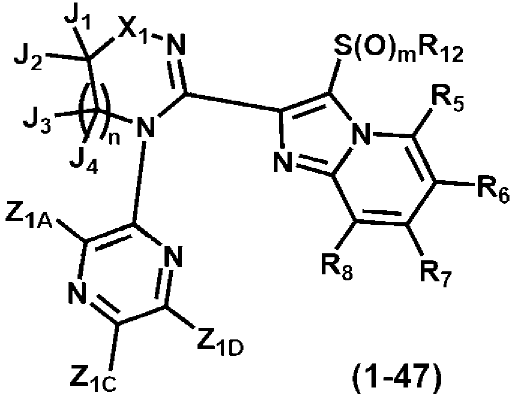 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1C}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-47), which is formula (1) where B is B-4, G₅, G₆, G₇, and G₈ are CR₅, CR₆, CR₇, and CR₈, respectively, and D is D-7, Z_{1A}, Z_{1C}, and Z_{1D} are substituents shown in Table 1-1, Table 1-3, and Table 1-4, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₂ is a substituent shown in Table 7, and m is combined from 0, 1, and 2.

A compound represented by formula (1-48), which is formula (1) where B is B-4, G₅, G₆, G₇, and G₈ are CR₅, CR₆, CR₇, and CR₈, respectively, and D is D-8, a salt thereof or an N-oxide thereof 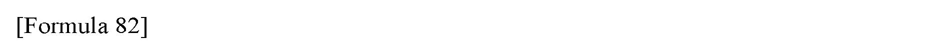 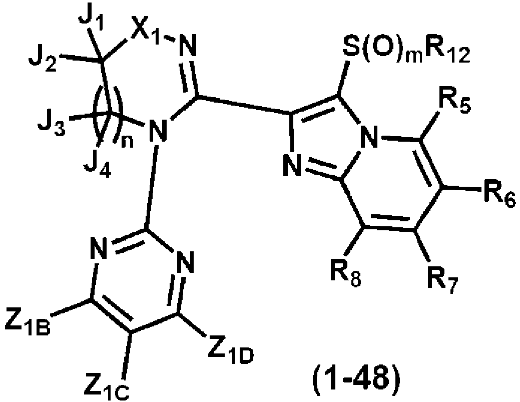 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1B}, Z_{1C}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-48), which is formula (1) where B is B-4, G₅, G₆, G₇, and G₈ are CR₅, CR₆, CR₇, and CR₈, respectively, and D is D-8, Z_{1B}, Z_{1C}, and Z_{1D} are substituents shown in Table 1-2, Table 1-3, and Table 1-4, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₂ is a substituent shown in Table 7, and m is combined from 0, 1, and 2.

A compound represented by formula (1-49), which is formula (1) where B is B-4, G₅, G₆, G₇, and G₈ are CR₅, CR₆, CR₇, and CR₈, respectively, and D is D-9, a salt thereof or an N-oxide thereof 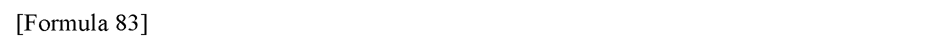 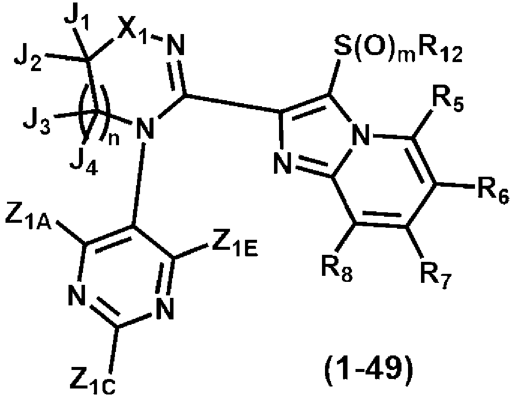 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1C}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-49), which is formula (1) where B is B-4, G₅, G₆, G₇, and G₈ are CR₅, CR₆, CR₇, and CR₈, respectively, and D is D-9, Z_{1A}, Z_{1C}, and Z_{1E} are substituents shown in Table 1-1, Table 1-3, and Table 1-5, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₂ is a substituent shown in Table 7, and m is combined from 0, 1, and 2.

A compound represented by formula (1-50), which is formula (1) where B is B-4, G₅, G₆, G₇, and G₈ are CR₅, CR₆, CR₇, and CR₈, respectively, and D is D-10, a salt thereof or an N-oxide thereof 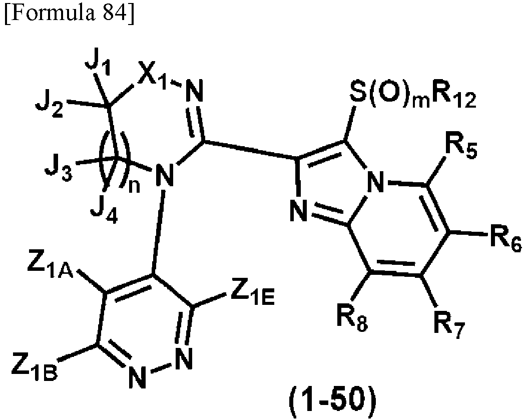 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-50), which is formula (1) where B is B-4, G₅, G₆, G₇, and G₈ are CR₅, CR₆, CR₇, and CR₈, respectively, and D is D-10, Z_{1A}, Z_{1B}, and Z_{1E} are substituents shown in Table 1-1, Table 1-2, and Table 1-5, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₂ is a substituent shown in Table 7, and m is combined from 0, 1, and 2.

A compound represented by formula (1-51), which is formula (1) where B is B-5, G₅, G₆, G₇, and G₈ are CR₅, CR₆, CR₇, and CR₈, respectively, and D is D-1, a salt thereof or an N-oxide thereof 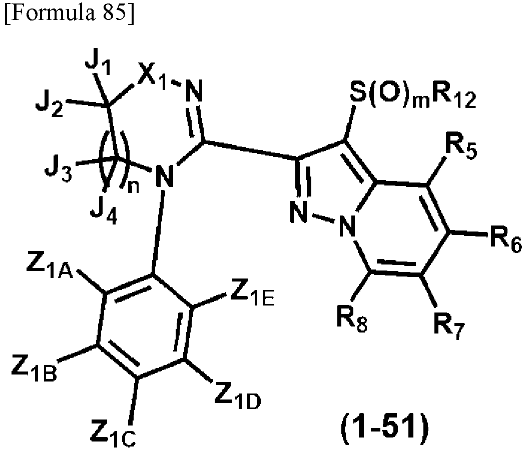 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1C}, Z_{1D}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-51), which is formula (1) where B is B-5, G₅, G₆, G₇, and G₈ are CR₅, CR₆, CR₇, and CR₈, respectively, and D is D-1, Z_{1A}, Z_{1B}, Z_{1C}, Z_{1D}, and Z_{1E} are substituents shown in Table 1-1, Table 1-2, Table 1-3, Table 1-4, and Table 1-5, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₂ is a substituent shown in Table 7, and m is combined from 0, 1, and 2.

A compound represented by formula (1-52), which is formula (1) where B is B-5, G₅, G₆, G₇, and G₈ are CR₅, CR₆, CR₇, and CR₈, respectively, and D is D-2, a salt thereof or an N-oxide thereof 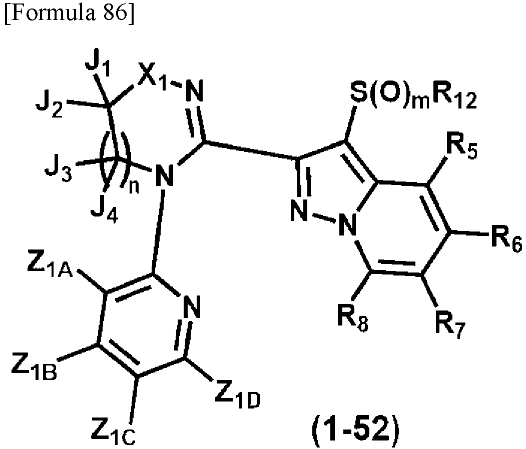 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1C}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-52), which is formula (1) where B is B-5, G₅, G₆, G₇, and G₈ are CR₅, CR₆, CR₇, and CR₈, respectively, and D is D-2, Z_{1A}, Z_{1B}, Z_{1C}, and Z_{1D} are substituents shown in Table 1-1, Table 1-2, Table 1-3, and Table 1-4, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₂ is a substituent shown in Table 7, and m is combined from 0, 1, and 2.

A compound represented by formula (1-53), which is formula (1) where B is B-5, G₅, G₆, G₇, and G₈ are CR₅, CR₆, CR₇, and CR₈, respectively, and D is D-3, a salt thereof or an N-oxide thereof 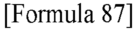 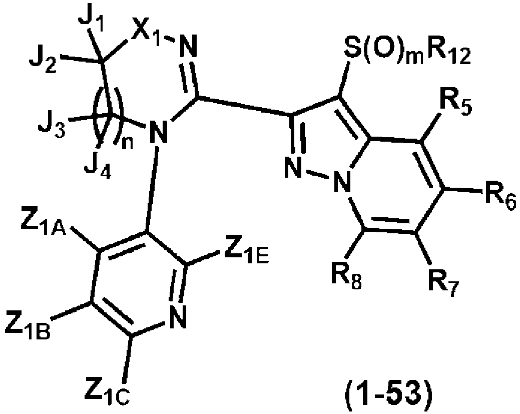 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1C}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-53), which is formula (1) where B is B-5, G₅, G₆, G₇, and G₈ are CR₅, CR₆, CR₇, and CR₈, respectively, and D is D-3, Z_{1A}, Z_{1B}, Z_{1C}, and Z_{1E} are substituents shown in Table 1-1, Table 1-2, Table 1-3, and Table 1-5, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₂ is a substituent shown in Table 7, and m is combined from 0, 1, and 2.

A compound represented by formula (1-54), which is formula (1) where B is B-5, G₅, G₆, G₇, and G₈ are CR₅, CR₆, CR₇, and CR₈, respectively, and D is D-4, a salt thereof or an N-oxide thereof 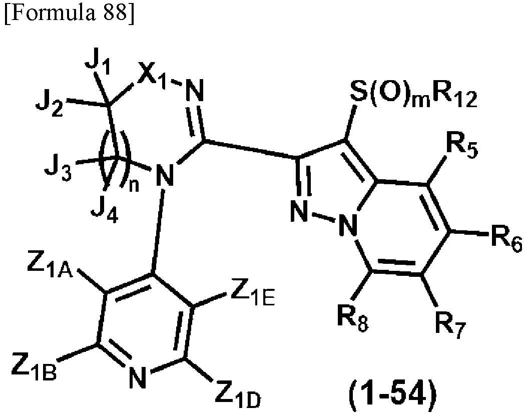 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1D}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-54), which is formula (1) where B is B-5, G₅, G₆, G₇, and G₈ are CR₅, CR₆, CR₇, and CR₈, respectively, and D is D-4, Z_{1A}, Z_{1B}, Z_{1D}, and Z_{1E} are substituents shown in Table 1-1, Table 1-2, Table 1-4, and Table 1-5, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₂ is a substituent shown in Table 7, and m is combined from 0, 1, and 2.

A compound represented by formula (1-55), which is formula (1) where B is B-5, G₅, G₆, G₇, and G₈ are CR₅, CR₆, CR₇, and CR₈, respectively, and D is D-5, a salt thereof or an N-oxide thereof 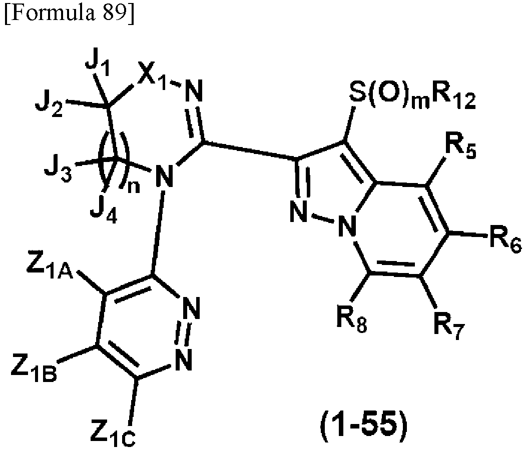 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, and Z_{1C} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-55), which is formula (1) where B is B-5, G₅, G₆, G₇, and G₈ are CR₅, CR₆, CR₇, and CR₈, respectively, and D is D-5, Z_{1A}, Z_{1B}, and Z_{1C} are substituents shown in Table 1-1, Table 1-2, and Table 1-3, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₂ is a substituent shown in Table 7, and m is combined from 0, 1, and 2.

A compound represented by formula (1-56), which is formula (1) where B is B-5, G₅, G₆, G₇, and G₈ are CR₅, CR₆, CR₇, and CR₈, respectively, and D is D-6, a salt thereof or an N-oxide thereof 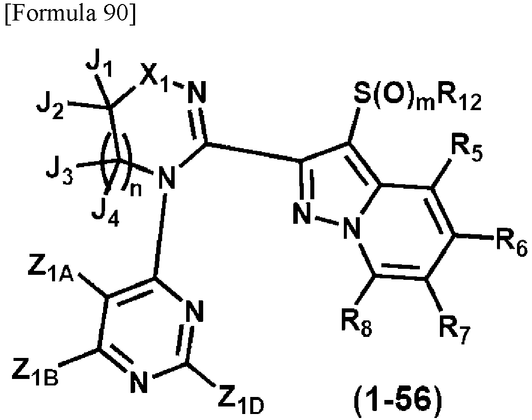 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-56), which is formula (1) where B is B-5, G₅, G₆, G₇, and G₈ are CR₅, CR₆, CR₇, and CR₈, respectively, and D is D-6, Z_{1A}, Z_{1B}, and Z_{1D} are substituents shown in Table 1-1, Table 1-2, and Table 1-4, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₂ is a substituent shown in Table 7, and m is combined from 0, 1, and 2.

A compound represented by formula (1-57), which is formula (1) where B is B-5, G₅, G₆, G₇, and G₈ are CR₅, CR₆, CR₇, and CR₈, respectively, and D is D-7, a salt thereof or an N-oxide thereof 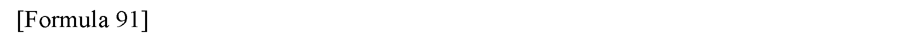 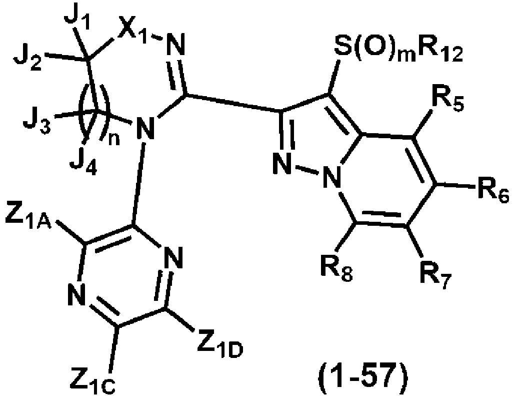 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1C}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-57), which is formula (1) where B is B-5, G₅, G₆, G₇, and G₈ are CR₅, CR₆, CR₇, and CR₈, respectively, and D is D-7, Z_{1A}, Z_{1C}, and Z_{1D} are substituents shown in Table 1-1, Table 1-3, and Table 1-4, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₂ is a substituent shown in Table 7, and m is combined from 0, 1, and 2.

A compound represented by formula (1-58), which is formula (1) where B is B-5, G₅, G₆, G₇, and G₈ are CR₅, CR₆, CR₇, and CR₈, respectively, and D is D-8, a salt thereof or an N-oxide thereof 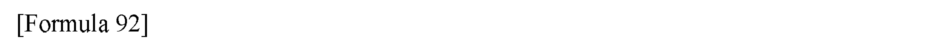 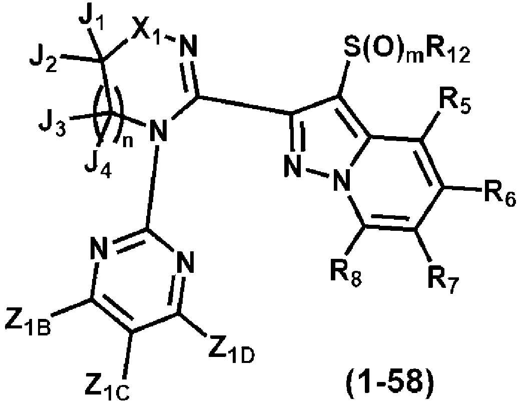 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1B}, Z_{1C}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-58), which is formula (1) where B is B-5, G₅, G₆, G₇, and G₈ are CR₅, CR₆, CR₇, and CR₈, respectively, and D is D-8, Z_{1B}, Z_{1C}, and Z_{1D} are substituents shown in Table 1-2, Table 1-3, and Table 1-4, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₂ is a substituent shown in Table 7, and m is combined from 0, 1, and 2.

A compound represented by formula (1-59), which is formula (1) where B is B-5, G₅, G₆, G₇, and G₈ are CR₅, CR₆, CR₇, and CR₈, respectively, and D is D-9, a salt thereof or an N-oxide thereof 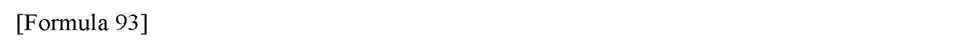 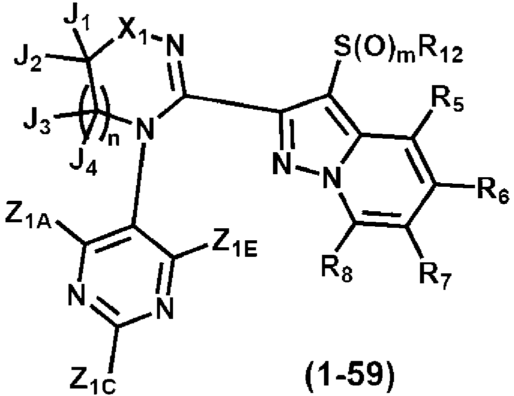 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1C}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-59), which is formula (1) where B is B-5, G₅, G₆, G₇, and G₈ are CR₅, CR₆, CR₇, and CR₈, respectively, and D is D-9, Z_{1A}, Z_{1C}, and Z_{1E} are substituents shown in Table 1-1, Table 1-3, and Table 1-5, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₂ is a substituent shown in Table 7, and m is combined from 0, 1, and 2.

A compound represented by formula (1-60), which is formula (1) where B is B-5, G₅, G₆, G₇, and G₈ are CR₅, CR₆, CR₇, and CR₈, respectively, and D is D-10, a salt thereof or an N-oxide thereof 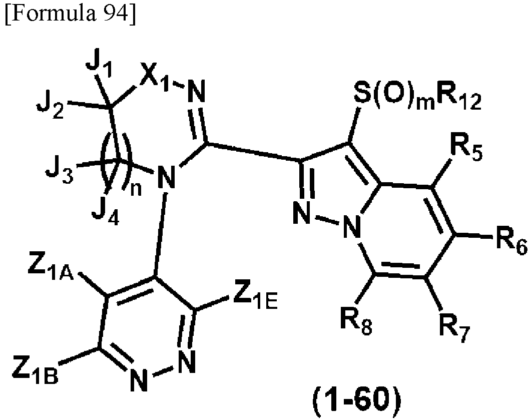 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-60), which is formula (1) where B is B-5, G₅, G₆, G₇, and G₈ are CR₅, CR₆, CR₇, and CR₈, respectively, and D is D-10, Z_{1A}, Z_{1B}, and Z_{1E} are substituents shown in Table 1-1, Table 1-2, and Table 1-5, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₂ is a substituent shown in Table 7, and m is combined from 0, 1, and 2.

A compound represented by formula (1-61), which is formula (1) where B is B-6, G₅, G₆, G₇, and G₈ are CR₅, CR₆, CR₇, and CR₈, respectively, and D is D-1, a salt thereof or an N-oxide thereof 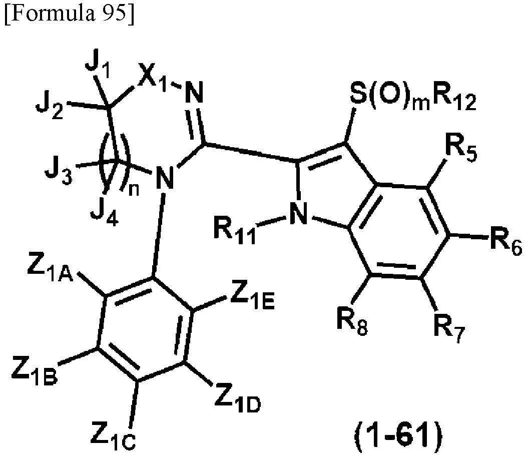 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1C}, Z_{1D}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₁ is a group selected from the group consisting of a hydrogen atom, a (C₁ to C₆) alkyl group, a halo (C₁ to C₆) alkyl group, a (C₃ to C₆) cycloalkyl group, a halo (C₃ to C₆) cycloalkyl group, a (C₁ to C₆) alkoxy group, a halo (C₁ to C₆) alkoxy group, a (C₁ to C₆) alkylcarbonyl group, a halo (C₁ to C₆) alkylcarbonyl group, a (C₄ to C₇) cycloalkylcarbonyl group, and a halo (C₄ to C₇) cycloalkylcarbonyl group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-61), which is formula (1) where B is B-3, G₅, G₆, G₇, and G₈ are CR₅, CR₆, CR₇, and CR₈, respectively, and D is D-1, Z_{1A}, Z_{1B}, Z_{1C}, Z_{1D}, and Z_{1E} are substituents shown in Table 1-1, Table 1-2, Table 1-3, Table 1-4, and Table 1-5, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₁ is a substituent shown in Table 12, R₁₂ is a substituent shown in Table 7, and m is combined from 0, 1, and 2.

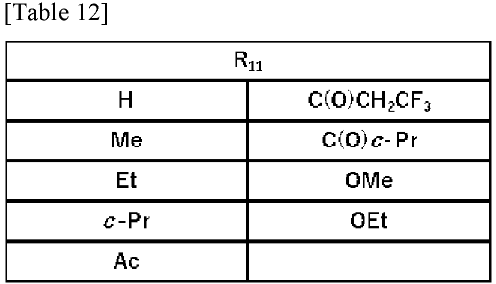

A compound represented by formula (1-62), which is formula (1) where B is B-6, G₅, G₆, G₇, and G₈ are CR₅, CR₆, CR₇, and CR₈, respectively, and D is D-2, a salt thereof or an N-oxide thereof 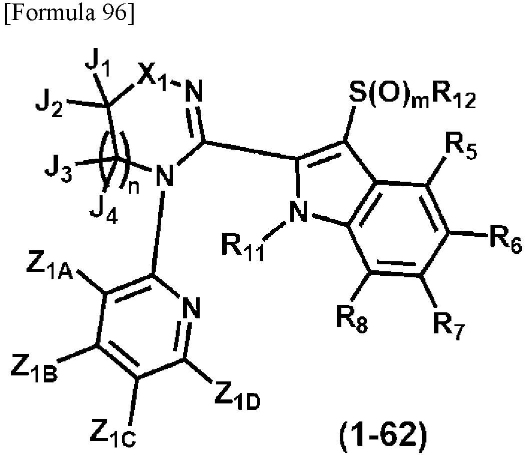 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1C}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₁ is a group selected from the group consisting of a hydrogen atom, a (C₁ to C₆) alkyl group, a halo (C₁ to C₆) alkyl group, a (C₃ to C₆) cycloalkyl group, a halo (C₃ to C₆) cycloalkyl group, a (C₁ to C₆) alkoxy group, a halo (C₁ to C₆) alkoxy group, a (C₁ to C₆) alkylcarbonyl group, a halo (C₁ to C₆) alkylcarbonyl group, a (C₄ to C₇) cycloalkylcarbonyl group, and a halo (C₄ to C₇) cycloalkylcarbonyl group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or
   a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-62), which is formula (1) where B is B-6, G₅, G₆, G₇, and G₈ are CR₅, CR₆, CR₇, and CR₈, respectively, and D is D-2, Z_{1A}, Z_{1B}, Z_{1C}, and Z_{1D} are substituents shown in Table 1-1, Table 1-2, Table 1-3, and Table 1-4, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₁ is a substituent shown in Table 12, R₁₂ is a substituent shown in Table 7, and m is combined from 0, 1, and 2.

A compound represented by formula (1-63), which is formula (1) where B is B-6, G₅, G₆, G₇, and G₈ are CR₅, CR₆, CR₇, and CR₈, respectively, and D is D-3, a salt thereof or an N-oxide thereof 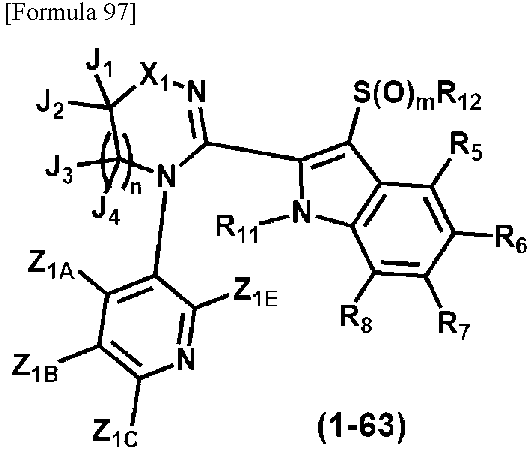 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1C}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₁ is a group selected from the group consisting of a hydrogen atom, a (C₁ to C₆) alkyl group, a halo (C₁ to C₆) alkyl group, a (C₃ to C₆) cycloalkyl group, a halo (C₃ to C₆) cycloalkyl group, a (C₁ to C₆) alkoxy group, a halo (C₁ to C₆) alkoxy group, a (C₁ to C₆) alkylcarbonyl group, a halo (C₁ to C₆) alkylcarbonyl group, a (C₄ to C₇) cycloalkylcarbonyl group, and a halo (C₄ to C₇) cycloalkylcarbonyl group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-63), which is formula (1) where B is B-6, G₅, G₆, G₇, and G₈ are CR₅, CR₆, CR₇, and CR₈, respectively, and D is D-3, Z_{1A}, Z_{1B}, Z_{1C}, and Z_{1E} are substituents shown in Table 1-1, Table 1-2, Table 1-3, and Table 1-5, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₁ is a substituent shown in Table 12, R₁₂ is a substituent shown in Table 7, and m is combined from 0, 1, and 2.

A compound represented by formula (1-64), which is formula (1) where B is B-6, G₅, G₆, G₇, and G₈ are CR₅, CR₆, CR₇, and CR₈, respectively, and D is D-4, a salt thereof or an N-oxide thereof 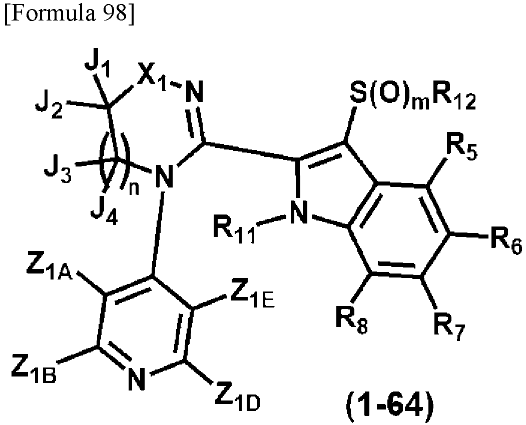 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1D}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₁ is a group selected from the group consisting of a hydrogen atom, a (C₁ to C₆) alkyl group, a halo (C₁ to C₆) alkyl group, a (C₃ to C₆) cycloalkyl group, a halo (C₃ to C₆) cycloalkyl group, a (C₁ to C₆) alkoxy group, a halo (C₁ to C₆) alkoxy group, a (C₁ to C₆) alkylcarbonyl group, a halo (C₁ to C₆) alkylcarbonyl group, a (C₄ to C₇) cycloalkylcarbonyl group, and a halo (C₄ to C₇) cycloalkylcarbonyl group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-64), which is formula (1) where B is B-6, G₅, G₆, G₇, and G₈ are CR₅, CR₆, CR₇, and CR₈, respectively, and D is D-4, Z_{1A}, Z_{1B}, Z_{1D}, and Z_{1E} are substituents shown in Table 1-1, Table 1-2, Table 1-4, and Table 1-5, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₁ is a substituent shown in Table 12, R₁₂ is a substituent shown in Table 7, and m is combined from 0, 1, and 2.

A compound represented by formula (1-65), which is formula (1) where B is B-6, G₅, G₆, G₇, and G₈ are CR₅, CR₆, CR₇, and CR₈, respectively, and D is D-5, a salt thereof or an N-oxide thereof 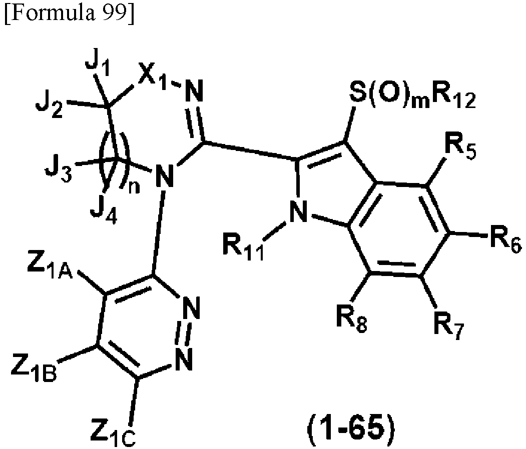 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, and Z_{1C} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₁ is a group selected from the group consisting of a hydrogen atom, a (C₁ to C₆) alkyl group, a halo (C₁ to C₆) alkyl group, a (C₃ to C₆) cycloalkyl group, a halo (C₃ to C₆) cycloalkyl group, a (C₁ to C₆) alkoxy group, a halo (C₁ to C₆) alkoxy group, a (C₁ to C₆) alkylcarbonyl group, a halo (C₁ to C₆) alkylcarbonyl group, a (C₄ to C₇) cycloalkylcarbonyl group, and a halo (C₄ to C₇) cycloalkylcarbonyl group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-65), which is formula (1) where B is B-6, G₅, G₆, G₇, and G₈ are CR₅, CR₆, CR₇, and CR₈, respectively, and D is D-5, Z_{1A}, Z_{1B}, and Z_{1C} are substituents shown in Table 1-1, Table 1-2, and Table 1-3, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₁ is a substituent shown in Table 12, R₁₂ is a substituent shown in Table 7, and m is combined from 0, 1, and 2.

A compound represented by formula (1-66), which is formula (1) where B is B-6, G₅, G₆, G₇, and G₈ are CR₅, CR₆, CR₇, and CR₈, respectively, and D is D-6, a salt thereof or an N-oxide thereof 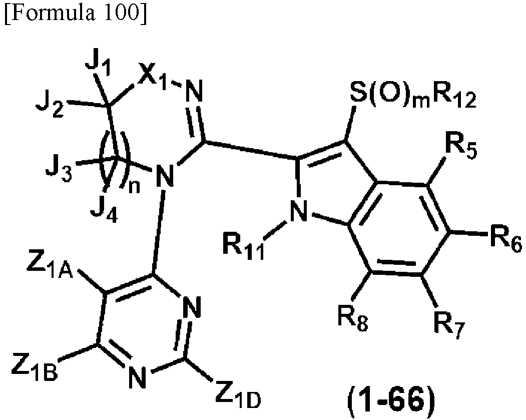 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₁ is a group selected from the group consisting of a hydrogen atom, a (C₁ to C₆) alkyl group, a halo (C₁ to C₆) alkyl group, a (C₃ to C₆) cycloalkyl group, a halo (C₃ to C₆) cycloalkyl group, a (C₁ to C₆) alkoxy group, a halo (C₁ to C₆) alkoxy group, a (C₁ to C₆) alkylcarbonyl group, a halo (C₁ to C₆) alkylcarbonyl group, a (C₄ to C₇) cycloalkylcarbonyl group, and a halo (C₄ to C₇) cycloalkylcarbonyl group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-66), which is formula (1) where B is B-6, G₅, G₆, G₇, and G₈ are CR₅, CR₆, CR₇, and CR₈, respectively, and D is D-6, Z_{1A}, Z_{1B}, and Z_{1D} are substituents shown in Table 1-1, Table 1-2, and Table 1-4, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₁ is a substituent shown in Table 12, R₁₂ is a substituent shown in Table 7, and m is combined from 0, 1, and 2.

A compound represented by formula (1-67), which is formula (1) where B is B-6, G₅, G₆, G₇, and G₈ are CR₅, CR₆, CR₇, and CR₈, respectively, and D is D-7, a salt thereof or an N-oxide thereof 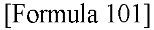 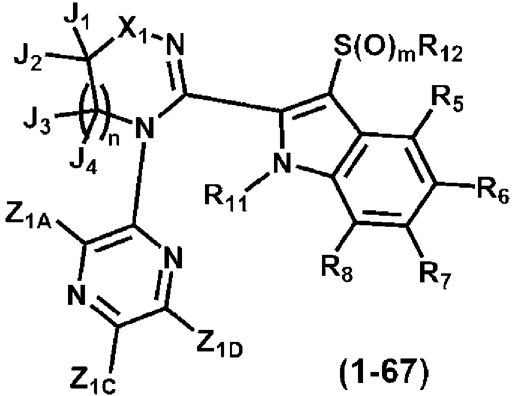 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1C}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₁ is a group selected from the group consisting of a hydrogen atom, a (C₁ to C₆) alkyl group, a halo (C₁ to C₆) alkyl group, a (C₃ to C₆) cycloalkyl group, a halo (C₃ to C₆) cycloalkyl group, a (C₁ to C₆) alkoxy group, a halo (C₁ to C₆) alkoxy group, a (C₁ to C₆) alkylcarbonyl group, a halo (C₁ to C₆) alkylcarbonyl group, a (C₄ to C₇) cycloalkylcarbonyl group, and a halo (C₄ to C₇) cycloalkylcarbonyl group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or
   a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-67), which is formula (1) where B is B-6, G₅, G₆, G₇, and G₈ are CR₅, CR₆, CR₇, and CR₈, respectively, and D is D-7, Z_{1A}, Z_{1C}, and Z_{1D}, are substituents shown in Table 1-1, Table 1-3, and Table 1-4, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₁ is a substituent shown in Table 12, R₁₂ is a substituent shown in Table 7, and m is combined from 0, 1, and 2.

A compound represented by formula (1-68), which is formula (1) where B is B-6, G₅, G₆, G₇, and G₈ are CR₅, CR₆, CR₇, and CR₈, respectively, and D is D-8, a salt thereof or an N-oxide thereof 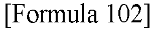 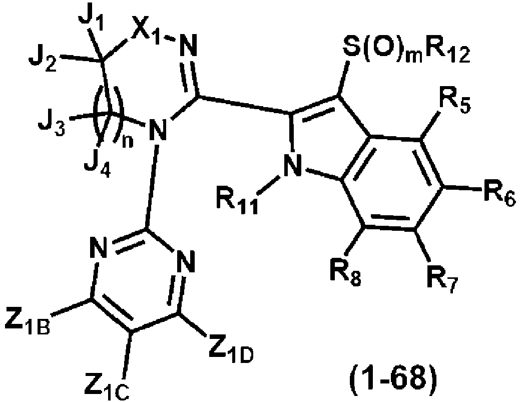 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1B}, Z_{1C}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₁ is a group selected from the group consisting of a hydrogen atom, a (C₁ to C₆) alkyl group, a halo (C₁ to C₆) alkyl group, a (C₃ to C₆) cycloalkyl group, a halo (C₃ to C₆) cycloalkyl group, a (C₁ to C₆) alkoxy group, a halo (C₁ to C₆) alkoxy group, a (C₁ to C₆) alkylcarbonyl group, a halo (C₁ to C₆) alkylcarbonyl group, a (C₄ to C₇) cycloalkylcarbonyl group, and a halo (C₄ to C₇) cycloalkylcarbonyl group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-68), which is formula (1) where B is B-6, G₅, G₆, G₇, and G₈ are CR₅, CR₆, CR₇, and CR₈, respectively, and D is D-8, Z_{1B}, Z_{1C}, and Z_{1D} are substituents shown in Table 1-2, Table 1-3, and Table 1-4, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₁ is a substituent shown in Table 12, R₁₂ is a substituent shown in Table 7, and m is combined from 0, 1, and 2.

A compound represented by formula (1-69), which is formula (1) where B is B-6, G₅, G₆, G₇, and G₈ are CR₅, CR₆, CR₇, and CR₈, respectively, and D is D-9, a salt thereof or an N-oxide thereof 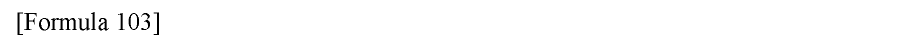 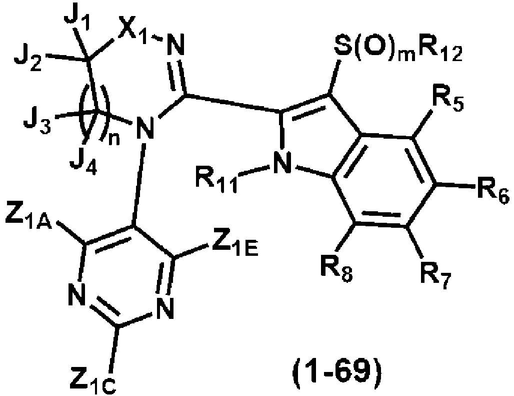 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1C}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₁ is a group selected from the group consisting of a hydrogen atom, a (C₁ to C₆) alkyl group, a halo (C₁ to C₆) alkyl group, a (C₃ to C₆) cycloalkyl group, a halo (C₃ to C₆) cycloalkyl group, a (C₁ to C₆) alkoxy group, a halo (C₁ to C₆) alkoxy group, a (C₁ to C₆) alkylcarbonyl group, a halo (C₁ to C₆) alkylcarbonyl group, a (C₄ to C₇) cycloalkylcarbonyl group, and a halo (C₄ to C₇) cycloalkylcarbonyl group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-69), which is formula (1) where B is B-6, G₅, G₆, G₇, and G₈ are CR₅, CR₆, CR₇, and CR₈, respectively, and D is D-9, Z_{1A}, Z_{1C}, and Z_{1E} are substituents shown in Table 1-1, Table 1-3, and Table 1-5, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₁ is a substituent shown in Table 12, R₁₂ is a substituent shown in Table 7, and m is combined from 0, 1, and 2.

A compound represented by formula (1-70), which is formula (1) where B is B-6, G₅, G₆, G₇, and G₈ are CR₅, CR₆, CR₇, and CR₈, respectively, and D is D-10, a salt thereof or an N-oxide thereof 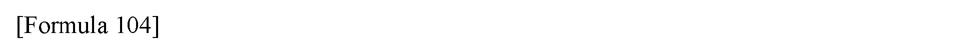 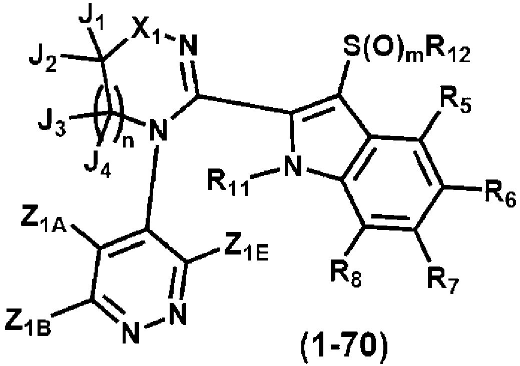 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₅, R₆, R₇, and R₈ are each independently a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₂ to C₆) alkynyl optionally having a substituent, a halo (C₂ to C₆) alkynyl optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a -C(O)NY₄Y₅ group, a -CH=NY₆ group, a cyano group, a nitro group, a formyl group, a hydroxy group, a mercapto group, a formyl group, a carboxy group, and an SF₅ group,
Y₃, Y₄, Y₅, and Y₆ are defined as above,
R₁₁ is a group selected from the group consisting of a hydrogen atom, a (C₁ to C₆) alkyl group, a halo (C₁ to C₆) alkyl group, a (C₃ to C₆) cycloalkyl group, a halo (C₃ to C₆) cycloalkyl group, a (C₁ to C₆) alkoxy group, a halo (C₁ to C₆) alkoxy group, a (C₁ to C₆) alkylcarbonyl group, a halo (C₁ to C₆) alkylcarbonyl group, a (C₄ to C₇) cycloalkylcarbonyl group, and a halo (C₄ to C₇) cycloalkylcarbonyl group,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or
   a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-70), which is formula (1) where B is B-6, G₅, G₆, G₇, and G₈ are CR₅, CR₆, CR₇, and CR₈, respectively, and D is D-10, Z_{1A}, Z_{1B}, and Z_{1E} are substituents shown in Table 1-1, Table 1-2, and Table 1-5, respectively, X₁ is a structure shown in Table 2, J₁ and J₂ are substituents shown in Table 3-1, J₃ and J₄ are substituents shown in Table 3-2, n is 0 or 1, R₅, R₆, R₇, and R₈ are each independently a substituent shown in Table 9-1 and Table 9-2, R₁₁ is a substituent shown in Table 12, R₁₂ is a substituent shown in Table 7, and m is combined from 0, 1, and 2.

A compound represented by formula (1-71), which is formula (1) where B is B-8 and D is D-1, a salt thereof or an N-oxide thereof 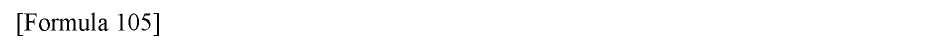 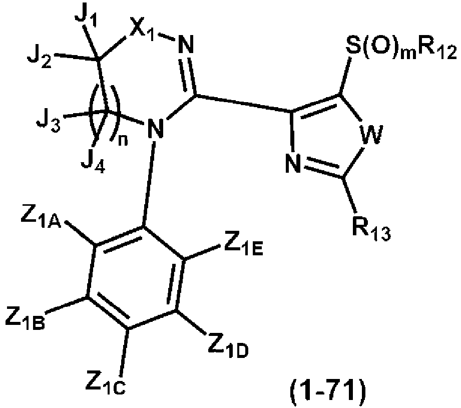 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1C}, Z_{1D}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₁₃ is a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkynyl group optionally having a substituent, a halo (C₂ to C₆) alkynyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(O)NY₄Y₅ group, a -CH=NY₆ group, a cyano group, a nitro group, a formyl group, a hydroxy group, a mercapto group, a formyl group, a carboxy group, and an SF₅ group,
W is an oxygen atom, a sulfur atom, or NY₇,
Y₃, Y₄, Y₅, Y₆, and Y₇ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-71), which is formula (1) where B is B-8 and D is D-1, Z_{1A}, Z_{1B}, Z_{1C}, Z_{1D}, and Z_{1E} are substituents shown in Table 1-1, Table 1-2, Table 1-3, Table 1-4, and Table 1-5, respectively, X₁ is a structure shown in Table 2, J₁ and J₂ are substituents shown in Table 3-1, J₃ and J₄ are substituents shown in Table 3-2, n is 0 or 1, R₁₃ is a substituent shown in Table 13-1 to Table 13-2, W is a substituent shown in Table 14, R₁₂ is a substituent shown in Table 7, and m is combined from 0, 1, and 2.

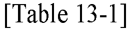

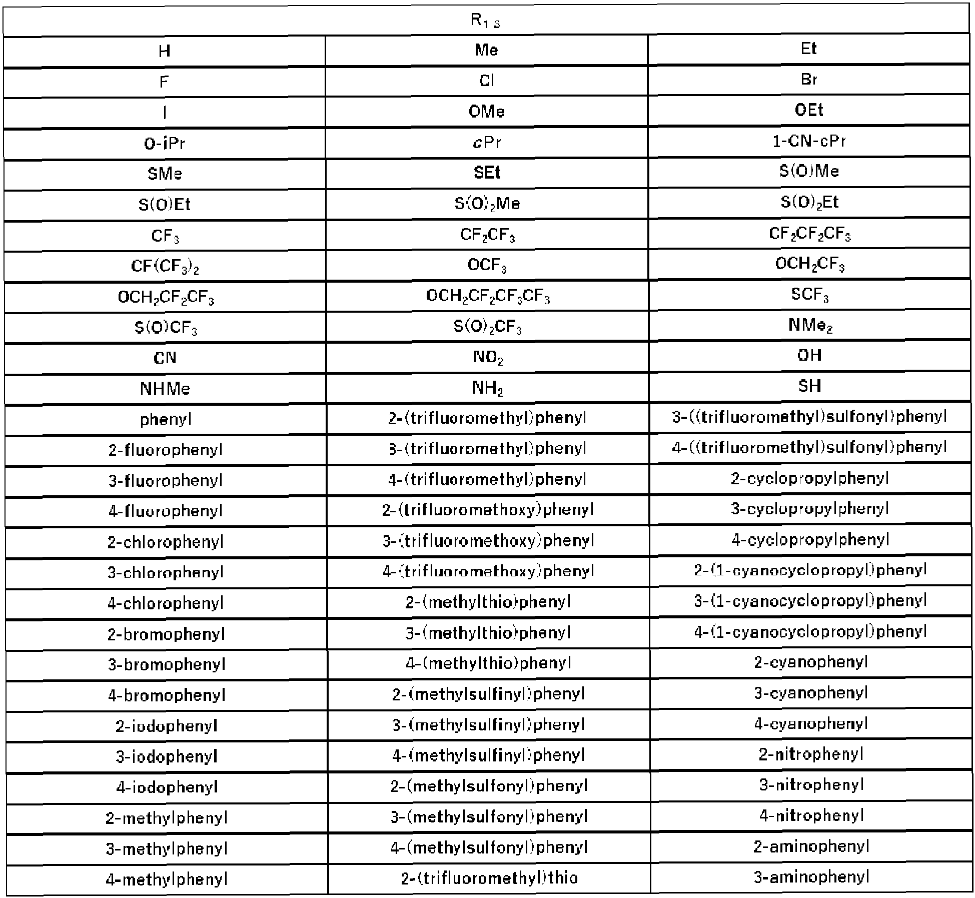

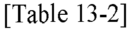

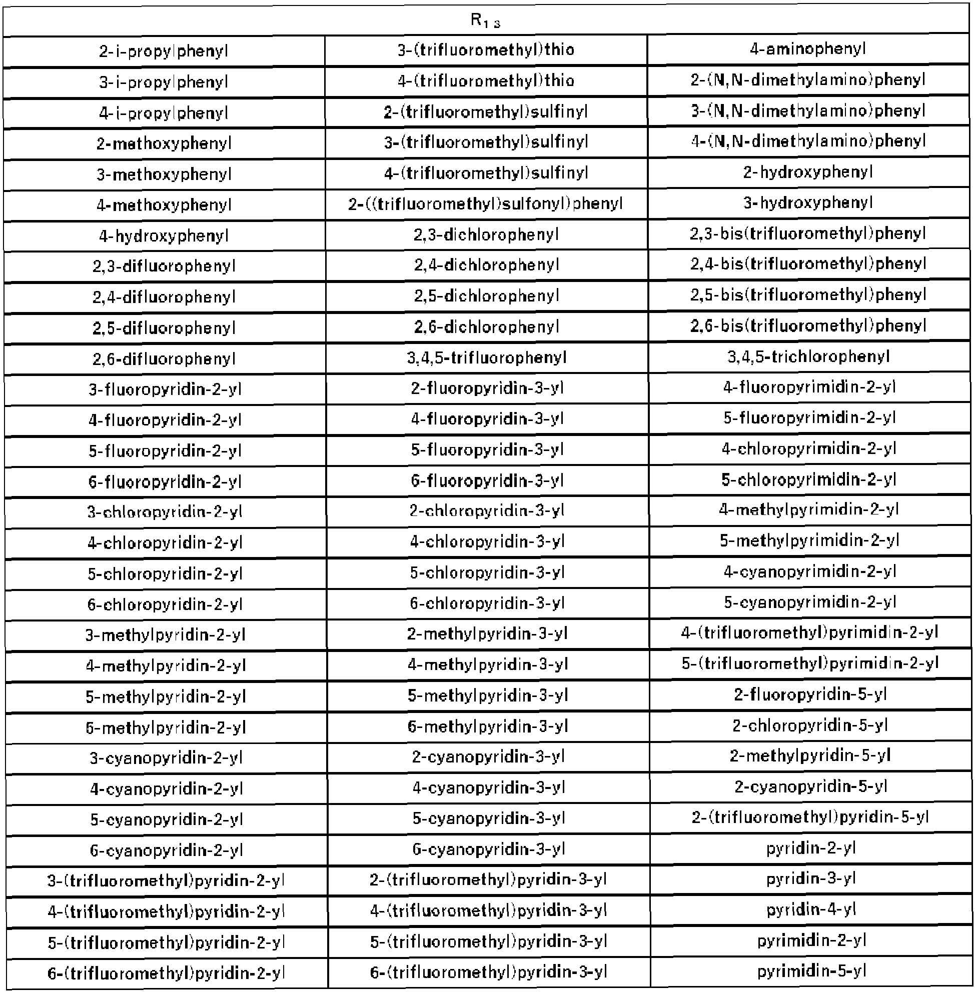

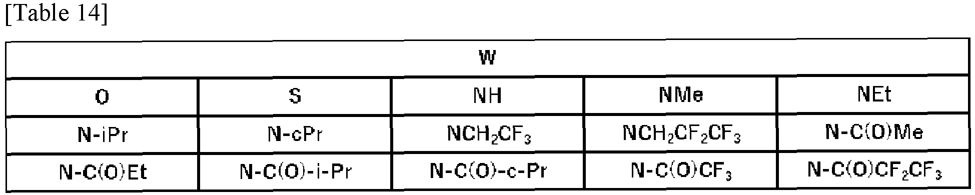

A compound represented by formula (1-72), which is formula (1) where B is B-8 and D is D-2, a salt thereof or an N-oxide thereof 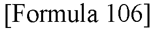 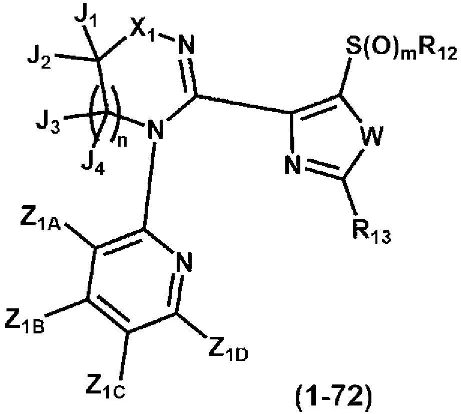 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1C}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₁₃ is a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkynyl group optionally having a substituent, a halo (C₂ to C₆) alkynyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(O)NY₄Y₅ group, a -CH=NY₆ group, a cyano group, a nitro group, a formyl group, a hydroxy group, a mercapto group, a formyl group, a carboxy group, and an SF₅ group,
W is an oxygen atom, a sulfur atom, or NY₇,
Y₃, Y₄, Y₅, Y₆, and Y₇ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-72), which is formula (1) where B is B-8 and D is D-2, Z_{1A}, Z_{1B}, Z_{1C}, and Z_{1D} are substituents shown in Table 1-1, Table 1-2, Table 1-3, and Table 1-4, respectively, X₁ is a structure shown in Table 2, J₁ and J₂ are substituents shown in Table 3-1, J₃ and J₄ are substituents shown in Table 3-2, n is 0 or 1, R₁₃ is a substituent shown in Table 13-1 to Table 13-2, W is a substituent shown in Table 14, R₁₂ is a substituent shown in Table 7, and m is combined from 0, 1, and 2.

A compound represented by formula (1-73), which is formula (1) where B is B-8 and D is D-3, a salt thereof or an N-oxide thereof 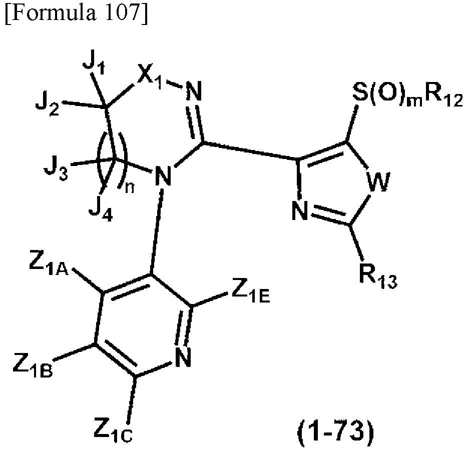 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1C}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₁₃ is a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkynyl group optionally having a substituent, a halo (C₂ to C₆) alkynyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(O)NY₄Y₅ group, a -CH=NY₆ group, a cyano group, a nitro group, a formyl group, a hydroxy group, a mercapto group, a formyl group, a carboxy group, and an SF₅ group,
W is an oxygen atom, a sulfur atom, or NY₇,
Y₃, Y₄, Y₅, Y₆, and Y₇ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-73), which is formula (1) where B is B-8 and D is D-3, Z₁ₐ, Z_{1B}, Z_{1C}, and Z_{1E} are substituents shown in Table 1-1, Table 1-2, Table 1-3, and Table 1-5, respectively, X₁ is a structure shown in Table 2, J₁ and J₂ are substituents shown in Table 3-1, J₃ and J₄ are substituents shown in Table 3-2, n is 0 or 1, R₁₃ is a substituent shown in Table 13-1 to Table 13-2, W is a substituent shown in Table 14, R₁₂ is a substituent shown in Table 7, and m is combined from 0, 1, and 2.

A compound represented by formula (1-74), which is formula (1) where B is B-8 and D is D-4, a salt thereof or an N-oxide thereof 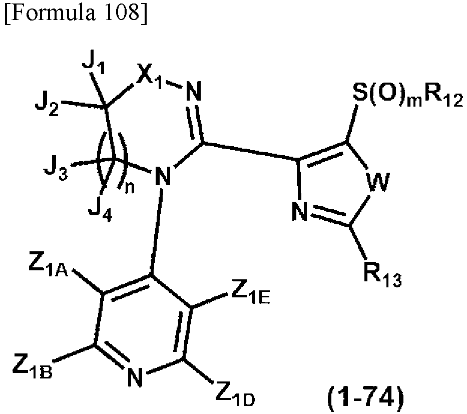 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, Z_{1D}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₁₃ is a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkynyl group optionally having a substituent, a halo (C₂ to C₆) alkynyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(O)NY₄Y₅ group, a -CH=NY₆ group, a cyano group, a nitro group, a formyl group, a hydroxy group, a mercapto group, a formyl group, a carboxy group, and an SF₅ group,
W is an oxygen atom, a sulfur atom, or NY₇,
Y₃, Y₄, Y₅, Y₆, and Y₇ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-74), which is formula (1) where B is B-8 and D is D-4, Z_{1A}, Z_{1B}, Z_{1D}, and Z_{1E} are substituents shown in Table 1-1, Table 1-2, Table 1-4, and Table 1-5, respectively, X₁ is a structure shown in Table 2, J₁ and J₂ are substituents shown in Table 3-1, J₃ and J₄ are substituents shown in Table 3-2, n is 0 or 1, R₁₃ is a substituent shown in Table 13-1 to Table 13-2, W is a substituent shown in Table 14, R₁₂ is a substituent shown in Table 7, and m is combined from 0, 1, and 2.

A compound represented by formula (1-75), which is formula (1) where B is B-8 and D is D-5, a salt thereof or an N-oxide thereof 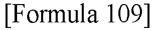 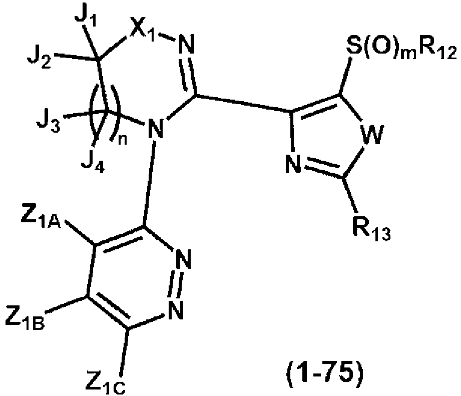 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, and Z_{1C} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₁₃ is a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkynyl group optionally having a substituent, a halo (C₂ to C₆) alkynyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(O)NY₄Y₅ group, a -CH=NY₆ group, a cyano group, a nitro group, a formyl group, a hydroxy group, a mercapto group, a formyl group, a carboxy group, and an SF₅ group,
W is an oxygen atom, a sulfur atom, or NY₇,
Y₃, Y₄, Y₅, Y₆, and Y₇ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-75), which is formula (1) where B is B-8 and D is D-5, Z_{1A}, Z_{1B}, and Z_{1C} are substituents shown in Table 1-1, Table 1-2, and Table 1-3, respectively, X₁ is a structure shown in Table 2, J₁ and J₂ are substituents shown in Table 3-1, J₃ and J₄ are substituents shown in Table 3-2, n is 0 or 1, R₁₃ is a substituent shown in Table 13-1 to Table 13-2, W is a substituent shown in Table 14, R₁₂ is a substituent shown in Table 7, and m is combined from 0, 1, and 2.

A compound represented by formula (1-76), which is formula (1) where B is B-8 and D is D-6, a salt thereof or an N-oxide thereof 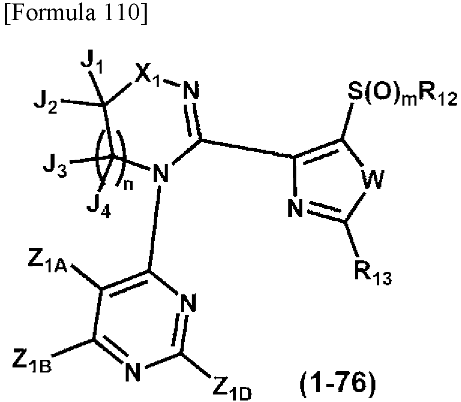 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₁₃ is a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkynyl group optionally having a substituent, a halo (C₂ to C₆) alkynyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(O)NY₄Y₅ group, a -CH=NY₆ group, a cyano group, a nitro group, a formyl group, a hydroxy group, a mercapto group, a formyl group, a carboxy group, and an SF₅ group,
W is an oxygen atom, a sulfur atom, or NY₇,
Y₃, Y₄, Y₅, Y₆, and Y₇ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-76), which is formula (1) where B is B-8 and D is D-6, Z_{1A}, Z_{1B}, and Z_{1D} are substituents shown in Table 1-1, Table 1-2, and Table 1-4, respectively, X₁ is a structure shown in Table 2, J₁ and J₂ are substituents shown in Table 3-1, J₃ and J₄ are substituents shown in Table 3-2, n is 0 or 1, R₁₃ is a substituent shown in Table 13-1 to Table 13-2, W is a substituent shown in Table 14, R₁₂ is a substituent shown in Table 7, and m is combined from 0, 1, and 2.

A compound represented by formula (1-77), which is formula (1) where B is B-8 and D is D-7, a salt thereof or an N-oxide thereof 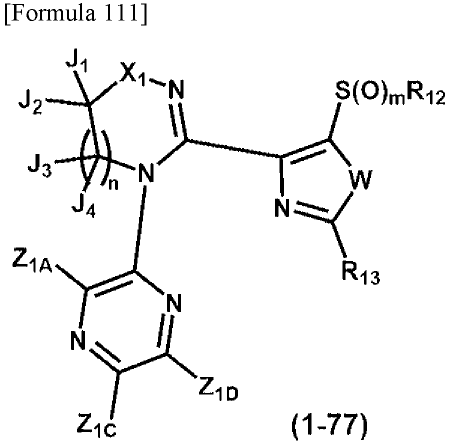 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1C}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₁₃ is a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkynyl group optionally having a substituent, a halo (C₂ to C₆) alkynyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(O)NY₄Y₅ group, a -CH=NY₆ group, a cyano group, a nitro group, a formyl group, a hydroxy group, a mercapto group, a formyl group, a carboxy group, and an SF₅ group,
W is an oxygen atom, a sulfur atom, or NY₇,
Y₃, Y₄, Y₅, Y₆, and Y₇ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-77), which is formula (1) where B is B-8 and D is D-7, Z_{1A}, Z_{1C}, and Z_{1D} substituents shown in Table 1-1, Table 1-3, and Table 1-4, respectively, X₁ is a structure shown in Table 2, J₁ and J₂ are substituents shown in Table 3-1, J₃ and J₄ are substituents shown in Table 3-2, n is 0 or 1, R₁₃ is a substituent shown in Table 13-1 to Table 13-2, W is a substituent shown in Table 14, R₁₂ is a substituent shown in Table 7, and m is combined from 0, 1, and 2.

A compound represented by formula (1-78), which is formula (1) where B is B-8 and D is D-8, a salt thereof or an N-oxide thereof 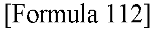 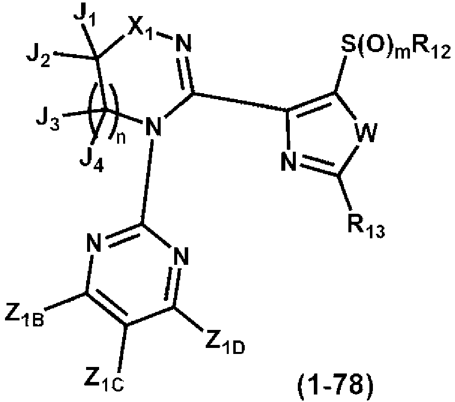 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1B}, Z_{1C}, and Z_{1D} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₁₃ is a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkynyl group optionally having a substituent, a halo (C₂ to C₆) alkynyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(O)NY₄Y₅ group, a -CH=NY₆ group, a cyano group, a nitro group, a formyl group, a hydroxy group, a mercapto group, a formyl group, a carboxy group, and an SF₅ group,
W is an oxygen atom, a sulfur atom, or NY₇,
Y₃, Y₄, Y₅, Y₆, and Y₇ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-78), which is formula (1) where B is B-8 and D is D-8, Z_{1B}, Z_{1C}, and Z_{1D} are substituents shown in Table 1-2, Table 1-3, and Table 1-4, respectively, X₁ is a structure shown in Table 2, J₁ and J₂ are substituents shown in Table 3-1, J₃ and J₄ are substituents shown in Table 3-2, n is 0 or 1, R₁₃ is a substituent shown in Table 13-1 to Table 13-2, W is a substituent shown in Table 14, R₁₂ is a substituent shown in Table 7, and m is combined from 0, 1, and 2.

A compound represented by formula (1-79), which is formula (1) where B is B-8 and D is D-9, a salt thereof or an N-oxide thereof 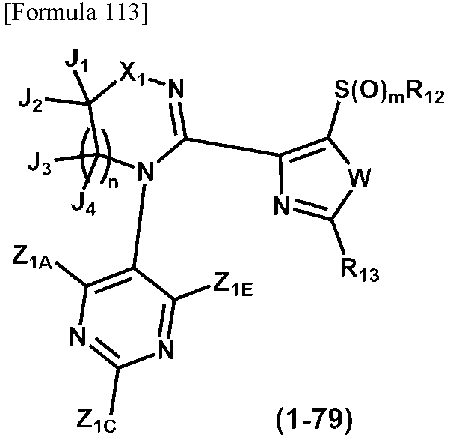 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1C}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₁₃ is a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkynyl group optionally having a substituent, a halo (C₂ to C₆) alkynyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(O)NY₄Y₅ group, a -CH=NY₆ group, a cyano group, a nitro group, a formyl group, a hydroxy group, a mercapto group, a formyl group, a carboxy group, and an SF₅ group,
W is an oxygen atom, a sulfur atom, or NY₇,
Y₃, Y₄, Y₅, Y₆, and Y₇ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-79), which is formula (1) where B is B-8 and D is D-9, Z_{1A}, Z_{1C}, and Z_{1E} are substituents shown in Table 1-1, Table 1-3, and Table 1-5, respectively, X₁ is a structure shown in Table 2, J₁ and J₂ are substituents shown in Table 3-1, J₃ and J₄ are substituents shown in Table 3-2, n is 0 or 1, R₁₃ is a substituent shown in Table 13-1 to Table 13-2, W is a substituent shown in Table 14, R₁₂ is a substituent shown in Table 7, and m is combined from 0, 1, and 2.

A compound represented by formula (1-80), which is formula (1) where B is B-8 and D is D-10, a salt thereof or an N-oxide thereof 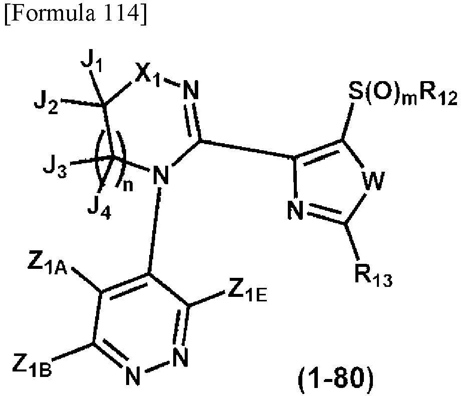 wherein
X₁ is an oxygen atom, a sulfur atom, NY₁, or C(J₅J₆),
n is 0 or 1,
J₁, J₂, J₃, and J₄ are each independently a hydrogen atom, a halogen atom, a (C₁ to C₆) alkyl group optionally having a substituent, a halo (C₁ to C₆) alkyl group optionally having a substituent, a (C₁ to C₆) alkoxy group optionally having a substituent, a halo (C₁ to C₆) alkoxy group optionally having a substituent, a hydroxy group, or a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆, J₇ and J₈, and Y₁ and Y₂ are defined as above,
Z_{1A}, Z_{1B}, and Z_{1E} are each independently a group selected from the group consisting of a hydrogen atom, a halogen atom, a (C₁ to C₆) alkyl group optionally having a substituent, a halo (C₁ to C₆) alkyl group optionally having a substituent, a (C₃ to C₆) cycloalkyl group optionally having a substituent, a halo (C₃ to C₆) cycloalkyl group optionally having a substituent, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkoxycarbonyl group optionally having a substituent, a halo (C₁ to C₆) alkoxycarbonyl group optionally having a substituent, a (C₁ to C₆) alkylcarbonyloxy group, a halo (C₁ to C₆) alkylcarbonyloxy group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, an NY₄Y₅ group, a C(O)NY₄Y₅ group, a cyano group, a nitro group, a hydroxy group, a mercapto group, a formyl group, a carboxy group, and an SF₅ group,
R₁₃ is a group selected from the group consisting of a hydrogen atom, a halogen atom, a (C₁ to C₆) alkyl group optionally having a substituent, and a halo (C₁ to C₆) alkyl group optionally having a substituent, a (C₃ to C₆) cycloalkyl group optionally having a substituent, a halo (C₃ to C₆) cycloalkyl optionally having a substituent group, a (C₁ to C₆) alkoxy group optionally having a substituent, a halo (C₁ to C₆) alkoxy group optionally having a substituent, a (C₂ to C₆) alkenyl group optionally having a substituent, a halo (C₂ to C₆) alkenyl group optionally having a substituent, a (C₂ to C₆) alkynyl group optionally having a substituent, a halo (C₂ to C₆) alkynyl group optionally having a substituent, a (C₂ to C₆) alkenyloxy group optionally having a substituent, a halo (C₂ to C₆) alkenyloxy group optionally having a substituent, a (C₁ to C₆) alkylthio group optionally having a substituent, a halo (C₁ to C₆) alkylthio group optionally having a substituent, a (C₁ to C₆) alkylsulfinyl group optionally having a substituent, a halo (C₁ to C₆) alkylsulfinyl group optionally having a substituent, a (C₁ to C₆) alkylsulfonyl group optionally having a substituent, a halo (C₁ to C₆) alkylsulfonyl group optionally having a substituent, a (C₁ to C₆) alkylcarbonyl group optionally having a substituent, a halo (C₁ to C₆) alkylcarbonyl group optionally having a substituent, a (C₁ to C₆) alkylcarbonyloxy group optionally having a substituent, a halo (C₁ to C₆) alkylcarbonyloxy group optionally having a substituent, a (C₁ to C₆) alkoxycarbonyl group optionally having a substituent, a halo (C₁ to C₆) alkoxycarbonyl group optionally having a substituent, a (C₁ to C₆) alkoxycarbonyloxy group optionally having a substituent, a halo (C₁ to C₆) alkoxycarbonyloxy group optionally having a substituent, a (C₁ to C₆) alkylsulfonyloxy group optionally having a substituent, a halo (C₁ to C₆) alkylsulfonyloxy group optionally having a substituent, a phenyl group optionally having a substituent, a heterocyclic group optionally having a substituent, a phenoxy group optionally having a substituent, a pyridyloxy group optionally having a substituent, a -NY₄Y₅ group, a - C(O)NY₄Y₅ group, a -CH=NY₆ group, a cyano group, a nitro group, a formyl group, a hydroxy group, a mercapto group, a formyl group, a carboxy group, and an SF₅ group,
W is an oxygen atom, a sulfur atom, or NY₇,
Y₃, Y₄, Y₅, Y₆, and Y₇ are defined as above,
R₁₂ is a group selected from the group consisting of a (C₁ to C₆) alkyl group, a halo (C₁ to C₆) alkyl group, a (C₃ to C₆) cycloalkyl group, a halo (C₃ to C₆) cycloalkyl group, and an NYₐY_{b} group,
Yₐ and Y_{b} are each independently a group selected from the group consisting of a hydrogen atom, a (C₁ to C₆) alkyl group, a halo (C₁ to C₆) alkyl group, a (C₃ to C₆) cycloalkyl group, and a halo (C₃ to C₆) cycloalkyl group, or a group forming an aliphatic 3- to 10-membered cyclic amino group unsubstituted or optionally substituted with one or more halogen atoms or (C₁ to C₆) alkyl groups, formed by connecting Yₐ and Y_{b} to each other, and
m is an integer of 0 to 2,
provided that when R₁₂ is an NYₐY_{b} group, m is 1 or 2.

The compound of the present invention wherein in formula (1-80), which is formula (1) where B is B-8 and D is D-10, Z_{1A}, Z_{1B}, and Z_{1E} are substituents shown in Table 1-1, Table 1-2, and Table 1-5, respectively, X₁ is a structure shown in Table 2, J₁ and J₂ are substituents shown in Table 3-1, J₃ and J₄ are substituents shown in Table 3-2, n is 0 or 1, R₁₃ is a substituent shown in Table 13-1 to Table 13-2, W is a substituent shown in Table 14, R₁₂ is a substituent shown in Table 7, and m is combined from 0, 1, and 2.

### EXAMPLES

### <Synthesis Examples>

Hereinafter, the present invention will be specifically described with reference to Examples, but the present invention is not limited to these Examples. In NMR data, "s" stands for singlet, "d" stands for doublet, "t" stands for triplet, "q" stands for quartet, "m" stands for multiplet, "br" stands for broad, and J stands for coupling constant.

### Synthesis Example 1-1: Preparation of 3-(ethylthio)-5-(4-(trifluoromethoxy)phenyl)picolinonitrile

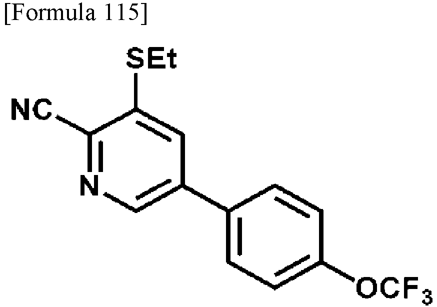

5-Bromo-3-(ethylthio)picolinonitrile (4.50 g, 18.5 mmol) prepared by the method disclosed in International Publication No. WO2017/016922 and (4-(trifluoromethoxy)phenyl)boronic acid (5.72 g, 27.8 mmol) were dissolved in 1,4-dioxane (160 mL), and a 2 M sodium carbonate aqueous solution (40 mL) was added. The inside of the system was degassed with a pump and then purged with nitrogen, and dichlorobis(triphenylphosphine)palladium(II) (1.30 g, 1.85 mmol) was added. Degassing and nitrogen purge were carried out again, and then the mixture was heated and stirred under reflux for 2 hours. After cooling to room temperature, the reaction solution was diluted with ethyl acetate and water and filtered by using a filter aid to remove insoluble matter. Water was further added to the filtrate, the resulting mixture was extracted twice with ethyl acetate, and the resulting organic layer was dried over anhydrous magnesium sulfate and then filtered. After that, the filtrate was concentrated under reduced pressure, and the resulting crude product was purified by silica gel chromatography to obtain the target product (4.69 g, 78.2%).
¹H NMR (CDCl₃) δ 8.69 (1H, d, J = 1.8 Hz), 7.87 (1H, d, J = 2.1 Hz), 7.80 (2H, d, J = 8.4 Hz), 7.70 (2H, d, J = 8.4 Hz), 3.13 (2H, q, J = 7.5 Hz), 1.43 (3H, t, J = 7.5 Hz)

### Synthesis Example 1-2: Preparation of 3-(ethylthio)-5-(4-(trifluoromethoxy)phenyl)-picolinic acid

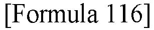

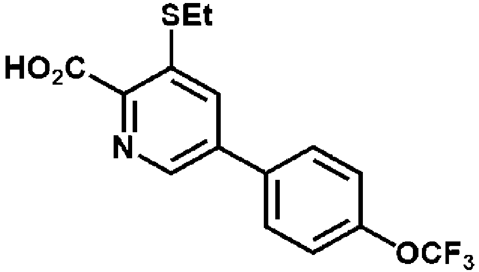

The product (2.00 g, 6.17 mmol) of Synthesis Example 1-1 was dissolved in ethanol (60 mL) and dioxane (20 mL), a 25% sodium hydroxide aqueous solution (50.0 g, 308 mmol) was added thereto, and the resulting mixture was stirred under reflux for 5 hours. After cooling to room temperature, the reaction solution was concentrated under reduced pressure. The resulting residue was diluted with water and 1 N hydrochloric acid, and then the resulting mixture was extracted three times with ethyl acetate. The resulting organic layer was dried over anhydrous magnesium sulfate and then filtered. The filtrate was concentrated under reduced pressure, then the resulting composition was suspended in a mixture of ethyl acetate and hexane, and the resulting suspension was filtered to obtain the target product (2.18 g, quant.) as a filtered product.
¹H NMR (CDCl₃) δ 10.90 (1H, br), 8.47 (1H, d, J = 1.8 Hz), 7.49 (1H, d, J = 1.5 Hz), 7.66-7.61 (2H, m), 7.39 (2H, d, J = 8.1 Hz), 3.04 (2H, q, J = 7.5 Hz), 1.48 (3H, t, J = 7.5 Hz)

### Synthesis Example 1-3: Preparation of 3-(ethylthio)-5-(4-(trifluoromethoxy)phenyl)-N-(5-(trifluoromethyl)pyridin-2-yl)picolinamide

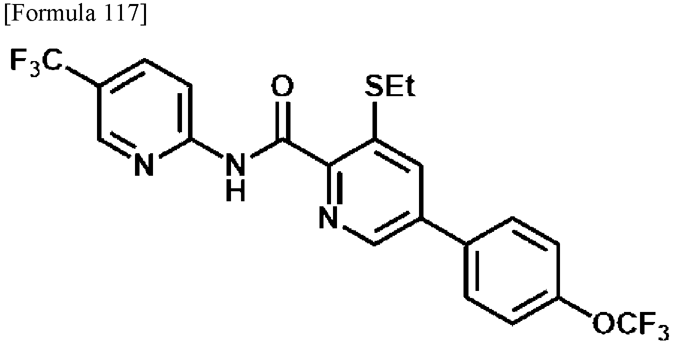

The product (5.22 g, 15.2 mmol) of Synthesis Example 1-2 was dissolved in dichloromethane (140 mL), oxalyl chloride (2.61 mL, 30.4 mmol) and dimethylformamide (500 µL) were added thereto, and the resulting mixture was stirred at room temperature for 1 hour. After that, the reaction solution was concentrated under reduced pressure, and the resulting residue was dissolved in tetrahydrofuran (120 mL). 2-Amino-5-trifluoromethylpyridine (3.20 g, 19.7 mmol) and triethylamine (11 mL, 78.9 mmol) were added thereto, and the resulting mixture was stirred at room temperature for 4.5 hours. Water and saturated brine were added to the reaction solution, the resulting mixture was extracted three times with ethyl acetate, and the resulting organic layer was dried over anhydrous magnesium sulfate and then filtered. After that, the filtrate was concentrated under reduced pressure, and the resulting crude product was purified by silica gel chromatography to obtain the target product (3.46 g, 46.7%).
¹H NMR (CDCl₃) δ 10.83 (1H, s), 8.63-8.60 (2H, m), 8.53 (1H, d, J = 1.8 Hz), 7.97 (1H, dd, J = 9.0, 1.8 Hz), 7.81 (1H, d, J = 1.5 Hz), 7.65 (2H, d, J = 8.7 Hz), 7.39 (2H, d, J = 8.4 Hz), 3.04 (2H, q, J = 7.5 Hz), 1.49 (3H, t, J = 7.2 Hz)

### Synthesis Example 1-4: Preparation of 3-(ethylsulfonyl)-5-(4-(trifluoromethoxy)phenyl)-N-(5-(trifluoromethyl)pyridin-2-yl)picolinamide

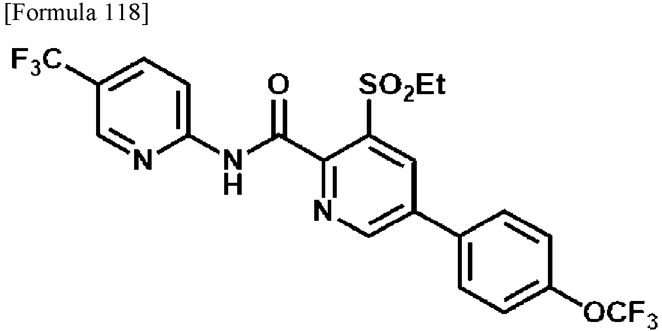

The product (190 mg, 0.390 mmol) of Synthesis Example 1-3 was dissolved in dichloromethane (7 mL), 3-chloroperbenzoic acid (65%, 208 mg, 0.783 mmol) was added thereto, and the resulting mixture was stirred at room temperature for 7 hours. A sodium hydrogen carbonate aqueous solution was added to the reaction solution, and then the resulting mixture was extracted with ethyl acetate. The resulting organic layer was dried over anhydrous magnesium sulfate and filtered. After that, the filtrate was concentrated under reduced pressure, and the resulting crude product was purified by silica gel chromatography to obtain the target product (177 mg, 87.4%).
¹H NMR (CDCl₃) δ 10.35 (1H, s), 9.04 (1H, d, J = 2.1 Hz), 8.82 (1H, d, J = 2.1 Hz), 8.65 (1H, br), 8.56 (1H, d, J = 8.7 Hz), 8.01 (1H, dd, J = 9.0, 2.4 Hz), 7.76-7.73 (2H, m), 7.43 (2H, d, J = 8.1 Hz), 4.00 (2H, q, J = 7.5 Hz), 1.41 (3H, t, J = 7.5 Hz)

### Synthesis Example 1-5: Preparation of 3-(ethylsulfonyl)-N'-hydroxy-5-(4-(trifluoromethoxy)phenyl)-N-(5-(trifluoromethyl)pyridin-2-yl)picolinimidamide

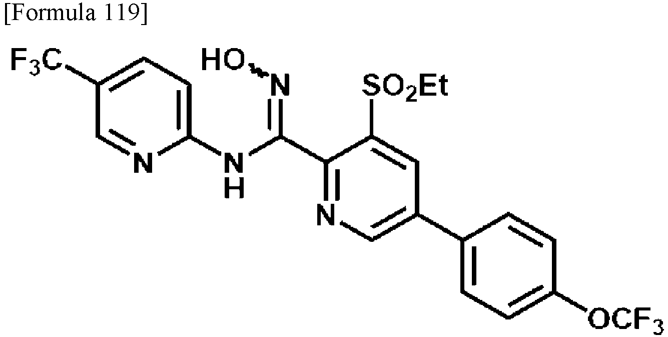

3-(Ethylsulfonyl)-5-(4-(trifluoromethoxy)phenyl)-N-(5-(trifluoromethyl)pyridin-2-yl)picolinamide (218 mg, 0.42 mmol) prepared in Synthesis Example 1-4 was dissolved in acetonitrile (7 mL), carbon tetrachloride (122 µL, 1.25 mmol) and triphenylphosphine (0.66 g, 2.52 mmol) were added, and the resulting mixture was stirred under reflux for 1 hour. After cooling to room temperature, a 50% hydroxylamine aqueous solution (1 mL) was added dropwise, and the resulting mixture was stirred at room temperature overnight. Most of acetonitrile in the reaction solution was distilled off, and the residue was purified by silica gel chromatography to obtain the target product (185 mg, 87.7%).
¹H NMR (CDCl₃) δ 8.97 (1H, d, J = 2.4 Hz), 8.57 (1H, d, J = 2.1 Hz), 8.14 (1H, s), 7.76-7.69 (2H, m), 7.65 (1H, dd, J = 8.7, 2.4 Hz), 7.40 (2H, d, J = 8.1 Hz), 6.63 (1H, d, J = 8.7 Hz), 3.60 (2H, q, J = 7.5 Hz), 1.36 (3H, t, J = 7.5 Hz)

### Synthesis Example 1-6: Preparation of 3-(3-(ethylsulfonyl)-5-(4-(trifluoromethoxy)phenyl)pyridin-2-yl)-4-(5-(trifluoromethyl)pyridin-2-yl)-1,2,4-oxadiazol-5(4H)-one) (Compound No. 4)

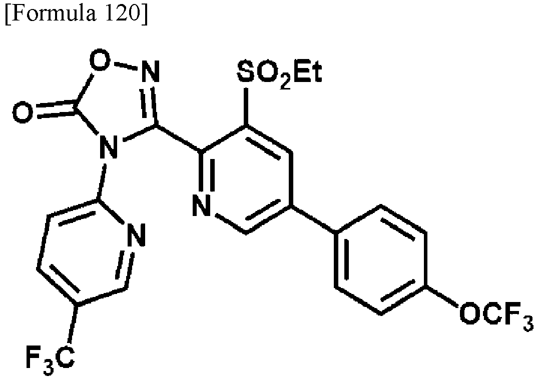

3-(Ethylsulfonyl)-N'-hydroxy-5-(4-(trifluoromethoxy)phenyl)-N-(6-(trifluoromethyl)pyridin-3-yl)picolinimidamide (110 mg, 0.21 mmol) prepared in Synthesis Example 1-5 was dissolved in tetrahydrofuran (13 mL), 1,1'-carbonyldiimidazole (52 mg, 0.31 mmol) was added, and the resulting mixture was stirred at room temperature for 3 hours. Most of tetrahydrofuran in the reaction solution was distilled off, and the residue was purified by silica gel chromatography to obtain the target product (85 mg, 73.4%).
¹H NMR (CDCl₃) δ 9.05 (1H, d, J = 2.1 Hz), 8.58 (1H, d, J = 2.1 Hz), 8.26 (2H, d, J = 9.3 Hz), 8.08 (1H, dd, J = 2.4, 7.9 Hz), 7.78-7.74 (2H, m), 7.45 (2H, d, J = 7.8 Hz), 3.51 (2H, q, J = 7.5 Hz), 1.39 (3H, t, J = 7.5 Hz)

### Synthesis Example 2-1: Preparation of 3-(ethylsulfonyl)-5-(4-fluorophenyl)-N'-hydroxy-N-(5-(trifluoromethyl)pyridin-2-yl)picolinimidamide

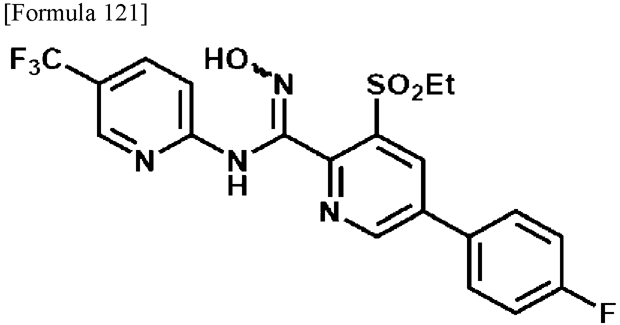

The target product was obtained by the same method as in Synthesis Example 5-2 of International Publication No. WO2021/177410.
¹H NMR (CDCl₃) δ 8.95 (1H, d, J = 2.1 Hz) 8.54 (1H, d, J = 2.4 Hz) 8.27 (1H, s) 8.14 (1H, m) 7.79 (1H, br) 7.67 (2H, m) 7.60 (1H, dd, J = 2.1, 8.7 Hz) 7.24 (2H, m) 6.58 (1H, d, J = 8.7 Hz) 3.57 (2H, q, J = 7.5 Hz) 1.34 (3H, t, J = 7.5 Hz)

### Synthesis Example 2-2: Preparation of 3-(3-(ethylsulfonyl)-5-(4-fluorophenyl)pyridin-2-yl)-4-(5-(trifluoromethyl)pyridin-2-yl)-1,2,4-oxadiazol-5(4H)-one (Compound No. 2)

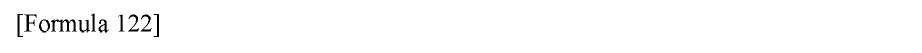

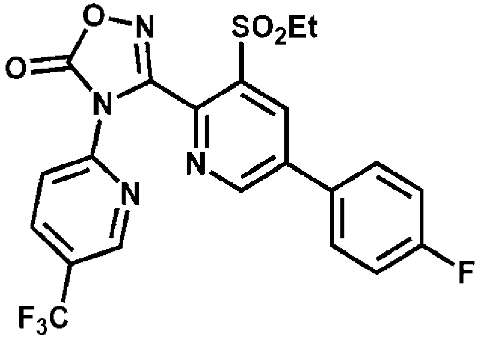

3-(Ethylsulfonyl)-5-(4-fluorophenyl)-N'-hydroxy-N-(5-(trifluoromethyl)pyridin-2-yl)picolinimidamide (100 mg, 0.21 mmol) prepared in Synthesis Example 2-1 was dissolved in tetrahydrofuran (15 mL), 1,1'-carbonyldiimidazole (42 mg, 0.26 mmol) was added, and the resulting mixture was stirred at room temperature for 1 hour. 1,1'-Carbonyldiimidazole (21 mg, 0.13 mmol) was added to the reaction solution, and the resulting mixture was further stirred at room temperature for 3 hours. Most of tetrahydrofuran in the reaction solution was distilled off, and the residue was purified by silica gel chromatography to obtain the target product (40 mg, 38.0%).
¹H NMR (CDCl₃) δ 9.03 (1H, d, J = 2.1 Hz), 8.56 (1H, d, J = 2.1 Hz), 8.27-8.24 (2H, m), 8.07 (1H, dd, J = 1.8, 8.7 Hz), 7.73-7.68 (2H, m), 7.31-7.25 (2H, m), 3.51 (2H, q, J = 7.2 Hz), 1.39 (3H, t, J = 7.5 Hz)

### Synthesis Example 2-3: Preparation of 3-(3-(ethylsulfonyl)-5-(4-fluorophenyl)pyridin-2-yl)-4-(5-(trifluoromethyl)pyridin-2-yl)-1,2,4-oxadiazol-5(4H)-thione (Compound No. 18)

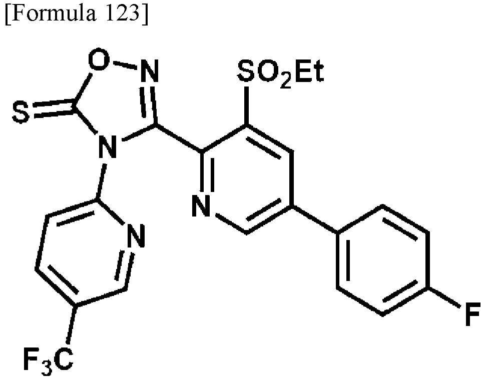

3-(Ethylsulfonyl)-5-(4-fluorophenyl)-N'-hydroxy-N-(5-(trifluoromethyl)pyridin-2-yl)picolinimidamide (80 mg, 0.17 mmol) prepared in Synthesis Example 2-1 was dissolved in tetrahydrofuran (10 mL), 1,8-diazabicyclo[5.4.0]-7-undecene (76 µL, 0.51 mmol), 1,1'-thiocarbonyldiimidazole (61 mg, 3.42 mmol) was added, and the resulting mixture was stirred at room temperature for 3 hours. Water was added to the reaction solution, and then the resulting mixture was extracted with ethyl acetate. The resulting organic layer was washed with an ammonium chloride aqueous solution and saturated brine, dried over anhydrous magnesium sulfate, and filtered. After that, the filtrate was concentrated under reduced pressure, and the resulting crude product was purified by silica gel chromatography to obtain the target product (80 mg, 92.8%).
¹H NMR (CDCl₃) δ 8.95 (1H, d, J = 2.1 Hz), 8.54 (1H, d, J = 2.1 Hz), 8.46 (1H, d, J = 2.1 Hz), 8.41 (1H, d, J = 8.7 Hz), 8.13 (1H, dd, J = 8.6, 2.4 Hz), 7.68-7.63 (2H, m), 7.30-7.23 (2H, m), 3.54 (2H, q, J = 7.5 Hz), 1.40 (3H, t, J = 7.5 Hz)

### Synthesis Example 2-4: Preparation of 3-(3-(ethylsulfonyl)-5-(4-fluorophenyl)pyridin-2-yl)-4-(5-(trifluoromethyl)pyridin-2-yl)-4,5-dihydro-1,2,4-oxadiazole (Compound No. 27)

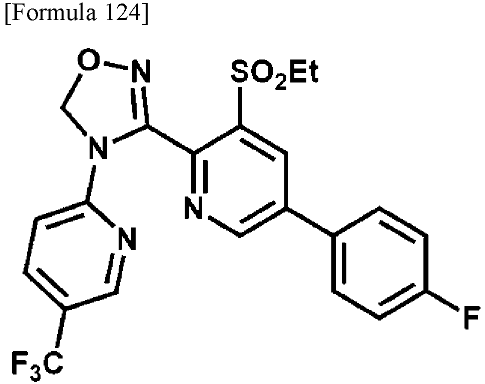

3-(Ethylsulfonyl)-5-(4-fluorophenyl)-N'-hydroxy-N-(5-(trifluoromethyl)pyridin-2-yl)picolinimidamide (70 mg, 0.15 mmol) prepared in Synthesis Example 2-1 was dissolved in N,N-dimethylformamide (3 mL), potassium carbonate (104 mg, 0.75 mmol) and bromochloromethane (302 µL, 4.50 mmol) were added, and the resulting mixture was stirred at 70°C for 3 hours. After cooling to room temperature, water was added to the reaction solution, and the resulting mixture was extracted with ethyl acetate. The resulting organic layer was dried over anhydrous magnesium sulfate and filtered. After that, the filtrate was concentrated under reduced pressure, and the resulting crude product was purified by silica gel chromatography to obtain the target product (36 mg, 50.0%).
¹H NMR (CDCl₃) δ 9.10 (1H, d, J = 2.1 Hz), 8.58 (1H, d, J = 2.1 Hz), 8.36-8.35 (1H, m), 7.73-7.68 (2H, m), 7.61 (1H, dd, J = 8.7, 2.1 Hz), 7.30-7.25 (2H, m), 6.19 (1H, d, J = 8.7 Hz), 6.16 (2H, s), 3.64 (2H, q, J = 7.4 Hz), 1.38 (3H, t, J = 7.5 Hz)

### Synthesis Example 2-5: Preparation of 3-(3-(ethylsulfonyl)-5-(4-fluorophenyl)pyridin-2-yl)-4-(5-(trifluoromethyl)pyridin-2-yl)-5,6-dihydro-4H-1,2,4-oxadiazine (Compound No. 29)

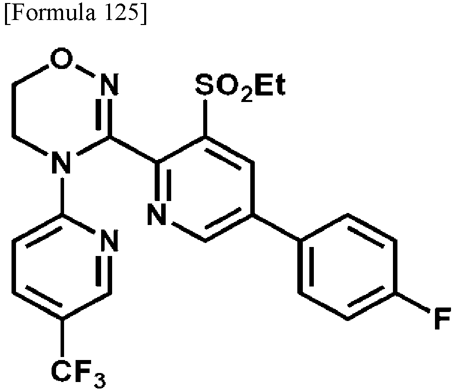

3-(Ethylsulfonyl)-5-(4-fluorophenyl)-N'-hydroxy-N-(5-(trifluoromethyl)pyridin-2-yl)picolinimidamide (70 mg, 0.15 mmol) prepared in Synthesis Example 2-1 was dissolved in N,N-dimethylformamide (3 mL), potassium carbonate (104 mg, 0.75 mmol) and 1,2-dibromoethane (332 µL, 4.50 mmol) were added, and the resulting mixture was stirred at 70°C for 4 hours. After cooling to room temperature, water was added to the reaction solution, and the resulting mixture was extracted with ethyl acetate. The resulting organic layer was dried over anhydrous magnesium sulfate and filtered. After that, the filtrate was concentrated under reduced pressure, and the resulting crude product was purified by silica gel chromatography to obtain the target product (60 mg, 80.9%).
¹H NMR (CDCl₃) δ 8.79 (1H, d, J = 2.4 Hz), 8.53 (1H, d, J = 2.1 Hz), 8.45 (1H, br), 7.63-7.7.58 (2H, m), 7.55 (1H, dd, J = 8.7, 2.4 Hz), 7.25-7.18 (2H, m), 6.82 (1H, d, J = 8.7 Hz), 4.46-4.44 (2H, m), 4.26-4.23 (2H, m), 3.77 (2H, q, J = 7.5 Hz), 1.39 (3H, t, J = 7.5 Hz)

### Synthesis Example 1-4: Preparation of 3-(ethylsulfonyl)-5-(4-(trifluoromethoxy)phenyl)-N-(5-(trifluoromethyl)pyridin-2-yl)picolinohydrazonamide

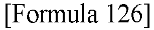

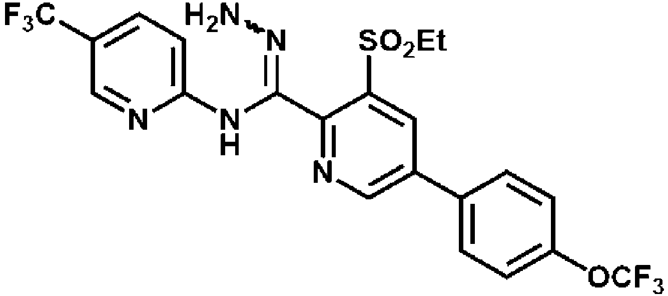

3-(Ethylsulfonyl)-5-(4-(trifluoromethoxy)phenyl)-N-(5-(trifluoromethyl)pyridin-2-yl)picolinamide (373 mg, 0.72 mmol) prepared in Synthesis Example 1-4 was dissolved in acetonitrile (20 mL), carbon tetrachloride (667 µL, 3.59 mmol) and triphenylphosphine (1.89 g, 7.20 mmol) were added, and the resulting mixture was stirred under reflux for 1 hour. In a separate flask, hydrazine hydrate (0.72 mL, 14.4 mmol), sodium hydrogen carbonate, and a molecular sieve were mixed, this mixture was added to the reaction solution cooled to room temperature, and the resulting mixture was stirred at room temperature for 3 hours. Most of acetonitrile in the reaction solution was distilled off, and the residue was purified by silica gel chromatography to obtain the target product (195 mg, 50.9%).
¹H NMR (CDCl₃) δ 8.89 (1H, d, J = 2.4 Hz), 8.60 (1H, d, J = 2.4 Hz), 8.44 (1H, t, J = 1.2 Hz), 7.71-7.65 (3H, m), 7.61 (1H, br), 7.37 (2H, d, J = 7.8 Hz), 6.70 (1H, d, J = 8.7 Hz), 5.86 (2H, br), 3.81 (2H, q, J = 7.4 Hz), 1.39 (3H, t, J = 7.5 Hz)

### Synthesis Example 3-2

### Preparation of 5-(3-(ethylsulfonyl)-5-(4-(trifluoromethoxy)phenyl)pyridin-2-yl)-4-(5-(trifluoromethyl)pyridin-2-yl)-2,4-dihydro-3H-1,2,4-triazol-3-one) (Compound No. 5)

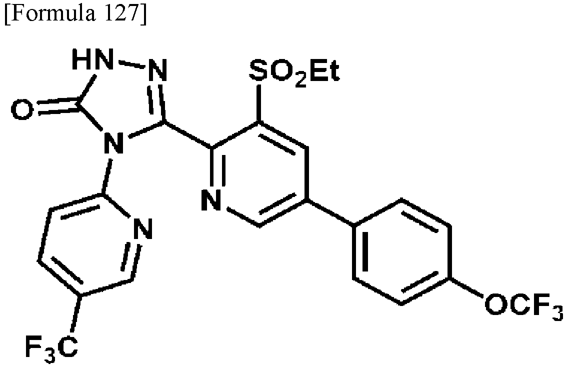

3-(Ethylsulfonyl)-5-(4-(trifluoromethoxy)phenyl)-N-(5-(trifluoromethyl)pyridin-2-yl)picolinohydrazoneamide (80 mg, 0.15 mmol) prepared in Synthesis Example 3-1 was dissolved in tetrahydrofuran (10 mL), 1,1-carbonyldiimidazole (31 mg, 0.18 mmol) was added, and the resulting mixture was stirred at room temperature for 5 hours. Most of tetrahydrofuran in the reaction solution was distilled off, and the residue was purified by silica gel chromatography to obtain the target product (68 mg, 81.7%).
¹H NMR (CDCl₃) δ 11.32 (1H, br), 8.94 (1H, d, J = 2.1 Hz), 8.60 (1H, d, J = 2.1 Hz), 8.32 (1H, s), 8.25 (1H, d, J = 8.4 Hz), 8.04 (1H, dd, J = 2.1, 8.7 Hz), 7.73 (2H, d, J = 8.7 Hz), 7.40 (2H, d, J = 8.7 Hz), 3.60 (2H, q, J = 7.2 Hz), 1.36 (3H, t, J = 7.2 Hz)

### Synthesis Example 4-1: Preparation of 3-(ethylsulfonyl)-5-(4-fluorophenyl)-N-(5-(trifluoromethyl)pyridin-2-yl)picolinimidoyl chloride

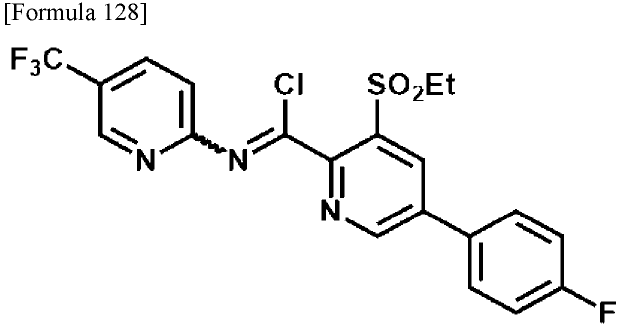

3-(Ethylsulfonyl)-5-(4-(trifluoromethoxy)phenyl)-N-(5-(trifluoromethyl)pyridin-2-yl)picolinamide (280 mg, 0.62 mmol) prepared in Synthesis Example 1-4 was dissolved in acetonitrile (20 mL), carbon tetrachloride (894 µL, 9.26 mmol) and triphenylphosphine (0.97 g, 3.71 mmol) were added, and the resulting mixture was stirred under reflux for 1 hour. After cooling to room temperature, most of acetonitrile in the reaction solution was distilled off, and the residue was purified by silica gel chromatography to obtain the target product (220 mg, 75.5%).

### Synthesis Example 4-2: Preparation of methyl 2-(((3-(ethylsulfonyl)-5-(4-fluorophenyl)pyridin-2-yl)((5-(trifluoromethyl)pyridin-2-yl)amino)methylene)amino)acetate

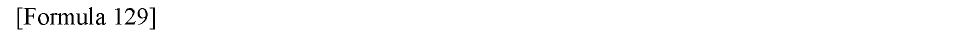

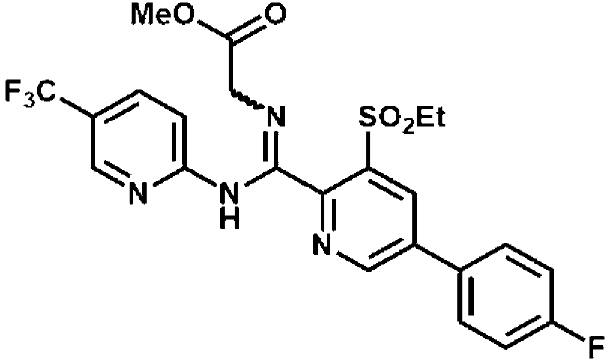

Glycine methyl ester hydrochloride (38 mg, 0.30 mmol) was dissolved in N,N-dimethylformamide (0.5 mL), N,N-diisopropylethylamine (131 µL, 0.75 mmol) was added, and the resulting mixture was stirred at room temperature for 5 minutes. A solution of 3-(ethylsulfonyl)-5-(4-fluorophenyl)-N-(5-(trifluoromethyl)pyridin-2-yl)picolinimidoyl chloride (53 mg, 62 mmol) prepared in Synthesis Example 4-1 in N,N-dimethylformamide (2.5 mL) was added, and the resulting mixture was stirred at room temperature for 14 hours. Water was added to the reaction solution, and the resulting mixture was extracted with ethyl acetate. The resulting organic layer was dried over anhydrous magnesium sulfate and filtered. After that, the filtrate was concentrated under reduced pressure, and the resulting crude product was purified by silica gel chromatography to obtain the target product (91 mg, quant.).
¹H NMR (CDCl₃) δ 11.84 (1H, br), 9.04 (1H, br), 8.73 (1H, br), 8.51 (1H, d, J = 2.1 Hz), 7.83 (1H, d, J = 7.5 Hz), 7.67-7.62 (2H, m), 7.29-7.23 (2H, m), 7.11 (1H, d, J = 8.4 Hz), 4.02 (2H, d, J = 5.1 Hz), 3.79 (3H, s), 3.70 (2H, q, J = 7.4 Hz), 1.35 (3H, t, J = 7.5 Hz)

### Synthesis Example 4-3: Preparation of 2-(3-(ethylsulfonyl)-5-(4-fluorophenyl)pyridin-2-yl)-5-(methoxymethylene)-5-(5-(trifluoromethyl)pyridin-2-yl)-3,5-dihydro-4H-imidazol-4-one (Compound No. 34)

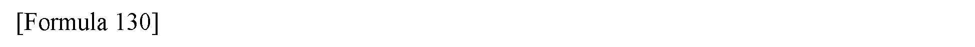

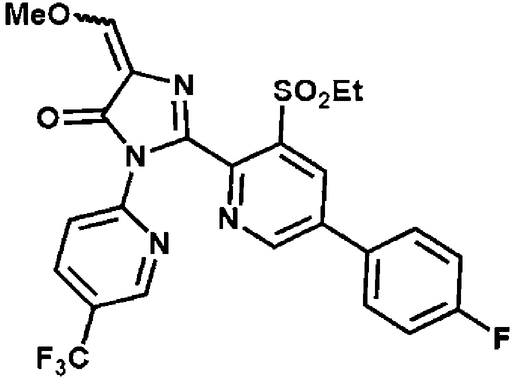

Methyl 2-(((3-(ethylsulfonyl)-5-(4-fluorophenyl)pyridin-2-yl)((5-(trifluoromethyl)pyridin-2-yl)amino)methylene)amino)acetate (53 mg, 0.11 mmol) prepared in Synthesis Example 4-2 was dissolved in acetonitrile (2 mL), N,N-dimethylformamide dimethyl acetal (45 µL, 0.34 mmol) was added, and the resulting mixture was stirred under reflux for 1 hour. After cooling to room temperature, most of acetonitrile in the reaction solution was distilled off, and the residue was purified by silica gel chromatography to obtain the target product (52 mg, 84.6%).
¹H NMR (CDCl₃) δ 8.89 (1H, d, J = 2.1 Hz), 8.71 (1H, br), 8.59 (1H, d, J = 2.1 Hz), 8.38 (1H, s), 7.87 (1H, dd, J = 8.5, 2.1 Hz), 7.68-7.63 (2H, m), 7.27-7.18 (3H, m), 3.95 (3H, s), 3.87 (2H, q, J = 7.5 Hz), 1.41 (3H, t, J = 7.5 Hz)

### Synthesis Example 5-1: Preparation of 3-(benzylthio)-5-bromopicolinonitrile

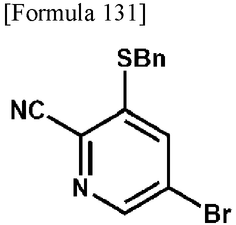

Commercially available 5-bromo-3-nitropicolinonitrile (2.28 g, 10.0 mmol) was dissolved in tetrahydrofuran (40 mL), and under ice cooling, sodium hydride (480 mg, 11.0 mmol) and benzyl mercaptan (1.29 mL, 11.0 mmol) were added, water was added to the reaction solution, and the resulting mixture was extracted with ethyl acetate. The resulting organic layer was dried over anhydrous magnesium sulfate and filtered. After that, the filtrate was concentrated under reduced pressure, and the resulting crude product was purified by silica gel chromatography to obtain the target product (2.48 g, 81.2%).
¹H NMR (CDCl₃) 8.50 (1H, d, J = 2.1 Hz), 7.76 (1H, d, J = 2.1 Hz), 7.34-7.29 (5H, m), 4.24 (2H, s)

### Synthesis Example 5-2: Preparation of 3-(3-(benzylthio)-5-(4-(trifluoromethoxy)phenyl)pyridin-2-yl)-4-(5-(trifluoromethyl)pyridin-2-yl)-1,2,4-oxadiazol-5(4H)-one)

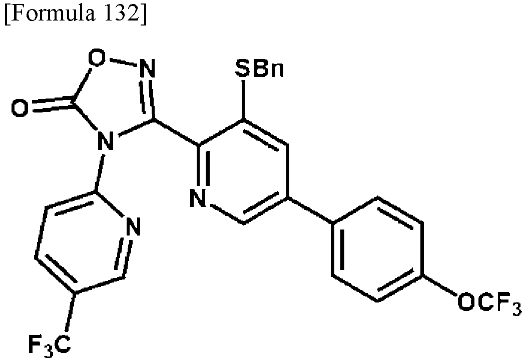

The desired product was obtained in the same manner as in Synthesis Example 1-1, 1-2, 1-3, 1-5, and 1-6 above except that 3-(benzylthio)-5-bromopicolinonitrile was used instead of 3-(ethylthio)-5-bromopicolinonitrile used in Synthesis Example 1-1.
¹H NMR (CDCl₃) 8.52 (1H, d, J = 2.1 Hz), 8.32 (1H, d, J = 0.9 Hz) 8.12 (1H, dd, J = 2.1, 8.4 Hz), 8.07 (1H, d, J = 8.4 Hz), 7.67 (1H, d, J = 2.1 Hz), 7.43 (2H, dt, J = 9.0, 2.4 Hz), 7.33-7.24 (7H, m), 4.16 (2H, s)

### Synthesis Example 5-3: Preparation of N,N-dimethyl-2-(5-oxo-4-(5-(trifluoromethyl)pyridin-2-yl)-4,5-dihydro-1,2,4-oxadiazol-3-yl)-5-(4-(trifluoromethoxy)phenyl)pyridine-3-sulfonamide (Compound No. 195)

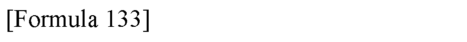

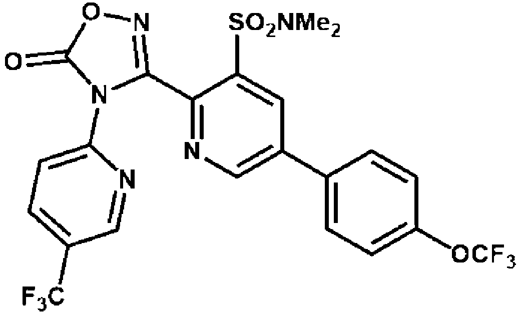

3-(3-(Benzylthio)-5-(4-(trifluoromethoxy)phenyl)pyridin-2-yl)-4-(5-(trifluoromethyl)pyridin-2-yl)-1,2,4-oxadiazol-5(4H)-one) (135 mg, 0.23 mmol) prepared in Synthesis Example 5-2 was dissolved in acetonitrile (3 mL) and acetic acid (0.3 mL), 1,3-dichloro-5,5-dimethylhydantoin (113 mg, 0.57 mmol) was added, and the resulting mixture was stirred at room temperature for 10 minutes. Tetrahydrofuran (4 mL) and a 50% dimethylamine aqueous solution (0.2 mL) were added, and the resulting mixture was further stirred at room temperature for 10 minutes. The reaction solution was diluted with ethyl acetate, and washed with water, a sodium hydrogen carbonate aqueous solution, and saturated brine. The resulting organic layer was dried over anhydrous magnesium sulfate and filtered. After that, the filtrate was concentrated under reduced pressure, and the resulting crude product was purified by silica gel chromatography to obtain the target product (71.4 mg, 54.2%).
¹H NMR (CDCl₃) 9.03 (1H, d, J = 2.1 Hz), 8.40 (1H, d, J = 2.1 Hz), 8.31 (1H, d, J = 8.7 Hz), 8.22 (1H, s), 8.07 (1H, dd, J = 2.1, 8.7 Hz), 7.76-7.73 (2H, m), 7.44 (2H, d, J = 8.4 Hz), 2.90 (6H, s)

Hereinafter, the structures of compounds of the present invention and NMR data of the compounds will be shown. The compounds of the present invention are not limited to these compounds.

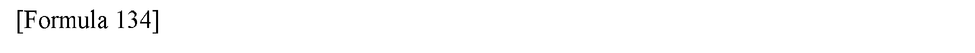

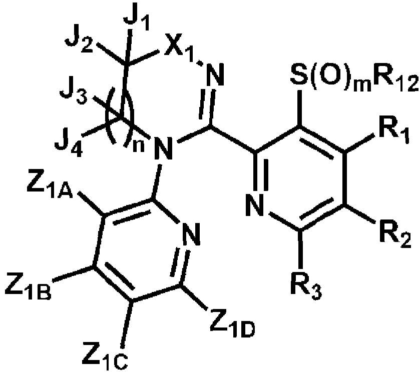

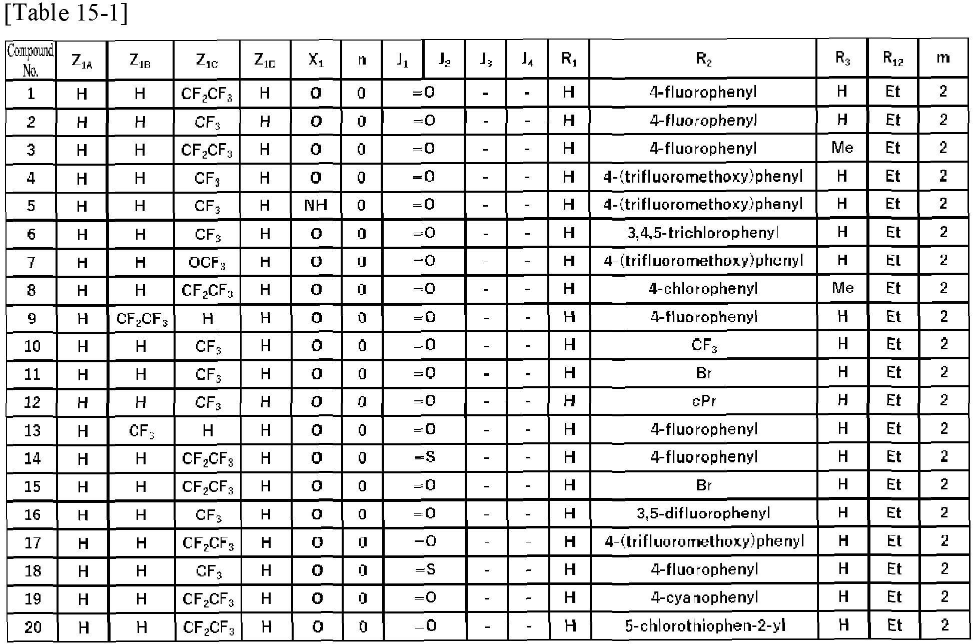

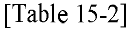

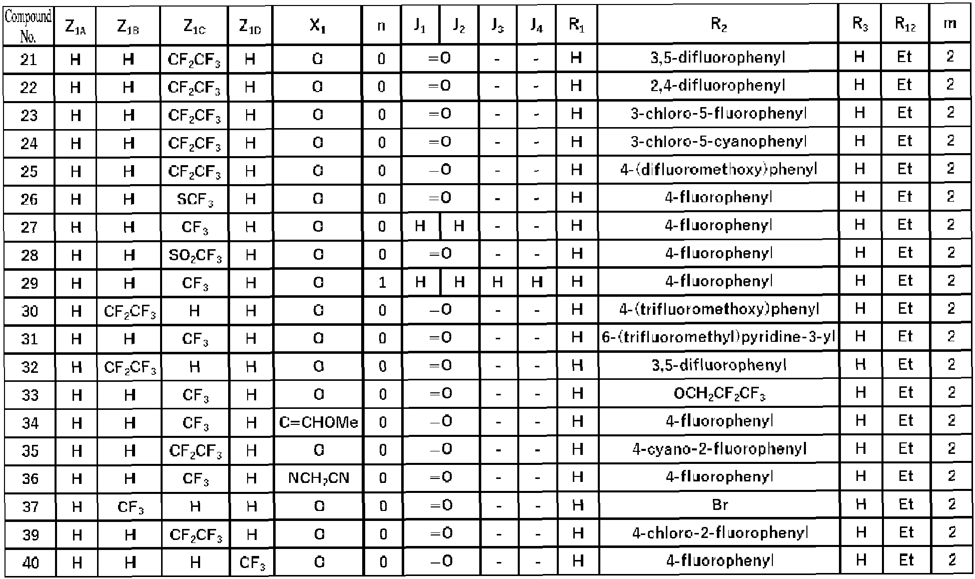

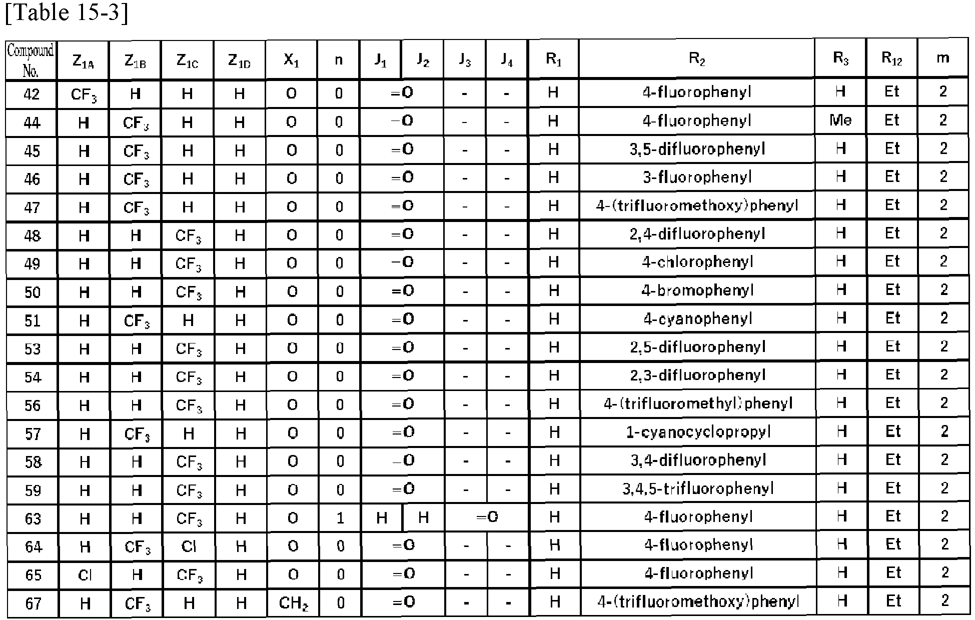

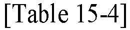

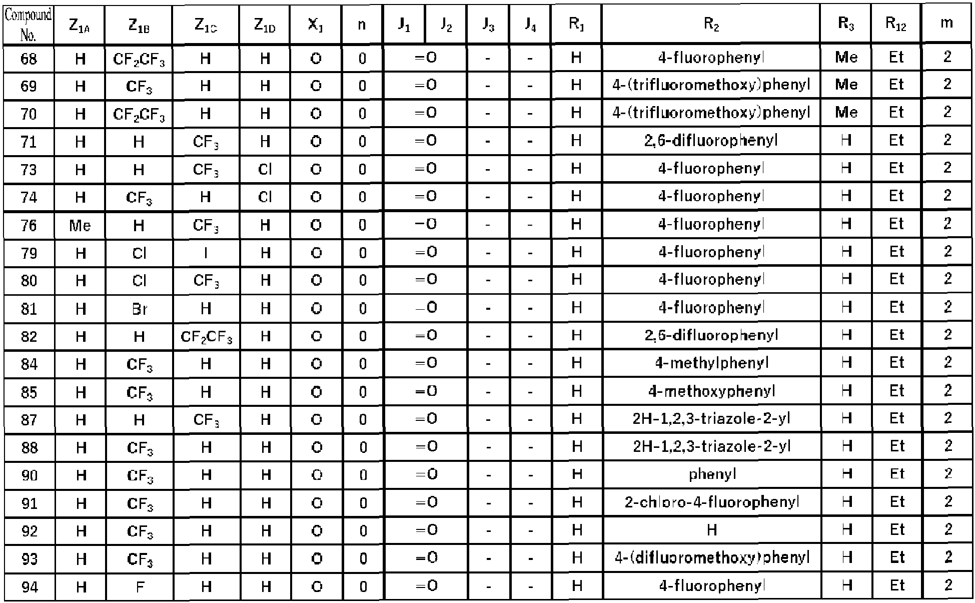

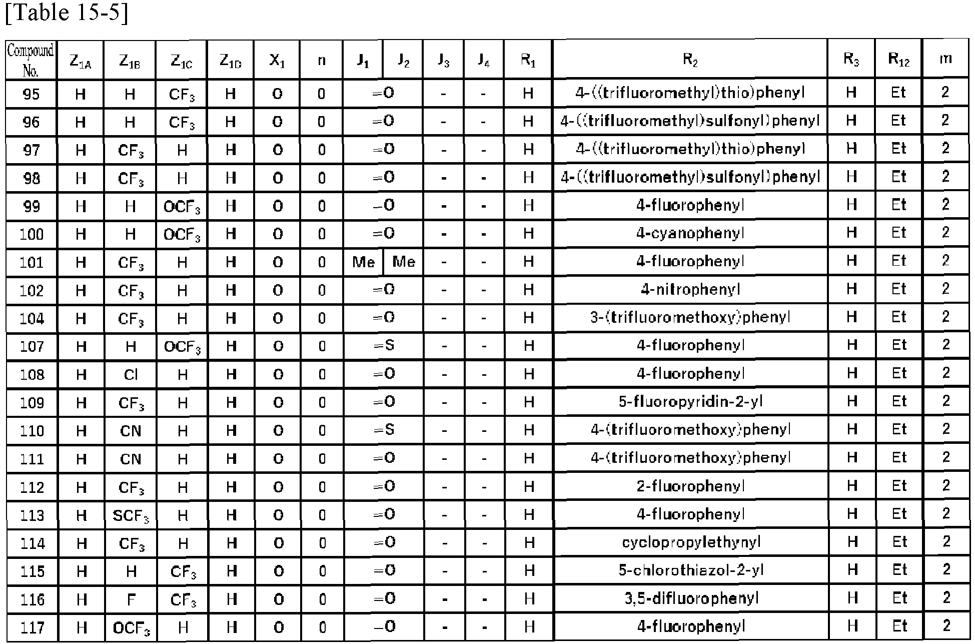

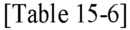

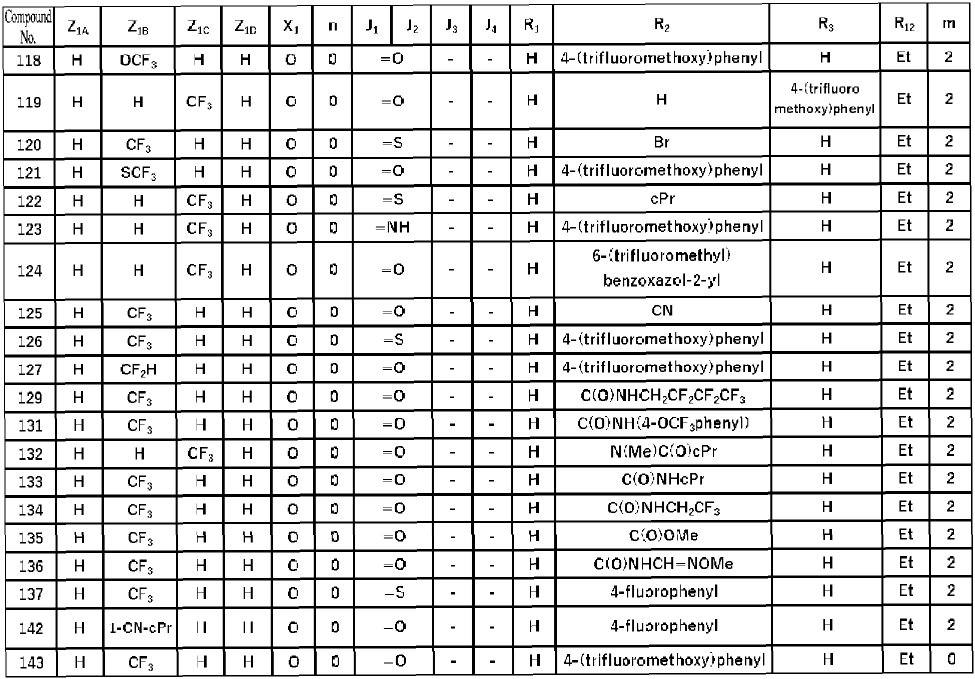

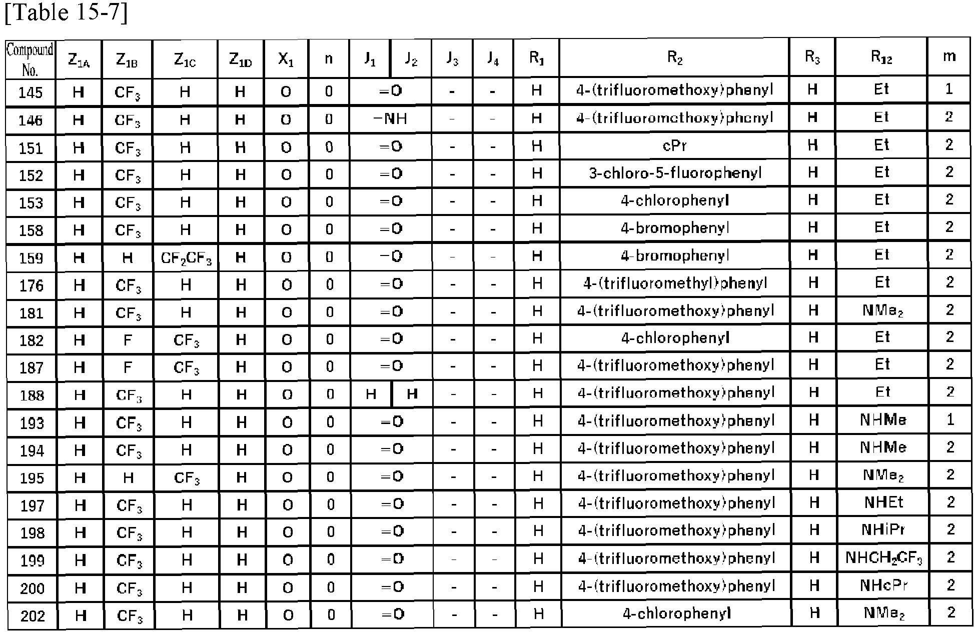

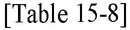

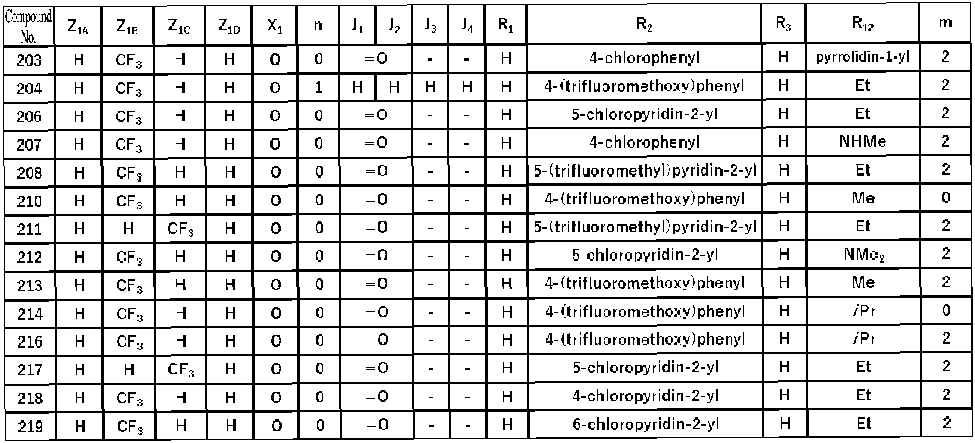

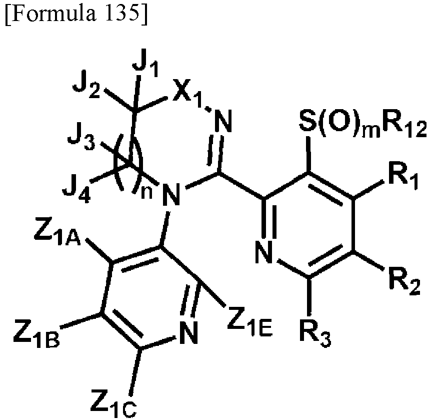

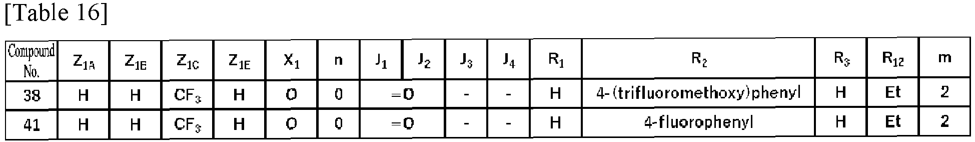

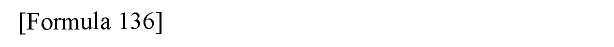

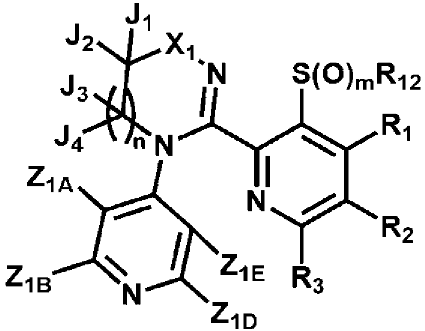

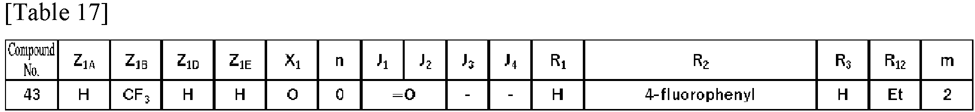

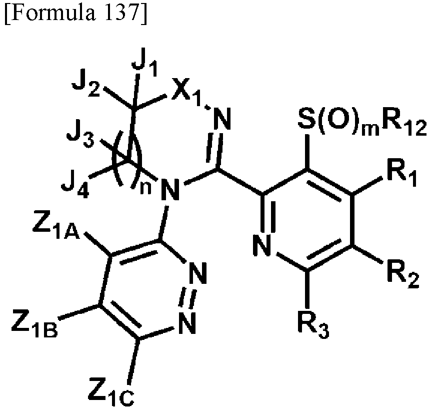

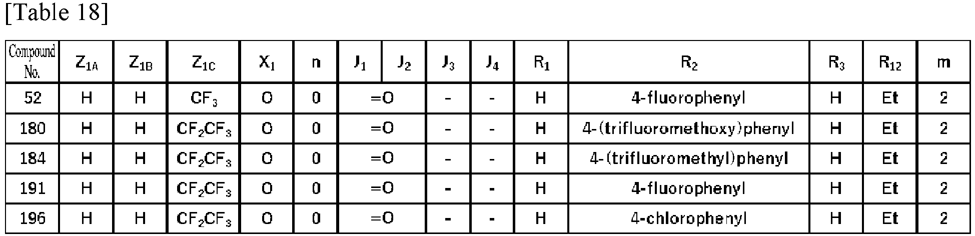

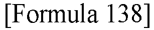

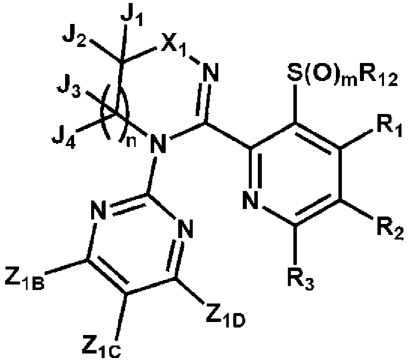

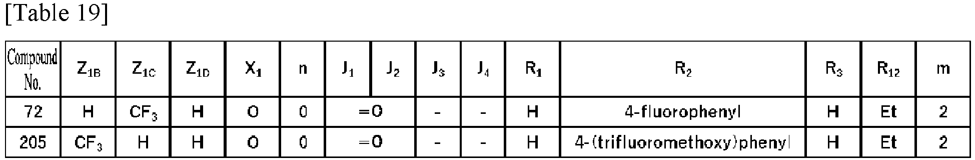

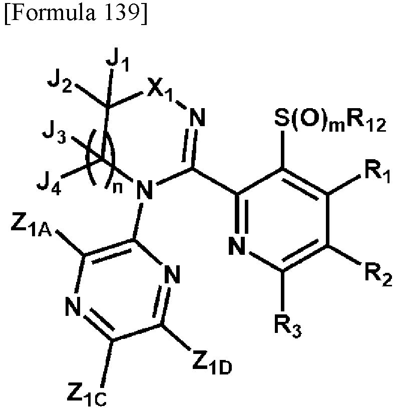

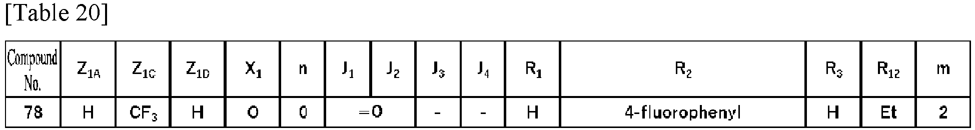

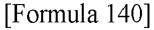

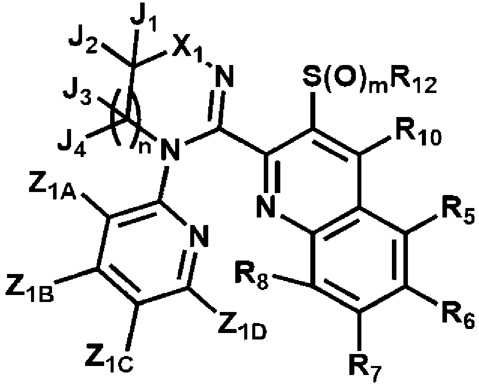

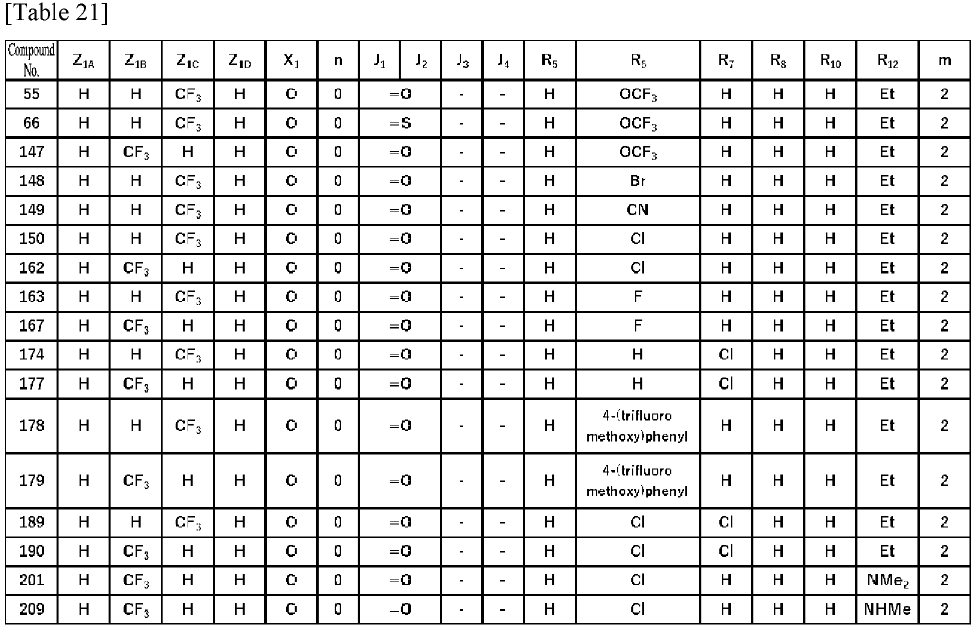

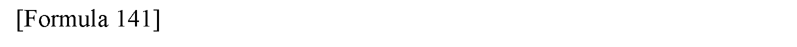

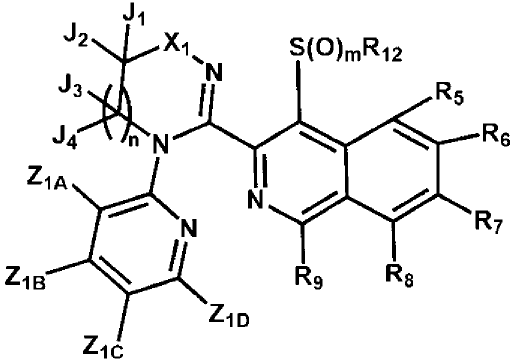

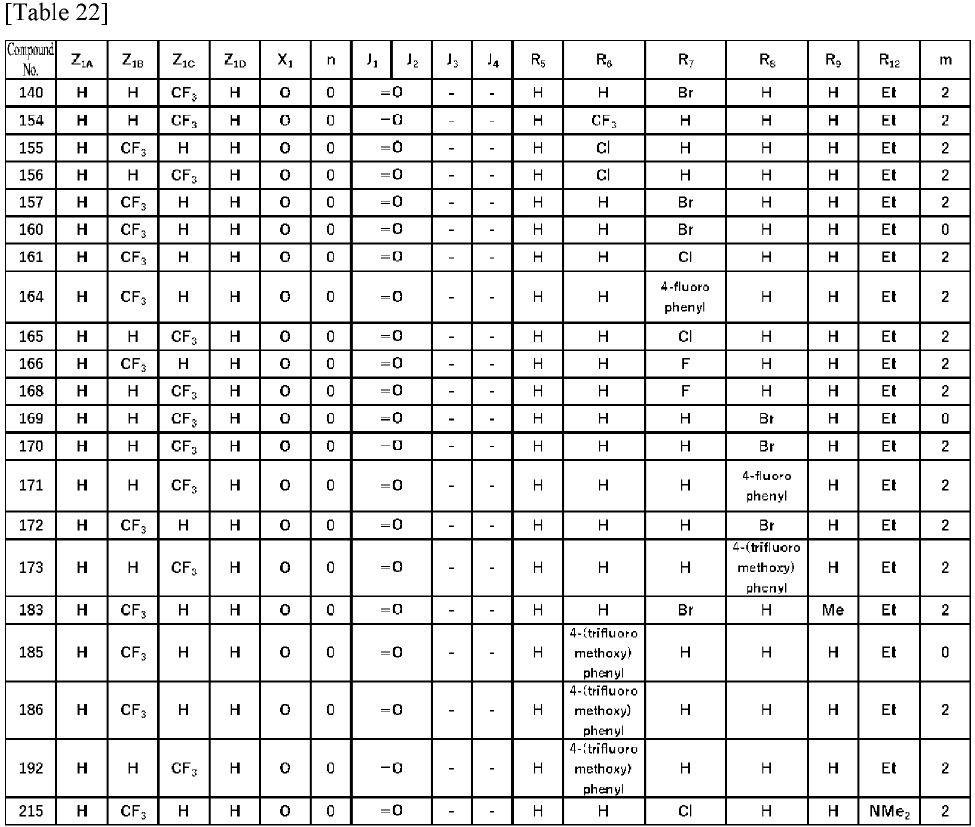

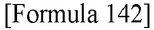

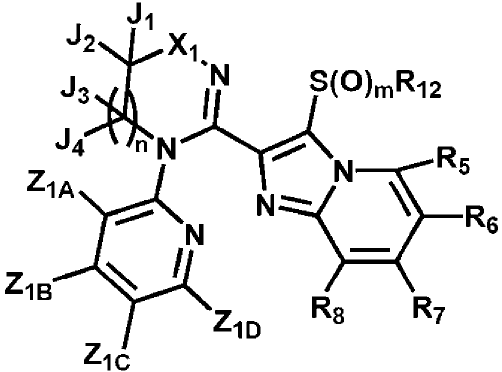

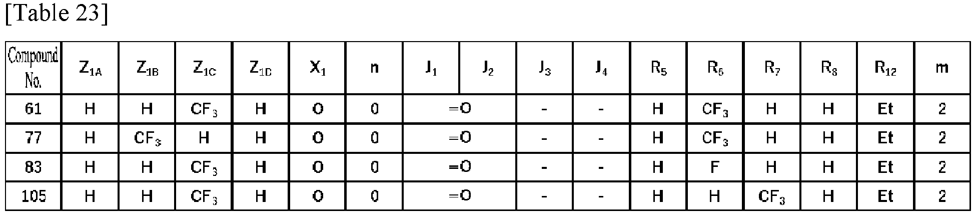

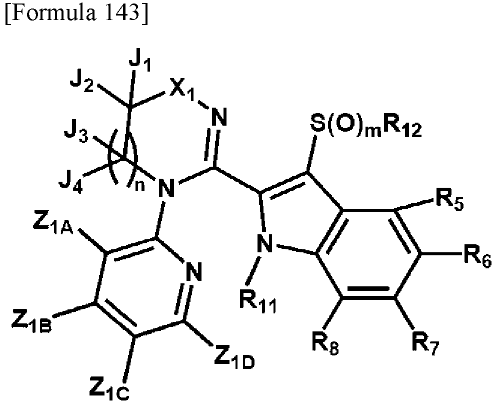

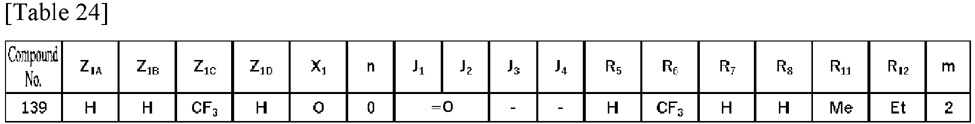

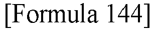

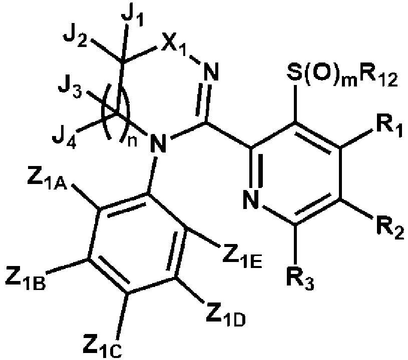

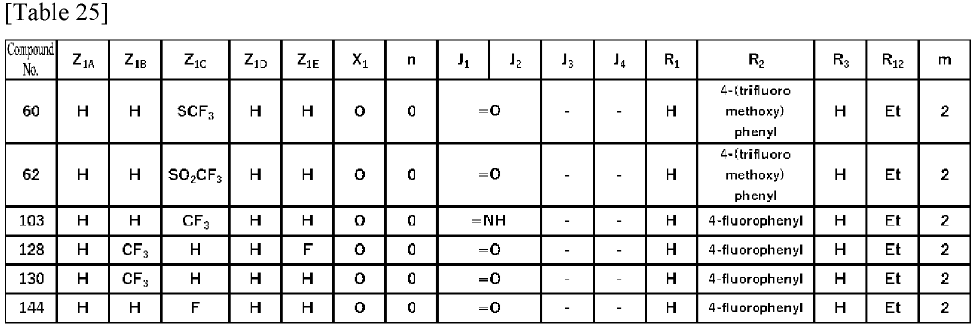

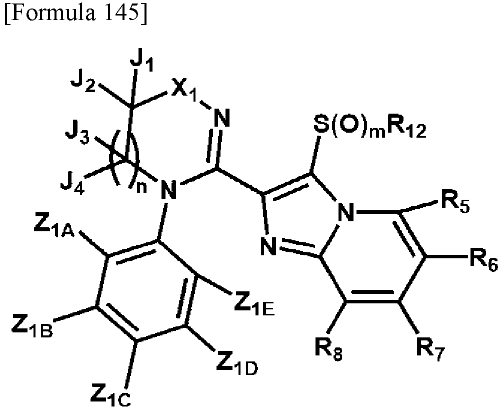

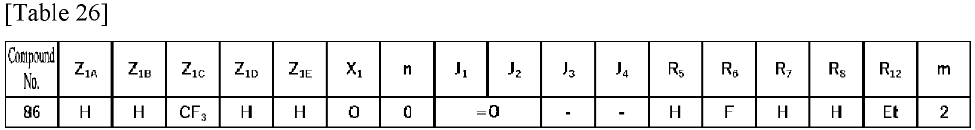

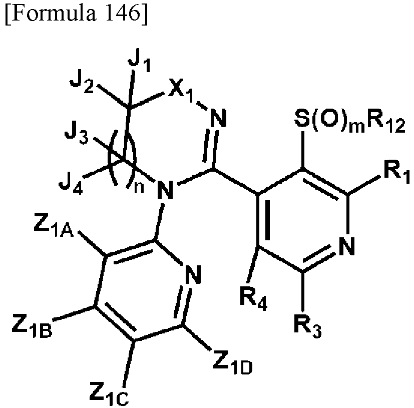

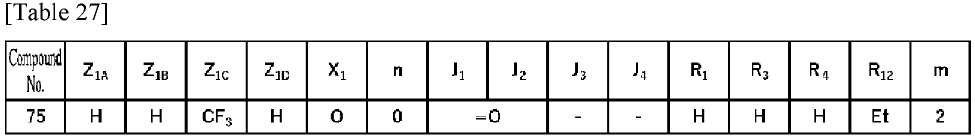

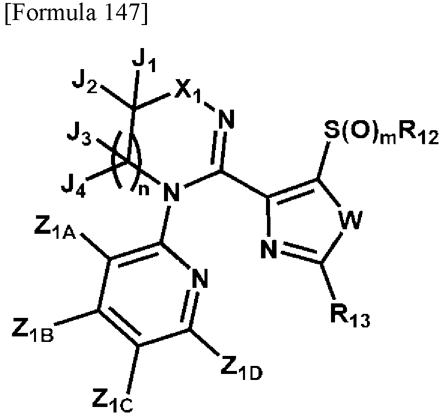

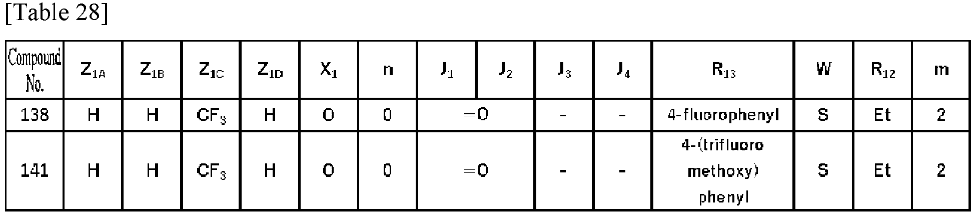

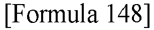

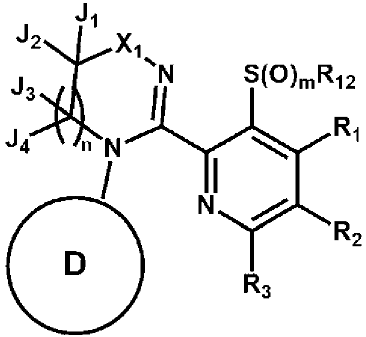

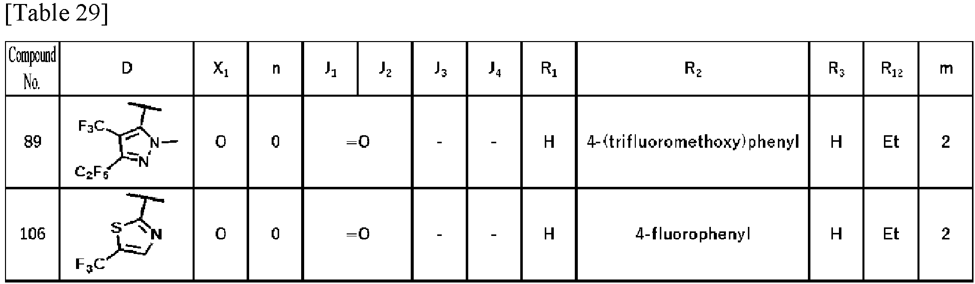

Group D: Compound 89 ; 1-methyl-3-(pentafluoroethyl)-4-(trifluoromethyl)-1H-pyrazol-5-yl Compound 106 ; 5-(trifluoromethyl)thiazol-2-yl

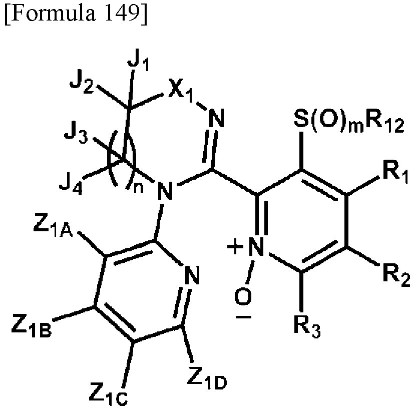

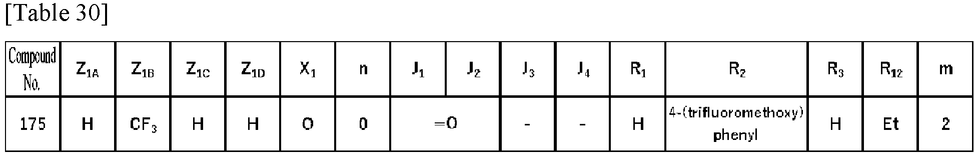

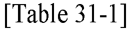

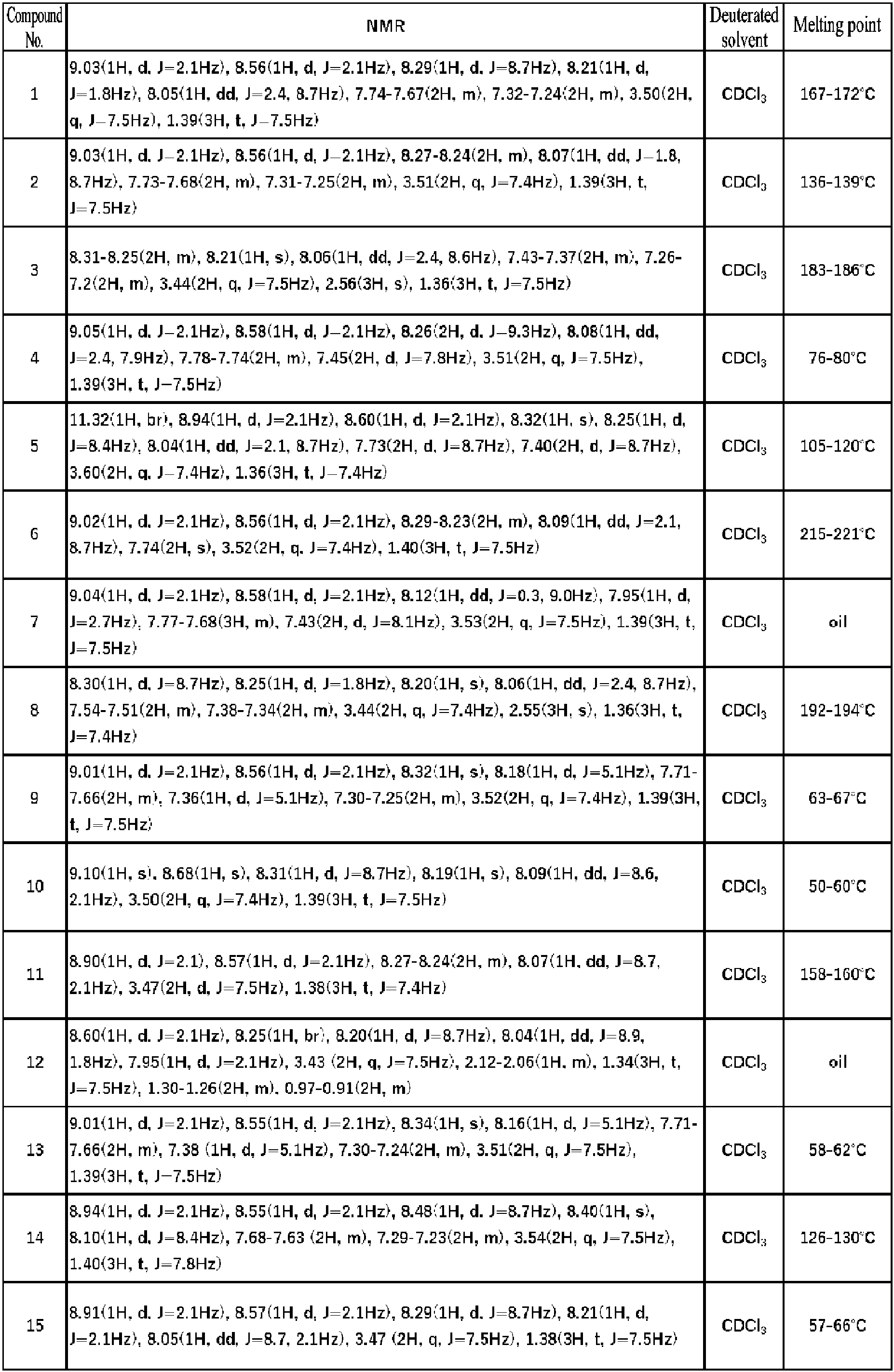

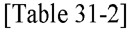

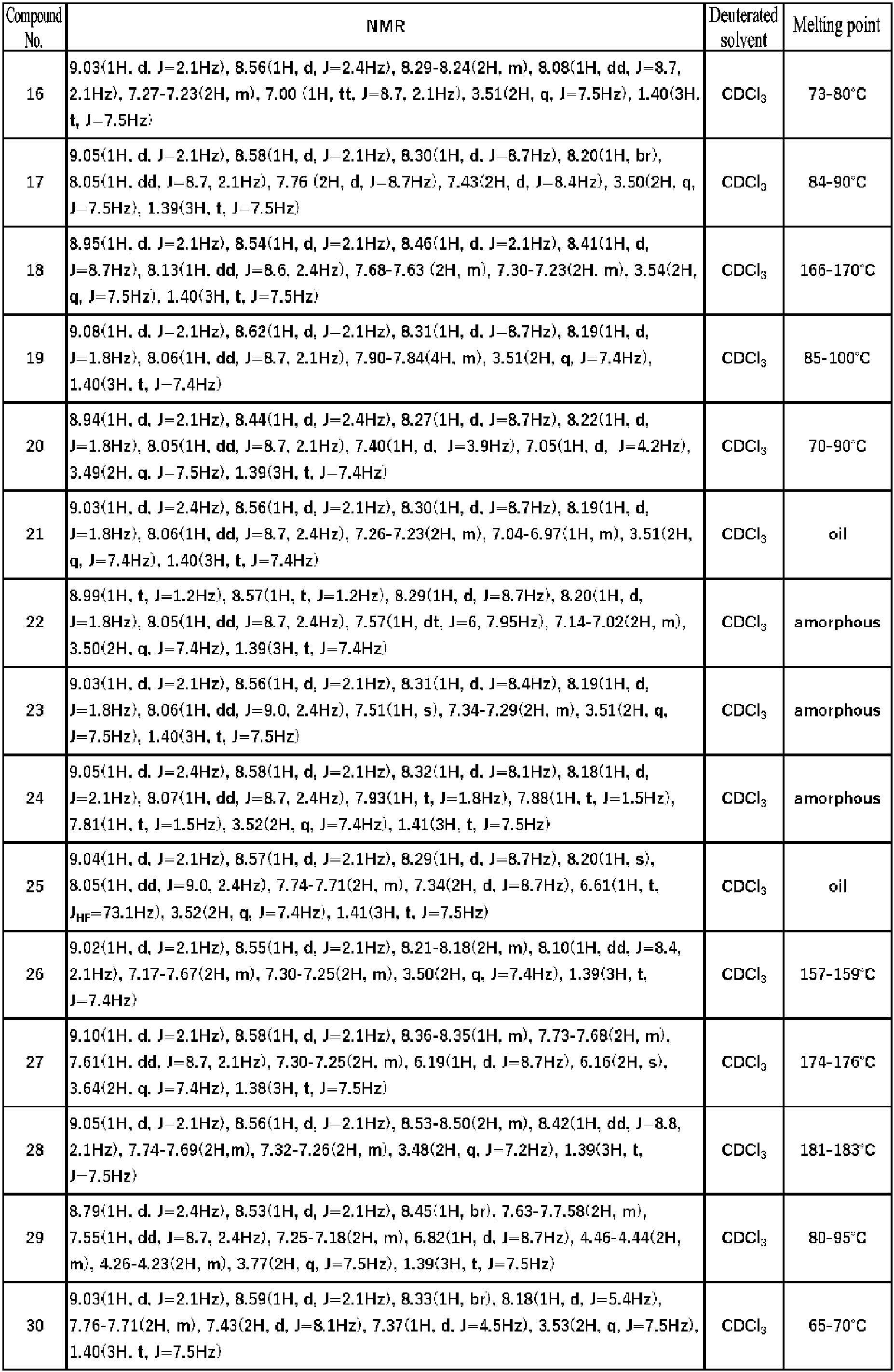

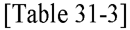

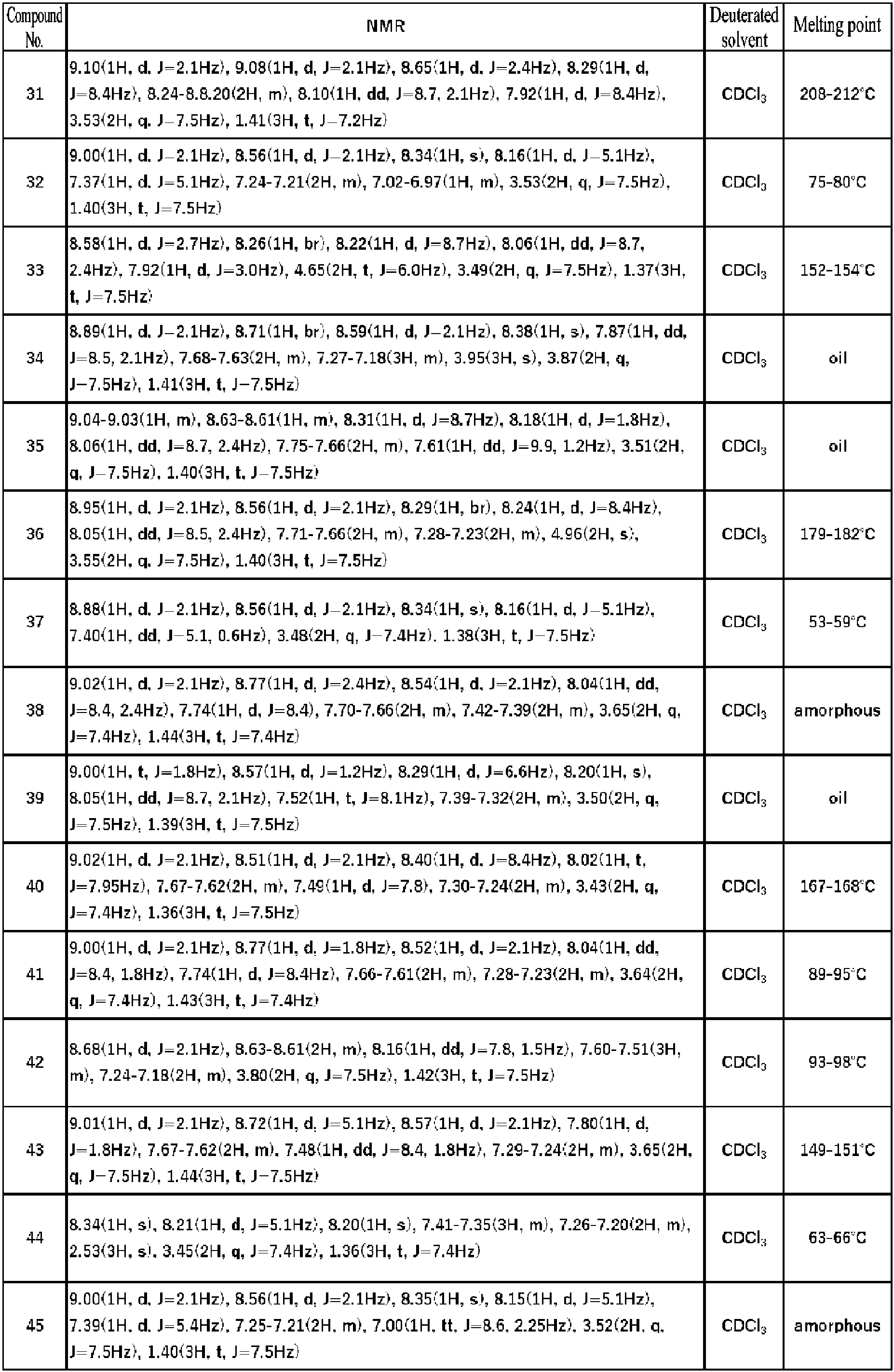

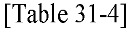

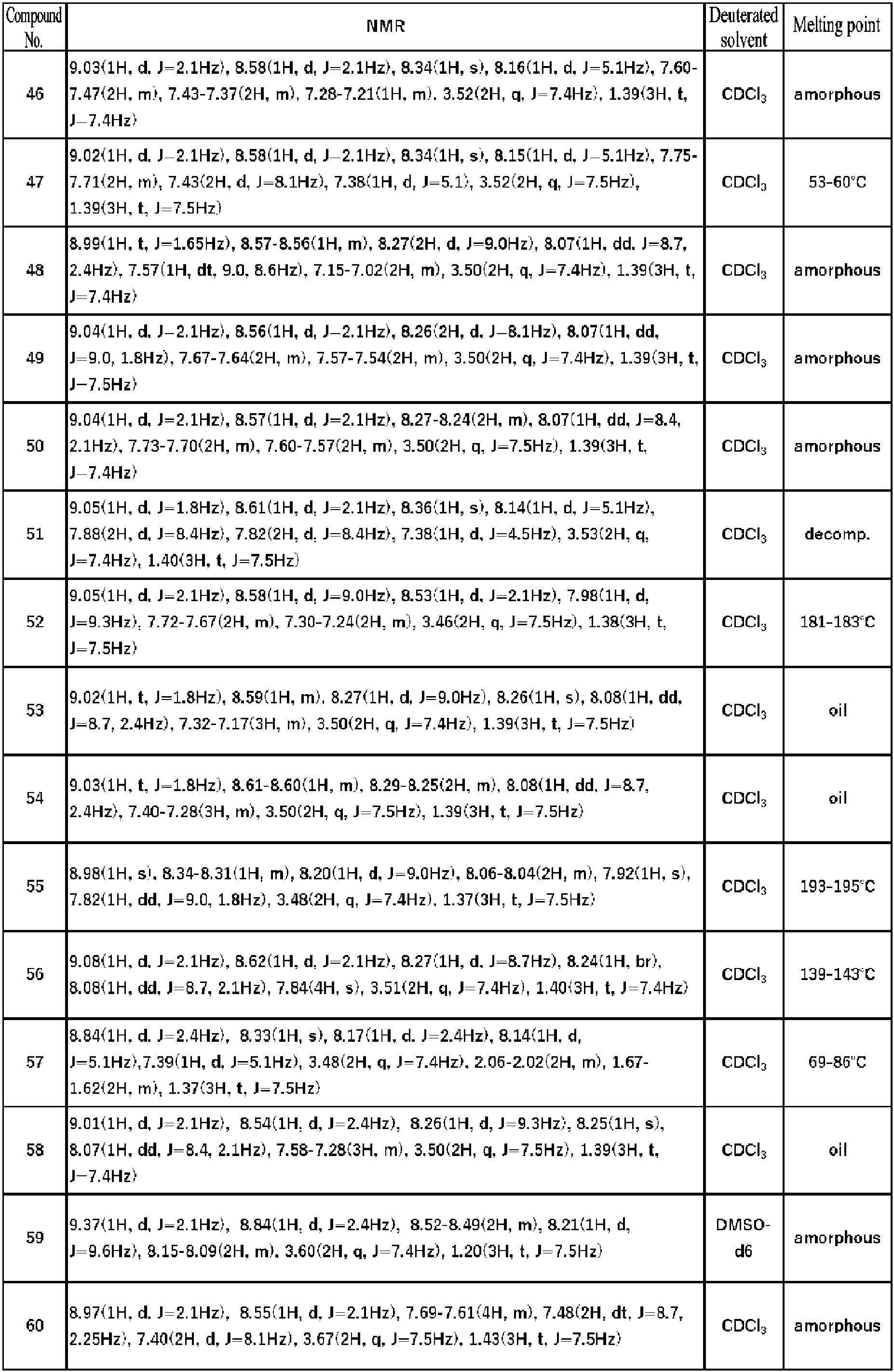

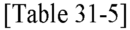

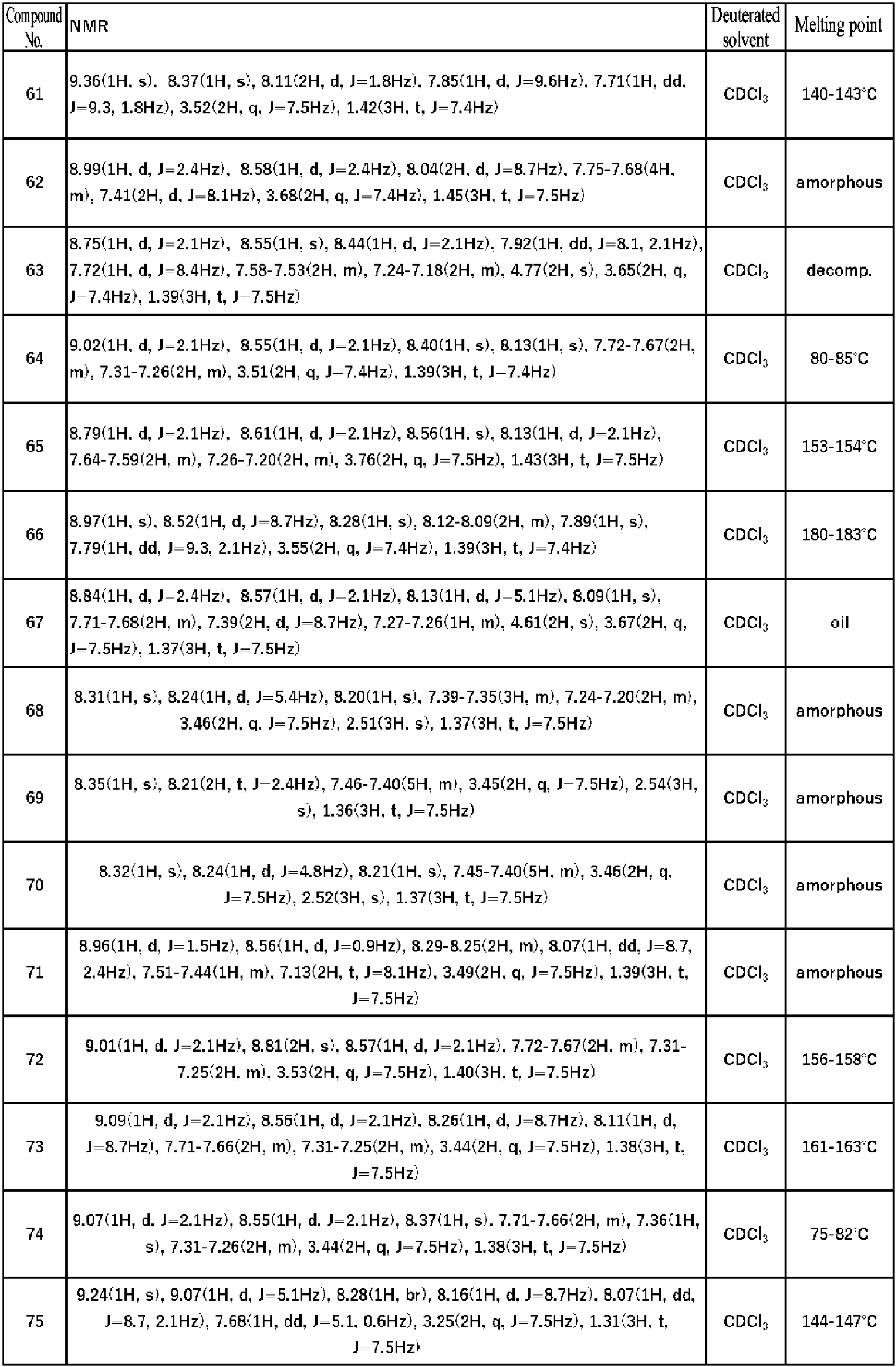

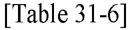

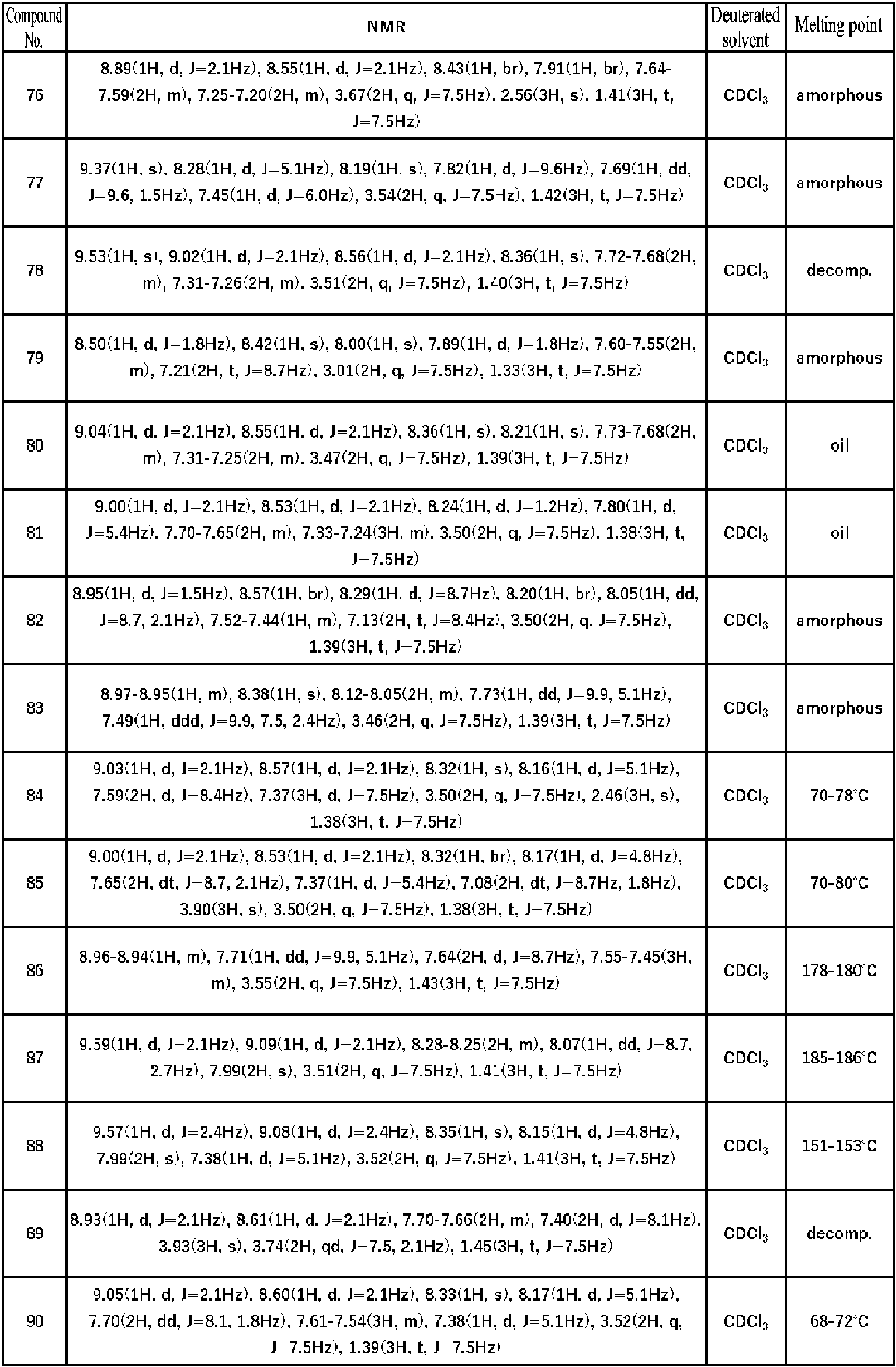

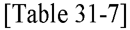

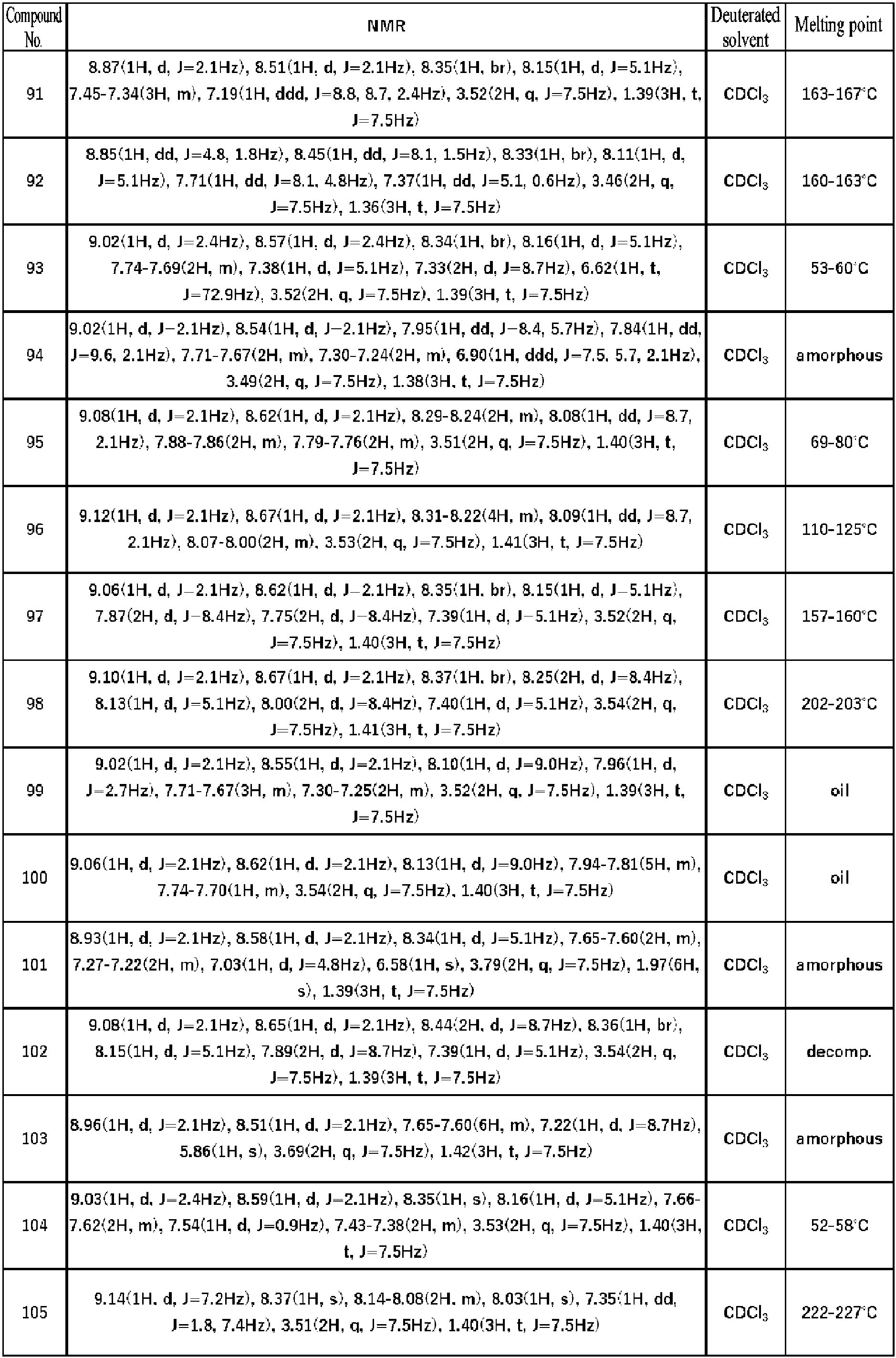

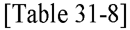

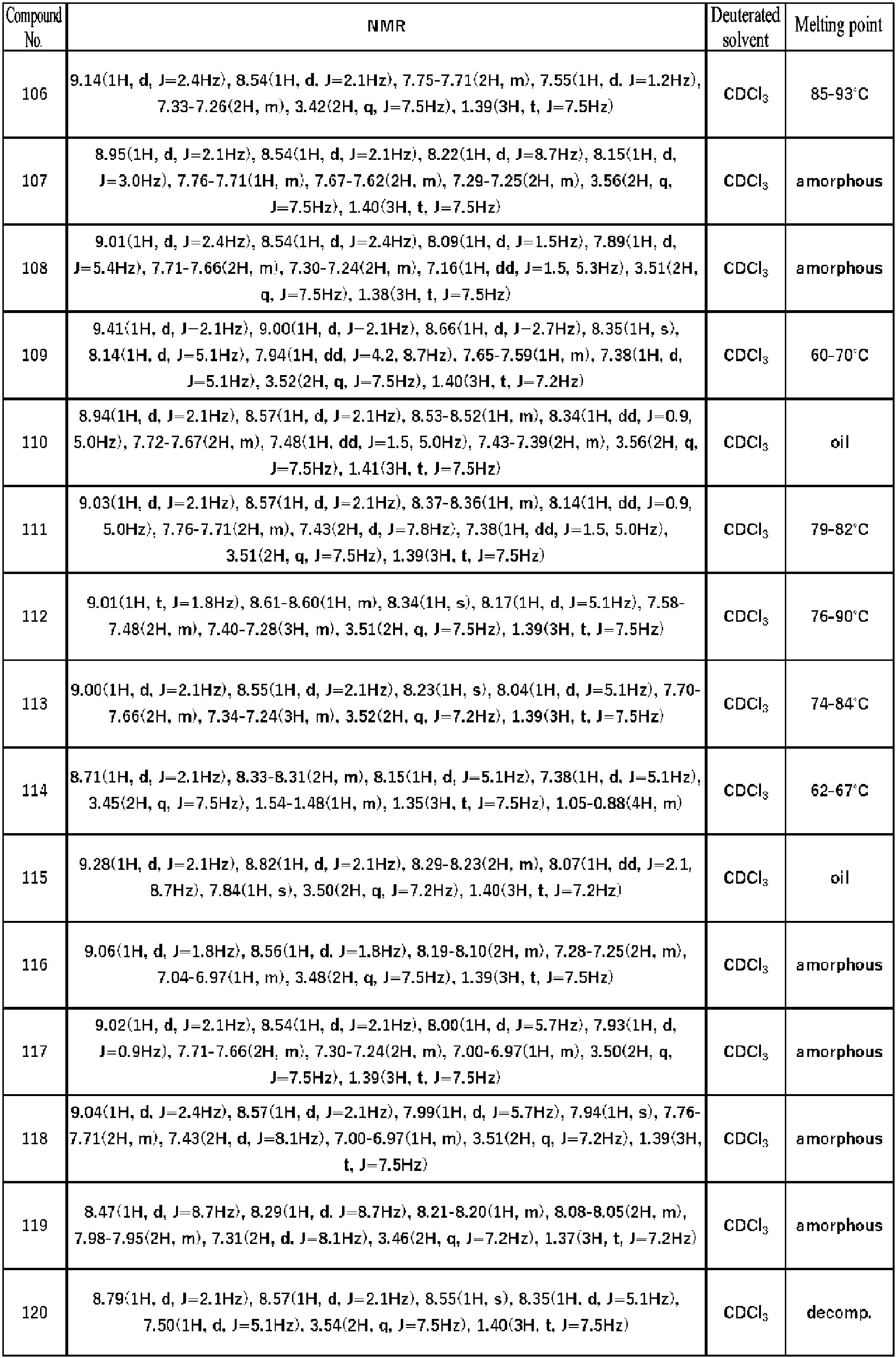

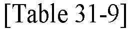

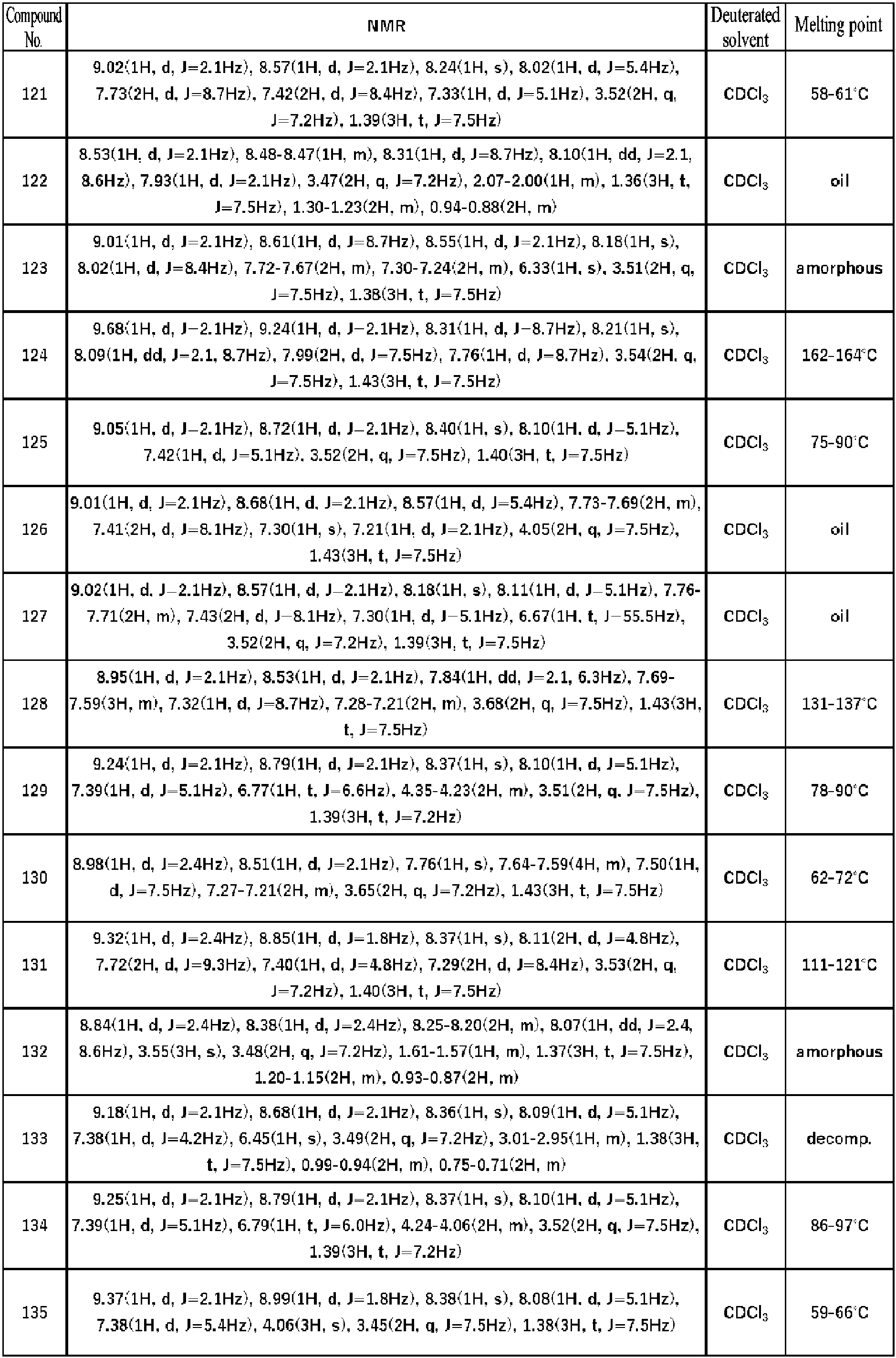

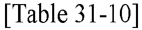

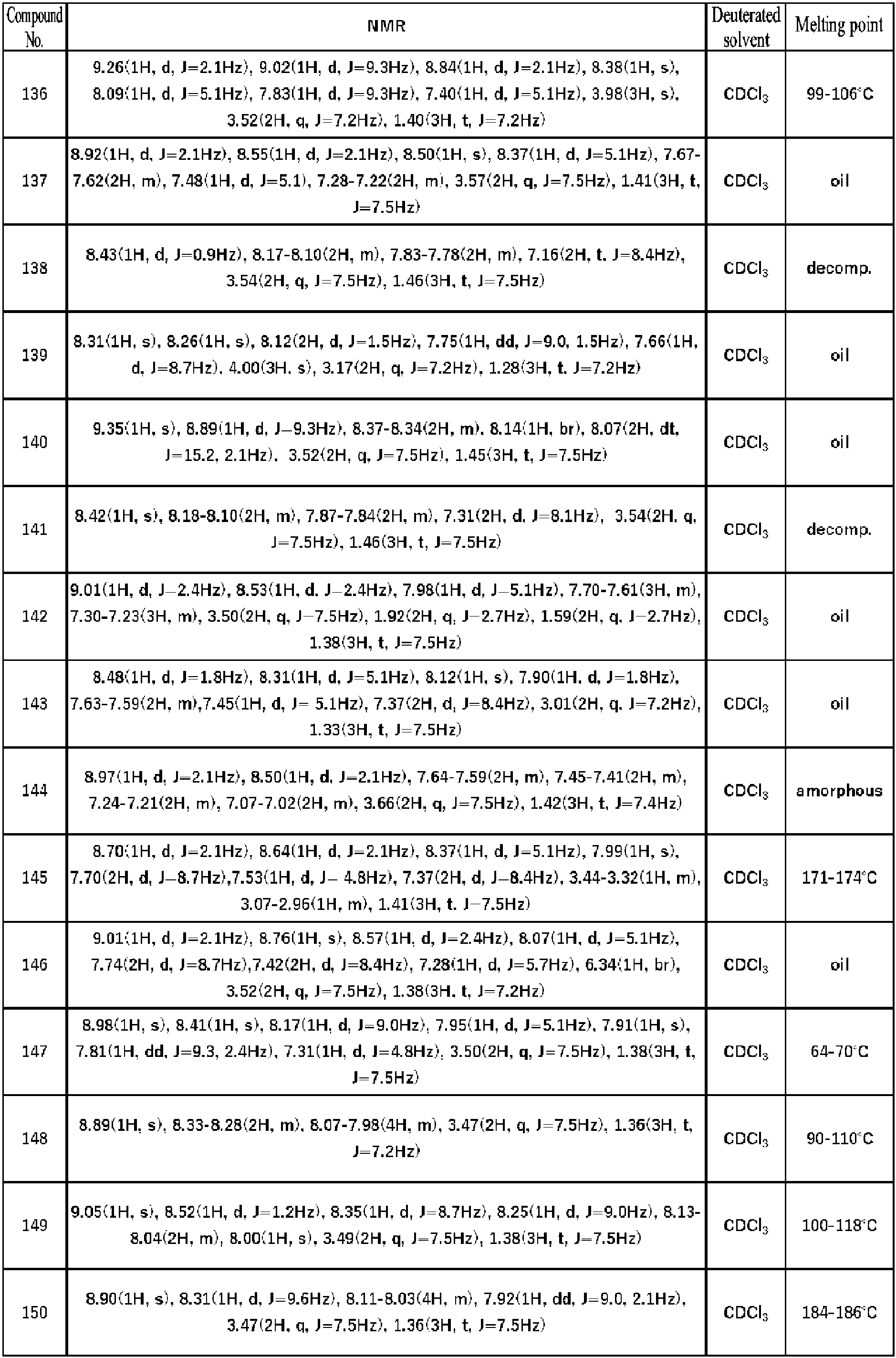

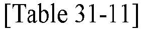

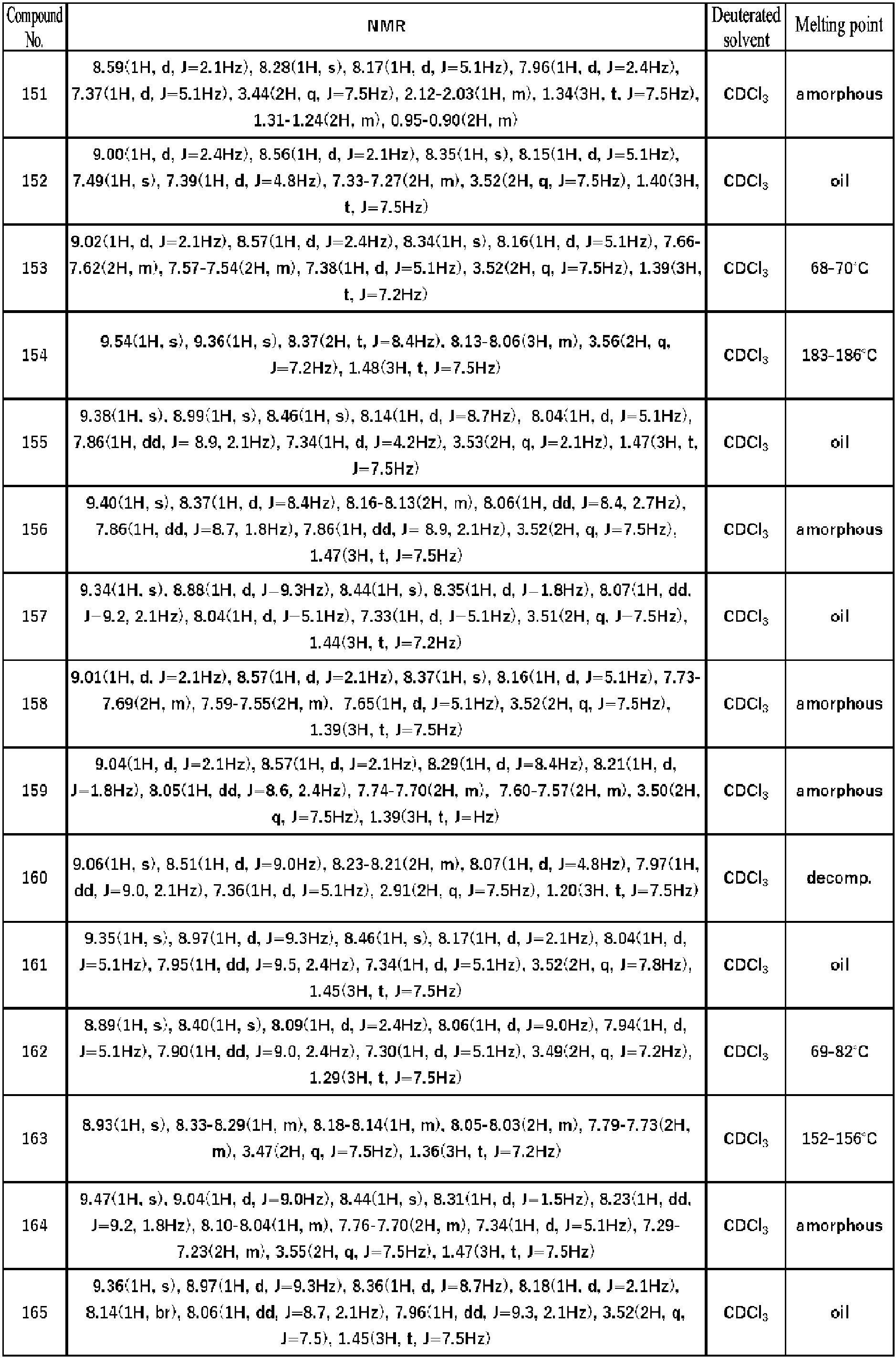

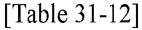

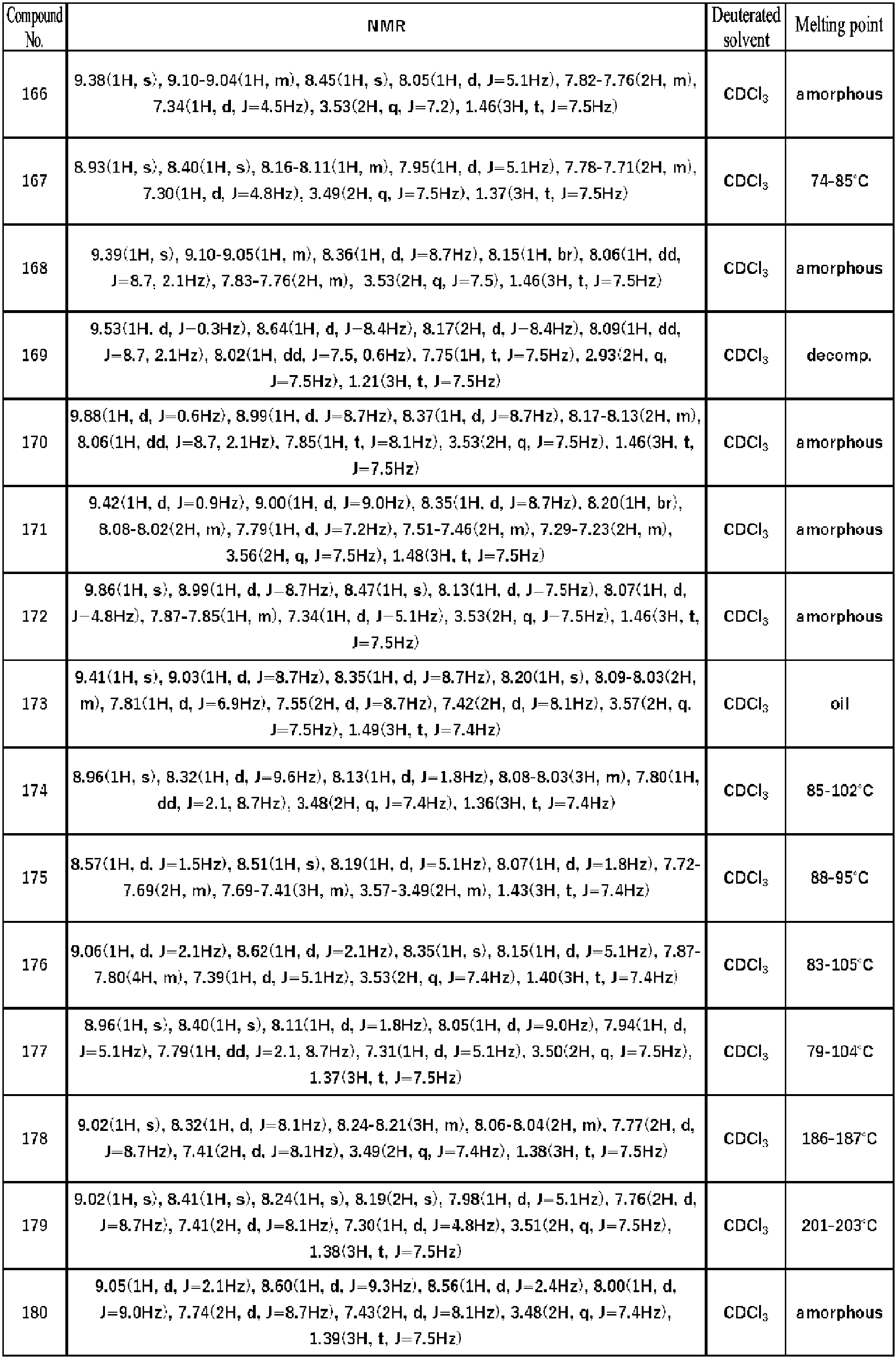

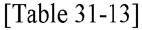

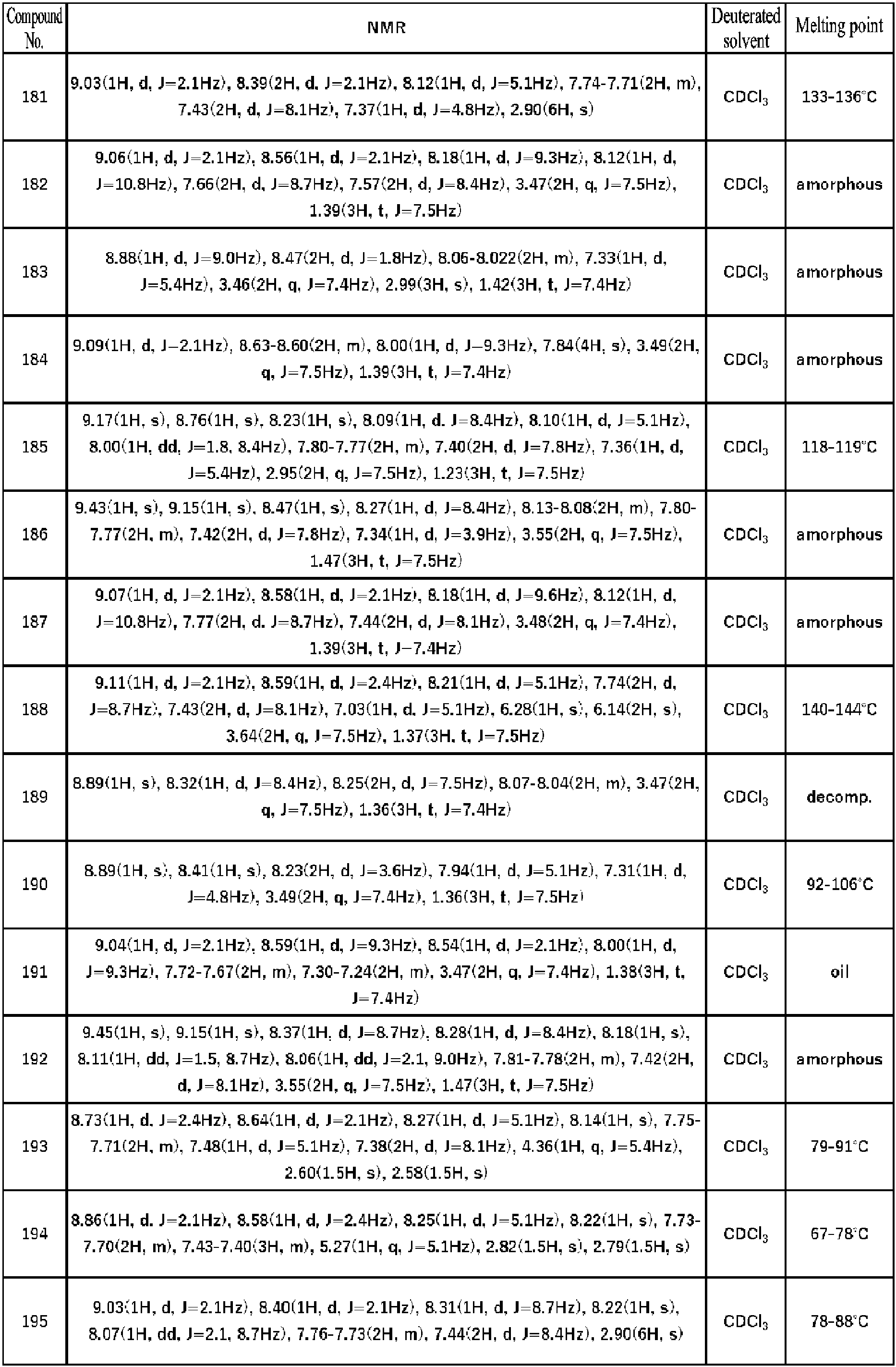

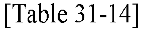

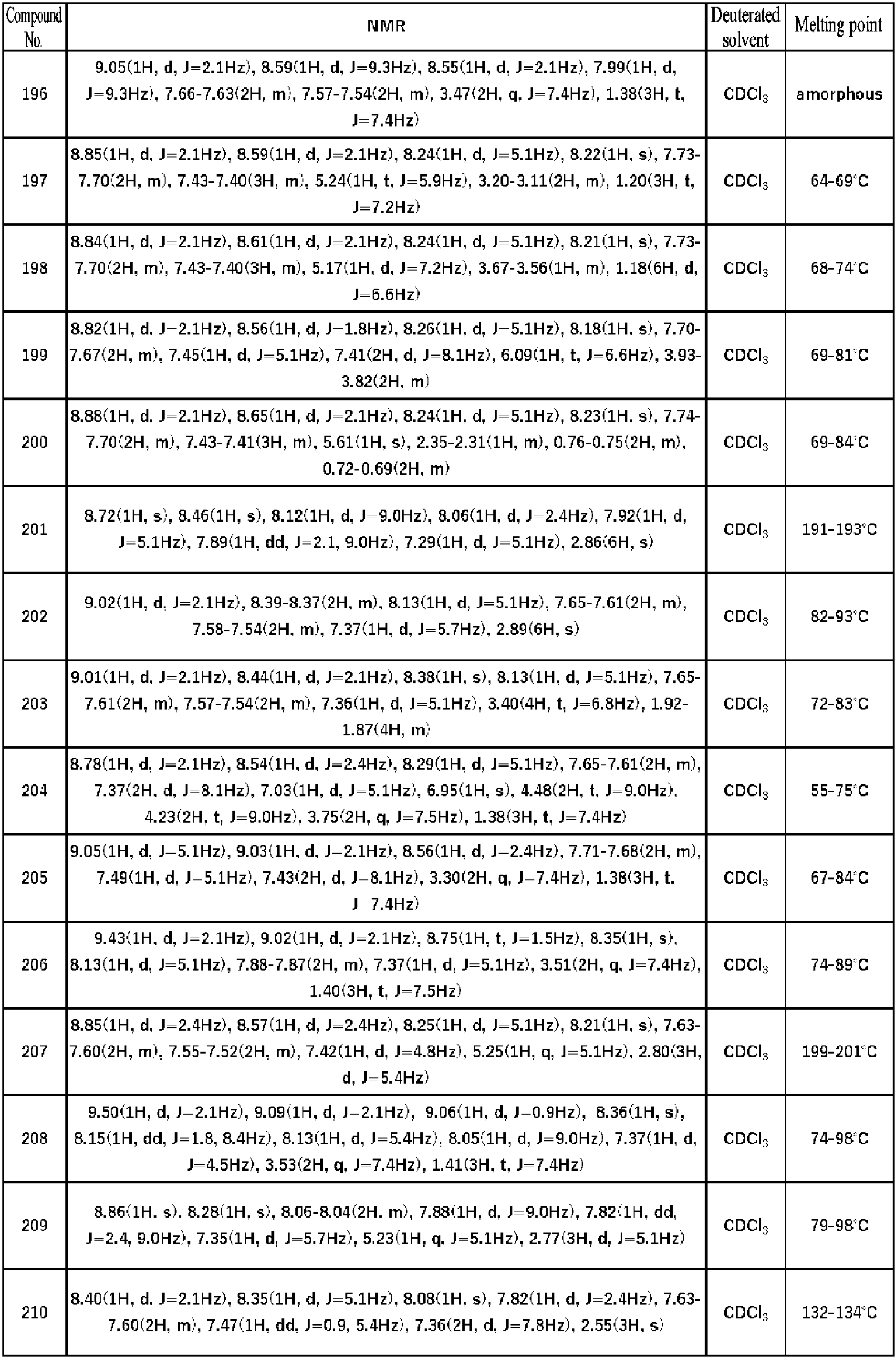

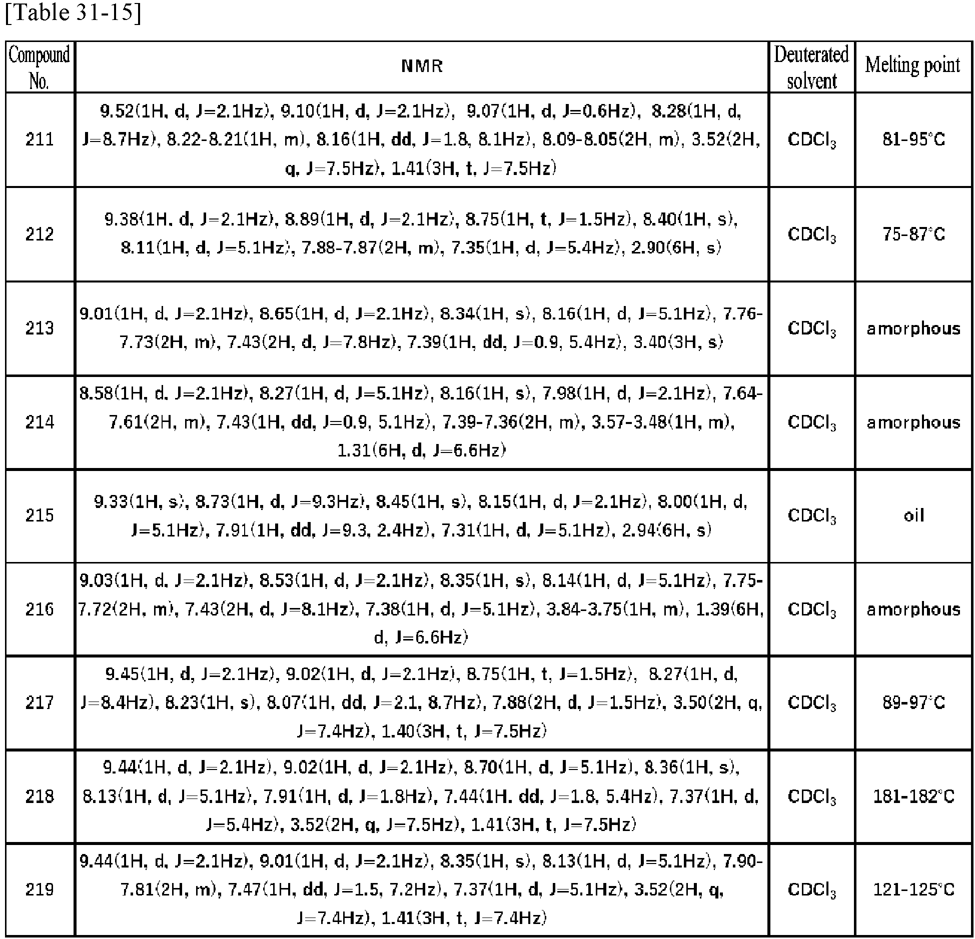

### <Formulation Examples>

Examples of an agrochemical formulation containing the compound represented by formula (1) according to the present invention as an active ingredient will be listed below.

### Formulation Example 1 [wettable powder]

The following ingredients were uniformly mixed and ground to obtain a wettable powder.

| | |
|---|---|
| Compound of the present invention | 30% by weight |
| Clay | 30% by weight |
| Diatomaceous earth | 35% by weight |
| SAN X P252 | 4% by weight |
| (Calcium lignosulfonate: trade name of Nippon Paper Industries Co., Ltd.) | |
| Sorpol 8070 (Sodium lauryl sulfate: trade name of Toho Chemical Industry Co., Ltd.) | 1% by weight |

### Formulation Example 2 [dust]

The following ingredients were uniformly mixed to obtain a dust.

| | |
|---|---|
| Compound of the present invention | 2% by weight |
| Clay | 90% by weight |
| Talc | 7% by weight |
| Calcium stearate | 1% by weight |

### Formulation Example 3 [emulsifiable concentrate]

The following ingredients were uniformly mixed and dissolved to obtain an emulsifiable concentrate.

| | |
|---|---|
| Compound of the present invention | 20% by weight |
| N,N-Dimethylformamide | 20% by weight |
| T-SOL 150 (Aromatic solvent: product name of JXTG Energy Corporation) | 50% by weight |
| Newkalgen CL-H | 10% by weight |

(POE alkyl phenyl ether: product name of TAKEMOTO OIL & FAT Co., Ltd.)

### Formulation Example 4 [emulsifiable concentrate]

The following ingredients were uniformly mixed and dissolved to obtain an emulsifiable concentrate.

| | |
|---|---|
| Compound of the present invention | 5% by weight |
| Xylene | 42.5% by weight |
| DMSO | 42.5% by weight |
| Newkalgen 2003 | 10% by weight |

(Mixture of POE allyl phenyl ether formaldehyde condensate and metal alkylbenzenesulfonate: product name of TAKEMOTO OIL & FAT Co., Ltd.)

### Formulation Example 5 [granule]

The following ingredients were uniformly ground and mixed, water was added, and the resulting mixture was thoroughly kneaded, and then granulated and dried to obtain a granule.

| | |
|---|---|
| Compound of the present invention | 5% by weight |
| Bentonite | 40% by weight |
| Talc | 10% by weight |
| Clay | 43% by weight |
| SAN X P252 | 2% by weight |

(Calcium lignosulfonate: trade name of Nippon Paper Industries Co., Ltd.)

### Formulation Example 6 [flowable]

All the amounts of the following ingredients excluding a 1% xanthan gum aqueous solution and an appropriate amount of water were premixed, and then the resulting mixture was ground by using a wet grinding mill. After that, the 1% xanthan gum aqueous solution and the remaining water were added to the obtained ground product to obtain 100% by weight of a flowable.

| | |
|---|---|
| Compound of the present invention | 25% by weight |
| Sorpol 7556 (POE styryl phenyl ether sulfate: product name of Toho Chemical Industry Co., Ltd.) | 5% by weight |
| Propylene glycol | 6% by weight |
| Bentonite | 1% by weight |
| 1% Xanthan gum aqueous solution | 3% by weight |
| Water | 60% by weight |

### Formulation Example 7 [granule]

The following ingredients were uniformly ground and mixed, water was added, and the resulting mixture was thoroughly kneaded, and then granulated and dried to obtain a granule.

| | |
|---|---|
| Compound of the present invention | 5% by weight |
| Bentonite | 40% by weight |
| Talc | 10% by weight |
| Clay | 43% by weight |
| SAN X P252 | 2% by weight |

(Calcium lignosulfonate: trade name of Nippon Paper Industries Co., Ltd.)

### <Biological Test Examples>

The following tests demonstrate the control efficacy of the compounds of the invention against certain pests. "Control efficacy" represents inhibition of growth (including mortality rate) of harmful invertebrates that remarkably reduces feeding. However, the pests subject to control achieved by the compounds are not limited to these species.

Compound numbers refer to compounds shown in Tables 15 to 30.

The insect mortality rate and the feeding damage prevention effect shown in the Biological Test Examples were calculated based on the following expression. Insect mortality rate (%) = (sum of number of abnormal insects and number of dead insects)/(total number of insects) × 100 Feeding damage prevention effect (%) = 100 × (total leaf area - leaf area damaged by subject insect pest)/(total leaf area)

### Biological Test Example 1: Cotton aphid (Aphis gossypii) control test (leaf piece application treatment)

A cucumber leaf was cut into a piece having a diameter of 3.5 cm and placed on absorbent cotton moistened with water. Two adult cotton aphids were released thereon, and allowed to lay nymphs for 24 hours, and then the adults were removed. 2 mL of a diluted solution of the test compound diluted to 200 ppm was applied onto the resulting cucumber leaf by using an spray tower. The cucumber leaf was air-dried and then placed in a plastic cup together with the absorbent cotton, the cup was covered with a lid, and the nymphs were reared in a constant temperature chamber at 25°C. Five days after the treatment, the insects were observed to be alive or dead, and the insect mortality rate was calculated.

As a result, compounds 1, 2, 3, 4, 9, 10, 11, 12, 13, 15, 18, 19, 20, 22, 25, 26, 29, 31, 35, 37, 39, 44, 47, 48, 49, 50, 51, 52, 53, 54, 55, 57, 61, 64, 71, 72, 73, 75, 76, 77, 78, 82, 83, 84, 85, 87, 88, 90, 92, 93, 95, 97, 99, 100, 101, 102, 103, 104, 105, 106, 107, 109, 112, 114, 115, 117, 118, 120, 121, 122, 123, 127, 132, 134, 135, 137, 140, 142, 146, 147, 148, 150, 151, 152, 153, 154, 155, 157, 158, 159, 160, 161, 162, 163, 164, 165, 166, 167, 168, 170, 171, 172, 173, 174, 176, 177, 180, 181, 183, 188, 190, 191, 194, 196, 197, 201, 206, 209, 211, 212, 213, 215, 217, 218, and 219 of the present invention showed an insect mortality rate of 80% or more.

### Biological Test Example 2: Tobacco whitefly (Bemisia tabaci) control test (foliage application treatment)

A cucumber leaf was cut into a piece having a diameter of 6.0 cm and placed on absorbent cotton moistened with water. 2 mL of a diluted solution of the test compound diluted to 200 ppm was applied onto the resulting cucumber leaf by using an spray tower. The cucumber leaf was air-dried and then placed in a plastic cup, 20 adult tobacco whiteflies were released thereinto, the cup was covered with a lid, and these insects were reared in a constant temperature chamber at 25°C. Five days after the treatment, the insects were observed to be alive or dead, and the insect mortality rate was calculated.

As a result, compounds 15 and 75 showed an insect mortality rate of 80% or more.

### Biological Test Example 3: Diamondback moth (Plutella xylostella) control test (leaf piece dipping treatment)

A cabbage leaf was cut into a piece having a diameter of 5.0 cm, and this cabbage leaf piece was immersed in 20 mL of a diluted solution of the test compound diluted to 200 ppm, and air-dried. After air-drying, the cabbage leaf piece was placed in a plastic cup, 10 third instar diamondback moth nymphs were released thereinto, the cup was covered with a lid, and the nymphs were reared in a constant temperature chamber at 25°C. Five days after the treatment, the larvae were observed to be alive or dead, and the insect mortality rate was calculated.

As a result, compounds 1, 2, 3, 4, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 37, 38, 39, 40, 41, 43, 44, 45, 46, 47, 48, 49, 50, 51, 52, 53, 54, 55, 56, 57, 58, 59, 60, 61, 62, 63, 64, 65, 66, 68, 69, 70, 71, 72, 73, 74, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 126, 127, 128, 129, 130, 132, 134, 135, 137, 138, 139, 140, 142, 143, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 188, 190, 191, 192, 193, 194, 195, 196, 197, 199, 200, 201, 202, 204, 205, 206, 207, 208, 209, 210, 211, 212, 213, 214, 215, 216, 217, 218, and 219 of the present invention showed an insect mortality rate of 80% or more.

### Biological Test Example 4: Brown planthopper (Nilaparvata lugens) control test (foliage dipping treatment)

Ten rice seedlings were taken, immersed in 20 mL of a chemical solution of the test compound diluted to 200 ppm, and air-dried. After air drying, the rice seedlings are held in a glass cylinder (inner diameters of 4.5 cm × 14 cm) by using urethane, and the glass cylinder is placed upright in a plastic cup filled with 40 mL of water. Third instar brown planthopper nymphs were released thereinto, the plastic cup was covered with a powder paper lid, and the nymphs were reared in a constant temperature chamber at 25°C. Five days after the treatment, the larvae were observed to be alive or dead, and the insect mortality rate was calculated.

As a result, compounds 10, 15, 38, 51, 57, 65, 77, 78, 88, 92, 93, 100, 109, 132, 135, 136, 145, 147, 151, 167, 168, 169, 171, 174, 177, 201, and 202 of the present invention showed an insect mortality rate of 80% or more.

### Biological Test Example 5: Tobacco cutworm (Spodoptera litura) control test (leaf piece dipping treatment)

A cabbage leaf was cut into a piece having a diameter of 5.0 cm, and this cabbage leaf piece was immersed in 20 mL of a diluted solution of the test compound diluted to 200 ppm, and air-dried. After air-drying, the cabbage leaf piece was placed in a plastic cup, 5 second instar tobacco cutworm larvae were released thereinto, the cup was covered with a lid, and the larvae were reared in a constant temperature chamber at 25°C. Five days after the treatment, the larvae were observed to be alive or dead, and the insect mortality rate was calculated.

As a result, compounds 1, 2, 3, 4, 6, 7, 8, 9, 10, 12, 13, 14, 15, 16, 17, 18, 19, 20, 21, 22, 23, 24, 25, 26, 27, 28, 29, 30, 31, 32, 33, 35, 37, 38, 39, 40, 41, 43, 44, 45, 46, 47, 48, 49, 50, 51, 52, 53, 54, 55, 56, 57, 58, 59, 60, 61, 62, 64, 65, 66, 68, 69, 70, 71, 73, 74, 76, 77, 78, 79, 80, 81, 82, 84, 85, 87, 88, 89, 90, 91, 93, 94, 95, 96, 97, 98, 99, 100, 101, 102, 103, 104, 105, 106, 107, 108, 109, 110, 111, 112, 113, 114, 115, 116, 117, 118, 119, 120, 121, 123, 124, 126, 127, 129, 130, 137, 138, 139, 140, 141, 142, 143, 145, 146, 147, 148, 149, 150, 151, 152, 153, 154, 155, 156, 157, 158, 159, 160, 161, 162, 163, 164, 165, 167, 171, 172, 173, 174, 175, 176, 177, 178, 179, 180, 181, 182, 183, 184, 186, 187, 188, 189, 190, 191, 192, 193, 194, 195, 196, 197, 198, 199, 200, 201, 202, 203, 204, 206, 207, 208, 209, 210, 211, 212, 213, 214, 215, 216, 217, 218, and 219 of the present invention showed an insect mortality rate of 80% or more.

### Biological Test Example 6: Southern yellow thrips (Thrips palmi) control test (leaf piece application treatment)

A cucumber leaf was cut into a piece having a diameter of 1.5 cm and placed on absorbent cotton moistened with water. 2 mL of a diluted solution of the test compound diluted to 200 ppm was applied onto the resulting cucumber leaf by using an spray tower. The cucumber leaf was air-dried and then placed in a plastic cup together with the absorbent cotton. Five first instar southern yellow thrips larvae were released thereinto, the plastic cup was covered with a lid, and the larvae were reared in a constant temperature chamber at 25°C. Two days after the treatment, the insects were observed to be alive or dead, and the insect mortality rate and the feeding damage prevention effect were calculated.

As a result, compounds 47, 57, 78, 80, 93, 95, 96, 97, 98, 103, 105, 109, 123, 146, 153, 177, and 201 of the present invention showed an insect mortality rate or a feeding damage prevention effect of 50% or more.

## Claims

1. A compound represented by formula (1) or a salt thereof or an N-oxide thereof: 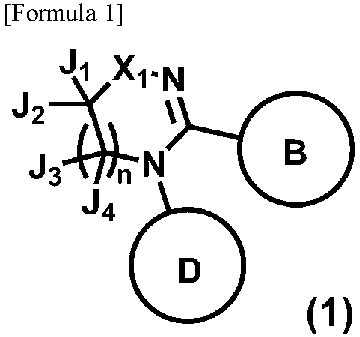 wherein
X₁ represents an oxygen atom, a sulfur atom, NY₁, or C(J₅J₆),
n represents 0 or 1,
J₁, J₂, J₃, and J₄ each independently represent
a hydrogen atom,
a halogen atom,
a (C₁ to C₆) alkyl group unsubstituted or arbitrarily substituted with Tₐ,
a halo (C₁ to C₆) alkyl group unsubstituted or arbitrarily substituted with Tₐ,
a (C₁ to C₆) alkoxy group unsubstituted or arbitrarily substituted with Tₐ,
a halo (C₁ to C₆) alkoxy group unsubstituted or arbitrarily substituted with Tₐ,
a hydroxy group, or
a group selected from the group consisting of
an O atom in a carbonyl group,
a S atom in a thiocarbonyl group,
an imino group substituted with Y₂, and
a methylene group substituted with J₇ and J₈, formed by two bonds of C-J₁ and C-J₂, or C-J₃ and C-J₄ together,
J₅ and J₆ each independently represent
a hydrogen atom,
a halogen atom,
a (C₁ to C₆) alkyl group unsubstituted or arbitrarily substituted with Tₐ,
a halo (C₁ to C₆) alkyl group unsubstituted or arbitrarily substituted with Tₐ, or
a group selected from the group consisting of
an O atom in a carbonyl group,
a S atom in a thiocarbonyl group,
an imino group substituted with Y₃, and
a methylene group substituted with J₇ and J₈, formed by two bonds of C-J₅ and C-J₆ together,
J₇ and J₈ each independently represent a group selected from the group consisting of
a hydrogen atom,
a halogen atom,
a (C₁ to C₆) alkyl group unsubstituted or arbitrarily substituted with Tₐ,
a halo (C₁ to C₆) alkyl group unsubstituted or arbitrarily substituted with Tₐ,
a (C₁ to C₆) alkoxy group unsubstituted or arbitrarily substituted with Tₐ,
a halo (C₁ to C₆) alkoxy group unsubstituted or arbitrarily substituted with Tₐ,
an amino group optionally substituted with one or two (C₁ to C₆) alkyl groups and/or halo (C₁ to C₆) alkyl groups, and
a hydroxy group,
D represents a group selected from the group consisting of
a phenyl group unsubstituted or arbitrarily substituted with Z₁, and
a heterocyclic group unsubstituted or arbitrarily substituted with Z₁,
B is a structure represented by B-1, B-2, B-3, B-4, B-5, B-6, or B-8 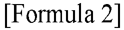 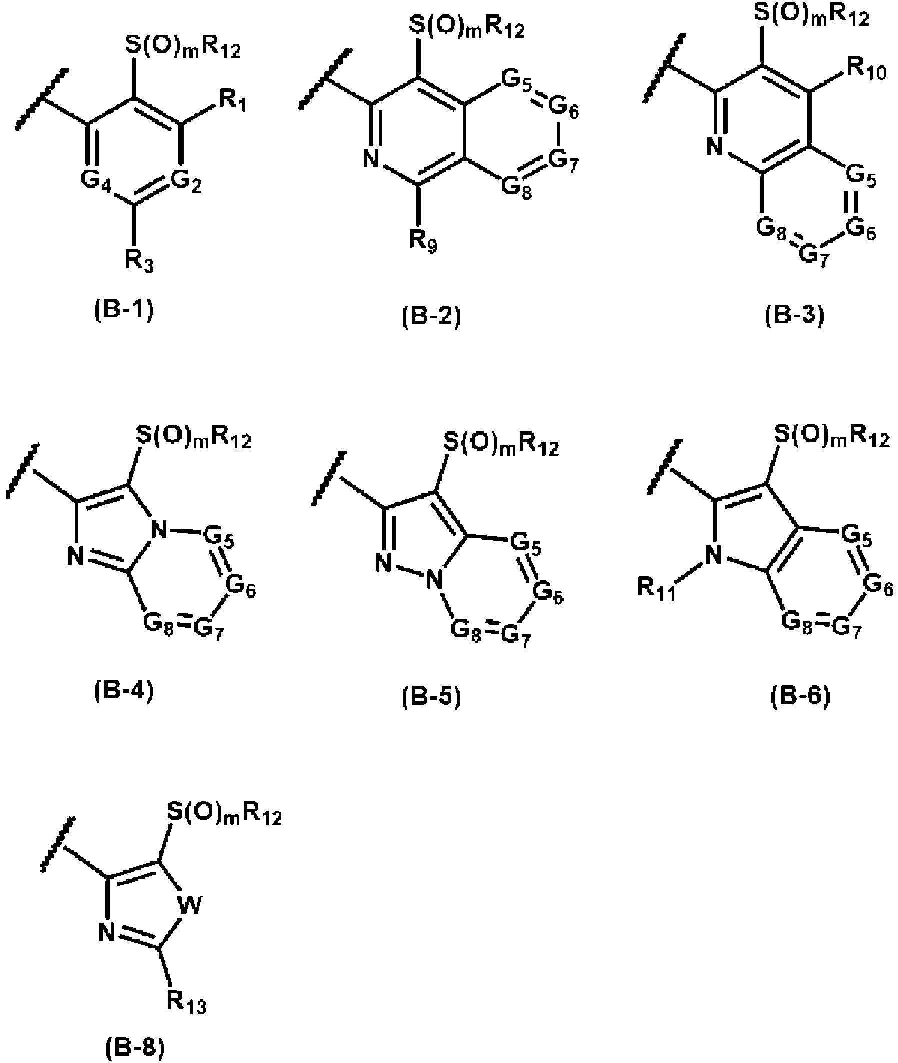
R₁₂ represents a group selected from the group consisting of
a (C₁ to C₆) alkyl group,
a halo (C₁ to C₆) alkyl group,
a (C₃ to C₆) cycloalkyl group,
a halo (C₃ to C₆) cycloalkyl group,
a (C₁ to C₆) alkoxy group,
a halo (C₁ to C₆) alkoxy group, and
an NYₐY_{b} group,
m represents 0, 1, or 2,
Yₐ and Y_{b} each independently represent a group selected from the group consisting of
a hydrogen atom,
a (C₁ to C₆) alkyl group,
a halo (C₁ to C₆) alkyl group,
a (C₃ to C₆) cycloalkyl group,
a halo (C₃ to C₆) cycloalkyl group,
a (C₁ to C₆) alkoxy group,
a halo (C₁ to C₆) alkoxy group,
a (C₁ to C₆) alkenyl group, and
a halo (C₁ to C₆) alkenyl group, or
a group forming an aliphatic 3- to 10-membered cyclic amino group or cyclic amide group unsubstituted or optionally substituted with one or more halogen atoms or (C₁ to C₆) alkyl groups, formed by connecting Yₐ and Y_{b} to each other,
provided that when R₁₂ is an NYₐY_{b} group, m represents 1 or 2,
G₂ represents a nitrogen atom or C(R₂),
G₄ represents a nitrogen atom or C(R₄),
provided that either or both of G₂ and G₄ is/are a nitrogen atom(s),
G₅ represents a nitrogen atom or C(R₅),
G₆ represents a nitrogen atom or C(R₆),
G₇ represents a nitrogen atom or C(R₇),
G₈ represents a nitrogen atom or C(R₈),
R₁, R₂, R₃, R₄, R₅, R₆, R₇, R₈, and R₁₃ each independently represent a group selected from the group consisting of
a hydrogen atom,
a halogen atom,
a (C₁ to C₆) alkyl group unsubstituted or arbitrarily substituted with T₁,
a halo (C₁ to C₆) alkyl group unsubstituted or arbitrarily substituted with T₁,
a (C₃ to C₆) cycloalkyl group unsubstituted or arbitrarily substituted with T₁,
a halo (C₃ to C₆) cycloalkyl group unsubstituted or arbitrarily substituted with T₁,
a (C₁ to C₆) alkoxy group unsubstituted or arbitrarily substituted with T₁,
a halo (C₁ to C₆) alkoxy group unsubstituted or arbitrarily substituted with T₁,
a (C₂ to C₆) alkenyl group unsubstituted or arbitrarily substituted with T₁,
a halo (C₂ to C₆) alkenyl group unsubstituted or arbitrarily substituted with T₁,
a (C₂ to C₆) alkynyl group unsubstituted or arbitrarily substituted with T₁,
a halo (C₂ to C₆) alkynyl group unsubstituted or arbitrarily substituted with T₁,
a (C₂ to C₆) alkenyloxy group unsubstituted or arbitrarily substituted with T₁,
a halo (C₂ to C₆) alkenyloxy group unsubstituted or arbitrarily substituted with T₁,
a (C₁ to C₆) alkylthio group unsubstituted or arbitrarily substituted with T₁,
a halo (C₁ to C₆) alkylthio group unsubstituted or arbitrarily substituted with T₁,
a (C₁ to C₆) alkylsulfinyl group unsubstituted or arbitrarily substituted with T₁,
a halo (C₁ to C₆) alkylsulfinyl group unsubstituted or arbitrarily substituted with T₁,
a (C₁ to C₆) alkylsulfonyl group unsubstituted or arbitrarily substituted with T₁,
a halo (C₁ to C₆) alkylsulfonyl group unsubstituted or arbitrarily substituted with T₁,
a (C₁ to C₆) alkylcarbonyl group unsubstituted or arbitrarily substituted with T₁,
a halo (C₁ to C₆) alkylcarbonyl group unsubstituted or arbitrarily substituted with T₁,
a (C₁ to C₆) alkylcarbonyloxy group unsubstituted or arbitrarily substituted with T₁,
a halo (C₁ to C₆) alkylcarbonyloxy group unsubstituted or arbitrarily substituted with T₁,
a (C₁ to C₆) alkoxycarbonyl group unsubstituted or arbitrarily substituted with T₁,
a halo (C₁ to C₆) alkoxycarbonyl group unsubstituted or arbitrarily substituted with T₁,
a (C₁ to C₆) alkoxycarbonyloxy group unsubstituted or arbitrarily substituted with T₁,
a halo (C₁ to C₆) alkoxycarbonyloxy group unsubstituted or arbitrarily substituted with T₁,
a (C₁ to C₆) alkylsulfonyloxy group unsubstituted or arbitrarily substituted with T₁,
a halo (C₁ to C₆) alkylsulfonyloxy group unsubstituted or arbitrarily substituted with T₁,
a phenyl group unsubstituted or arbitrarily substituted with Z₂,
a heterocyclic group unsubstituted or arbitrarily substituted with Z₂,
a phenoxy group unsubstituted or arbitrarily substituted with Z₂,
a pyridyloxy group unsubstituted or arbitrarily substituted with Z₂,
an NY₄Y₅ group,
a C(O)NY₄Y₅ groups,
a CH=NY₆ groups,
a cyano group,
a nitro group,
a hydroxy group,
a mercapto group,
a formyl group,
a carboxy group, and
an SF₅ group,
R₉ and R₁₀ each independently represent a group selected from the group consisting of
a hydrogen atom,
a halogen atom,
a (C₁ to C₆) alkyl group,
a halo (C₁ to C₆) alkyl group,
a (C₃ to C₆) cycloalkyl group,
a halo (C₃ to C₆) cycloalkyl group,
a (C₁ to C₆) alkoxy group,
a halo (C₁ to C₆) alkoxy group,
an NY₄Y₅ group,
a C(O)NY₄Y₅ group,
a cyano group,
a nitro group,
a hydroxy group,
a (C₁ to C₆) alkylcarbonyloxy group,
a halo (C₁ to C₆) alkylcarbonyloxy group,
a (C₁ to C₆) alkoxycarbonyloxy group, and
a halo (C₁ to C₆) alkoxycarbonyloxy group,
R₁₁ represents a group selected from the group consisting of
a hydrogen atom,
a (C₁ to C₆) alkyl group,
a halo (C₁ to C₆) alkyl group,
a (C₃ to C₆) cycloalkyl group,
a halo (C₃ to C₆) cycloalkyl group,
a (C₁ to C₆) alkoxy group,
a halo (C₁ to C₆) alkoxy group,
a (C₁ to C₆) alkylcarbonyl group,
a halo (C₁ to C₆) alkylcarbonyl group,
a (C₄ to C₇) cycloalkylcarbonyl group, and
a halo (C₄ to C₇) cycloalkylcarbonyl group,
Z₁ and Z₂ each independently represent a group selected from the group consisting of
a halogen atom,
a (C₁ to C₆) alkyl group unsubstituted or arbitrarily substituted with T₂,
a halo (C₁ to C₆) alkyl group unsubstituted or arbitrarily substituted with T₂,
a (C₃ to C₆) cycloalkyl group unsubstituted or arbitrarily substituted with T₂,
a halo (C₃ to C₆) cycloalkyl group unsubstituted or arbitrarily substituted with T₂,
a (C₁ to C₆) alkoxy group unsubstituted or arbitrarily substituted with T₂,
a halo (C₁ to C₆) alkoxy group unsubstituted or arbitrarily substituted with T₂,
a (C₂ to C₆) alkenyl group unsubstituted or arbitrarily substituted with T₂,
a halo (C₂ to C₆) alkenyl group unsubstituted or arbitrarily substituted with T₂,
a (C₂ to C₆) alkenyloxy group unsubstituted or arbitrarily substituted with T₂,
a halo (C₂ to C₆) alkenyloxy group unsubstituted or arbitrarily substituted with T₂,
a (C₁ to C₆) alkylthio group unsubstituted or arbitrarily substituted with T₂,
a halo (C₁ to C₆) alkylthio group unsubstituted or arbitrarily substituted with T₂,
a (C₁ to C₆) alkylsulfinyl group unsubstituted or arbitrarily substituted with T₂,
a halo (C₁ to C₆) alkylsulfinyl group unsubstituted or arbitrarily substituted with T₂,
a (C₁ to C₆) alkylsulfonyl group unsubstituted or arbitrarily substituted with T₂,
a halo (C₁ to C₆) alkylsulfonyl group unsubstituted or arbitrarily substituted with T₂,
a (C₁ to C₆) alkylcarbonyl group unsubstituted or arbitrarily substituted with T₂,
a halo (C₁ to C₆) alkylcarbonyl group unsubstituted or arbitrarily substituted with T₂,
a (C₁ to C₆) alkoxycarbonyl group unsubstituted or arbitrarily substituted with T₂,
a halo (C₁ to C₆) alkoxycarbonyl group that is unsubstituted or arbitrarily substituted with T₂,
a (C₁ to C₆) alkylcarbonyloxy group unsubstituted or arbitrarily substituted with T₂,
a halo (C₁ to C₆) alkylcarbonyloxy group unsubstituted or arbitrarily substituted with T₂,
a (C₁ to C₆) alkoxycarbonyloxy group unsubstituted or arbitrarily substituted with T₂,
a halo (C₁ to C₆) alkoxycarbonyloxy group unsubstituted or arbitrarily substituted with T₂,
a (C₁ to C₆) alkylsulfonyloxy group unsubstituted or arbitrarily substituted with T₂,
a halo (C₁ to C₆) alkylsulfonyloxy group unsubstituted or arbitrarily substituted with T₂,
an NY₄Y₅ group,
a C(O)NY₄Y₅ group,
a cyano group,
a nitro group,
a hydroxy group,
a mercapto group,
a formyl group,
a carboxy group,
an SF₅ group,
or
a group forming a 5- or 6-membered alicyclic ring unsubstituted or optionally substituted with one or more halogen atoms or (C₁ to C₆) alkyl groups, formed by connecting two adjacent Z₁ or two adjacent Z₂ to each other, or a group forming a 1,3-dioxole group or a 1,4-dioxin group formed by two adjacent Z₁ or two adjacent Z₂ together with a carbon atom to which the two Z₁ or the two Z₂ are attached, wherein the 1,3-dioxole group and the 1,4-dioxin group are optionally substituted with one or two or more independent halogen atoms,
Y₁, Y₂, and Y₃ each independently represent a group selected from the group consisting of
a hydrogen atom,
a (C₁ to C₆) alkyl group unsubstituted or arbitrarily substituted with T₃,
a halo (C₁ to C₆) alkyl group unsubstituted or arbitrarily substituted with T₃,
a (C₃ to C₆) cycloalkyl group unsubstituted or arbitrarily substituted with T₃,
a halo (C₃ to C₆) cycloalkyl group unsubstituted or arbitrarily substituted with T₃,
a (C₂ to C₆) alkenyl group unsubstituted or arbitrarily substituted with T₃,
a halo (C₂ to C₆) alkenyl group unsubstituted or arbitrarily substituted with T₃,
a (C₁ to C₆) alkoxy group unsubstituted or arbitrarily substituted with T₃,
a halo (C₁ to C₆) alkoxy group unsubstituted or arbitrarily substituted with T₃,
a (C₁ to C₆) alkylcarbonyl group unsubstituted or arbitrarily substituted with T₃,
a halo (C₁ to C₆) alkylcarbonyl group unsubstituted or arbitrarily substituted with T₃,
a (C₃ to C₆) cycloalkylcarbonyl group unsubstituted or arbitrarily substituted with T₃,
a halo (C₃ to C₆) cycloalkylcarbonyl group unsubstituted or arbitrarily substituted with T₃,
a (C₁ to C₆) alkoxycarbonyl group unsubstituted or arbitrarily substituted with T₃,
a halo (C₁ to C₆) alkoxycarbonyl group unsubstituted or arbitrarily substituted with T₃,
a (C₁ to C₆) alkylsulfonyl group unsubstituted or arbitrarily substituted with T₃,
a halo (C₁ to C₆) alkylsulfonyl group unsubstituted or arbitrarily substituted with T₃, and
a hydroxy group,
Tₐ and T₁ each independently represent a group selected from the group consisting of
a (C₃ to C₆) cycloalkyl group,
a halo (C₃ to C₆) cycloalkyl group,
a (C₁ to C₆) alkoxy group,
a halo (C₁ to C₆) alkoxy group,
a (C₁ to C₆) alkylcarbonyl group,
a halo (C₁ to C₆) alkylcarbonyl group,
a (C₁ to C₆) alkoxycarbonyl group,
a halo (C₁ to C₆) alkoxycarbonyl group,
a (C₁ to C₆) alkylthio group,
a halo (C₁ to C₆) alkylthio group,
a (C₁ to C₆) alkylsulfinyl group,
a halo (C₁ to C₆) alkylsulfinyl group,
a (C₁ to C₆) alkylsulfonyl group,
a halo (C₁ to C₆) alkylsulfonyl group,
an NY₄Y₅ group,
a C(O)NY₄Y₅ group,
a cyano group,
a nitro group,
a hydroxy group,
a mercapto group,
a formyl group,
a carboxy group,
a phenyl group unsubstituted or arbitrarily substituted with Z₃,
a heterocycle unsubstituted or arbitrarily substituted with Z₃,
a phenoxy group unsubstituted or arbitrarily substituted with Z₃, and
a pyridyloxy group unsubstituted or arbitrarily substituted with Z₃,
T₂ and T₃ each independently represents a group selected from the group consisting of
a (C₃ to C₆) cycloalkyl group,
a halo (C₃ to C₆) cycloalkyl group,
a (C₁ to C₆) alkoxy group,
a halo (C₁ to C₆) alkoxy group,
a (C₁ to C₆) alkylcarbonyl group,
a halo (C₁ to C₆) alkylcarbonyl group,
a (C₁ to C₆) alkoxycarbonyl group,
a halo (C₁ to C₆) alkoxycarbonyl group,
a (C₁ to C₆) alkylthio group,
a halo (C₁ to C₆) alkylthio group,
a (C₁ to C₆) alkylsulfinyl group,
a halo (C₁ to C₆) alkylsulfinyl group,
a (C₁ to C₆) alkylsulfonyl group,
a halo (C₁ to C₆) alkylsulfonyl group,
an NY₄Y₅ group,
a C(O)NY₄Y₅ group,
a cyano group,
a nitro group,
a hydroxy group,
a mercapto group,
a formyl group, and
a carboxy group,
Z₃, or when a plurality thereof is present, each independently, represents a group selected from the group consisting of
a halogen atom,
a (C₁ to C₆) alkyl group,
a halo (C₁ to C₆) alkyl group,
a (C₃ to C₆) cycloalkyl group,
a halo (C₃ to C₆) cycloalkyl group,
a 1-cyano-(C₃ to C₆) cycloalkyl group,
a (C₁ to C₆) alkoxy group,
a halo (C₁ to C₆) alkoxy group,
a (C₁ to C₆) alkylthio group,
a halo (C₁ to C₆) alkylthio group,
a (C₁ to C₆) alkylsulfinyl group,
a halo (C₁ to C₆) alkylsulfinyl group,
a (C₁ to C₆) alkylsulfonyl group,
a halo (C₁ to C₆) alkylsulfonyl group,
a (C₁ to C₆) alkylcarbonyl group,
a halo (C₁ to C₆) alkylcarbonyl group,
a (C₁ to C₆) alkoxycarbonyl group,
a halo (C₁ to C₆) alkoxycarbonyl group,
an NY₄Y₅ group,
a C(O)NY₄Y₅ group,
a cyano group,
a nitro group,
a hydroxy group,
a mercapto group,
a formyl group,
a carboxy group, and
an SF₅ group,
Y₄ and Y₅ each independently represent a group selected from the group consisting of
a hydrogen atom,
a (C₁ to C₆) alkyl group,
a halo (C₁ to C₆) alkyl group,
a (C₃ to C₆) cycloalkyl group,
a halo (C₃ to C₆) cycloalkyl group,
a (C₁ to C₆) alkoxy group,
a halo (C₁ to C₆) alkoxy group,
a (C₁ to C₆) alkylcarbonyl group,
a halo (C₁ to C₆) alkylcarbonyl group,
a (C₃ to C₆) cycloalkylcarbonyl group,
a halo (C₃ to C₆) cycloalkylcarbonyl group,
a (C₁ to C₆) alkoxycarbonyl group,
a halo (C₁ to C₆) alkoxycarbonyl group,
a (C₁ to C₆) alkylsulfonyl group,
a halo (C₁ to C₆) alkylsulfonyl group, and
a phenyl group unsubstituted or arbitrarily substituted with Z₃,
Y₆, or when a plurality thereof is present, each independently, represents a group selected from the group consisting of
a hydrogen atom,
a (C₁ to C₆) alkyl group unsubstituted or arbitrarily substituted with T₃,
a halo (C₁ to C₆) alkyl group unsubstituted or arbitrarily substituted with T ₃,
a (C₃ to C₆) cycloalkyl group,
a halo (C₃ to C₆) cycloalkyl group,
a (C₁ to C₆) alkoxy group unsubstituted or arbitrarily substituted with a cyano group,
a halo (C₁ to C₆) alkoxy group,
a (C₃ to C₆) cycloalkoxy group,
a halo (C₃ to C₆) cycloalkoxy group,
a halo (C₂ to C₆) alkenyl group,
a (C₂ to C₆) alkenyloxy group, and
a halo (C₂ to C₆) alkenyloxy group,
W represents an oxygen atom, a sulfur atom, or NY₇, and
Y₇ is a group selected from the group consisting of
a hydrogen atom,
a (C₁ to C₆) alkyl group,
a halo (C₁ to C₆) alkyl group,
a (C₁ to C₆) alkylcarbonyl group, and
a halo (C₁ to C₆) alkylcarbonyl group.

2. A compound represented by formula (1) or a salt thereof or an N-oxide thereof: 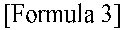 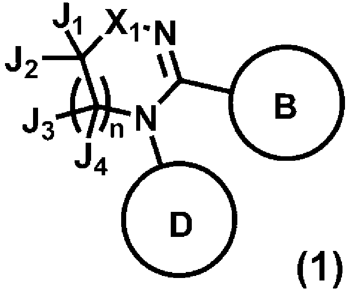 wherein
X₁ represents an oxygen atom, a sulfur atom, NY₁, or C(J₅J₆),
n represents 0 or 1,
J₁, J₂, J₃, and J₄ each independently represent a hydrogen atom, a halogen atom, a (C₁ to C₆) alkyl group unsubstituted or arbitrarily substituted with Tₐ, a halo (C₁ to C₆) alkyl group unsubstituted or arbitrarily substituted with Tₐ, a (C₁ to C₆) alkoxy group unsubstituted or arbitrarily substituted with Tₐ, a halo (C₁ to C₆) alkoxy group unsubstituted or arbitrarily substituted with Tₐ, a hydroxy group, or
a group selected from the group consisting of an O atom in a carbonyl group, a S atom in a thiocarbonyl group, an imino group substituted with Y₂, and a methylene group substituted with J₇ and J₈, formed by two bonds of C-J₁ and C-J₂, or C-J₃ and C-J₄ together,
J₅ and J₆ each independently represent a hydrogen atom, a halogen atom, a (C₁ to C₆) alkyl group unsubstituted or arbitrarily substituted with Tₐ, a halo (C₁ to C₆) alkyl group unsubstituted or arbitrarily substituted with Tₐ, or
a group selected from the group consisting of an O atom in a carbonyl group, a S atom in a thiocarbonyl group, an imino group substituted with Y₃, and a methylene group substituted with J₇ and J₈, formed by two bonds of C-J₅ and C-J₆ together,
J₇ and J₈ each independently represent a group selected from the group consisting of a hydrogen atom, a halogen atom, a (C₁ to C₆) alkyl group unsubstituted or arbitrarily substituted with Tₐ, a halo (C₁ to C₆) alkyl group unsubstituted or arbitrarily substituted with Tₐ, a (C₁ to C₆) alkoxy group unsubstituted or arbitrarily substituted with Tₐ, a halo (C₁ to C₆) alkoxy group unsubstituted or arbitrarily substituted with Tₐ, an amino group optionally substituted with one or two (C₁ to C₆) alkyl groups and/or halo (C₁ to C₆) alkyl groups, and a hydroxy group,
D represents a group selected from the group consisting of a phenyl group unsubstituted or arbitrarily substituted with Z₁, and a heterocyclic group unsubstituted or arbitrarily substituted with Z₁,
B is a structure represented by B-1, B-2, B-3, B-4, B-5, or B-6 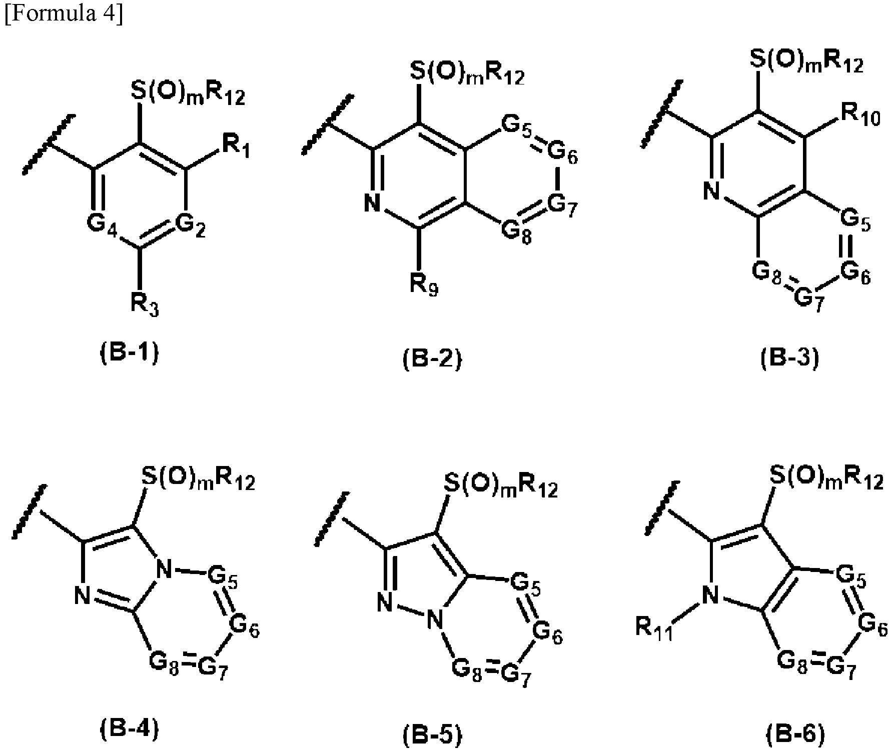
R₁₂ represents a group selected from the group consisting of a (C₁ to C₆) alkyl group, a halo (C₁ to C₆) alkyl group, a (C₃ to C₆) cycloalkyl group, and a halo (C₃ to C₆) cycloalkyl group,
m represents 0, 1, or 2,
G₂ represents a nitrogen atom or C(R₂),
G₄ represents a nitrogen atom or C(R₄),
provided that either or both of G₂ and G₄ is/are a nitrogen atom(s),
G₅ represents a nitrogen atom or C(R₅),
G₆ represents a nitrogen atom or C(R₆),
G₇ represents a nitrogen atom or C(R₇),
G₈ represents a nitrogen atom or C(R₈),
R₁, R₂, R₃, R₄, R₅, R₆, R₇, and R₈ each independently represent a group selected from the group consisting of
a hydrogen atom, a halogen atom, a (C₁ to C₆) alkyl group unsubstituted or arbitrarily substituted with T₁, a halo (C₁ to C₆) alkyl group unsubstituted or arbitrarily substituted with T₁, a (C₃ to C₆) cycloalkyl group unsubstituted or arbitrarily substituted with T₁, a halo (C₃ to C₆) cycloalkyl group unsubstituted or arbitrarily substituted with T₁, a (C₁ to C₆) alkoxy group unsubstituted or arbitrarily substituted with T₁, a halo (C₁ to C₆) alkoxy group unsubstituted or arbitrarily substituted with T₁, a (C₂ to C₆) alkenyl group unsubstituted or arbitrarily substituted with T₁, a halo (C₂ to C₆) alkenyl group unsubstituted or arbitrarily substituted with T₁, a (C₂ to C₆) alkynyl group unsubstituted or arbitrarily substituted with T₁, a halo (C₂ to C₆) alkynyl group unsubstituted or arbitrarily substituted with T₁, a (C₂ to C₆) alkenyloxy group unsubstituted or arbitrarily substituted with T₁, a halo (C₂ to C₆) alkenyloxy group unsubstituted or arbitrarily substituted with T₁, a (C₁ to C₆) alkylthio group unsubstituted or arbitrarily substituted with T₁, a halo (C₁ to C₆) alkylthio group unsubstituted or arbitrarily substituted with T₁, a (C₁ to C₆) alkylsulfinyl group unsubstituted or arbitrarily substituted with T₁, a halo (C₁ to C₆) alkylsulfinyl group unsubstituted or arbitrarily substituted with T₁, a (C₁ to C₆) alkylsulfonyl group unsubstituted or arbitrarily substituted with T₁, a halo (C₁ to C₆) alkylsulfonyl group unsubstituted or arbitrarily substituted with T₁, a (C₁ to C₆) alkylcarbonyl group unsubstituted or arbitrarily substituted with T₁, a halo (C₁ to C₆) alkylcarbonyl group unsubstituted or arbitrarily substituted with T₁, a (C₁ to C₆) alkylcarbonyloxy group unsubstituted or arbitrarily substituted with T₁, a halo (C₁ to C₆) alkylcarbonyloxy group substituted or arbitrarily substituted with T₁, a (C₁ to C₆) alkoxycarbonyl group unsubstituted or arbitrarily substituted with T₁, a halo (C₁ to C₆) alkoxycarbonyl group unsubstituted or arbitrarily substituted with T₁, a (C₁ to C₆) alkylsulfonyloxy group unsubstituted or arbitrarily substituted with T₁, a (C₁ to C₆) alkoxycarbonyloxy group unsubstituted or arbitrarily substituted with T₁, a halo (C₁ to C₆) alkoxycarbonyloxy group unsubstituted or arbitrarily substituted with T₁, a halo (C₁ to C₆) alkylsulfonyloxy group unsubstituted or arbitrarily substituted with T₁, a phenyl group unsubstituted or arbitrarily substituted with Z₂, a heterocyclic group unsubstituted or arbitrarily substituted with Z₂, a phenoxy group unsubstituted or arbitrarily substituted with Z₂, a pyridyloxy group unsubstituted or arbitrarily substituted with Z₂, an NY₄Y₅ group, a C(O)NY₄Y₅ group, a CH=NY₆ group, a cyano group, a nitro group, a hydroxy group, a mercapto group, a formyl group, a carboxy group, and an SF₅ group,
R₉ and R₁₀ each independently represent a group selected from the group consisting of
a hydrogen atom, a halogen atom, a (C₁ to C₆) alkyl group, a halo (C₁ to C₆) alkyl group, a (C₃ to C₆) cycloalkyl group, a halo (C₃ to C₆) cycloalkyl group, a (C₁ to C₆) alkoxy group, a halo (C₁ to C₆) alkoxy group, a (C₁ to C₆) alkylcarbonyloxy group, a halo (C₁ to C₆) alkylcarbonyloxy group, a (C₁ to C₆) alkoxycarbonyloxy group, a halo (C₁ to C₆) alkoxycarbonyloxy group, an NY₄Y₅ group, a C(O)NY₄Y₅ group, a cyano group, a nitro group, and a hydroxy group,
R₁₁ represents a group selected from the group consisting of a hydrogen atom, a (C₁ to C₆) alkyl group, a halo (C₁ to C₆) alkyl group, a (C₃ to C₆) cycloalkyl group, a halo (C₃ to C₆) cycloalkyl group, a (C₁ to C₆) alkoxy group, a halo (C₁ to C₆) alkoxy group, a (C₁ to C₆) alkylcarbonyl group, a halo (C₁ to C₆) alkylcarbonyl group, a (C₄ to C₇) cycloalkylcarbonyl group, and a halo (C₄ to C₇) cycloalkylcarbonyl group,
Z₁ and Z₂ each independently represent a group selected from the group consisting of a halogen atom, a (C₁ to C₆) alkyl group unsubstituted or arbitrarily substituted with T₂, or a halo (C₁ to C₆) alkyl group unsubstituted or arbitrarily substituted with T₂, a (C₃ to C₆) cycloalkyl group unsubstituted or arbitrarily substituted with T₂, a halo (C₃ to C₆) cycloalkyl group unsubstituted or arbitrarily substituted with T₂, a (C₁ to C₆) alkoxy group unsubstituted or arbitrarily substituted with T₂, a halo (C₁ to C₆) alkoxy group unsubstituted or arbitrarily substituted with T₂, a (C₂ to C₆) alkenyl group unsubstituted or arbitrarily substituted with T₂, a halo (C₂ to C₆) alkenyl group unsubstituted or arbitrarily substituted with T₂, a (C₂ to C₆) alkenyloxy group unsubstituted or arbitrarily substituted with T₂, a halo (C₂ to C₆) alkenyloxy group unsubstituted or arbitrarily substituted with T₂, a (C₁ to C₆) alkylthio group unsubstituted or arbitrarily substituted with T₂, a halo (C₁ to C₆) alkylthio group unsubstituted or arbitrarily substituted with T₂, a (C₁ to C₆) alkylsulfinyl group unsubstituted or arbitrarily substituted with T₂, a halo (C₁ to C₆) alkylsulfinyl group unsubstituted or arbitrarily substituted with T₂, a (C₁ to C₆) alkylsulfonyl group unsubstituted or arbitrarily substituted with T₂, a halo (C₁ to C₆) alkylsulfonyl group unsubstituted or arbitrarily substituted with T₂, a (C₁ to C₆) alkylcarbonyl group unsubstituted or
arbitrarily substituted with T₂, a halo (C₁ to C₆) alkylcarbonyl group unsubstituted or arbitrarily substituted with T₂, a (C₁ to C₆) alkoxycarbonyl group unsubstituted or arbitrarily substituted with T₂, a halo (C₁ to C₆) alkoxycarbonyl group unsubstituted or arbitrarily substituted with T₂, a (C₁ to C₆) alkylcarbonyloxy group unsubstituted or arbitrarily substituted with T₂, a halo (C₁ to C₆) alkylcarbonyloxy group unsubstituted or arbitrarily substituted with T₂, a (C₁ to C₆) alkoxycarbonyloxy group unsubstituted or arbitrarily substituted with T₂, a halo (C₁ to C₆) alkoxycarbonyloxy group unsubstituted or arbitrarily substituted with T₂, a (C ₁ to C₆) alkylsulfonyloxy group unsubstituted or arbitrarily substituted with T₂, a halo (C₁ to C₆) alkylsulfonyloxy group unsubstituted or arbitrarily substituted with T₂, an NY₄Y₅ group, a C(O)NY₄Y₅ group, a cyano group, a nitro group, a hydroxy group, a mercapto group, a formyl group, a carboxy group, and an SF₅ group, or
a group forming a 5- or 6-membered alicyclic ring unsubstituted or optionally substituted with one or more halogen atoms or (C₁ to C₆) alkyl groups, formed by connecting two adjacent Z₁ or Z₂ to each other, or a group forming a 1,3-dioxole group or a 1,4-dioxin group formed by two adjacent Z₁ or Z₂ together with a carbon atom to which the two Z₁ or Z₂ are attached, wherein the 1,3-dioxole group and the 1,4-dioxin group are optionally substituted with one or two or more independent halogen atoms,
Y₁, Y₂, and Y₃ each independently represent a group selected from the group consisting of a hydrogen atom, a (C₁ to C₆) alkyl group unsubstituted or arbitrarily substituted with T₃, a halo (C₁ to C₆) alkyl group unsubstituted or arbitrarily substituted with T₃, a (C₃ to C₆) cycloalkyl group unsubstituted or arbitrarily substituted with T₃, a halo (C₃ to C₆) cycloalkyl group unsubstituted or arbitrarily substituted with T₃, a (C₂ to C₆) alkenyl group unsubstituted or
arbitrarily substituted with T₃, a halo (C₂ to C₆) alkenyl group unsubstituted or arbitrarily substituted with T₃, a (C₁ to C₆) alkoxy group unsubstituted or arbitrarily substituted with T₃, a halo (C₁ to C₆) alkoxy group unsubstituted or arbitrarily substituted with T₃, a (C₁ to C₆) alkylcarbonyl group unsubstituted or arbitrarily substituted with T₃, a halo (C₁ to C₆) alkylcarbonyl group unsubstituted or arbitrarily substituted with T₃, a (C₃ to C₆) cycloalkylcarbonyl group unsubstituted or arbitrarily substituted with T₃, a halo (C₃ to C₆) cycloalkylcarbonyl group unsubstituted or arbitrarily substituted with T₃, a (C₁ to C₆) alkoxycarbonyl group unsubstituted or arbitrarily substituted with T₃, a halo (C₁ to C₆) alkoxycarbonyl group unsubstituted or arbitrarily substituted with T₃, a (C₁ to C₆) alkylsulfonyl group unsubstituted or arbitrarily substituted with T₃, a halo (C₁ to C₆) alkylsulfonyl group unsubstituted or arbitrarily substituted with T₃, and a hydroxy group,
Tₐ and T₁ each independently represent a group selected from the group consisting of a (C₃ to C₆) cycloalkyl group, a halo (C₃ to C₆) cycloalkyl group, a (C₁ to C₆) alkoxy group, a halo (C₁ to C₆) alkoxy group, a (C₁ to C₆) alkylcarbonyl group, a halo (C₁ to C₆) alkylcarbonyl group, a (C₁ to C₆) alkoxycarbonyl group, a halo (C₁ to C₆) alkoxycarbonyl group, a (C₁ to C₆) alkylthio group, a halo (C₁ to C₆) alkylthio group, a (C₁ to C₆) alkylsulfinyl group, a halo (C₁ to C₆) alkylsulfinyl group, a (C₁ to C₆) alkylsulfonyl group, a halo (C₁ to C₆) alkylsulfonyl group, an NY₄Y₅ group, a C(O)NY₄Y₅ group, a cyano group, a nitro group, a hydroxy group, a mercapto group, a formyl group, a carboxy group, a phenyl group unsubstituted or arbitrarily substituted with Z₃, a heterocyclic group unsubstituted or arbitrarily substituted with Z₃, a phenoxy group unsubstituted or arbitrarily substituted with Z₃, and a pyridyloxy group unsubstituted or arbitrarily substituted with Z₃,
T₂ and T₃ each independently represents a group selected from the group consisting of a (C₃ to C₆) cycloalkyl group, a halo (C₃ to C₆) cycloalkyl group, a (C₁ to C₆) alkoxy group, a halo (C₁ to C₆) alkoxy group, a (C₁ to C₆) alkylcarbonyl group, a halo (C₁ to C₆) alkylcarbonyl group, a (C₁ to C₆) alkoxycarbonyl group, a halo (C₁ to C₆) alkoxycarbonyl group, a (C₁ to C₆) alkylthio group, a halo (C₁ to C₆) alkylthio group, a (C₁ to C₆) alkylsulfinyl group, a halo (C₁ to C₆) alkylsulfinyl group, a (C₁ to C₆) alkylsulfonyl group, a halo (C₁ to C₆) alkylsulfonyl group, an NY₄Y₅ group, a C(O)NY₄Y₅ group, a cyano group, a nitro group, a hydroxy group, a mercapto group, a formyl group, and a carboxy group,
Z₃, or when a plurality thereof is present, each independently, represents a group selected from the group consisting of a halogen atom, a (C₁ to C₆) alkyl group, a halo (C₁ to C₆) alkyl group, a (C₃ to C₆) cycloalkyl group, a halo (C₃ to C₆) cycloalkyl group, a 1-cyano-(C₃ to C₆) cycloalkyl group, a (C₁ to C₆) alkoxy group, a halo (C₁ to C₆) alkoxy group, a (C₁ to C₆) alkylthio group,
a halo (C₁ to C₆) alkylthio group, a (C₁ to C₆) alkylsulfinyl group, a halo (C₁ to C₆) alkylsulfinyl group, a (C₁ to C₆) alkylsulfonyl group, a halo (C₁ to C₆) alkylsulfonyl group, a (C₁ to C₆) alkylcarbonyl group, a halo (C₁ to C₆) alkylcarbonyl group, a (C₁ to C₆) alkoxycarbonyl group, a halo (C₁ to C₆) alkoxycarbonyl group, an NY₄Y₅ group, a C(O)NY₄Y₅ group, a cyano group, a nitro group, a hydroxy group, a mercapto group, a formyl group, a carboxy group, and an SF₅ group,
Y₄ and Y₅ each independently represent a group selected from the group consisting of a hydrogen atom, a (C₁ to C₆) alkyl group, a halo (C₁ to C₆) alkyl group, a (C₃ to C₆) cycloalkyl group, a halo (C₃ to C₆) cycloalkyl group, a (C₁ to C₆) alkoxy group, a halo (C₁ to C₆) alkoxy group, a (C₁ to C₆) alkylcarbonyl group, a halo (C₁ to C₆) alkylcarbonyl group, a (C₃ to C₆) cycloalkylcarbonyl group, a halo (C₃ to C₆) alkylcarbonyl group, a (C₁ to C₆) alkoxycarbonyl group, a halo (C₁ to C₆) alkoxycarbonyl group, a (C₁ to C₆) alkylsulfonyl group,
a halo (C₁ to C₆) alkylsulfonyl group, and a phenyl group unsubstituted or arbitrarily substituted with Z₃, and
Y₆, or when a plurality thereof is present, each independently, represents a group selected from the group consisting of a hydrogen atom, a (C₁ to C₆) alkyl group unsubstituted or arbitrarily substituted with T₃, a halo (C₁ to C₆) alkyl group unsubstituted or arbitrarily substituted with T₃,
a (C₃ to C₆) cycloalkyl group, a halo (C₃ to C₆) cycloalkyl group, a (C₁ to C₆) alkoxy group unsubstituted or arbitrarily substituted with a cyano group, a halo (C₁ to C₆) alkoxy group, a (C₃ to C₆) cycloalkoxy group, a halo (C₃ to C₆) cycloalkoxy group, a (C₂ to C₆) alkenyl group, a halo (C₂ to C₆) alkenyl group, a (C₂ to C₆) alkenyloxy group, and a halo (C₂ to C₆) alkenyloxy group.

3. The compound according to claim 1 or 2 or a salt thereof or an N-oxide thereof, wherein
X₁ is an oxygen atom, a sulfur atom, NY₁, or C(J₅J₆),
two bonds of C-J₅ and C-J₆ are taken together to be a group selected from the group consisting of an O atom in a carbonyl group, a S atom in a thiocarbonyl group, an imino group substituted with Y₃, and a methylene group substituted with J₇ and J₈, and
J₇ and J₈ are each independently a group selected from the group consisting of a hydrogen atom, a halogen atom, a (C₁ to C₆) alkyl group unsubstituted or arbitrarily substituted with Tₐ, a (C₁ to C₆) alkoxy group unsubstituted or arbitrarily substituted with Tₐ, an amino group optionally substituted with one or two (C₁ to C₆) alkyl groups and/or halo (C₁ to C₆) alkyl groups, and a hydroxy group.

4. The compound according to any one of claims 1 to 3 or a salt thereof or an N-oxide thereof, wherein
D is D-1, D-2, D-3, D-4, D-5, D-6, D-7, D-8, D-9, or D-10 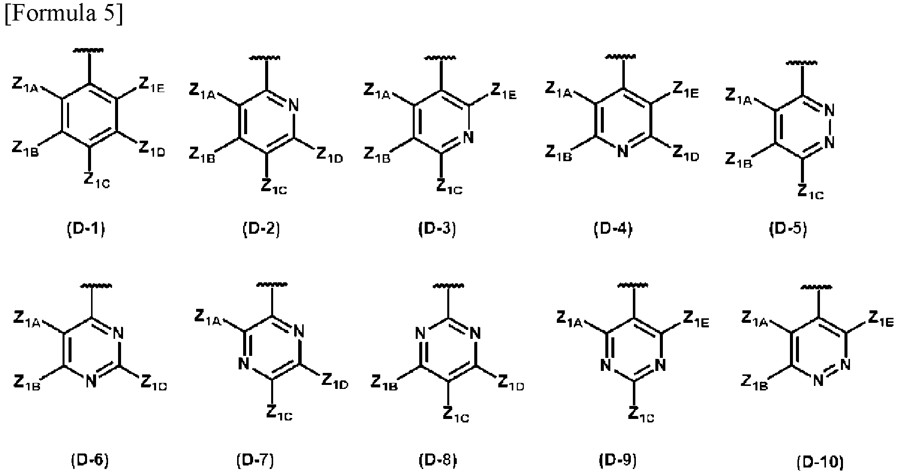 wherein
Z_{1A}, Z_{1B}, Z_{1C}, Z_{1D}, and Z_{1E} are each independently a group selected from the group consisting of a hydrogen atom, a halogen atom, a (C₁ to C₆) alkyl group unsubstituted or arbitrarily substituted with T₂, a halo (C₁ to C₆) alkyl group unsubstituted or arbitrarily substituted with T₂, a (C₃ to C₆) cycloalkyl group unsubstituted or arbitrarily substituted with T₂, a halo (C₃ to C₆) cycloalkyl group unsubstituted or arbitrarily substituted with T₂, a (C₁ to C₆) alkoxy group unsubstituted or arbitrarily substituted with T₂, a halo (C₁ to C₆) alkoxy group unsubstituted or arbitrarily substituted with T₂, a (C₂ to C₆) alkenyl group unsubstituted or arbitrarily substituted with T₂, a halo (C₂ to C₆) alkenyl group unsubstituted or arbitrarily substituted with T₂, a (C₂ to C₆) alkenyloxy group unsubstituted or arbitrarily substituted with T₂, a halo (C₂ to C₆) alkenyloxy group unsubstituted or arbitrarily substituted with T₂, a (C₁ to C₆) alkylthio group unsubstituted or arbitrarily substituted with T₂, a halo (C₁ to C₆) alkylthio group unsubstituted or arbitrarily substituted with T₂, a (C₁ to C₆) alkylsulfinyl group unsubstituted or arbitrarily substituted with T₂, a halo (C₁ to C₆) alkylsulfinyl group unsubstituted or arbitrarily substituted with T₂, a (C₁ to C₆) alkylsulfonyl group unsubstituted or arbitrarily substituted with T₂, a halo (C₁ to C₆) alkylsulfonyl group unsubstituted or arbitrarily substituted with T₂, a (C₁ to C₆) alkylcarbonyl group unsubstituted or arbitrarily substituted with T₂, a halo (C₁ to C₆) alkylcarbonyl group unsubstituted or arbitrarily substituted with T₂, a (C₁ to C₆) alkoxycarbonyl group unsubstituted or arbitrarily substituted with T₂, a halo (C₁ to C₆) alkoxycarbonyl group unsubstituted or arbitrarily substituted with T₂, a (C₁ to C₆) alkylcarbonyloxy group unsubstituted or arbitrarily substituted with T₂, a halo (C₁ to C₆) alkylcarbonyloxy group unsubstituted or arbitrarily substituted with T₂, a (C₁ to C₆) alkoxycarbonyloxy group unsubstituted or arbitrarily substituted with T₂, a halo (C₁ to C₆) alkoxycarbonyloxy group unsubstituted or arbitrarily substituted with T₂, a (C₁ to C₆) alkylsulfonyloxy group unsubstituted or arbitrarily substituted with T₂, a halo (C₁ to C₆) alkylsulfonyloxy group unsubstituted or arbitrarily substituted with T₂, an NY₄Y₅ group, a C(O)NY₄Y₅ group, a cyano group, a nitro group, a hydroxy group, a mercapto group, a formyl group, a carboxy group, and an SF₅ group.

5. The compound according to any one of claims 1 to 4 or a salt thereof or an N-oxide thereof, wherein B is a structure represented by B-1.

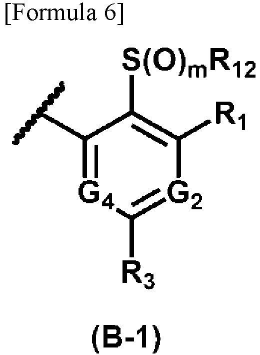

6. The compound according to any one of claims 1 to 4 or a salt thereof or an N-oxide thereof, wherein B is a structure represented by B-2.

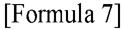

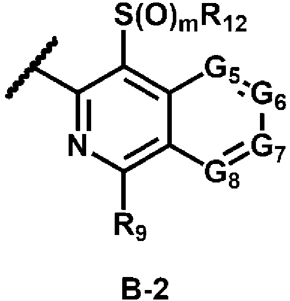

7. The compound according to any one of claims 1 to 4 or a salt thereof or an N-oxide thereof, wherein B is a structure represented by B-3.

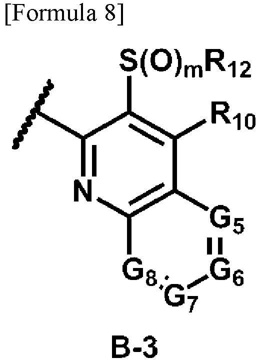

8. The compound according to any one of claims 1 to 4 or a salt thereof or an N-oxide thereof, wherein B is a structure represented by B-4.

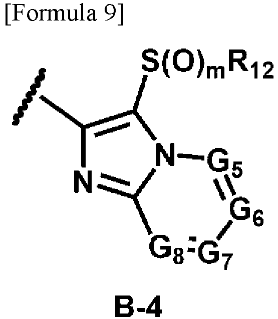

9. The compound according to any one of claims 1 to 4 or a salt thereof or an N-oxide thereof, wherein B is a structure represented by B-5.

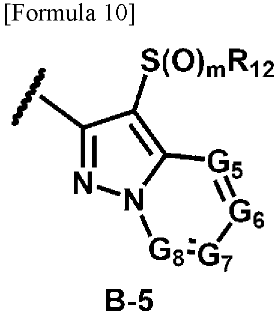

10. The compound according to any one of claims 1 to 4 or a salt thereof or an N-oxide thereof, wherein B is a structure represented by B-6.

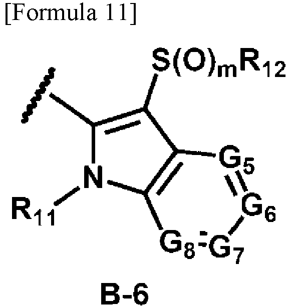

11. The compound according to any one of claims 1 to 4 or a salt thereof or an N-oxide thereof, wherein B is a structure represented by B-7 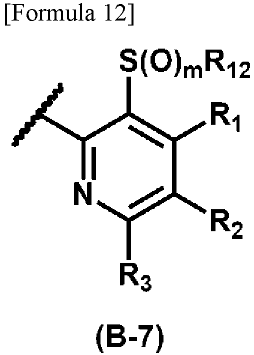 wherein R₁ is a hydrogen atom.

12. A pest control agent comprising the compound according to any one of claims 1 to 11 or a salt thereof or an N-oxide thereof.
